# EUROPEAN PATENT APPLICATION

(11) **EP 4 844 066 A2**
(43) Date of publication of application: **25.11.2026**
(21) Application number: 26210819.4
(22) Date of filing: 06.02.2024
(51) Int. Cl.: C12N 15/113

(54) **INHIBITORS OF EXPRESSION AND/OR FUNCTION**

(30) Priority: 06.02.2023 EP 23155094; 15.12.2023 EP 23217288; 22.12.2023 EP 23219939
(62) Document number(s) of the earlier application(s) in accordance with Art. 76 EPC: 24703564.5 / 4 662 315
(71) Applicant: Tangram Therapeutics PLC, London W2 6BD (GB)
(72) Inventor: WHITMORE, Alan Victor, London, W2 6BD (GB); MALANDRAKI-MILLER, Sophia, London, W2 6BD (GB); DE SANTIAGO, Ines, London, W2 6BD (GB); PURSELL, Natalie, London, W2 6BD (US); GIANNOUSTAS, Gaye Saginc, London, W2 6BD (GB)
(74) Representative: CMS Cameron McKenna Nabarro Olswang LLP

(57) **Abstract**

The present invention relates to inhibitors, and compositions containing inhibitors, and uses of the same in the treatment or prevention of a metabolic disease or disorder, such as a metabolic disease or disorder associated with non-alcoholic fatty liver disease (NAFLD), and/or obesity and/or a disease or disorder associated with adipogenesis.

## Description

### FIELD

The present invention provides inhibitors, such as nucleic acid compounds, such as siRNA, suitable for therapeutic use. Additionally, the present invention provides methods of making these compounds, as well as methods of using such compounds for the treatment of various diseases and condition

### BACKGROUND OF THE INVENTION

Inhibitors, such as oligonucleoside/ oligonucleotide compounds which are inhibitors of gene expression and/or expression or function of other targets such as LNCRNAs, can have important therapeutic applications in medicine. Oligonucleotides/ oligonucleosides can be used to silence genes that are responsible for a particular disease. Gene-silencing prevents formation of a protein by inhibiting translation. Importantly, gene-silencing agents are a promising alternative to traditional small, organic compounds that inhibit the function of the protein linked to the disease. siRNA, antisense RNA, and micro-RNA are oligonucleoside /oligonucleotides that prevent the formation of proteins by gene-silencing.

A number of modified siRNA compounds in particular have been developed in the last two decades for diagnostic and therapeutic purposes, including siRNA / RNAi therapeutic agents for the treatment of various diseases including central-nervous-system diseases, inflammatory diseases, metabolic disorders, oncology, infectious diseases, and ocular diseases.

The present invention relates to inhibitors, such oligomers e.g. nucleic acids, e.g. oligonucleoside /oligonucleotide compounds, and their use in the treatment and / or prevention of disease.

The *SLC25A5* gene belongs to the ANT gene family, which itself belongs to the superfamily that includes genes encoding brown fat mitochondrial uncoupling proteins and mitochondrial phosphate carrier proteins. This gene is a member of the mitochondrial carrier subfamily of solute carrier protein genes. The product of this gene, adenine nucleotide translocator 2 (ANT2), functions as a major constituent of the mitochondrial permeability-transition pore complex that catalyses the exchange of mitochondrial ATP with cytosolic ADP. As a result of its antiporter function, ANT2 maintains mitochondrial membrane potential by regulating ADP/ATP ratios in oxidative phosphorylation. ANT2 facilitates uncoupling of the mitochondrial membrane when acylated by SIRT4. Though uncoupling the membrane potential typically leads to apoptosis, ANT2 was found to be antiapoptotic. As a result, it is postulated to mediate the TFIIH-dependent response to DNA damage as a component of the MMS19-XPD.

### STATEMENTS OF INVENTION

The invention is defined as in the claims and relates to, *inter alia*:
In one aspect, the invention relates to an inhibitor of expression and / or function of SLC25A5/ANT2, wherein said inhibitor is conjugated to one or more ligand moieties.

In a further aspect, the invention relates to an inhibitor according to the invention, wherein said inhibitor is an siRNA oligomer.

In another aspect, the invention relates to an inhibitor of expression and / or function of SLC25A5/ANT2, wherein said inhibitor is an siRNA oligomer.

In a further aspect, the invention relates to an inhibitor according to the invention, wherein said inhibitor comprises an siRNA oligomer conjugated to one or more ligand moieties.

In a further aspect, the invention relates to an inhibitor according to the invention, wherein said one or more ligand moieties comprise one or more GalNAc ligands or comprise one or more GalNAc ligand derivatives.

In a further aspect, the invention relates to an inhibitor according to the invention, wherein said one or more ligand moieties comprise one or more GalNAc ligand derivatives.

In a further aspect, the invention relates to an inhibitor according to the invention, wherein the target of the inhibitor is SLC25A5/ANT2.

In a further aspect, the invention relates to an inhibitor according to the invention, wherein the inhibitor is a nucleic acid for inhibiting expression of SLC25A5 comprising a duplex region that comprises a first strand and a second strand that is at least partially complementary to the first strand, wherein
(i) at least partially complementary to a portion of RNA transcribed from the SLC25A5 gene, and
(ii) comprises at least 17 contiguous nucleosides differing by 0 or 1 nucleosides from any one of the first strand sequences as listed in Table 2.

In a further aspect, the invention relates to an inhibitor according to the invention, wherein the inhibitor is a nucleic acid for inhibiting expression of SLC25A5 comprising a duplex region that comprises a first strand and a second strand that is at least partially complementary to the first strand, wherein said first strand is:
(i) at least partially complementary to a portion of RNA transcribed from the SLC25A5 gene, and
(ii) comprises at least 17 contiguous nucleosides differing by 0 or 1 nucleosides from any one of the first strand modified sequences as listed in Table 3.

In a further aspect, the invention relates to an inhibitor according to the invention, wherein the first strand comprises nucleosides 2-18 of any one of the sequences defined in claim 8 or 9, in particular wherein the first strand comprises nucleosides 2-18 of any one of the sequences defined in Tables 2 or 3.

In a further aspect, the invention relates to an inhibitor according to the invention, wherein the second strand comprises a nucleoside sequence of at least 17 contiguous nucleosides differing by 0 or 1 nucleosides from any one of the second strand sequences as listed in Table 2, and wherein the second strand has a region of at least 85% complementarity over the 17 contiguous nucleosides to the first strand.

In a further aspect, the invention relates to an inhibitor according to the invention, wherein the second strand comprises a nucleoside sequence of at least 17 contiguous nucleosides differing by 0 or 1 nucleosides from any one of the second strand modified sequences as listed in Table 4, and wherein the second strand has a region of at least 85% complementarity over the 17 contiguous nucleosides to the first strand.

In a further aspect, the invention relates to an inhibitor according to the invention, wherein the first strand comprises any one of the first strand sequences as listed in Table 2.

In a further aspect, the invention relates to an inhibitor according to the invention, wherein the first strand comprises any one of the first strand modified sequences as listed in Table 3.

In a further aspect, the invention relates to an inhibitor according to the invention, wherein the second strand comprises any one of the second strand sequences as listed in Table 2.

In a further aspect, the invention relates to an inhibitor according to the invention, wherein the second strand comprises any one of the first strand modified sequences as listed in Table 4.

In a further aspect, the invention relates to an inhibitor according to the invention, wherein the first strand comprises any one of the following sequences: SEQ ID NO:304, SEQ ID NO:323, SEQ ID NO:439, SEQ ID NO:453 and SEQ ID NO:496.

In a further aspect, the invention relates to an inhibitor according to the invention, wherein the first strand comprises any one of the following sequences: SEQ ID NO:856, SEQ ID NO:875, SEQ ID NO:991, SEQ ID NO:1005 and SEQ ID NO:1048.

In a further aspect, the invention relates to an inhibitor according to the invention, wherein the second strand comprises any one of the following sequences: SEQ ID NO:580, SEQ ID NO:599, SEQ ID NO:715, SEQ ID NO:729 and SEQ ID NO:772.

In a further aspect, the invention relates to an inhibitor according to the invention, wherein the second strand comprises any one of the following sequences: SEQ ID NO:1132, SEQ ID NO:1151, SEQ ID NO:1267, SEQ ID NO:1281 and SEQ ID NO:1324.

In a further aspect, the invention relates to an inhibitor according to the invention, comprising first and second strands that comprise, consist of, or consist essentially of a nucleoside sequence differing by 0 or 1 nucleosides from any one of the following combinations of first and second sequences:

| Unmodified first strand | Unmodified second strand |
|---|---|
| SEQ ID NO: 304 | SEQ ID NO: 580 |
| SEQ ID NO: 323 | SEQ ID NO: 599 |
| SEQ ID NO: 439 | SEQ ID NO: 715 |
| SEQ ID NO: 453 | SEQ ID NO: 729 |
| SEQ ID NO: 496 | SEQ ID NO: 772 |

In a further aspect, the invention relates to an inhibitor according to the invention, comprising first and second strands that comprise, consist of, or consist essentially of a nucleoside sequence differing by 0 or 1 nucleosides from any one of the following combinations of first and second sequences:

| Unmodified first strand | Unmodified second strand |
|---|---|
| SEQ ID NO: 304 | SEQ ID NO: 580 |

In a further aspect, the invention relates to an inhibitor according to the invention, comprising first and second strands that comprise, consist of, or consist essentially of a nucleoside sequence differing by 0 or 1 nucleosides from any one of the following combinations of first and second sequences:

| Unmodified first strand | Unmodified second strand |
|---|---|
| SEQ ID NO: 856 | SEQ ID NO: 1132 |
| SEQ ID NO: 875 | SEQ ID NO: 1151 |
| SEQ ID NO: 991 | SEQ ID NO: 1267 |
| SEQ ID NO: 1005 | SEQ ID NO: 1281 |
| SEQ ID NO: 1048 | SEQ ID NO: 1324 |

In a further aspect, the invention relates to an inhibitor according to the invention, comprising first and second strands that comprise, consist of, or consist essentially of a nucleoside sequence differing by 0 or 1 nucleosides from any one of the following combinations of first and second sequences:

| Unmodified first strand | Unmodified second strand |
|---|---|
| SEQ ID NO: 856 | SEQ ID NO: 1132 |

In a further aspect, the invention relates to an inhibitor according to the invention, which is an siRNA oligomer having a first and a second strand wherein:
i) the first strand of the siRNA has a length in the range of 15 to 30 nucleosides, preferably 19 to 25 nucleosides, more preferably 23 or 25; even more preferably 23; and / or
ii) the second strand of the siRNA has a length in the range of 15 to 30 nucleosides, preferably 19 to 25 nucleosides, more preferably 21 nucleosides.

In a further aspect, the invention relates to an inhibitor according to the invention, wherein the first strand has a length in the range of 17 to 30 nucleosides, preferably 19 to 25 nucleosides, more preferably 19 or 23 nucleosides.

In a further aspect, the invention relates to an inhibitor according to the invention, wherein the second strand has a length in the range of 17 to 30 nucleosides, preferably 19 to 25 nucleosides, more preferably 19 or 21 or 23 nucleosides.

In a further aspect, the invention relates to an inhibitor according to the invention, wherein the duplex region of the nucleic acid is between 17 and 30 nucleosides in length, more preferably is 19 or 21 or 23 nucleosides in length.

In a further aspect, the invention relates to an inhibitor according to the invention, wherein the region of complementarity between the first strand and the portion of RNA transcribed from the SLC25A5 gene is between 17 and 30 nucleosides in length.

In a further aspect, the invention relates to an inhibitor according to the invention, wherein the nucleic acid further comprises one or more single-stranded nucleoside overhangs, optionally wherein the overhang is present on the first or second strand, preferably at the 3' terminus of the first or second strand, and/or wherein the overhang comprises 1 to 4 nucleosides, more preferably 2 nucleosides.

In a further aspect, the invention relates to an inhibitor according to the invention, wherein the nucleic acid is an siRNA oligonucleoside.

In a further aspect, the invention relates to an inhibitor according to the invention, wherein the second sense strand further comprises one or more abasic nucleosides in a terminal region of the second strand, and wherein said abasic nucleoside(s) is / are connected to an adjacent nucleoside through a reversed internucleoside linkage.

In a further aspect, the invention relates to an inhibitor according to the invention, wherein the second strand comprises:
i) 2, or more than 2, abasic nucleosides in a terminal region of the second strand; and / or
ii) 2, or more than 2, abasic nucleosides in either the 5' or 3' terminal region of the second strand; and / or
iii) 2, or more than 2, abasic nucleosides in either the 5' or 3' terminal region of the second strand, wherein the abasic nucleosides are present in an overhang as herein described; and / or
iv) 2, or more than 2, consecutive abasic nucleosides in a terminal region of the second strand, wherein preferably one such abasic nucleoside is a terminal nucleoside; and / or
v) 2, or more than 2, consecutive abasic nucleosides in either the 5' or 3' terminal region of the second strand, wherein preferably one such abasic nucleoside is a terminal nucleoside in either the 5' or 3' terminal region of the second strand; and / or
vi) a reversed internucleoside linkage connects at least one abasic nucleoside to an adjacent basic nucleoside in a terminal region of the second strand; and / or
vii) a reversed internucleoside linkage connects at least one abasic nucleoside to an adjacent basic nucleoside in either the 5' or 3' terminal region of the second strand; and / or
viii) an abasic nucleoside as the penultimate nucleoside which is connected via the reversed linkage to the nucleoside which is not the terminal nucleoside (called the antepenultimate nucleoside herein); and / or
ix) abasic nucleosides as the 2 terminal nucleosides connected via a 5'-3' linkage when reading the strand in the direction towards that terminus;
x) abasic nucleosides as the 2 terminal nucleosides connected via a 3'-5' linkage when reading the strand in the direction towards the terminus comprising the terminal nucleosides;
xi) abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
xii) abasic nucleosides as the terminal 2 positions, wherein the penultimate nucleoside is connected via the reversed linkage to the antepenultimate nucleoside, and wherein either
   (1) the reversed linkage is a 5-5' reversed linkage and the linkage between the terminal and penultimate abasic nucleosides is 3'5' when reading towards the terminus comprising the terminal and penultimate abasic nucleosides; or
   (2) the reversed linkage is a 3-3' reversed linkage and the linkage between the terminal and penultimate abasic nucleosides is 5'3' when reading towards the terminus comprising the terminal and penultimate abasic nucleosides.

In a further aspect, the invention relates to an inhibitor according to the invention, wherein the reversed internucleoside linkage is at a terminal region which is distal to the 5' terminal region of the second strand, or at a terminal region which is distal to the 3' terminal region of the second strand.

In a further aspect, the invention relates to an inhibitor according to the invention, wherein the reversed internucleoside linkage is a 3'3 reversed linkage.

In a further aspect, the invention relates to an inhibitor according to the invention, wherein the reversed internucleoside linkage is a 5'5 reversed linkage.

In a further aspect, the invention relates to an inhibitor according to the invention, wherein the second sense strand further comprises one or more abasic nucleosides in a terminal region of the second strand, and wherein said abasic nucleoside(s) is / are connected to an adjacent nucleoside through a reversed internucleoside linkage.

In a further aspect, the invention relates to an inhibitor according to the invention, wherein the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein:
(a) said penultimate abasic nucleoside is connected to an adjacent first basic nucleoside in an adjacent 5' near terminal region through a reversed internucleoside linkage; and
(b) the reversed linkage is a 5-5' reversed linkage; and
(c) the linkage between the terminal and penultimate abasic nucleosides is 3'5' when reading towards the terminus comprising the terminal and penultimate abasic nucleosides.12. An inhibitor, or inhibitor for use, according claim 10 or 11, wherein the reversed internucleoside linkage is at a terminal region which is distal to the 5' terminal region of the second strand, or at a terminal region which is distal to the 3' terminal region of the second strand.

In a further aspect, the invention relates to an inhibitor according to the invention, wherein
(i) the first strand and the second strand each has a length of 23 nucleosides;
(ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand;
(iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and
(iv) the second strand of the nucleic acid is conjugated directly or indirectly to one or more ligand moieties at the 3' terminal region of the second strand.

In a further aspect, the invention relates to an inhibitor according to the invention, wherein, wherein the 2 consecutive inverted abasic nucleosides in the 5' terminal region of the second strand present as the following 5' terminal motif 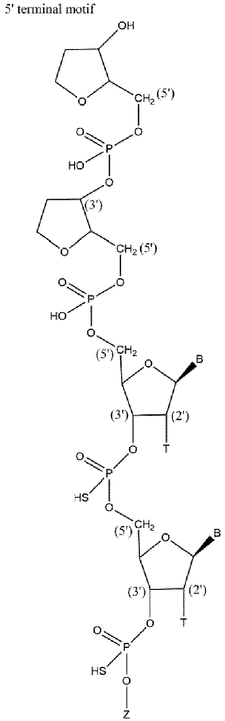 wherein:
T represents a 2'Me ribose modification,
B represents the nucleoside bases of the first two basic nucleosides in the 5' terminal region of the second strand, and
Z represents the remaining 19 contiguous basic nucleosides of said second strand.

In a further aspect, the invention relates to an inhibitor according to the invention, wherein one or more nucleosides on the first strand and / or the second strand is / are modified, to form modified nucleosides.

In a further aspect, the invention relates to an inhibitor according to the invention, wherein the modification is a modification at the 2'-OH group of the ribose sugar, optionally selected from 2'-Me or 2'-F modifications.

In a further aspect, the invention relates to an inhibitor according to the invention, wherein the first strand comprises a 2'-F at any of position 14, position 2, position 6, or any combination thereof, counting from position 1 of said first strand.

In a further aspect, the invention relates to an inhibitor according to the invention, wherein the second strand comprises a 2'-F modification at position 7 and / or 9, and / or 11 and / or 13, counting from position 1 of said second strand.

In a further aspect, the invention relates to an inhibitor according to the invention, wherein the first and second strand each comprise 2'-Me and 2'-F modifications.

In a further aspect, the invention relates to an inhibitor according to the invention, which is an siRNA, wherein the siRNA comprises at least one thermally destabilizing modification, suitably at one or more of positions 1 to 9 of the first strand counting from position 1 of the first strand, and / or at one or more of positions on the second strand aligned with positions 1 to 9 of the first strand, wherein the destabilizing modification is selected from a modified unlocked nucleic acid (UNA) and a glycol nucleic acid (GNA), preferably a glycol nucleic acid.

In a further aspect, the invention relates to an inhibitor according to the invention, wherein the siRNA comprises at least one thermally destabilizing modification at position 7 of the first strand, counting from position 1 of the first strand.

In a further aspect, the invention relates to an inhibitor according to the invention, which is an siRNA, wherein the siRNA comprises 3 or more 2'-F modifications at positions 7 to 13 of the second strand, such as 4, 5, 6 or 7 2'-F modifications at positions 7 to 13 of the second strand, counting from position 1 of said second strand

In a further aspect, the invention relates to an inhibitor according to the invention, which is an siRNA, wherein said second strand comprises at least 3, such as 4, 5 or 6, 2'-Me modifications at positions 1 to 6 of the second strand, counting from position 1 of said second strand.

In a further aspect, the invention relates to an inhibitor according to the invention, which is an siRNA, wherein said first strand comprises at least 5 2'-Me consecutive modifications at the 3' terminal region, preferably including the terminal nucleoside at the 3' terminal region, or at least within 1 or 2 nucleosides from the terminal nucleoside at the 3' terminal region.

In a further aspect, the invention relates to an inhibitor according to the invention, which is an siRNA wherein said first strand comprises 7 2'-Me consecutive modifications at the 3' terminal region, preferably including the terminal nucleoside at the 3' terminal region.

In a further aspect, the invention relates to an inhibitor according to the invention, wherein the siRNA oligomer further comprises one or more phosphorothioate internucleoside linkages.

In a further aspect, the invention relates to an inhibitor according to the invention, wherein said one or more phosphorothioate internucleoside linkages are respectively between at least three consecutive positions in a 5' or 3' near terminal region of the second strand, whereby said near terminal region is preferably adjacent said terminal region wherein said one or more abasic nucleosides of said second strand is / are located as defined herein.

In a further aspect, the invention relates to an inhibitor according to the invention, wherein said one or more phosphorothioate internucleoside linkages are respectively between at least three consecutive positions in a 5' and / or 3' terminal region of the first strand, whereby preferably a terminal position at the 5' and / or 3' terminal region of said first strand is attached to its adjacent position by a phosphorothioate internucleoside linkage.

In a further aspect, the invention relates to an inhibitor according to the invention, wherein the oligomer is an siRNA and the second strand of the siRNA is conjugated directly or indirectly to one or more ligand moiety(s), wherein said ligand moiety is typically present at a terminal region of the second strand, preferably at the 3' terminal region thereof.

In a further aspect, the invention relates to an inhibitor according to the invention, wherein the ligand moiety comprises
i) one or more GalNAc ligands; and / or
ii) one or more GalNAc ligand derivatives; and / or
iii) one or more GalNAc ligands and / or GalNAc ligand derivatives conjugated to said siRNA through a linker.

In a further aspect, the invention relates to an inhibitor according to the invention, wherein said one or more GalNAc ligands and / or GalNAc ligand derivatives are conjugated directly or indirectly to the 5' or 3' terminal region of the second strand of the siRNA oligomer, preferably at the 3' terminal region thereof.

In a further aspect, the invention relates to an inhibitor according to the invention, wherein the ligand moiety comprises

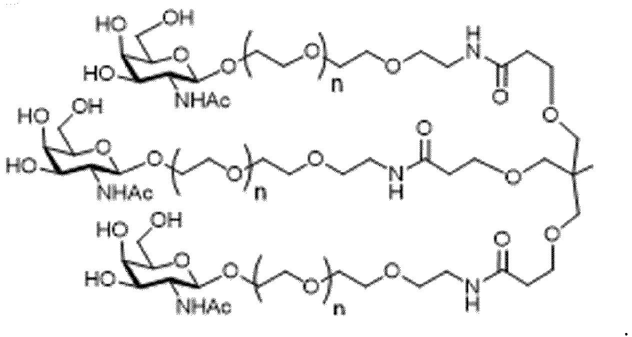

In a further aspect, the invention relates to an inhibitor according to the invention, comprising the structure: 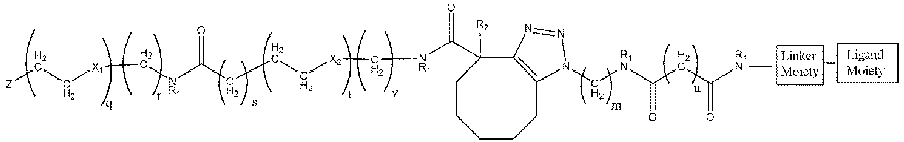 wherein:
R₁ at each occurrence is independently selected from the group consisting of hydrogen, methyl and ethyl;
R₂ is selected from the group consisting of hydrogen, hydroxy, -OC₁₋₃alkyl, -C(=O)OC₁₋₃alkyl, halo and nitro;
X₁ and X₂ at each occurrence are independently selected from the group consisting of methylene, oxygen and sulfur;
m is an integer of from 1 to 6;
n is an integer of from 1 to 10;
q, r, s, t, v are independently integers from 0 to 4, with the proviso that:
   (i) q and r cannot both be 0 at the same time; and
   (ii) s, t and v cannot all be 0 at the same time;
Z is an oligonucleoside moiety.

In a further aspect, the invention relates to an inhibitor according to the invention, comprising the structure 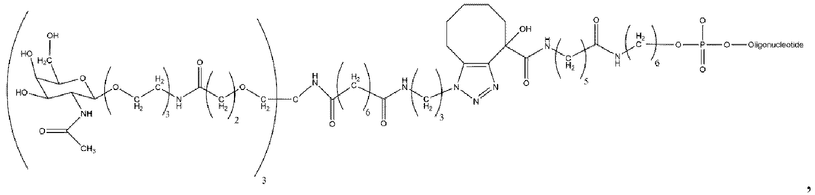 wherein oligonucleotide represents the contiguous nucleosides of the second strand.

In a further aspect, the invention relates to an inhibitor according to the invention, comprising the structure 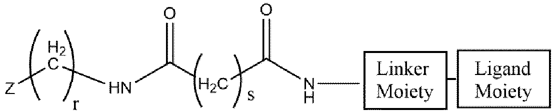 wherein:
r and s are independently an integer selected from 1 to 16; and
Z is an oligonucleoside moiety.

In a further aspect, the invention relates to an inhibitor according to the invention, comprising the structure 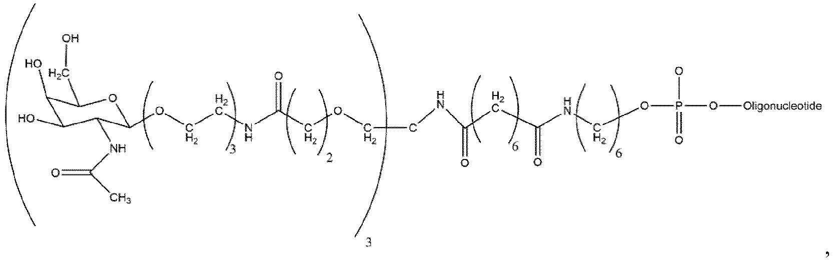 wherein oligonucleotide represents the contiguous nucleosides of the second strand.

In a further aspect, the invention relates to an inhibitor according to the invention, wherein the structure is conjugated to the 3' terminal region of the second strand.

In a further aspect, the invention relates to an inhibitor according to the invention, formulated as a pharmaceutical composition with an excipient and / or carrier.

In another aspect, the invention relates to a pharmaceutical composition comprising an inhibitor according to one or more preceding aspects, in combination with a pharmaceutically acceptable excipient or carrier.

In a further aspect, the invention relates to the pharmaceutical composition according to the invention, further comprising a GLP-1 agonist and/or a THR-beta agonist.

In a further aspect, the invention relates to the pharmaceutical composition according to the invention, wherein the GLP-1 agonist is a GLP-1/GIP dual agonist, a GLP-1/FGF21 dual agonist, a GLP-1/GCGR dual agonist, or a GLP-1/GIP/GCGR triple agonist.

In a further aspect, the invention relates to the pharmaceutical composition according to the invention, wherein the GLP-1 agonist is semaglutide.

In a further aspect, the invention relates to the pharmaceutical composition according to the invention, wherein the THR-beta agonist is resmetirom.

In a further aspect, the invention relates to the pharmaceutical composition according to the invention, wherein the GLP-1/GIP dual agonist is tirzepatide.

In a further aspect, the invention relates to the pharmaceutical composition according to the invention, further comprising one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

In another aspect, the invention relates to an inhibitor according to the invention or a pharmaceutical composition according to the invention for use in therapy.

In another aspect, the invention relates to an inhibitor according to the invention or a pharmaceutical composition according to the invention for use in the prevention and/or treatment of metabolic disease or disorder, such as a metabolic disease or disorder associated with non-alcoholic fatty liver disease (NAFLD), and/or obesity and/or a disease or disorder associated with adipogenesis and/or for use in reducing adipogenesis.

In a further aspect, the invention relates to an inhibitor for use according to the invention or a pharmaceutical composition for use according to the invention, wherein the inhibitor or the pharmaceutical composition is to be used in combination with a GLP-1 agonist and/or a THR-beta agonist.

In a further aspect, the invention relates to an inhibitor for use according to the invention or a pharmaceutical composition for use according to the invention, wherein the GLP-1 agonist is a GLP-1/GIP dual agonist, a GLP-1/FGF21 dual agonist, a GLP-1/GCGR dual agonist, or a GLP-1/GIP/GCGR triple agonist.

In a further aspect, the invention relates to an inhibitor for use according to the invention or a pharmaceutical composition for use according to the invention, wherein the GLP-1 agonist is semaglutide.

In a further aspect, the invention relates to an inhibitor for use according to the invention or a pharmaceutical composition for use according to the invention, wherein the THR-beta agonist is resmetirom.

In a further aspect, the invention relates to an inhibitor for use according to the invention or a pharmaceutical composition for use according to the invention, wherein the GLP-1/GIP dual agonist is tirzepatide.

In a further aspect, the invention relates to an inhibitor for use according to the invention or a pharmaceutical composition for use according to the invention, wherein the inhibitor or the pharmaceutical composition is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

In another aspect, the invention relates to the use of SLC25A5/ANT2 as a target for identifying one or more therapeutic agents for the treatment or prevention of a metabolic disease or disorder, such as a metabolic disease or disorder associated with non-alcoholic fatty liver disease (NAFLD), and/or obesity and/or a disease or disorder associated with adipogenesis and/or for reducing adipogenesis.

In another aspect, the invention relates to a method of treating or preventing metabolic disease or disorder, such as a metabolic disease or disorder associated with non-alcoholic fatty liver disease (NAFLD), and/or obesity and/or a disease or disorder associated with adipogenesis and/or a method of reducing adipogenesis, which comprises administering to a patient an inhibitor of expression and/or function of SLC25A5/ANT2, such as an inhibitor according to the invention.

In a further aspect, the invention relates to a method according to the invention, wherein the inhibitor of expression and/or function of SLC25A5/ANT2 is administered together with a GLP-1 agonist and/or a THR-beta agonist.

In a further aspect, the invention relates to a method according to the invention, wherein the GLP-1 agonist is a GLP-1/GIP dual agonist, a GLP-1/FGF21 dual agonist, a GLP-1/GCGR dual agonist, or a GLP-1/GIP/GCGR triple agonist.

In a further aspect, the invention relates to a method according to the invention, wherein the GLP-1 agonist is semaglutide.

In a further aspect, the invention relates to a method according to the invention, wherein the GLP-1/GIP dual agonist is tirzepatide.

In a further aspect, the invention relates to a method according to the invention, wherein the THR-beta agonist is resmetirom.

In a further aspect, the invention relates to a method according to the invention, wherein the inhibitor of expression and/or function of SLC25A5/ANT2 is administered together with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

In another aspect, the invention relates to the use of an inhibitor according to the invention or a pharmaceutical composition according to the invention, in the preparation of a medicament for the treatment of a metabolic disease or disorder, such as a metabolic disease or disorder associated with non-alcoholic fatty liver disease (NAFLD), and/or obesity and/or a disease or disorder associated with adipogenesis and/or for reducing adipogenesis
In another aspect, the invention relates to SLC25A5/ANT2 for use as a biomarker of a metabolic disease or disorder, such as a metabolic disease or disorder associated with non-alcoholic fatty liver disease (NAFLD), and/or obesity and/or a disease or disorder associated with adipogenesis.

In another aspect, the invention relates to SLC25A5/ANT2 for use in an *in vivo* method of predicting susceptibility to prevention and/or treatment of metabolic disease or disorder, such as a metabolic disease or disorder associated with non-alcoholic fatty liver disease (NAFLD), and/or obesity and/or a disease or disorder associated with adipogenesis, typically by monitoring the sequence and/ or level of expression and / or function of SLC25A5/ANT2 in a sample obtained from a patient.

In another aspect, the invention relates to a method of predicting susceptibility to a metabolic disease or disorder, such as a metabolic disease or disorder associated with non-alcoholic fatty liver disease (NAFLD), and/or obesity and/or a disease or disorder associated with adipogenesis, in a patient, said method comprising:
(a) obtaining a sample from the patient,
(b) detecting the sequence and / or expression and / or function of SLC25A5/ANT2 in said sample obtained from the patient,
(c) predicting susceptibility to a disease related to a metabolic disease or disorder, such as a metabolic disease or disorder associated with non-alcoholic fatty liver disease (NAFLD) and/or obesity and/or a disease or disorder associated with adipogenesis, based on the sequence and / or expression and / or function of SLC25A5/ANT2 in said sample obtained from the patient,
(d) preferably administering to the diagnosed patient an effective amount of an inhibitor of SLC25A5/ANT2.

### FIGURES

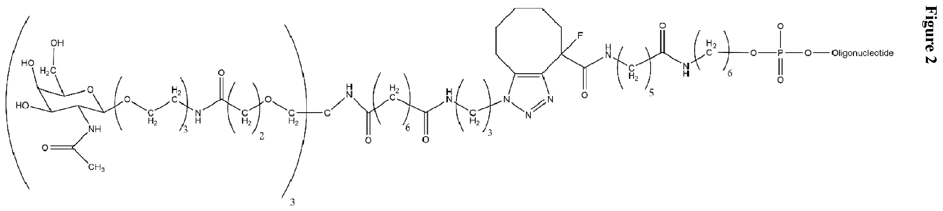

**Figure 1a****:** An exemplary linear configuration for a conjugate.
**Figure 1b**: An exemplary branched configuration for a conjugate.
**Figure 2****:** Linker and ligand portions of constructs suitable for use according to the present invention including tether 1a. While Figure 2 depicts the linker to be conjugated to an oligonucleotide, it is to be understood that the present invention also encompasses conjugates of the same linker with an oligonucleoside as disclosed herein.
   It should also be understood that while Figure 2 depicts as a product molecules based on the linker and ligand portions as specifically depicted in Figure 2 attached to an oligonucleoside moiety as also depicted herein, this product may alternatively further comprise, or consist essentially of, molecules wherein the linker and ligand portions are essentially as depicted in Figure 2 attached to an oligonucleoside moiety but having the F substituent as shown in Figure 2 on the cyclo-octyl ring replaced by a substituent, which could occur as a result of hydrolytic displacement, such as an OH substituent, or the OH substituent could be synthesized as a linker in its own right. In this way, (a) tether 1a constructs can consist essentially of molecules having linker and ligand portions specifically as depicted in Figure 2, with a F substituent on the cyclo-octyl ring; or (b) tether 1a constructs can consist essentially of molecules having linker and ligand portions essentially as depicted in Figure 2 but having the F substituent as shown in Figure 2 on the cyclo-octyl ring replaced by an OH substituent, or (c) tether 1a constructs can comprise a mixture of molecules as defined in (a) and/or (b).

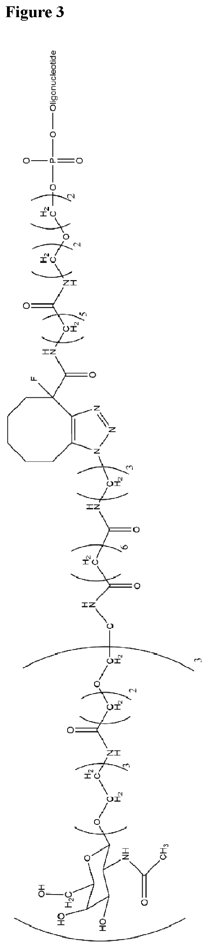

**Figure 3****:** Linker and ligand portions of constructs suitable for use according to the present invention including tether 1b. While Figure 3 depicts the linker to be conjugated to an oligonucleotide, it is to be understood that the present invention also encompasses conjugates of the same linker with an oligonucleoside as disclosed herein.
   The comments made in relation to Figure 2 and the possible replacement of the F substituent as shown in Figure 2 on the cyclo-octyl ring replaced by a substituent, which could occur as a result of hydrolytic displacement, such as an OH substituent, or the OH substituent could be synthesized as a linker in its own right, apply equally to tether 1b constructs. In this way, (a) tether 1b constructs can consist essentially of molecules having linker and ligand portions specifically as depicted in Figure 3, with a F substituent on the cyclo-octyl ring; or (b) tether 1b constructs can consist essentially of molecules having linker and ligand portions essentially as depicted in Figure 3 but having the F substituent as shown in Figure 3 on the cyclo-octyl ring replaced by an OH substituent, or (c) tether 1b constructs can comprise a mixture of molecules as defined in (a) and/or (b).

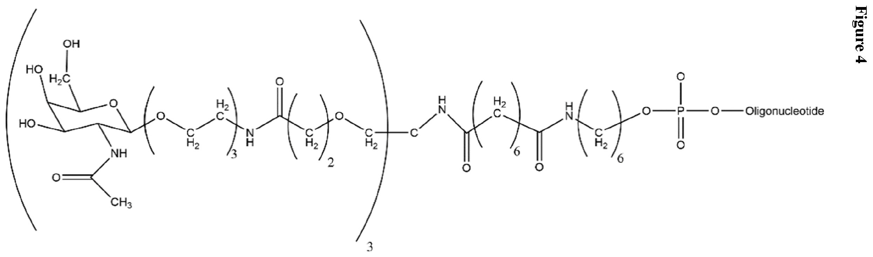

**Figure 4****:** Linker and ligand portions of constructs suitable for use according to the present invention including tether 2a. While Figure 4 depicts the linker to be conjugated to an oligonucleotide, it is to be understood that the present invention also encompasses conjugates of the same linker with an oligonucleoside as disclosed herein.

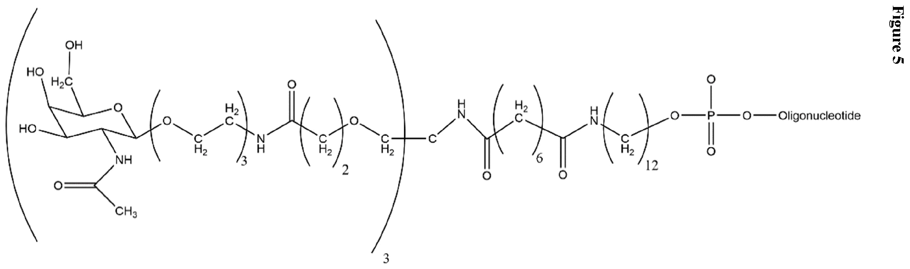

**Figure 5****:** Linker and ligand portions of constructs suitable for use according to the present invention including tether 2b. While Figure 5 depicts the linker to be conjugated to an oligonucleotide, it is to be understood that the present invention also encompasses conjugates of the same linker with an oligonucleoside as disclosed herein.

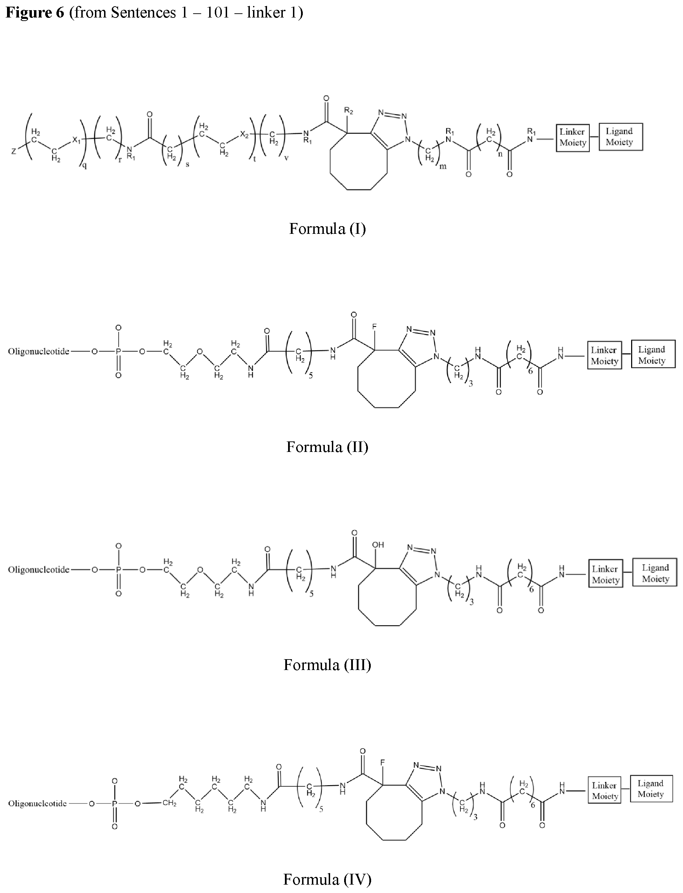

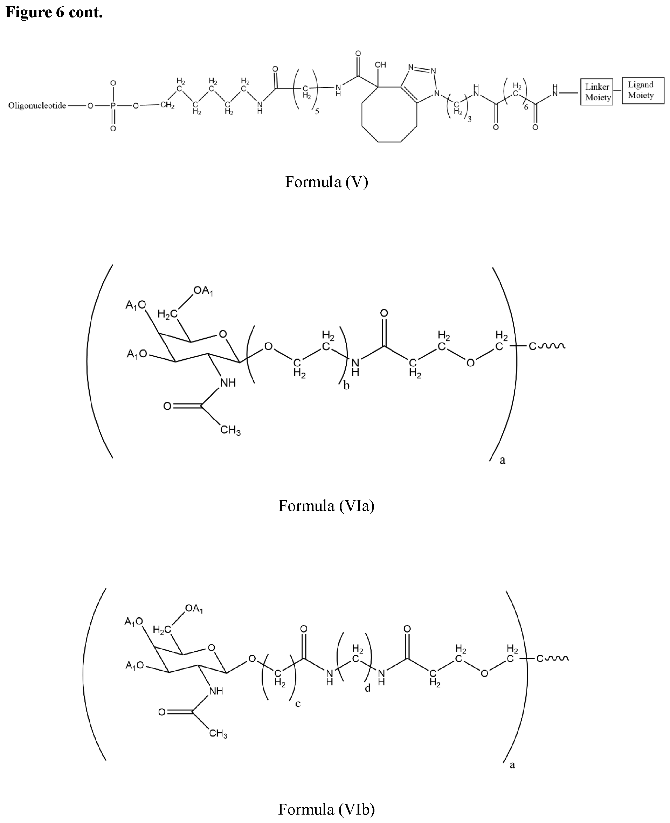

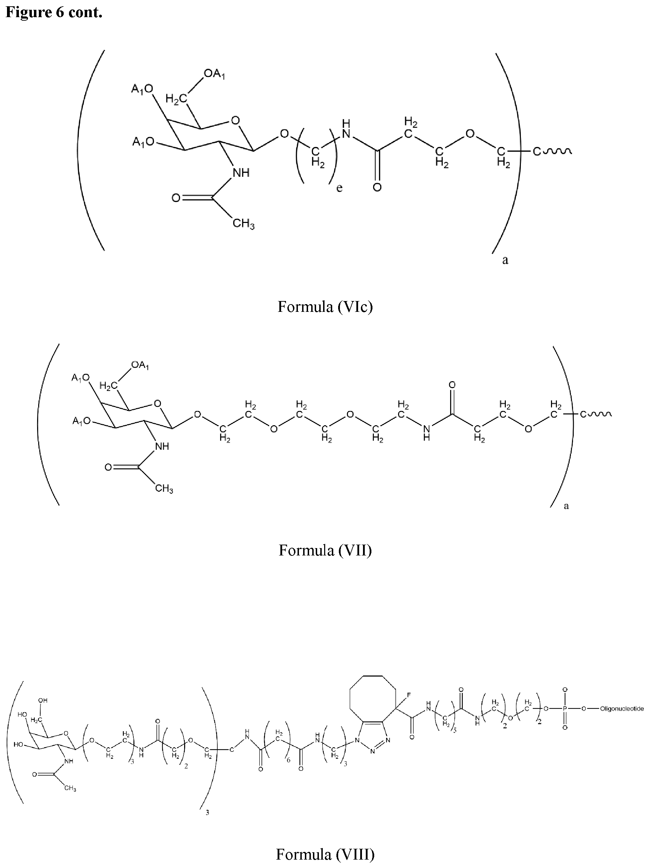

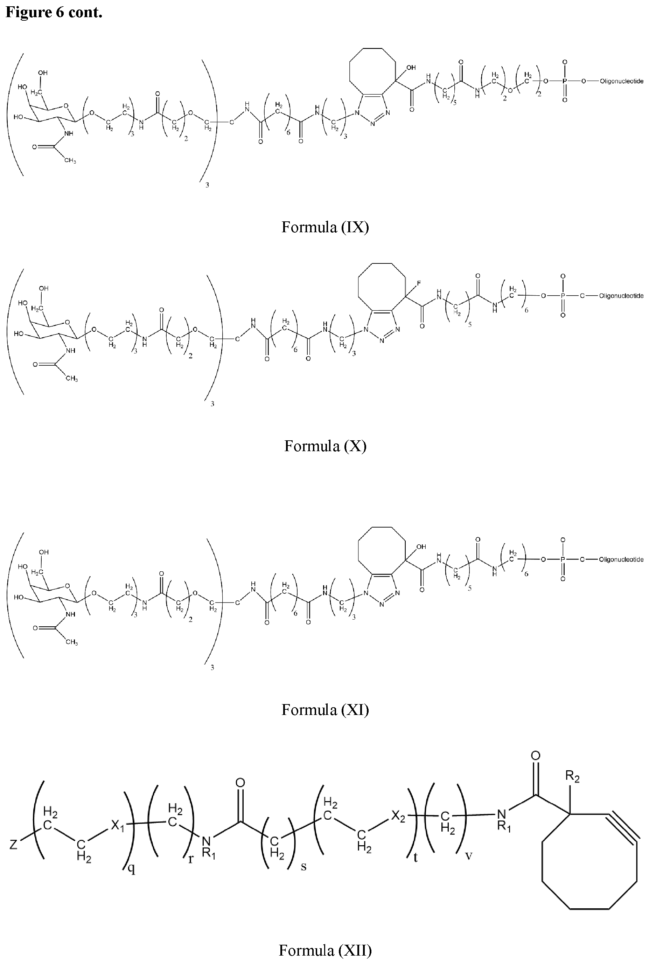

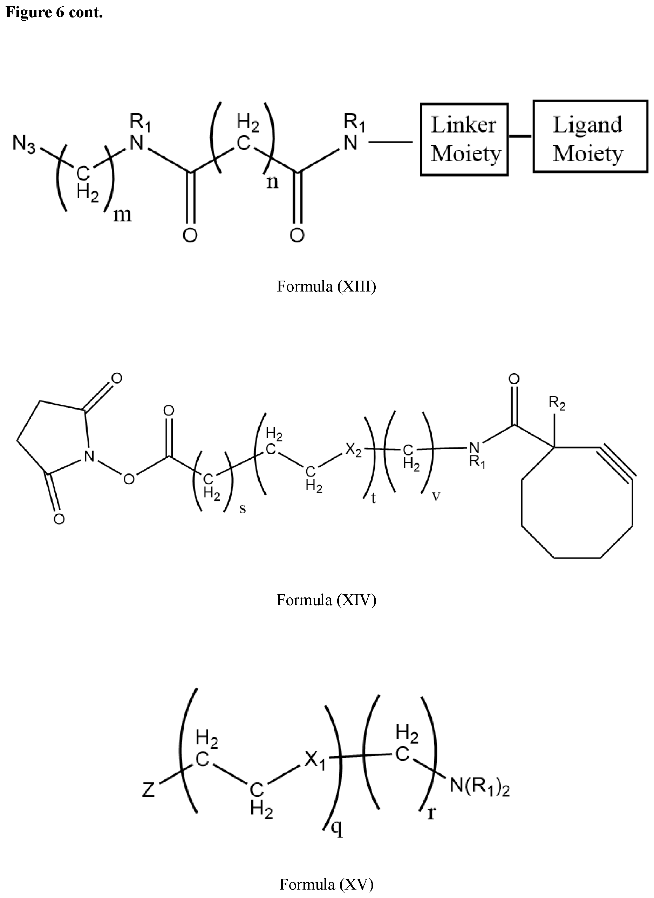

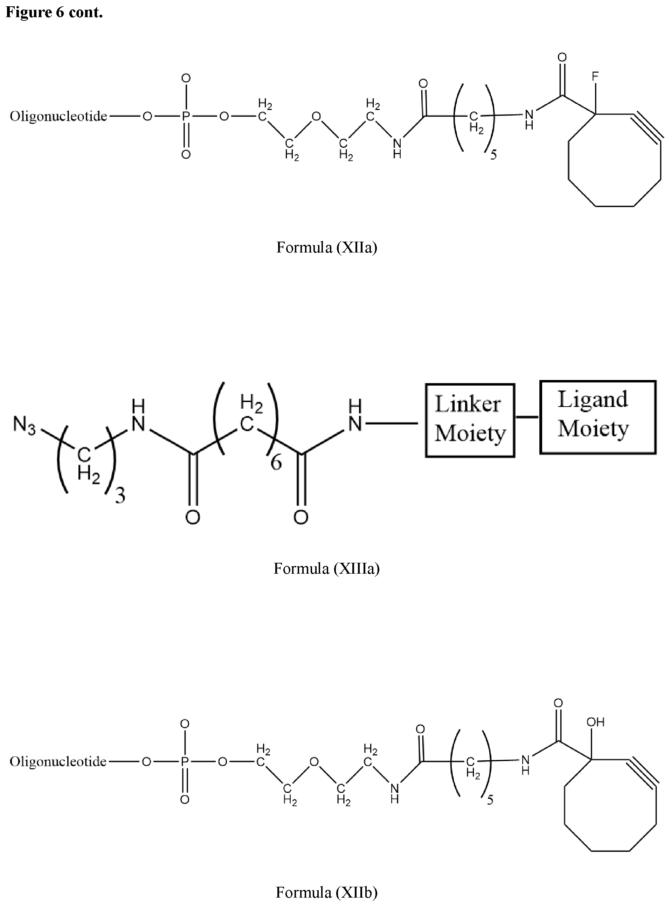

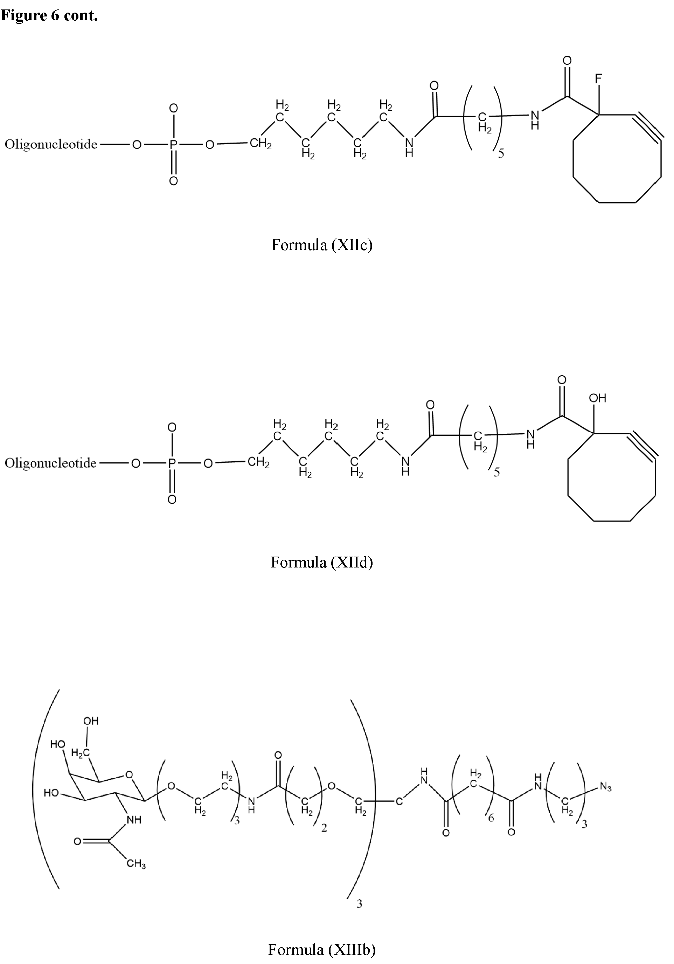

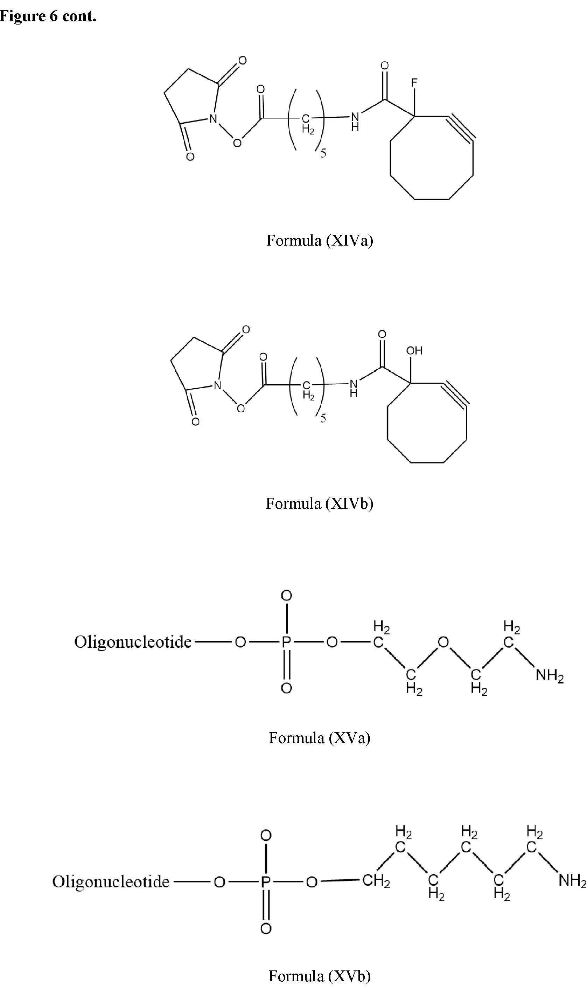

**Figure 6****:** Formulae described in Sentences 1-101 disclosed herein.

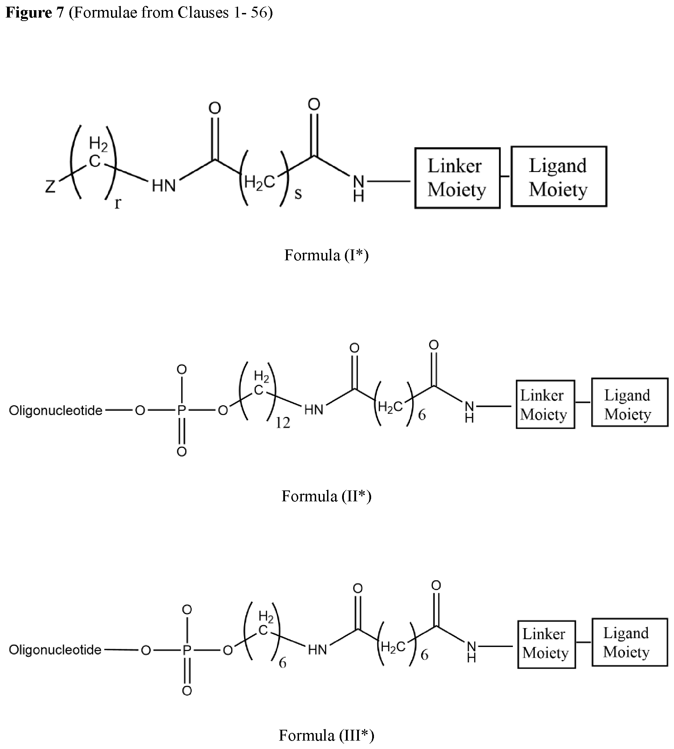

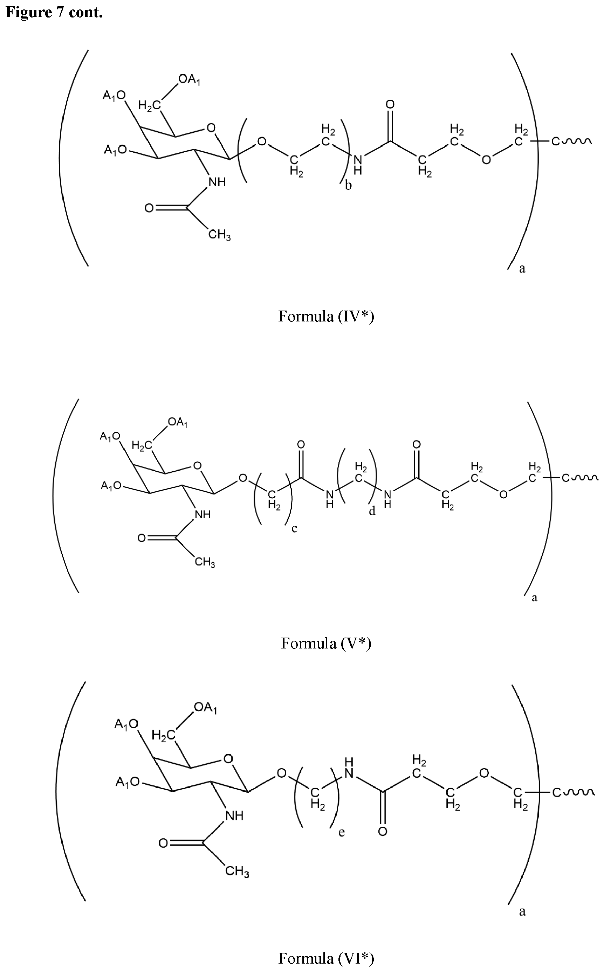

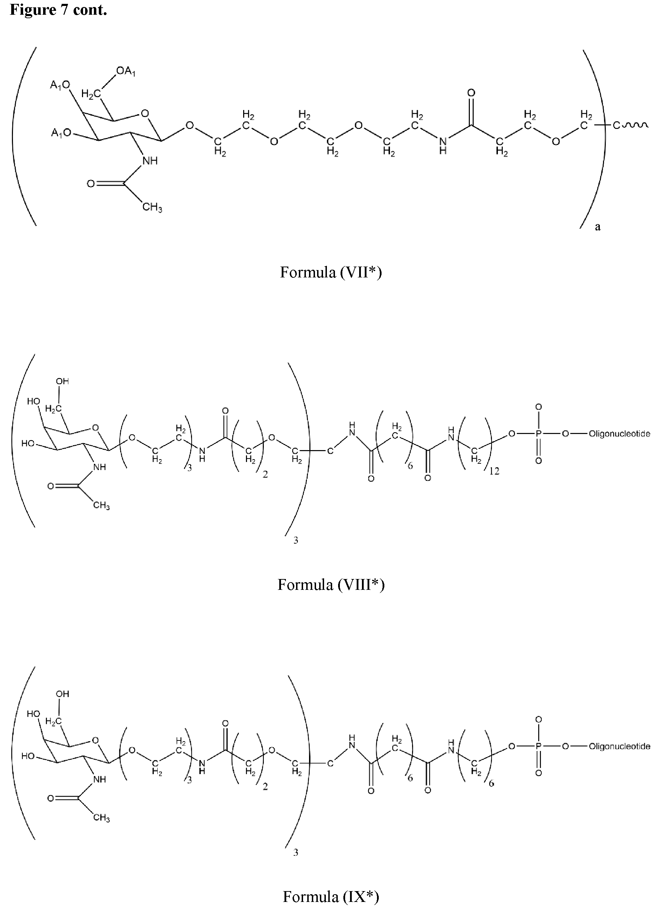

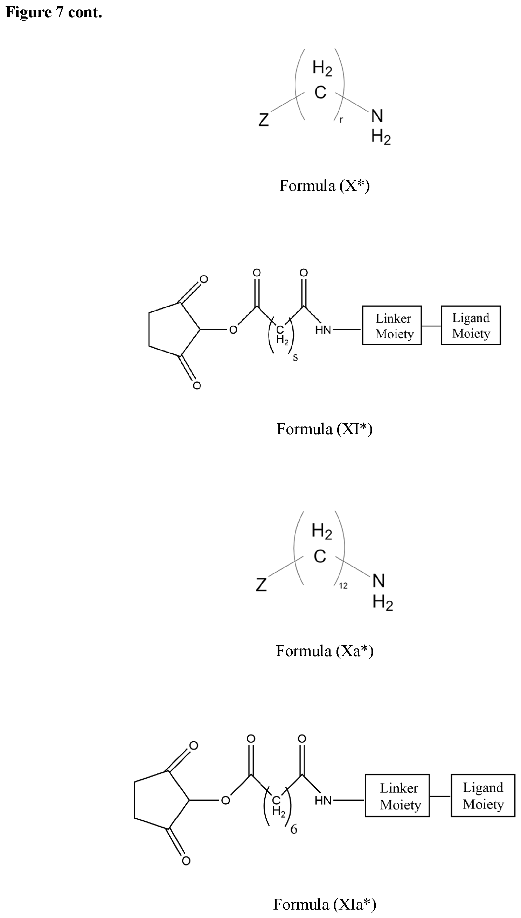

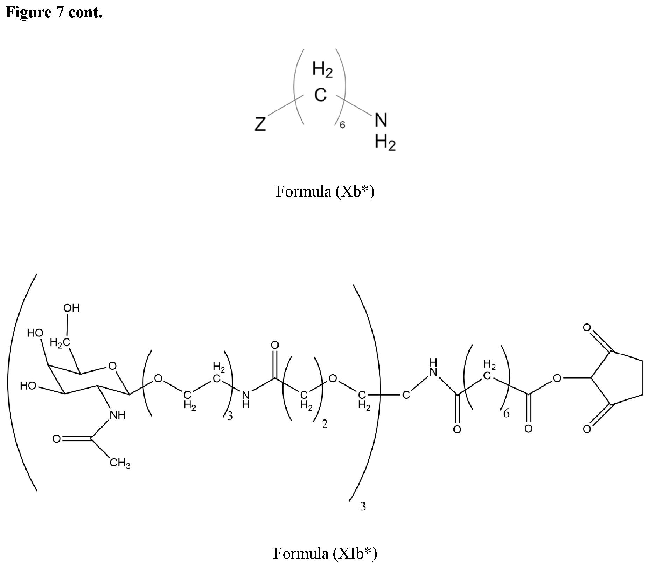

**Figure 7****:** Formulae described in Clauses 1-56 disclosed herein.

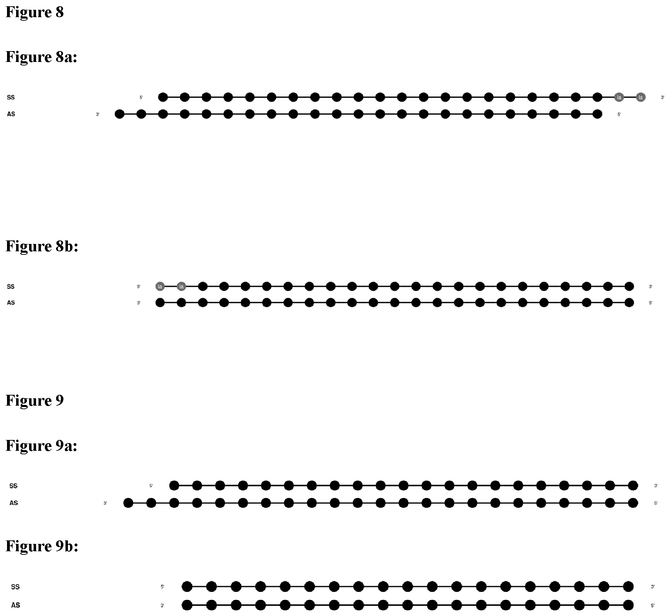

**Figures 8a and 8b****:** Inverted abasic constructs that can be used with nucleic acid sequences according to the present invention as described herein. For Figure 8a, a GalNAc linker is attached to the 5' end region of the sense strand in use (not depicted in Figure 8a). For Figure 8b, a GalNAc linker is attached to the 3' end region of the sense strand in use (not depicted in Figure 8b).
   iaia as shown at the 3' end region of the sense strand in Figure 8a represents (i) two abasic nucleosides provided as the penultimate and terminal nucleosides at the 3' end region of the sense strand, (ii) wherein a 3'-3' reversed linkage is provided between the antepenultimate nucleoside (namely at position 21 of the sense strand, wherein position 1 is the terminal 5' nucleoside of the sense strand) and the adjacent penultimate abasic residue of the sense strand, and (iii) the linkage between the terminal and penultimate abasic nucleosides is 5'-3' when reading towards the 3' end region comprising the terminal and penultimate abasic nucleosides.
   iaia as shown at the 5' end region of the sense strand in Figure 8b represents (i) two abasic nucleosides provided as the penultimate and terminal nucleosides at the 5' end region of the sense strand, (ii) wherein a 5'-5' reversed linkage is provided between the antepenultimate nucleoside (namely at position 1 of the sense strand, not including the iaia motif at the 5' end region of the sense strand in the nucleoside position numbering on the sense strand) and the adjacent penultimate abasic residue of the sense strand, and (iii) the linkage between the terminal and penultimate abasic nucleosides is 3'-5' when reading towards the 5' end region comprising the terminal and penultimate abasic nucleosides.
**Figures 9a and 9b****:** Duplex constructs according to Table 5.

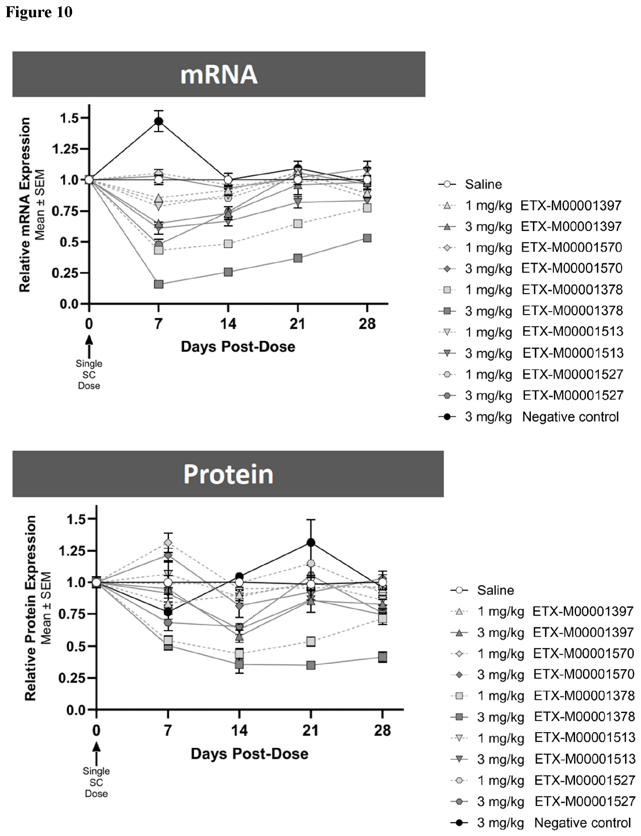

**Figures 10****:** Summary of mRNA and protein knockdown effects of a single dose of GalNAc-siRNAs, ETX-M00001397, ETX-M00001570, ETX-M00001378, ETX-M00001513, and ETX-M00001527 (1 mg/kg or 3 mg/kg) in mouse liver tissues. As a negative control, a non-target specific siRNA was used. The y-axis values are the relative mRNA or protein expression and data is normalized to the saline control group (n=4). Each data point represents the relative mRNA or protein expression as Mean ± SEM from n=4 experiment.

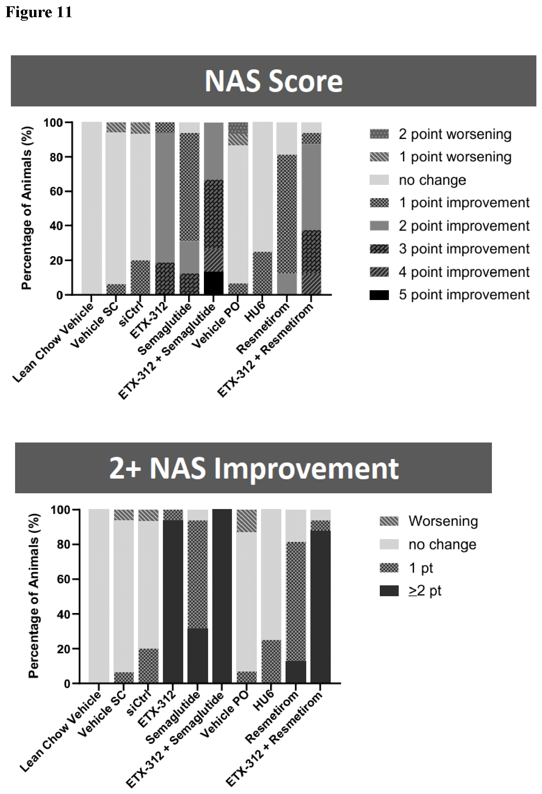

**Figure 11****:** Liver samples stained with H&E were given a score for NAFLD Activity Score (NAS) using the clinical criteria outlined by Kleiner et al. (2005). Total NAS represents the sum of scores for steatosis, inflammation, and ballooning, and ranges from 0-8. NAS score was determined by Gubra Histopathological Objective Scoring Technology (GHOST) deep learning app developed by Gubra using the VIS software (Visiopharm, Denmark) for a more accurate and objective method for staging disease in DIO-NASH mouse models. Results are presented as change in NAS score (improvement or worsening) at study termination compared to the pre-dose biopsy. Also shown is the percentage of animals with at least a 1- or 2-point improvement in NAS.

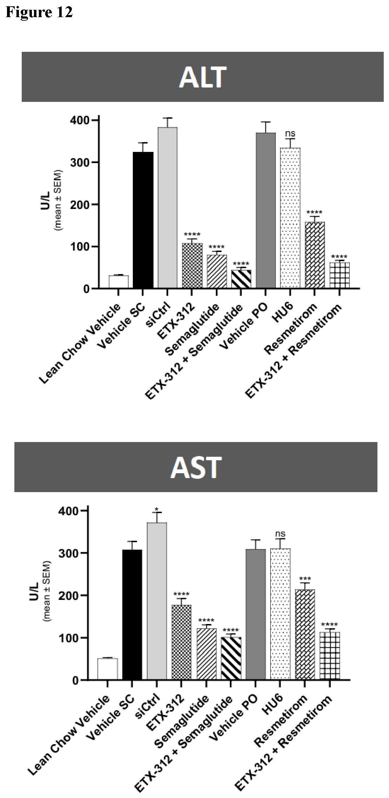

**Figure 12****:** ALT and AST were measured in plasma samples after 12 weeks ETX-312 (ETX-M00001378) treatment using commercial kits (Roche Diagnostics), on the cobas c501 autoanalyzer. ALT and AST levels were increased in DIO-NASH mice (vehicle sc, siCtrl, vehicle PO). Treatment with ETX-312 (ETX-M00001378) alone or in combination with semaglutide or resmetirom significantly reduced both ALT and AST levels. Results are presented as absolute levels as Mean ± SEM from n=16 experiment. * p < 0.05; *** p < 0.001; **** p < 0.0001

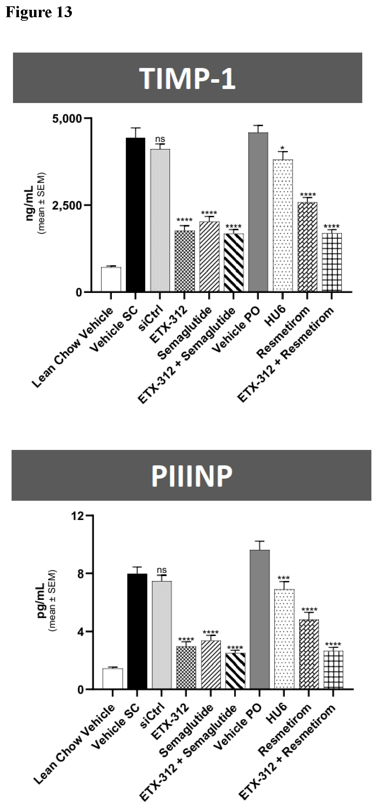

**Figure 13****:** TIMP-1 and PIIINP are non-invasive blood biomarkers for NAFLD/NASH that predict hepatic fibrosis. TIMP-1 was measured in plasma collected in EDTA tubes using a commercial ELISA kit (R&D Systems). PIIINP was measured in plasma collected in EDTA tubes using a commercial ELISA kit (Cusabio). TIMP-1 and PIIINP levels were increased in DIO-NASH mice (vehicle sc, siCtrl, vehicle PO). Treatment with ETX-312 (ETX-M00001378) alone or in combination with semaglutide or resmetirom significantly reduced both TIMP-1 and PIIINP levels. Results are presented as absolute levels as Mean ± SEM from n=16 experiment. An outlier analysis was conducted by comparing the studentised residuals of the linear model fitted to the subcutaneous treatment subset of the data to the critical Bonferroni alpha level (0.05/88 = ~0.00057). One animal in the semaglutide group was identified as an outlier which was verified by an influence analysis. This animal was concluded as both highly outlying and influential and was excluded from all TIMP-1 and PIIINP analysis. * p < 0.05; *** p < 0.001; **** p < 0.0001

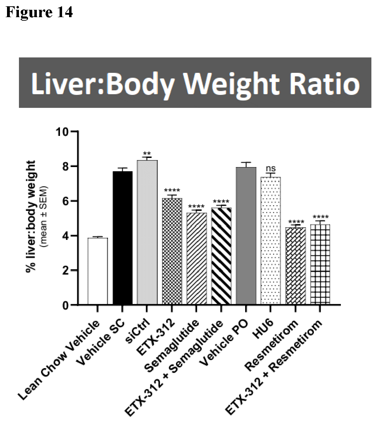

**Figure 14****:** Terminal liver weight to body weight ratio demonstrates hepatomegaly in DIO-NASH mice. Treatment with ETX-312 (ETX-M00001378) alone or in combination with semaglutide or resmetirom significantly reduced liver to body weight ratio. Results are presented as percent liver:body weight as Mean ± SEM from n=16 experiment. ** p < 0.01; **** p < 0.0001

### DETAILED DESCRIPTION

The present invention, inter alia, provides inhibitors, for example oligomers such as nucleic acids, such as inhibitory RNA molecules (which may be referred to as iRNA or siRNA ), and compositions containing the same which can affect expression of a target, for example by binding to mRNA transcribed from a gene. The target may be within a cell, e.g. a cell within a subject, such as a human. The inhibitors can be used to prevent and/or treat medical conditions associated with the e.g. the expression of a target gene.

In particular the present invention identifies inhibitors of expression and/or function of SLC25A5/ANT2 as useful in the prevention and/or treatment of a metabolic disease or disorder, such as a metabolic disease or disorder associated with non-alcoholic fatty liver disease (NAFLD) and/or obesity and/or a disease or disorder associated with adipogenesis.

ADP/ATP translocase 2 (ANT2) is a protein that in humans is encoded by the *SLC25A5* gene on the X chromosome. This protein functions as an antiporter for ADP/ATP exchange between the mitochondrial matrix and cytoplasm.

The present invention relates to an inhibitor of expression and/or function of SLC25A5/ANT2. Accordingly, in certain embodiments, the invention relates to an inhibitor of expression of the *SLC25A5* gene, such as an siRNA that targets an mRNA transcribed from the *SLC25A5* gene. In certain embodiments, the invention relates to an inhibitor of function of the gene product ANT2. Both options are encompassed when it referred herein to an inhibitor of SLC25A5/ANT2 or an inhibitor of the invention.

In humans, ANT2 is encoded by the *SLC25A5* gene (SEQ ID NO:1381).

SEQ ID NO:1381 (SLC25A5)

ATCGGCCATTTGCTTCGCTCCGCCCCGCAGCGCCGGAGTCAAAGCCGGTTCCCGGCCCAGTCCCGTCCT
GCAGCAGTCTGCCTCCTCTTTCAACATGACAGATGCCGCTGTGTCCTTCGCCAAGGACTTCCTGGCAGG
TGGAGTGGCCGCAGCCATCTCCAAGACGGCGGTAGCGCCCATCGAGCGGGTCAAGCTGCTGCTGCAG
GTACGTCCTGGGATCCAGGAGCCCAACCAGGAAGTGGGGGGAAGGGTCGCACAGAAGGCGGGCGCCC
GAGGGGTGGCGGGGAGCGAACTCTAAAGACATGGCCAGGGAAGCGGCTTAGGAGAGGCCAGAGCGG
GGCGCAGAGGCAGAACAGAAGTCAAACTGGTGGGAGGCGCCTTTAGTGACCTGAAGTAGTGAGTCTA
GGAAGGGGCCGGGGGCAGAGGGCAGGACCAGGCTCTCGGCATCTCCGAGGCGGCGGACTCGGATGG
AGCAGTTTCTGAGTGACGGCCTCCCCGGGCCTGGGCGTCAAGGGCGAAGGCCGAAAGCCGGCGTTAG
AAAGAGGAACGCCAGTTCTTACCGAAGACCTCAAGGTCGCGGCAAGGAGATAACTGCCCGGGGGAGG
CCATGCGCCCGGGTCCAGCGGCCTCCCAGCCCGCGGACGCGCTCAAACCTCGCCGGGCCGGAAGCCG
GCGCCGGGAAGCGCGTGTGCCTTTTACGTCCGCCCCCGCGCAGCCGCGGCCGCTGCCGCCGCGTCTCC
GCCTGCCTCCCTGCGCCGCGCGCTCTCCAGTGCCGGCTCTAGAGGGCGCTCCTGGGCTAGCGTGTAGG
GCTGGCGGCGGCGGCGCTCGGGTCACCTCTGGGAGCGGAGTGGGGGCGGAGCGAGACGGAAGCAGCT
CAGGAGACTTGAGGCGTAGGCTGCGGTCCCCAAGGTGACCGCGCCCTATGTGGGACTCGCCCTAATGC
CTCTGAACCTGGGTTTGAGGTAATGACCTTTCTCCTAGGTCTGAAGGTCACGGGTCCGCTGGAGGATG
CCCCCTCTCCACTCAGAGGGGTGGAGGCTTAATGCTACTGGTGCAGATCACCTCTTCCCCTGTGACAGC
CTCAGAGGGTTGGGAGGGTCCAGCCAGTATGATATACGAAGACTAGATTTGAGAGAGGGGAGCCTAC
CTTAAAGGGCATTGATCGAGATGGCATAAGCTCTTCTCTTTCCCTTCCCCATGGTTATAACTGTCCCTG
TTGGCTTCCTTCCTGTCTGTTAGGTGCAGCATGCCAGCAAGCAGATCACTGCAGATAAGCAATACAAA
GGCATTATAGACTGCGTGGTCCGTATTCCCAAGGAGCAGGGAGTTCTGTCCTTCTGGCGCGGTAACCT
GGCCAATGTCATCAGATACTTCCCCACCCAGGCTCTTAACTTCGCCTTCAAAGATAAATACAAGCAGA
TCTTCCTGGGTGGTGTGGACAAGAGAACCCAGTTTTGGCTCTACTTTGCAGGGAATCTGGCATCGGGT
GGTGCCGCAGGGGCCACATCCCTGTGTTTTGTGTACCCTCTTGATTTTGCCCGTACCCGTCTAGCAGCT
GATGTGGGTAAAGCTGGAGCTGAAAGGGAATTCCGAGGCCTCGGTGACTGCCTGGTTAAGATCTACA
AATCTGATGGGATTAAGGGCCTGTACCAAGGCTTTAACGTGTCTGTGCAGGGTATTATCATCTACCGA
GCCGCCTACTTCGGTATCTATGACACTGCAAAGGGTAAGTTTGCTGTGGGCTTTAACGTTGTGTTCTTA

GGAGACAGTTTAAAAGAGCATTGTACCAACCTAACAGTCCAAGAGCTAAAGAGTTGTTTTTTAATTG
CTAAAGGAAGCCAAGATCATCCAATGCAACCCTTGTGTACAGATGACGTGTTTAGGGGATGTGGGGA
AAGGAAGTCAGTAAAACTCTGCTTTTGGTAAAAGCATCTCTTTCCTATTCCCAGGAATGCTTCCGGAT
CCCAAGAACACTCACATCGTCATCAGCTGGATGATCGCACAGACTGTCACTGCTGTTGCCGGGTTGAC
TTCCTATCCATTTGACACTGTTCGCCGCCGCATGATGATGCAGTCAGGGCGCAAAGGAAGTAAGTTCC
ACTTGAGCAGAAGATAAAGTTGTAGTCGTGGGGCAATCTGCTGCCACAAACTGGTGATACATACCTTT
AAAATGGCTGTCTGTCCAAGTCAAGGGATGGGGTTGATAGCATCTGTGTCTGTTCCACAACTGCCTTTG
AGCTGGCCCTTCAGATGCCTATGAATGAGGGTGCTTAAATGGTGTTAGAGGTTAAGACCAATGGGTAG
TCTGTATTCCTGTGGTCATAGCATTAATATTTCAGTGTTGCCCATGCTAATGTGTGAATGTTGGATTTA
AAGCTGACGTTCTTAGAGGTGGGGCTCTGCTTTATTTAGCCTAGTGAATCTTAGGATTTTTCATCGGCC
TTCAGTCACTAACTCCATGTCTTTATTCTTTGCAGCTGACATCATGTACACAGGCACGCTTGACTGCTG
GCGGAAGATTGCTCGTGATGAAGGAGGCAAAGCTTTTTTCAAGGGTGCATGGTCCAATGTTCTCAGAG
GCATGGGTGGTGCTTTTGTGCTTGTCTTGTATGATGAAATCAAGAAGTACACATAAGTTATTTCCTAGG
ATTTTTCCCCCTGTGAACAGGCATGTTGTATTATATAACATATCTTGAGCATTCTTGACAGACTCCTGG
CTGTCAGTTTCTCAGTGGCAACTATTTACTGGTTGAAAATGGGAAGCAATAATATTCATCTGACCAGTT
TTCTCTTAAAGCCATTTCCATGATGATGATGATGGGACTCAATTGTATTTTTATTTCAGTCACTCCTGA
TAAATAACAAATTTGGAGAAATAAAAATATCTAAAATAAATTTTGTCTGCAGTATATTTTCATATAAA
AATGCATATTTGAGTGCTACATTCGAATAAATACTACCTTTTTAGTGAA

The inventors employed network analysis that allows them to allocate multiple genes or proteins to a smaller number of driver processes; and to mine these processes for impactful drug targets. The approach takes advantage of information that is usually ignored in standard gene set analyses - the known and predicted interactions between genes (and proteins) and the inclusion of other genes in the same or related pathways. In particular, the inventors analysed Genome Wide Association Studies (GWAS) metanalyses for Non-Alcoholic Fatty Liver Disease (NAFLD) using network models which highlighted SLC25A5/ANT2 as a preferred target for NAFLD among other known targets associated with NAFLD.

The inhibition disclosed herein may be of the gene SLC25A5 or protein ANT2 resulting from expression of the SLC25A5 gene and reference to SLC25A5/ANT2 hereby explicitly incorporates a reference to inhibition of the expression or function of the gene and, separately, of the protein product.

### DEFINITIONS

The "first strand", also called the antisense strand or guide strand herein and which can be used interchangeably herein, refers to the nucleic acid strand, e.g. the strand of an siRNA, e.g. a dsiRNA, which includes a region that is substantially complementary to a target sequence, e.g. to an mRNA. As used herein, the term "region of complementarity" refers to the region on the antisense strand that is substantially complementary to a sequence, for example a target sequence. Where the region of complementarity is not fully complementary to the target sequence, the mismatches can be in the internal or terminal regions of the molecule. In some embodiments, a double stranded nucleic acid e.g. an siRNA agent of the invention includes a nucleotide mismatch in the antisense strand.

The "second strand" (also called the sense strand or passenger strand herein, and which can be used interchangeably herein), refers to the strand of a nucleic acid e.g. siRNA that includes a region that is substantially complementary to a region of the antisense strand as that term is defined herein.

In the context of molecule comprising a nucleic acid provided with a ligand moiety, optionally also with a linker moiety, the nucleic acid of the invention may be referred to as an oligonucleotide moiety or oligonucleoside moiety

Oligonucleotides are short nucleic acid polymers. Whilst oligonucleotides contain phosphodiester bonds between the nucleoside component thereof (base plus sugar), the present invention is not limited to oligonucleotides always joined by such a phosphodiester bond between adjacent nucleosides, and other oligomers of nucleosides joined by bonds which are bonds other than a phosphate bond are contemplated. For example, a bond between nucleotides may be a phosphorothioate bond. Therefore, the term "oligonucleoside" herein covers both oligonucleotides and other oligomers of nucleosides. An oligonucleoside which is a nucleic acid having at least a portion which is an oligonucleotide is preferred according to the present invention. An oligonucleoside having one or more, or a majority of, phosphodiester backbone bonds between nucleosides is also preferred according to the present invention. An oligonucleoside having one or more, or a majority of, phosphodiester backbone bonds between nucleosides, and also having one or more phosphorothioate backbone bonds between nucleosides (typically in a terminal region of the first and / or second strands) is also preferred according to the present invention.

It is preferred herein that the nucleic acid according to the invention is a double stranded oligonucleoside comprising one or more phosphorothioate backbone bonds between nucleosides. Accordingly, in all instances in which the present application refers to an oligonucleotide, particularly in the chemical structures disclosed herein, the oligonucleotide may equally be an oligonucleoside as defined herein.

In some embodiments, a double stranded nucleic acid e.g. siRNA agent of the invention includes a nucleoside mismatch in the sense strand. In some embodiments, the nucleoside mismatch is, for example, within 5, 4, 3, 2, or 1 nucleosides from the 3 '-end of the nucleic acid e.g. siRNA.

In another embodiment, the nucleoside mismatch is, for example, in the 3'- terminal nucleoside of the nucleic acid e.g. siRNA.

A "target sequence" (which may be called a target RNA or a target mRNA) refers to a contiguous portion of the nucleoside sequence of an mRNA molecule formed during the transcription of a gene, including mRNA that is a product of RNA processing of a primary transcription product, or can be a contiguous portion of the nucleotide sequence of any RNA molecule such as a LNCRNA which it is desired to inhibit.

The target sequence may be from about 10-35 nucleosides in length, e.g., about 15-30 nucleosides in length. For example, the target sequence can be from about 15-30 nucleosides, 15-29, 15-28, 15-27, 15-26, 15-25, 15-24, 15-23, 15-22, 15-21, 15-20, 15-19, 15-18, 15-17, 18-30, 18-29, 18- 28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24, 20-23, 20-22, 20- 21, 21-30, 21-29, 21-28, 21-27, 21-26, 21-25, 21-24, 21-23, or 21-22 nucleosides in length. Ranges and lengths intermediate to the above recited ranges and lengths are also contemplated to be part of the invention.

The term "ribonucleoside" or "nucleoside" can also refer to a modified nucleoside as further detailed below.

A nucleic acid can be a DNA or an RNA, and can comprise modified nucleosides. RNA is a preferred nucleic acid.

The terms "iRNA", "siRNA", "RNAi agent," and "iRNA agent," "RNA interference agent" as used interchangeably herein, refer to an agent that contains RNA, and which mediates the targeted cleavage of an RNA transcript via an RNA-induced silencing complex (RISC) pathway. siRNA directs the sequence-specific degradation of mRNA through RNA interference (RNAi).

A double stranded RNA is referred to herein as a "double stranded siRNA (dsiRNA) agent", "double stranded siRNA (dsiRNA) molecule", "double stranded RNA (dsRNA) agent", "double stranded RNA (dsRNA) molecule", "dsiRNA agent", "dsiRNA molecule", or "dsiRNA", which refers to a complex of ribonucleic acid molecules, having a duplex structure comprising two antiparallel and substantially complementary nucleic acid strands, referred to as having "sense" and "antisense" orientations with respect to a target RNA. The majority of nucleosides of each strand of the nucleic acid, e.g. a dsRNA molecule, are preferably ribonucleosides, but in that case each or both strands can also include one or more non-ribonucleosides, e.g., a deoxyribonucleoside or a modified ribonucleoside. In addition, as used in this specification, an "siRNA" may include ribonucleosides with chemical modifications.

The term "modified nucleoside" refers to a nucleoside having, independently, a modified sugar moiety, a modified internucleoside linkage, or modified nucleobase, or any combination thereof. Thus, the term modified nucleoside encompasses substitutions, additions or removal of, e.g., a functional group or atom, to internucleoside linkages, sugar moieties, or nucleobases. Any such modifications, as used in a siRNA type molecule, are encompassed by "iRNA" or "RNAi agent" or "siRNA" or "siRNA agent" for the purposes of this specification and claims.

The duplex region of a nucleic acid of the invention e.g. a dsRNA may range from about 9 to 40 base pairs in length such as 9 to 36 base pairs in length, e.g., about 15- 30 base pairs in length, for example, about 9, 10, 11, 12, 13, 14, 15, 16, 17, 18, 19, 20, 21, 22, 23, 24, 25, 26, 27, 28, 29, 30, 31, 32, 33, 34, 35, or 36 base pairs in length, such as about 15-30, 15-29, 15-28, 15-27, 15-26, 15-25, 15-24, 15-23, 15-22, 15-21, 15-20, 15-19, 15-18, 15-17, 18-30, 18-29, 18-28, 18- 27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21- 30, 21-29, 21-28, 21-27, 21-26, 21-25, 21-24, 21-23, or 21-22 base pairs in length.

The two strands forming the duplex structure may be different portions of one larger molecule, or they may be separate molecules e.g. RNA molecules.

The term "nucleoside overhang" refers to at least one unpaired nucleoside that extends from the duplex structure of a double stranded nucleic acid. A ds nucleic acid can comprise an overhang of at least one nucleoside; alternatively, the overhang can comprise at least two nucleosides, at least three nucleosides, at least four nucleosides, at least five nucleosides, or more. A nucleoside overhang can comprise or consist of a nucleoside analog, including a deoxynucleoside. The overhang(s) can be on the sense strand, the antisense strand, or any combination thereof. Furthermore, the /nucleoside(s) of an overhang can be present on the 5'-end, 3'-end, or both ends of either an antisense or sense strand.

In certain embodiments, the antisense strand has a 1-10 nucleoside, e.g., 0-3, 1-3, 2-4, 2-5, 4-10, 5-10, 1, 2, 3, 4, 5, 6, 7, 8, 9, or 10 nucleoside overhang at the 3'-end or the 5'-end.

"Blunt" or "blunt end" means that there are no unpaired nucleoside at that end of the double stranded nucleic acid, i.e., no nucleoside overhang. The nucleic acids of the invention include those with no nucleoside overhang at one end or with no nucleoside overhangs at either end.

Unless otherwise indicated, the term "complementary," when used to describe a first nucleoside sequence in relation to a second nucleoside sequence, refers to the ability of an oligonucleoside comprising the first nucleoside sequence to hybridize and form a duplex structure under certain conditions with an oligonucleoside or polynucleoside comprising the second nucleoside sequence, as will be understood by the skilled person. Such conditions can, for example, be stringent conditions, where stringent conditions can include: 400 mM NaCl, 40 mM PIPES pH 6.4, 1 mM EDTA, 50°C or 70°C for 12-16 hours followed by washing (see, e.g., "Molecular Cloning: A Laboratory Manual, Sambrook, et al. (1989) Cold Spring Harbor Laboratory Press).

Complementary sequences within nucleic acid e.g. a dsiRNA, as described herein, include base-pairing of the oligonucleoside or polynucleoside comprising a first nucleoside sequence to an oligonucleoside or polynucleoside comprising a second nucleoside sequence over the entire length of one or both nucleoside sequences. Such sequences can be referred to as "fully complementary" with respect to each other herein. However, where a first sequence is referred to as "substantially complementary" or "partially complementary"with respect to a second sequence herein, the two sequences can be fully complementary, or they can form one or more mismatched base pairs, such as 2, 4, or 5 mismatched base pairs, but preferably not more than 5, while retaining the ability to hybridize under the conditions most relevant to their ultimate application, e.g., inhibition of gene expression via a RISC pathway. Overhangs shall not be regarded as mismatches with regard to the determination of complementarity. For example, a nucleic acid e.g. dsRNA comprising one oligonucleoside 17 nucleosides in length and another oligonucleoside 19 nucleosides in length, wherein the longer oligonucleoside comprises a sequence of 17 nucleosides that is fully complementary to the shorter oligonucleoside, can yet be referred to as "fully complementary".

"Complementary" sequences, as used herein, can also include, or be formed entirely from, non-Watson-Crick base pairs or base pairs formed from non-natural and modified nucleosides, in so far as the above requirements with respect to their ability to hybridize are fulfilled. Such non-Watson-Crick base pairs include, but are not limited to, G:U Wobble or Hoogstein base pairing.

The terms "complementary," "fully complementary" and "substantially/partially complementary" herein can be used with respect to the base matching between the sense strand and the antisense strand of a nucleic acid e.g. dsiRNA, or between the antisense strand of a double stranded nucleic acid e.g. siRNA agent and a target sequence.

Within the present invention, the second strand of the nucleic acid according to the invention, in particular a dsiRNA for inhibiting SLC25A5, is at least partially complementary to the first strand of said nucleic acid. In certain embodiments, a first and second strand of a nucleic acid according to the invention are partially complementary if they form a duplex region having a length of at least 17 base pairs and comprising not more than 1, 2, 3, 4, or 5 mismatched base pairs.

In certain embodiments, a first and second strand of the nucleic acid according to the invention are partially complementary if they form a duplex region having a length of 19 base pairs and comprising not more than 1, 2, 3, 4, or 5 mismatched base pairs. In certain embodiments, a first and second strand of the nucleic acid according to the invention are partially complementary if they form a duplex region having a length of 21 base pairs comprising not more than 1, 2, 3, 4, or 5 mismatched base pairs.

Alternatively, a first and second strand of the nucleic acid according to the invention are partially complementary if they form a duplex region having a length of at least 17 base pairs, wherein at least 14, 15, 16 or 17 of said base pairs are complementary base pairs, in particular Watson-Crick base pairs.

In certain embodiments, a first and second strand of the nucleic acid according to the invention are partially complementary if they form a duplex region having a length of 19 base pairs, wherein at least 14, 15, 16, 17, 18 or all 19 base pairs are complementary base pairs, in particular Watson-Crick base pairs. In certain embodiments, a first and second strand of the nucleic acid according to the invention are partially complementary if they form a duplex region having a length of 21 base pairs, wherein at least 16, 17, 18, 19, 20 or all 21 base pairs are complementary base pairs, in particular Watson-Crick base pairs.

As used herein, a nucleic acid that is "substantially complementary" or "partially complementary" to at least part of a messenger RNA (mRNA) refers to a polynucleoside that is substantially or partially complementary to a contiguous portion of the mRNA of interest (e.g., an mRNA encoding a gene). In certain embodiments, the contiguous portion of the mRNA is a sequence as listed in Table 1, i.e., any one of SEQ ID NOs:1-276. For example, a polynucleoside is complementary to at least a part of an mRNA of a gene of interest if the sequence is substantially or partially complementary to a non-interrupted portion of an mRNA encoding that gene.

Accordingly, in some preferred embodiments, the antisense oligonucleosides as disclosed herein are fully complementary to the target gene sequence.

In other embodiments, the antisense oligonucleosides disclosed herein are substantially or partially complementary to a target RNA sequence and comprise a contiguous nucleoside sequence which is at least about 80% complementary over its entire length to the equivalent region of the target RNA sequence, such as at least about 85%, 86%, 87%, 88%, 89%, about 90%, 91 %, 92%, 93%, 94%, 95%, 96%, 97%, 98%, or 99% complementary or 100% complementary.

In certain embodiments, the first (antisense) strand of a nucleic acid according to the invention is partially or fully complementary to a contiguous portion of RNA transcribed from the *SLC25A5* gene. In certain embodiments, the first strand of the nucleic acid according to the invention is partially or fully complementary to a contiguous portion of at least 17 nucleosides of the *SLC25A5* mRNA. In certain embodiments, the first strand of the nucleic acid according to the invention is partially or fully complementary to a contiguous portion of 17, 18, 19, 20, 21, 22 or 23 nucleosides of the *SLC25A5* mRNA. In certain embodiments, the first strand of the nucleic acid according to the invention is partially or fully complementary to a contiguous portion of 17, 18, 19, 20, 21, 22 or 23 nucleosides of any one of the sequences as listed in Table 1, i.e., any one of SEQ ID NOs: 1-276.

In certain embodiments, the first (antisense) strand of the nucleic acid according to the invention is partially complementary to a contiguous portion of the *SLC25A5* mRNA if it comprises a contiguous nucleoside sequence of at least 17 nucleosides, wherein at least 14, 15, 16 or 17 nucleosides of said contiguous nucleoside sequence are complementary to a contiguous portion of the *SLC25A5* mRNA. In certain embodiments, the first strand of the nucleic acid according to the invention comprises a contiguous nucleoside sequence of at least 17 nucleosides, wherein at least 14, 15, 16 or 17 nucleosides of said contiguous nucleoside sequence are complementary to a contiguous portion of any one of the sequences listed in Table 1, i.e., any one of SEQ ID NOs: 1-276. In certain embodiments, the first strand of the nucleic acid according to the invention comprises a contiguous nucleoside sequence of 19 nucleosides, wherein at least 14, 15, 16, 17, 18 or all 19 nucleosides of said contiguous nucleoside sequence are complementary to a contiguous portion of any one of the sequences listed in Table 1, i.e., any one of SEQ ID NOs: 1-276. In certain embodiments, the first strand of the nucleic acid according to the invention comprises a contiguous nucleoside sequence of 23 nucleosides, wherein at least 18, 19, 20, 21, 22 or all 23 nucleosides of said contiguous nucleoside sequence are complementary to a contiguous portion of any one of the sequences listed in Table 1, i.e., any one of SEQ ID NOs: 1-276.

In some embodiments, a nucleic acid e.g. an siRNA of the invention includes a sense strand that is substantially or partially complementary to an antisense oligonucleoside which, in turn, is complementary to a target gene sequence and comprises a contiguous nucleoside sequence. The nucleoside sequence of the sense strand is typically at least about 80% complementary over its entire length to the equivalent region of the nucleoside sequence of the antisense strand, such as about 85%, 86%, 87%, 88%, 89%, 90%, 91 %, 92%, 93%, 94%, 95%, 96%, 97%, 98%, or 99% complementary, or 100% complementary.

In some embodiments, a nucleic acid e.g. an siRNA of the invention includes an antisense strand that is substantially or partially complementary to the target sequence and comprises a contiguous nucleoside sequence which is at least 80% complementary over its entire length to the target sequence such as about 85%, 86%, 87%, 88%, 89%, 90%, 91 %, 92%, 93%, 94%, 95%, 96%, 97%, 98%, or 99% complementary, or 100% complementary.

As used herein, a "subject" is an animal, such as a mammal, including a primate (such as a human, a non-human primate, e.g., a monkey, and a chimpanzee), or a non-primate or a bird that expresses the target gene, either endogenously or heterologously, when the target gene sequence has sufficient complementarity to the nucleic acid e.g. iRNA agent to promote target knockdown. In certain preferred embodiments, the subject is a human.

The terms "treating" or "treatment" refer to a beneficial or desired result including, but not limited to, alleviation or amelioration of one or more symptoms associated with gene expression. "Treatment" can also mean prolonging survival as compared to expected survival in the absence of treatment.

The terms "prevent" or "prevention" as used herein are defined as eliminating or reducing the likelihood of occurrence of one or more symptoms of a disease or disorder. For example, the inhibitor disclosed herein can be used to prevent the occurrence of a metabolic disease or disorder associated with non-alcoholic fatty liver disease (NAFLD), and/or obesity and/or a disease or disorder associated with adipogenesis.

"Therapeutically effective amount," as used herein, is intended to include the amount of a nucleic acid e.g. an iRNA that, when administered to a patient for treating a subject having disease, is sufficient to effect treatment of the disease (e.g., by diminishing, ameliorating or maintaining the existing disease or one or more symptoms of disease or its related comorbidities).

The phrase "pharmaceutically acceptable" is employed herein to refer to compounds, materials, compositions, or dosage forms which are suitable for use in contact with the tissues of human subjects and animal subjects without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition, or vehicle, such as a liquid or solid filler, diluent, excipient, manufacturing aid or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject being treated.

Where a value or range of values of a parameter are recited, it is intended that values and ranges intermediate to the recited values are also intended to be part of this invention.

The articles "a" and "an" are used herein to refer to one or to more than one (i.e. to at least one) of the grammatical object of the article.

The term "including" is used herein to mean, and is used interchangeably with, the phrase "including but not limited to".

The term "or" is used herein to mean, and is used interchangeably with, the term "and/or," unless context clearly indicates otherwise. For example, "sense strand or antisense strand" is understood as "sense strand or antisense strand or sense strand and antisense strand."

The term "about" is used herein to mean within the typical ranges of tolerances in the art. For example, "about" can be understood as about 2 standard deviations from the mean. In certain embodiments, about means +10%. In certain embodiments, about means +5%. When about is present before a series of numbers or a range, it is understood that "about" can modify each of the numbers in the series or range.

The term "at least" prior to a number or series of numbers is understood to include the number adjacent to the term "at least", and all subsequent numbers or integers that could logically be included, as clear from context. For example, the number of nucleotides in a nucleic acid molecule must be an integer. For example, "at least 18 nucleosides of a 21 nucleoside nucleic acid molecule" means that 18, 19, 20, or 21 nucleosides have the indicated property. When at least is present before a series of numbers or a range, it is understood that "at least" can modify each of the numbers in the series or range.

As used herein, "no more than" or "less than" is understood as the value adjacent to the phrase and logical lower values or integers, as logical from context, to zero. For example, a duplex with an overhang of "no more than 2 nucleosides" has a 2, 1, or 0 nucleoside overhang. When "no more than" is present before a series of numbers or a range, it is understood that "no more than" can modify each of the numbers in the series or range.

The terminal region of a strand is the last 5 nucleotides from the 5' or the 3' end.

A nucleobase sequence is the sequence of the bases of the nucleic acid in an oligomer.

Various embodiments of the invention can be combined as determined appropriate by one of skill in the art.

### TARGET

A target for inhibition disclosed herein may be, without limitation, an mRNA, polypeptide, protein, or gene.

These targets are a target the inhibition of which helps in the prevention and/or treatment of a metabolic disease or disorder associated with non-alcoholic fatty liver disease (NAFLD) and/or obesity and/or a disease or disorder associated with adipogenesis and/or in reducing adipogenesis.

The target for inhibition is the gene SLC25A5 or a gene product thereof, such as an mRNA transcribed from the SLC25A5 gene or the ANT2 protein, and inhibition may be effected by inhibition of expression or function of the SLC25A5/ANT2 gene or protein or both.

In a preferred embodiment, the target is an mRNA expressed from the SLC25A5 gene. Exemplary target sequences on the SLC25A5 mRNA are listed below in Table 1.

Following Table 1 provides oligonucleoside mRNA target sequences of SLC25A5, together with the corresponding positions in transcript ENST00000317881.9. It is to be understood that SEQ ID NO: 1 to 276 refer to human (Homo sapiens) mRNA sequences.

**Table 1**

| SEQ ID NO | Oligonucleotide mRNA target sequence 5' → 3' | Starting position on ENST00000317881.9 |
|---|---|---|
| SEQ ID NO: 1 | ACUAUUUACUGGUUGAAAAUGGG | 1072 |
| SEQ ID NO: 2 | UACAAAGGCAUUAUAGACUGCGU | 223 |
| SEQ ID NO: 3 | UAUGAUGAAAUCAAGAAGUACAC | 943 |
| SEQ ID NO: 4 | AUGAUGAUGGGACUCAAUUGUAU | 1146 |
| SEQ ID NO: 5 | AGGCAUUAUAGACUGCGUGGUCC | 228 |
| SEQ ID NO: 6 | CAGUCACUCCUGAUAAAUAACAA | 1178 |
| SEQ ID NO: 7 | CAAUACAAAGGCAUUAUAGACUG | 220 |
| SEQ ID NO: 8 | GAGCCGCCUACUUCGGUAUCUAU | 635 |
| SEQ ID NO: 9 | CGAGCCGCCUACUUCGGUAUCUA | 634 |
| SEQ ID NO: 10 | AUAGACUGCGUGGUCCGUAUUCC | 235 |
| SEQ ID NO: 11 | AACACUCACAUCGUCAUCAGCUG | 691 |
| SEQ ID NO: 12 | UAACUUCGCCUUCAAAGAUAAAU | 333 |
| SEQ ID NO: 13 | GCCUACUUCGGUAUCUAUGACAC | 640 |
| SEQ ID NO: 14 | UAUUUACUGGUUGAAAAUGGGAA | 1074 |
| SEQ ID NO: 15 | UUUGCCCGUACCCGUCUAGCAGC | 478 |
| SEQ ID NO: 16 | GCAGUCAGGGCGCAAAGGAACUG | 792 |
| SEQ ID NO: 17 | AUUUACUGGUUGAAAAUGGGAAG | 1075 |
| SEQ ID NO: 18 | CCGAGCCGCCUACUUCGGUAUCU | 633 |
| SEQ ID NO: 19 | GGCCUCGGUGACUGCCUGGUUAA | 538 |
| SEQ ID NO: 20 | CACAUCGUCAUCAGCUGGAUGAU | 697 |
| SEQ ID NO: 21 | UAUUUCAGUCACUCCUGAUAAAU | 1173 |
| SEQ ID NO: 22 | GCUGGAUGAUCGCACAGACUGUC | 710 |
| SEQ ID NO: 23 | AAGCAAUACAAAGGCAUUAUAGA | 217 |
| SEQ ID NO: 24 | AGGCCUCGGUGACUGCCUGGUUA | 537 |
| SEQ ID NO: 25 | GGCUUUAACGUGUCUGUGCAGGG | 598 |
| SEQ ID NO: 26 | ACAAAGGCAUUAUAGACUGCGUG | 224 |
| SEQ ID NO: 27 | CUGUGAACAGGCAUGUUGUAUUA | 993 |
| SEQ ID NO: 28 | UCAUCUGACCAGUUUUCUCUUAA | 1107 |
| SEQ ID NO: 29 | CAAGGCUUUAACGUGUCUGUGCA | 595 |
| SEQ ID NO: 30 | AUGCAGUCAGGGCGCAAAGGAAC | 790 |
| SEQ ID NO: 31 | UGGAUGAUCGCACAGACUGUCAC | 712 |
| SEQ ID NO: 32 | AAAGGCAUUAUAGACUGCGUGGU | 226 |
| SEQ ID NO: 33 | GAUGAUCGCACAGACUGUCACUG | 714 |
| SEQ ID NO: 34 | GACUGCGUGGUCCGUAUUCCCAA | 238 |
| SEQ ID NO: 35 | AGCAAUACAAAGGCAUUAUAGAC | 218 |
| SEQ ID NO: 36 | GUCAUCAGCUGGAUGAUCGCACA | 703 |
| SEQ ID NO: 37 | GCAUUAUAGACUGCGUGGUCCGU | 230 |
| SEQ ID NO: 38 | AUUUUGCCCGUACCCGUCUAGCA | 476 |
| SEQ ID NO: 39 | UCACAUCGUCAUCAGCUGGAUGA | 696 |
| SEQ ID NO: 40 | CCUCUUGAUUUUGCCCGUACCCG | 469 |
| SEQ ID NO: 41 | UUAUAGACUGCGUGGUCCGUAUU | 233 |
| SEQ ID NO: 42 | GCCGCCUACUUCGGUAUCUAUGA | 637 |
| SEQ ID NO: 43 | UAGACUGCGUGGUCCGUAUUCCC | 236 |
| SEQ ID NO: 44 | CGUCAUCAGCUGGAUGAUCGCAC | 702 |
| SEQ ID NO: 45 | CAUUAUAGACUGCGUGGUCCGUA | 231 |
| SEQ ID NO: 46 | CGCCUACUUCGGUAUCUAUGACA | 639 |
| SEQ ID NO: 47 | UUUUAUUUCAGUCACUCCUGAUA | 1170 |
| SEQ ID NO: 48 | UCGUCAUCAGCUGGAUGAUCGCA | 701 |
| SEQ ID NO: 49 | UGAUUUUGCCCGUACCCGUCUAG | 474 |
| SEQ ID NO: 50 | UGCAAAGGGAAUGCUUCCGGAUC | 663 |
| SEQ ID NO: 51 | CCAAGGCUUUAACGUGUCUGUGC | 594 |
| SEQ ID NO: 52 | CAAAGGCAUUAUAGACUGCGUGG | 225 |
| SEQ ID NO: 53 | UCUUGAUUUUGCCCGUACCCGUC | 471 |
| SEQ ID NO: 54 | ACCGAGCCGCCUACUUCGGUAUC | 632 |
| SEQ ID NO: 55 | UUAUCAUCUACCGAGCCGCCUAC | 623 |
| SEQ ID NO: 56 | CUUGAUUUUGCCCGUACCCGUCU | 472 |
| SEQ ID NO: 57 | AUUAUCAUCUACCGAGCCGCCUA | 622 |
| SEQ ID NO: 58 | AUCAUCUACCGAGCCGCCUACUU | 625 |
| SEQ ID NO: 59 | GGCUCUUAACUUCGCCUUCAAAG | 327 |
| SEQ ID NO: 60 | CCCCACCCAGGCUCUUAACUUCG | 318 |
| SEQ ID NO: 61 | GUCCGUAUUCCCAAGGAGCAGGG | 247 |
| SEQ ID NO: 62 | AGGAGCAGGGAGUUCUGUCCUUC | 260 |
| SEQ ID NO: 63 | AUUCCCAAGGAGCAGGGAGUUCU | 253 |
| SEQ ID NO: 64 | GGUCCGUAUUCCCAAGGAGCAGG | 246 |
| SEQ ID NO: 65 | GAGCAGGGAGUUCUGUCCUUCUG | 262 |
| SEQ ID NO: 66 | UGGUCCGUAUUCCCAAGGAGCAG | 245 |
| SEQ ID NO: 67 | GGAGCAGGGAGUUCUGUCCUUCU | 261 |
| SEQ ID NO: 68 | AAGGAGCAGGGAGUUCUGUCCUU | 259 |
| SEQ ID NO: 69 | UAUUCCCAAGGAGCAGGGAGUUC | 252 |
| SEQ ID NO: 70 | CAAGGAGCAGGGAGUUCUGUCCU | 258 |
| SEQ ID NO: 71 | UCCCCACCCAGGCUCUUAACUUC | 317 |
| SEQ ID NO: 72 | GCUCUUAACUUCGCCUUCAAAGA | 328 |
| SEQ ID NO: 73 | AUUUCAGUCACUCCUGAUAAAUA | 1174 |
| SEQ ID NO: 74 | UUCAGUCACUCCUGAUAAAUAAC | 1176 |
| SEQ ID NO: 75 | UUUCAGUCACUCCUGAUAAAUAA | 1175 |
| SEQ ID NO: 76 | GAUGAUGGGACUCAAUUGUAUUU | 1148 |
| SEQ ID NO: 77 | AUGAUGGGACUCAAUUGUAUUUU | 1149 |
| SEQ ID NO: 78 | GUCACUCCUGAUAAAUAACAAAU | 1180 |
| SEQ ID NO: 79 | AUAUUCAUCUGACCAGUUUUCUC | 1103 |
| SEQ ID NO: 80 | UGAACAGGCAUGUUGUAUUAUAU | 996 |
| SEQ ID NO: 81 | AAAGGGAAUGCUUCCGGAUCCCA | 666 |
| SEQ ID NO: 82 | AAGGGAAUGCUUCCGGAUCCCAA | 667 |
| SEQ ID NO: 83 | UCCGAGGCCUCGGUGACUGCCUG | 533 |
| SEQ ID NO: 84 | GAACAGGCAUGUUGUAUUAUAUA | 997 |
| SEQ ID NO: 85 | GCAAUACAAAGGCAUUAUAGACU | 219 |
| SEQ ID NO: 86 | UUAUUUCAGUCACUCCUGAUAAA | 1172 |
| SEQ ID NO: 87 | UGAUGAUGGGACUCAAUUGUAUU | 1147 |
| SEQ ID NO: 88 | AUCUGACCAGUUUUCUCUUAAAG | 1109 |
| SEQ ID NO: 89 | UCUGACCAGUUUUCUCUUAAAGC | 1110 |
| SEQ ID NO: 90 | CAGAUAAGCAAUACAAAGGCAUU | 212 |
| SEQ ID NO: 91 | AGAUAAGCAAUACAAAGGCAUUA | 213 |
| SEQ ID NO: 92 | CUUUAACGUGUCUGUGCAGGGUA | 600 |
| SEQ ID NO: 93 | UUUAACGUGUCUGUGCAGGGUAU | 601 |
| SEQ ID NO: 94 | ACUCACAUCGUCAUCAGCUGGAU | 694 |
| SEQ ID NO: 95 | GAUGAUGAUGGGACUCAAUUGUA | 1145 |
| SEQ ID NO: 96 | AGACUGCGUGGUCCGUAUUCCCA | 237 |
| SEQ ID NO: 97 | GUGAACAGGCAUGUUGUAUUAUA | 995 |
| SEQ ID NO: 98 | UUUAUUUCAGUCACUCCUGAUAA | 1171 |
| SEQ ID NO: 99 | GAUAAGCAAUACAAAGGCAUUAU | 214 |
| SEQ ID NO: 100 | ACACUCACAUCGUCAUCAGCUGG | 692 |
| SEQ ID NO: 101 | AGCCGCCUACUUCGGUAUCUAUG | 636 |
| SEQ ID NO: 102 | CUCACAUCGUCAUCAGCUGGAUG | 695 |
| SEQ ID NO: 103 | CCGCCUACUUCGGUAUCUAUGAC | 638 |
| SEQ ID NO: 104 | GCUUUAACGUGUCUGUGCAGGGU | 599 |
| SEQ ID NO: 105 | GUUCGCCGCCGCAUGAUGAUGCA | 772 |
| SEQ ID NO: 106 | GGCAUUAUAGACUGCGUGGUCCG | 229 |
| SEQ ID NO: 107 | ACUGCGUGGUCCGUAUUCCCAAG | 239 |
| SEQ ID NO: 108 | UCAUCUACCGAGCCGCCUACUUC | 626 |
| SEQ ID NO: 109 | UUUUGCCCGUACCCGUCUAGCAG | 477 |
| SEQ ID NO: 110 | CACUCACAUCGUCAUCAGCUGGA | 693 |
| SEQ ID NO: 111 | AUUAUAGACUGCGUGGUCCGUAU | 232 |
| SEQ ID NO: 112 | GAGGCCUCGGUGACUGCCUGGUU | 536 |
| SEQ ID NO: 113 | CUGGAUGAUCGCACAGACUGUCA | 711 |
| SEQ ID NO: 114 | UUGAUUUUGCCCGUACCCGUCUA | 473 |
| SEQ ID NO: 115 | AUGAUCGCACAGACUGUCACUGC | 715 |
| SEQ ID NO: 116 | GGAUGAUCGCACAGACUGUCACU | 713 |
| SEQ ID NO: 117 | GAUUUUGCCCGUACCCGUCUAGC | 475 |
| SEQ ID NO: 118 | UAUCAUCUACCGAGCCGCCUACU | 624 |
| SEQ ID NO: 119 | UAUAGACUGCGUGGUCCGUAUUC | 234 |
| SEQ ID NO: 120 | CACUGCAGAUAAGCAAUACAAAG | 207 |
| SEQ ID NO: 121 | UCACUGCAGAUAAGCAAUACAAA | 206 |
| SEQ ID NO: 122 | AUCACUGCAGAUAAGCAAUACAA | 205 |
| SEQ ID NO: 123 | CAGCAAGCAGAUCACUGCAGAUA | 195 |
| SEQ ID NO: 124 | AGAUCACUGCAGAUAAGCAAUAC | 203 |
| SEQ ID NO: 125 | GAUCACUGCAGAUAAGCAAUACA | 204 |
| SEQ ID NO: 126 | AAGCAGAUCACUGCAGAUAAGCA | 199 |
| SEQ ID NO: 127 | AGCAGAUCACUGCAGAUAAGCAA | 200 |
| SEQ ID NO: 128 | CCAGCAAGCAGAUCACUGCAGAU | 194 |
| SEQ ID NO: 129 | CAGAUCACUGCAGAUAAGCAAUA | 202 |
| SEQ ID NO: 130 | UGAUAAAUAACAAAUUUGGAGAA | 1188 |
| SEQ ID NO: 131 | ACUCCUGAUAAAUAACAAAUUUG | 1183 |
| SEQ ID NO: 132 | CUGAAAGGGAAUUCCGAGGCCUC | 521 |
| SEQ ID NO: 133 | AUGUUGUAUUAUAUAACAUAUCU | 1005 |
| SEQ ID NO: 134 | AAUUCCGAGGCCUCGGUGACUGC | 530 |
| SEQ ID NO: 135 | UGAAAGGGAAUUCCGAGGCCUCG | 522 |
| SEQ ID NO: 136 | GUUGUAUUAUAUAACAUAUCUUG | 1007 |
| SEQ ID NO: 137 | GGAAUUCCGAGGCCUCGGUGACU | 528 |
| SEQ ID NO: 138 | AAGGGAAUUCCGAGGCCUCGGUG | 525 |
| SEQ ID NO: 139 | GGAGCUGAAAGGGAAUUCCGAGG | 517 |
| SEQ ID NO: 140 | CUGGAGCUGAAAGGGAAUUCCGA | 515 |
| SEQ ID NO: 141 | AGCUGAAAGGGAAUUCCGAGGCC | 519 |
| SEQ ID NO: 142 | UGGAGCUGAAAGGGAAUUCCGAG | 516 |
| SEQ ID NO: 143 | GAGCUGAAAGGGAAUUCCGAGGC | 518 |
| SEQ ID NO: 144 | AGGCAUGUUGUAUUAUAUAACAU | 1001 |
| SEQ ID NO: 145 | UGUUGUAUUAUAUAACAUAUCUU | 1006 |
| SEQ ID NO: 146 | UUGUAUUAUAUAACAUAUCUUGA | 1008 |
| SEQ ID NO: 147 | CAGGCAUGUUGUAUUAUAUAACA | 1000 |
| SEQ ID NO: 148 | GAAAGGGAAUUCCGAGGCCUCGG | 523 |
| SEQ ID NO: 149 | GGGAAUUCCGAGGCCUCGGUGAC | 527 |
| SEQ ID NO: 150 | AAAGGGAAUUCCGAGGCCUCGGU | 524 |
| SEQ ID NO: 151 | AGGGAAUUCCGAGGCCUCGGUGA | 526 |
| SEQ ID NO: 152 | GUGCUUGUCUUGUAUGAUGAAAU | 931 |
| SEQ ID NO: 153 | CUUCGCCUUCAAAGAUAAAUACA | 336 |
| SEQ ID NO: 154 | CUGCCUGGUUAAGAUCUACAAAU | 549 |
| SEQ ID NO: 155 | UAUCUAUGACACUGCAAAGGGAA | 651 |
| SEQ ID NO: 156 | CUUUUGUGCUUGUCUUGUAUGAU | 926 |
| SEQ ID NO: 157 | GGUGACUGCCUGGUUAAGAUCUA | 544 |
| SEQ ID NO: 158 | AUGACACUGCAAAGGGAAUGCUU | 656 |
| SEQ ID NO: 159 | UUCGGUAUCUAUGACACUGCAAA | 646 |
| SEQ ID NO: 160 | UAUUCAUCUGACCAGUUUUCUCU | 1104 |
| SEQ ID NO: 161 | ACUUCGGUAUCUAUGACACUGCA | 644 |
| SEQ ID NO: 162 | CUGGUUAAGAUCUACAAAUCUGA | 553 |
| SEQ ID NO: 163 | GUAUCUAUGACACUGCAAAGGGA | 650 |
| SEQ ID NO: 164 | CAAGGGUGCAUGGUCCAAUGUUC | 885 |
| SEQ ID NO: 165 | AGAUCUACAAAUCUGAUGGGAUU | 560 |
| SEQ ID NO: 166 | UACUUCGGUAUCUAUGACACUGC | 643 |
| SEQ ID NO: 167 | CAUGGGUGGUGCUUUUGUGCUUG | 915 |
| SEQ ID NO: 168 | GGUAUUAUCAUCUACCGAGCCGC | 619 |
| SEQ ID NO: 169 | GCGUGGUCCGUAUUCCCAAGGAG | 242 |
| SEQ ID NO: 170 | AUCUAUGACACUGCAAAGGGAAU | 652 |
| SEQ ID NO: 171 | UGCUUUUGUGCUUGUCUUGUAUG | 924 |
| SEQ ID NO: 172 | UGUACCAAGGCUUUAACGUGUCU | 590 |
| SEQ ID NO: 173 | GGGUGCAUGGUCCAAUGUUCUCA | 888 |
| SEQ ID NO: 174 | CGCCUUCAAAGAUAAAUACAAGC | 339 |
| SEQ ID NO: 175 | CAGGGUAUUAUCAUCUACCGAGC | 616 |
| SEQ ID NO: 176 | CCUACUUCGGUAUCUAUGACACU | 641 |
| SEQ ID NO: 177 | UGGUUAAGAUCUACAAAUCUGAU | 554 |
| SEQ ID NO: 178 | UGUGCAGGGUAUUAUCAUCUACC | 612 |
| SEQ ID NO: 179 | AAUCUGAUGGGAUUAAGGGCCUG | 569 |
| SEQ ID NO: 180 | GUCUGUGCAGGGUAUUAUCAUCU | 609 |
| SEQ ID NO: 181 | UCUGAUGGGAUUAAGGGCCUGUA | 571 |
| SEQ ID NO: 182 | UACCAAGGCUUUAACGUGUCUGU | 592 |
| SEQ ID NO: 183 | AAUAUUCAUCUGACCAGUUUUCU | 1102 |
| SEQ ID NO: 184 | UGACUGCCUGGUUAAGAUCUACA | 546 |
| SEQ ID NO: 185 | GGGUUGACUUCCUAUCCAUUUGA | 745 |
| SEQ ID NO: 186 | UCUAUGACACUGCAAAGGGAAUG | 653 |
| SEQ ID NO: 187 | GGUUAAGAUCUACAAAUCUGAUG | 555 |
| SEQ ID NO: 188 | GCAGGGUAUUAUCAUCUACCGAG | 615 |
| SEQ ID NO: 189 | ACUGCCUGGUUAAGAUCUACAAA | 548 |
| SEQ ID NO: 190 | AUCUGAUGGGAUUAAGGGCCUGU | 570 |
| SEQ ID NO: 191 | UCGGUAUCUAUGACACUGCAAAG | 647 |
| SEQ ID NO: 192 | GUACCCGUCUAGCAGCUGAUGUG | 485 |
| SEQ ID NO: 193 | GGUAUCUAUGACACUGCAAAGGG | 649 |
| SEQ ID NO: 194 | AGCUGAUGUGGGUAAAGCUGGAG | 498 |
| SEQ ID NO: 195 | CGUCUAGCAGCUGAUGUGGGUAA | 490 |
| SEQ ID NO: 196 | AAGGGCCUGUACCAAGGCUUUAA | 583 |
| SEQ ID NO: 197 | UGCCCGUACCCGUCUAGCAGCUG | 480 |
| SEQ ID NO: 198 | AGCAGCUGAUGUGGGUAAAGCUG | 495 |
| SEQ ID NO: 199 | GCCCGUACCCGUCUAGCAGCUGA | 481 |
| SEQ ID NO: 200 | CAGCUGAUGUGGGUAAAGCUGGA | 497 |
| SEQ ID NO: 201 | AGGGUAUUAUCAUCUACCGAGCC | 617 |
| SEQ ID NO: 202 | UAUUAUCAUCUACCGAGCCGCCU | 621 |
| SEQ ID NO: 203 | CCGUACCCGUCUAGCAGCUGAUG | 483 |
| SEQ ID NO: 204 | CUGAUGGGAUUAAGGGCCUGUAC | 572 |
| SEQ ID NO: 205 | GUGUCUGUGCAGGGUAUUAUCAU | 607 |
| SEQ ID NO: 206 | CUGCGUGGUCCGUAUUCCCAAGG | 240 |
| SEQ ID NO: 207 | UGCGUGGUCCGUAUUCCCAAGGA | 241 |
| SEQ ID NO: 208 | CCUGGUUAAGAUCUACAAAUCUG | 552 |
| SEQ ID NO: 209 | GUAUGAUGAAAUCAAGAAGUACA | 942 |
| SEQ ID NO: 210 | AAGAUCUACAAAUCUGAUGGGAU | 559 |
| SEQ ID NO: 211 | CUGUGCAGGGUAUUAUCAUCUAC | 611 |
| SEQ ID NO: 212 | CUGUACCAAGGCUUUAACGUGUC | 589 |
| SEQ ID NO: 213 | UAGCAGCUGAUGUGGGUAAAGCU | 494 |
| SEQ ID NO: 214 | GCAUGGGUGGUGCUUUUGUGCUU | 914 |
| SEQ ID NO: 215 | CUCGGUGACUGCCUGGUUAAGAU | 541 |
| SEQ ID NO: 216 | GUCUAGCAGCUGAUGUGGGUAAA | 491 |
| SEQ ID NO: 217 | UCUACAAAUCUGAUGGGAUUAAG | 563 |
| SEQ ID NO: 218 | CUACUUCGGUAUCUAUGACACUG | 642 |
| SEQ ID NO: 219 | GCAGCUGAUGUGGGUAAAGCUGG | 496 |
| SEQ ID NO: 220 | CCCGUACCCGUCUAGCAGCUGAU | 482 |
| SEQ ID NO: 221 | UGUCUGUGCAGGGUAUUAUCAUC | 608 |
| SEQ ID NO: 222 | UGUGCUUGUCUUGUAUGAUGAAA | 930 |
| SEQ ID NO: 223 | GUACCAAGGCUUUAACGUGUCUG | 591 |
| SEQ ID NO: 224 | AAGGGUGCAUGGUCCAAUGUUCU | 886 |
| SEQ ID NO: 225 | GUGACUGCCUGGUUAAGAUCUAC | 545 |
| SEQ ID NO: 226 | CUGAUGUGGGUAAAGCUGGAGCU | 500 |
| SEQ ID NO: 227 | CGUGGUCCGUAUUCCCAAGGAGC | 243 |
| SEQ ID NO: 228 | UGACACUGCAAAGGGAAUGCUUC | 657 |
| SEQ ID NO: 229 | GGGAUUAAGGGCCUGUACCAAGG | 577 |
| SEQ ID NO: 230 | UCUGUGCAGGGUAUUAUCAUCUA | 610 |
| SEQ ID NO: 231 | UUAACGUGUCUGUGCAGGGUAUU | 602 |
| SEQ ID NO: 232 | AGGGCCUGUACCAAGGCUUUAAC | 584 |
| SEQ ID NO: 233 | UAUGACACUGCAAAGGGAAUGCU | 655 |
| SEQ ID NO: 234 | CCUCGGUGACUGCCUGGUUAAGA | 540 |
| SEQ ID NO: 235 | GUGCUUUUGUGCUUGUCUUGUAU | 923 |
| SEQ ID NO: 236 | UGGGUGGUGCUUUUGUGCUUGUC | 917 |
| SEQ ID NO: 237 | CCCGUCUAGCAGCUGAUGUGGGU | 488 |
| SEQ ID NO: 238 | UAAGGGCCUGUACCAAGGCUUUA | 582 |
| SEQ ID NO: 239 | UUGUGCUUGUCUUGUAUGAUGAA | 929 |
| SEQ ID NO: 240 | ACAAAUCUGAUGGGAUUAAGGGC | 566 |
| SEQ ID NO: 241 | AUCUACAAAUCUGAUGGGAUUAA | 562 |
| SEQ ID NO: 242 | UGCAGGGUAUUAUCAUCUACCGA | 614 |
| SEQ ID NO: 243 | GCCUGUACCAAGGCUUUAACGUG | 587 |
| SEQ ID NO: 244 | UAAGAUCUACAAAUCUGAUGGGA | 558 |
| SEQ ID NO: 245 | GCUUUUGUGCUUGUCUUGUAUGA | 925 |
| SEQ ID NO: 246 | CUACAAAUCUGAUGGGAUUAAGG | 564 |
| SEQ ID NO: 247 | UUCAAGGGUGCAUGGUCCAAUGU | 883 |
| SEQ ID NO: 248 | UGGGAUUAAGGGCCUGUACCAAG | 576 |
| SEQ ID NO: 249 | CUUCGGUAUCUAUGACACUGCAA | 645 |
| SEQ ID NO: 250 | ACCAAGGCUUUAACGUGUCUGUG | 593 |
| SEQ ID NO: 251 | GGCAUGGGUGGUGCUUUUGUGCU | 913 |
| SEQ ID NO: 252 | GUUAAGAUCUACAAAUCUGAUGG | 556 |
| SEQ ID NO: 253 | UACCCGUCUAGCAGCUGAUGUGG | 486 |
| SEQ ID NO: 254 | CUAUGACACUGCAAAGGGAAUGC | 654 |
| SEQ ID NO: 255 | UCUAGCAGCUGAUGUGGGUAAAG | 492 |
| SEQ ID NO: 256 | CUGCAAAGGGAAUGCUUCCGGAU | 662 |
| SEQ ID NO: 257 | UUGCCCGUACCCGUCUAGCAGCU | 479 |
| SEQ ID NO: 258 | GGGUAUUAUCAUCUACCGAGCCG | 618 |
| SEQ ID NO: 259 | CCCUCUUGAUUUUGCCCGUACCC | 468 |
| SEQ ID NO: 260 | CGUACCCGUCUAGCAGCUGAUGU | 484 |
| SEQ ID NO: 261 | UACAAAUCUGAUGGGAUUAAGGG | 565 |
| SEQ ID NO: 262 | GUGUACCCUCUUGAUUUUGCCCG | 463 |
| SEQ ID NO: 263 | GGCCUGUACCAAGGCUUUAACGU | 586 |
| SEQ ID NO: 264 | GAUGGGAUUAAGGGCCUGUACCA | 574 |
| SEQ ID NO: 265 | GUAUUAUCAUCUACCGAGCCGCC | 620 |
| SEQ ID NO: 266 | GGGCCUGUACCAAGGCUUUAACG | 585 |
| SEQ ID NO: 267 | UGAUGGGAUUAAGGGCCUGUACC | 573 |
| SEQ ID NO: 268 | UAACGUGUCUGUGCAGGGUAUUA | 603 |
| SEQ ID NO: 269 | AAGGCAUUAUAGACUGCGUGGUC | 227 |
| SEQ ID NO: 270 | ACUUCCCCACCCAGGCUCUUAAC | 314 |
| SEQ ID NO: 271 | CAAUGUCAUCAGAUACUUCCCCA | 300 |
| SEQ ID NO: 272 | AUGUCAUCAGAUACUUCCCCACC | 302 |
| SEQ ID NO: 273 | AAUGUCAUCAGAUACUUCCCCAC | 301 |
| SEQ ID NO: 274 | CCAAUGUCAUCAGAUACUUCCCC | 299 |
| SEQ ID NO: 275 | UACUUCCCCACCCAGGCUCUUAA | 313 |
| SEQ ID NO: 276 | AUCUACCGAGCCGCCUACUUCGG | 628 |

It is to be understood that SEQ ID NOs: 1 to 276 relate to human (Homo sapiens) mRNA sequences.

### DISEASE/CONDITIONS

The present invention further provides methods of treatment of a subject in need thereof. The treatment methods of the invention include administering a nucleic acid such as an siRNA of the invention to a subject, e.g., a subject that would benefit from a reduction or inhibition of the expression of SLC25A5 gene, in a therapeutically effective amount e.g. a nucleic acid such as an siRNA targeting SLC25A5 or a pharmaceutical composition comprising the nucleic acid targeting SLC25A5. The disease to be treated is related to a metabolic disease or disorder, such as a metabolic disease or disorder associated with non-alcoholic fatty liver disease (NAFLD) and/or obesity and/or a disease or disorder associated with adipogenesis and/or adipogenesis.

The disease to be treated is related to a metabolic disease or disorder, such as a metabolic disease or disorder associated with non-alcoholic fatty liver disease (NAFLD) and/or obesity and/or a disease or disorder associated with adipogenesis and/or adipogenesis.

The inhibitor according to the invention may be used in the prevention and/or treatment of a metabolic disease or disorder. As used herein, the term "metabolic disease" refers to a disease or condition affecting a metabolic process in a subject and is often caused by disruption of normal metabolism.

The patient to be treated may be a patient that already has a metabolic disease or disorder or that is at risk of developing a metabolic disease or disorder. That is, in certain embodiments, the inhibitor of the present invention may be used in the treatment and/or management of an existing metabolic disease or disorder. Treatment and/or management of an existing metabolic disease or disorder with the inhibitor of the present invention may prevent worsening of the metabolic disease or disorder and/or reverse the metabolic disease or disorder. In some instances, treatment of an existing metabolic disease or disorder with the inhibitor of the present invention may even cure the metabolic disease or disorder. In certain embodiments, the inhibitor of the present invention may be used to prevent manifestation of a metabolic disease or disorder in a patient that is at risk of developing a metabolic disease or disorder.

The skilled person is capable of diagnosing whether a patient has a metabolic disease or disorder or is at risk of developing a metabolic disease or disorder. For example, a metabolic disease or disorder may be diagnosed based weight gain and/or one or more blood markers, including, without limitation, blood glucose levels, blood insulin levels, blood free fatty acid levels, blood HbA1c levels, blood fibrinogen levels, blood cholesterol levels and blood triglyceride levels. The skilled person is aware of threshold values of one or more blood markers that indicate the presence of a metabolic disease or disorder or the risk of developing a metabolic disease or disorder.

In certain embodiments, the metabolic disease is fatty liver disease, in particular non-alcoholic fatty liver disease (NAFLD). As used herein "fatty-liver disease" refers to a disease wherein fat is excessively accumulated in the liver and can cause severe diseases such as chronic hepatitis and hepatic cirrhosis. In patients with fatty liver disease, lipids, particularly neutral fat, accumulate in hepatocytes to the extent that the amount exceeds the physiologically permissible range. From a biochemical point of view, a standard for judgment of fatty liver is that the weight of neutral fat is about 10% (100 mg/g wet weight) or more of the wet weight of hepatic tissue. Fatty liver disease is generally detected by observation of elevated serum levels of liver-specific enzymes such as the transaminases ALT and AST, which serve as indices of hepatocyte injury, as well as by presentation of symptoms, which include fatigue and pain in the region of the liver, though definitive diagnosis often requires a biopsy and may be supported by imaging such as ultrasound and/or MRI. The term "NAFLD" or "non-alcoholic fatty liver disease", as used herein, relates to a condition occurring when fat is deposited in the liver (steatosis) not due to excessive alcohol use. It is related to insulin resistance and the metabolic syndrome.

In a preferred embodiment, the fatty liver disease is Non-Alcoholic SteatoHepatitis (NASH). NASH, as used herein, refers to a liver disease characterized by an accumulation of fat (lipid droplets), along with inflammation and degeneration of hepatocytes. Once initiated, the disease is accompanied with a high risk of cirrhosis, a state wherein liver functions are altered that can progress to liver insufficiency. Thereafter, NASH often progresses to liver cancer.

In certain embodiments, the invention relates to the inhibitor according to the invention for use in reducing one or more of steatosis, lobular inflammation, and/or hepatocytic ballooning.

In certain embodiments, the invention relates to the inhibitor according to the invention for use in treating and/or preventing a disease or disorder associated with increased steatosis, increased lobular inflammation, and/or increased hepatocytic ballooning.

In certain embodiments, the invention relates to the inhibitor according to the invention for use in the treatment and/or prevention of fatty liver disease, such as NAFLD or NASH, wherein the inhibitor according to the invention results in one or more of: reduced steatosis, reduced lobular inflammation, and/or reduced hepatocytic ballooning.

In certain embodiments, the invention relates to the inhibitor according to the invention for use in the treatment and/or prevention of liver steatosis.

In certain embodiments, the invention relates to the inhibitor according to the invention for use in the treatment and/or prevention of lobular inflammation of the liver.

In certain embodiments, the invention relates to the inhibitor according to the invention for use in the treatment and/or prevention of hepatocytic ballooning of the liver.

In certain embodiments, the invention relates to the inhibitor according to the invention for use in the treatment or prevention of liver fibrosis in a patient.

In certain embodiments, the invention relates to the inhibitor according to the invention for use in treating and/or preventing a disease or disorder associated with liver fibrosis.

In certain embodiments, the invention relates to the inhibitor according to the invention for use in the treatment and/or prevention of fatty liver disease, such as NAFLD or NASH, wherein the inhibitor according to the invention reduces fibrosis stage.

The person skilled in the art is aware of method to determine the level of liver steatosis, lobular inflammation, hepatocytic ballooning or fibrosis stage in a patient.

In certain embodiments, the invention relates to the inhibitor according to the invention for use in reducing the levels of one or more of Alanine transaminase (ALT), Aspartate transaminase (AST), Tissue inhibitor of metalloproteinases-1 (TIMP-1) and/or Type III Procollagen Peptide (PIIINP) in a patient.

In certain embodiments, the invention relates to the inhibitor according to the invention for use in treating and/or preventing a disease associated with elevated levels of one or more of Alanine transaminase (ALT), Aspartate transaminase (AST), Tissue inhibitor of metalloproteinases-1 (TIMP-1) and/or Type III Procollagen Peptide (PIIINP) in a patient.

In certain embodiments, the invention relates to the inhibitor according to the invention for use in treating and/or preventing a metabolic disease or disorder associated with elevated levels of one or more of Alanine transaminase (ALT), Aspartate transaminase (AST), Tissue inhibitor of metalloproteinases-1 (TIMP-1) and/or Type III Procollagen Peptide (PIIINP) in a patient.

In certain embodiments, the invention relates to the inhibitor according to the invention for use in treating and/or preventing a metabolic disease or disorder, wherein the inhibitor according to the invention reduces the levels of one or more of Alanine transaminase (ALT), Aspartate transaminase (AST), Tissue inhibitor of metalloproteinases-1 (TIMP-1) and/or Type III Procollagen Peptide (PIIINP) in a patient.

In certain embodiments, the invention relates to the inhibitor according to the invention for use in treating and/or preventing a fatty liver disease, such as NAFLD or NASH, wherein the inhibitor according to the invention reduces the levels of one or more of Alanine transaminase (ALT), Aspartate transaminase (AST), Tissue inhibitor of metalloproteinases-1 (TIMP-1) and/or Type III Procollagen Peptide (PIIINP) in a patient.

The skilled person is aware of methods and commercial kits to determine the levels of ALT, AST, TIMP-1 and PIIINP in a patient.

In certain In certain embodiments, the invention relates to the inhibitor according to the invention for use in reducing the ratio between liver weight and body weight.

In certain embodiments, the invention relates to the inhibitor according to the invention for use in treating and/or preventing a disease or disorder associated with increased liver weight.

In certain embodiments, the invention relates to the inhibitor according to the invention for use in treating and/or preventing a metabolic disease or disorder associated with increased liver weight.

In certain embodiments, the invention relates to the inhibitor according to the invention for use in treating and/or preventing a metabolic disease or disorder, wherein the inhibitor according to the invention reduces the ratio between liver weight and body weight in a patient.

In certain embodiments, the invention relates to the inhibitor according to the invention for use in treating and/or preventing a fatty liver disease, such as NAFLD or NASH, wherein the inhibitor according to the invention reduces the ratio between liver weight and body weight in a patient.

In certain embodiments, the metabolic disease is obesity. The term "obesity" as used herein refers to a condition in which the natural energy reserve, stored in the fatty tissue of animals, in particular humans and other mammals, is increased to a point where it is associated with certain health conditions or increased mortality. The term "obese" as used herein is defined for an adult human as having a body mass index (BMI) greater than 30. Obesity is commonly associated with excessive body weight gain, in particular diet-induced body weight gain. "(Diet-induced) body weight gain" is defined herein as body weight gain resulting from an excessive dietary intake, including an excessive dietary intake of fat, in particular saturated fat, and optionally an excessive dietary intake of simple sugars, including sucrose and fructose. For a given subject, an excessive dietary intake, in particular of fat and optionally of simple sugars, refers to the consumption of an amount of diet, in particular of fat and optionally of simple sugars, higher than the amount necessary to meet the physiological needs and maintain the energy balance of said subject. The effect of a treatment on reduction of - or prevention - of diet-induced body weight gain in a subject can be assessed by comparing body weight gain observed in a subject receiving the treatment with those observed in the same subject without treatment receiving the same diet and having the same level of physical activity.

In certain embodiments, a patient is at risk of developing obesity if the patient has a BMI greater than 25. In certain embodiments, a patient is obese if the patient has a BMI greater than 30.

The term "body mass index" as used herein means the ratio of weight in kg divided by the height in metres, squared.

A "disease associated with adipogenesis" refers to a medical condition characterized by the abnormal proliferation and differentiation of adipocytes (fat cells) within the body, leading to an excessive accumulation of adipose tissue. This condition often results in health complications such as obesity, metabolic disorders, and associated comorbidities.

Within the present invention, a "reduction of adipogenesis" refers to a medical or pharmaceutical intervention designed to decrease the formation and accumulation of adipocytes within the body. A reduction in adipogenesis may be determined and/or quantified based on the size and/or number of adipocytes in a tissue sample obtained from a patient. Alternatively, or in addition, a reduction in adipogenesis may be determined and/or quantified by gene expression analysis, measurement of adipogenic markers (i.e., by ELISA), assessment of lipid accumulation in a sample and/or measurement of triglyceride levels in cells or tissues.

Thus, in a particular embodiment, the invention relates to an inhibitor suitable for use, or for use, in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

### INHIBITORS

Inhibitors of the invention include nucleic acids such as siRNAs, antibodies and antigen binding fragments thereof, e.g., monoclonal antibodies, polypeptides, antibody-drug conjugates, and small molecules. Preferred are nucleic acids such as siRNA.

Certain preferred features of inhibitors of the invention, where these are oligonucelosides such as siRNA, are given below.

In certain embodiments, the nucleic acid comprises a first strand comprising a sequence that is at least partially complementary to a portion of RNA transcribed from the *SLC25A5* gene (SEQ ID NO:1381). In a preferred embodiment, the nucleic acid comprises a first strand comprising a sequence that is at least partially complementary to an SLC25A5 mRNA.

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 17 contiguous nucleosides differing by 0 or 1 nucleosides from any one of SEQ ID NO:277-552.

In certain embodiments, the first strand comprises nucleosides 2-18 of any one of the sequences set forth in SEQ ID NO: 277-552.

In certain embodiments, the first strand comprises any one of SEQ ID NO: 277-552.

In certain embodiments, the second strand comprises a nucleoside sequence of at least 17 contiguous nucleosides differing by 0 or 1 nucleosides from any one of SEQ ID NO: 553-828; wherein the second strand has a region of at least 85% complementarity over the 17 contiguous nucleosides to the first strand.

In certain embodiments, the second strand comprises any one of SEQ ID NO: 553-828.

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 21 contiguous nucleosides differing by 0 or 1 nucleosides from any one of SEQ ID NO: 277-552.

In certain embodiments, the first strand comprises nucleosides 2-22 of any one of the sequences set forth in SEQ ID NO: 277-552.

In certain embodiments, the second strand comprises a nucleoside sequence of at least 17 contiguous nucleosides differing by 0 or 1 nucleosides from any one of SEQ ID NO:553-828; wherein the second strand has a region of at least 85% complementarity over the 17 contiguous nucleosides to the first strand.

In certain embodiments, the second strand comprises any one of SEQ ID NO: 553-828.

In certain embodiments, the second strand comprises a nucleoside sequence of at least 19 contiguous nucleosides differing by 0 or 1 nucleosides from any one of SEQ ID NO: 553-828; wherein the second strand has a region of at least 85% complementarity over the 19 contiguous nucleosides to the first strand.

In certain embodiments, the second strand comprises a nucleoside sequence of at least 21 contiguous nucleosides differing by 0 or 1 nucleosides from any one of SEQ ID NO: 553-828; wherein the second strand has a region of at least 85% complementarity over the 21 contiguous nucleosides to the first strand.

In certain embodiments, the nucleic acid comprises a first strand that comprises, consists of, or consists essentially of a nucleoside sequence differing by 0 or 1 nucleosides from any one of SEQ ID NO: 277-552;
and a second strand that comprises, consists of, or consists essentially of a nucleoside sequence differing by 0 or 1 nucleosides from any one of SEQ ID NO: 553-828.

It is preferred herein that the duplex region is formed between a first (antisense) strand and a complementary second (sense) strand. Exemplary pairs of complementary antisense and sense strands are listed in Table 2 below.

Table 2 provides the unmodified first (antisense) and corresponding unmodified second (sense) strand sequences for siRNA oligonucleosides according to the present invention, together with the corresponding positions in the overall gene sequence of SEQ ID NO:1381 as follows.

**Table 2:**

| SEQ ID NO (AS) | Antisense Strand Base Sequence 5' → 3' (Shown as an Unmodified Nucleotide Sequence) | SEQ ID NO (SS) | Sense Strand Base Sequence 5' → 3' (Shown as an Unmodified Nucleotide Sequence) | Corresponding positions on ENST00000317 881.9 |
|---|---|---|---|---|
| SEQ ID NO: 304 | UUAAGAGAAAACUGGUCAG AUGA | SEQ ID NO: 580 | AUCUGACCAGUUUUCU CUUAA | 1107-1130 |
| SEQ ID NO: 323 | UAUCAGGAGUGACUGAAAU AAAA | SEQ ID NO: 599 | UUAUUUCAGUCACUCC UGAUA | 1170-1193 |
| SEQ ID NO: 439 | UCCCUUUGCAGUGUCAUAG AUAC | SEQ ID NO: 715 | AUCUAUGACACUGCAA AGGGA | 650-673 |
| SEQ ID NO: 453 | AUCAGAUUUGUAGAUCUUA ACCA | SEQ ID NO: 729 | GUUAAGAUCUACAAAU CUGAU | 554-577 |
| SEQ ID NO: 496 | AUCAGCUGCUAGACGGUA CGGG | SEQ ID NO: 772 | CGUACCCGUCUAGCAG CUGAU | 482-505 |
| SEQ ID NO: 277 | CCCAUUUUCAACCAGUAAA UAGU | SEQ ID NO: 553 | UAUUUACUGGUUGAAA AUGGG | 1072-1095 |
| SEQ ID NO: 278 | ACGCAGUCUAUAAUGCCUU UGUA | SEQ ID NO: 554 | CAAAGGCAUUAUAGAC UGCGU | 223-246 |
| SEQ ID NO: 279 | GUGUACUUCUUGAUUUCAU CAUA | SEQ ID NO: 555 | UGAUGAAAUCAAGAAG UACAC | 943-966 |
| SEQ ID NO: 280 | AUACAAUUGAGUCCCAUCA UCAU | SEQ ID NO: 556 | GAUGAUGGGACUCAAU UGUAU | 1146-1169 |
| SEQ ID NO: 281 | GGACCACGCAGUCUAUAAU GCCU | SEQ ID NO: 557 | GCAUUAUAGACUGCGU GGUCC | 228-251 |
| SEQ ID NO: 282 | UUGUUAUUUAUCAGGAGUG ACUG | SEQ ID NO: 558 | GUCACUCCUGAUAAAU AACAA | 1178-1201 |
| SEQ ID NO: 283 | CAGUCUAUAAUGCCUUUGU AUUG | SEQ ID NO: 559 | AUACAAAGGCAUUAUA GACUG | 220-243 |
| SEQ ID NO: 284 | AUAGAUACCGAAGUAGGCG GCUC | SEQ ID NO: 560 | GCCGCCUACUUCGGUA UCUAU | 635-658 |
| SEQ ID NO: 285 | UAGAUACCGAAGUAGGCGG CUCG | SEQ ID NO: 561 | AGCCGCCUACUUCGGU AUCUA | 634-657 |
| SEQ ID NO: 286 | GGAAUACGGACCACGCAGU CUAU | SEQ ID NO: 562 | AGACUGCGUGGUCCGU AUUCC | 235-258 |
| SEQ ID NO: 287 | CAGCUGAUGACGAUGUGAG UGUU | SEQ ID NO: 563 | CACUCACAUCGUCAUC AGCUG | 691-714 |
| SEQ ID NO: 288 | AUUUAUCUUUGAAGGCGAA GUUA | SEQ ID NO: 564 | ACUUCGCCUUCAAAGA UAAAU | 333-356 |
| SEQ ID NO: 289 | GUGUCAUAGAUACCGAAGU AGGC | SEQ ID NO: 565 | CUACUUCGGUAUCUAU GACAC | 640-663 |
| SEQ ID NO: 290 | UUCCCAUUUUCAACCAGUA AAUA | SEQ ID NO: 566 | UUUACUGGUUGAAAAU GGGAA | 1074-1097 |
| SEQ ID NO: 291 | GCUGCUAGACGGGUACGGG CAAA | SEQ ID NO: 567 | UGCCCGUACCCGUCUA GCAGC | 478-501 |
| SEQ ID NO: 292 | CAGUUCCUUUGCGCCCUGA CUGC | SEQ ID NO: 568 | AGUCAGGGCGCAAAGG AACUG | 792-815 |
| SEQ ID NO: 293 | CUUCCCAUUUUCAACCAGU AAAU | SEQ ID NO: 569 | UUACUGGUUGAAAAUG GGAAG | 1075-1098 |
| SEQ ID NO: 294 | AGAUACCGAAGUAGGCGGC UCGG | SEQ ID NO: 570 | GAGCCGCCUACUUCGG UAUCU | 633-656 |
| SEQ ID NO: 295 | UUAACCAGGCAGUCACCGA GGCC | SEQ ID NO: 571 | CCUCGGUGACUGCCUG GUUAA | 538-561 |
| SEQ ID NO: 296 | AUCAUCCAGCUGAUGACGA UGUG | SEQ ID NO: 572 | CAUCGUCAUCAGCUGG AUGAU | 697-720 |
| SEQ ID NO: 297 | AUUUAUCAGGAGUGACUGA AAUA | SEQ ID NO: 573 | UUUCAGUCACUCCUGA UAAAU | 1173-1196 |
| SEQ ID NO: 298 | GACAGUCUGUGCGAUCAUC CAGC | SEQ ID NO: 574 | UGGAUGAUCGCACAGA CUGUC | 710-733 |
| SEQ ID NO: 299 | UCUAUAAUGCCUUUGUAUU GCUU | SEQ ID NO: 575 | GCAAUACAAAGGCAUU AUAGA | 217-240 |
| SEQ ID NO: 300 | UAACCAGGCAGUCACCGAG GCCU | SEQ ID NO: 576 | GCCUCGGUGACUGCCU GGUUA | 537-560 |
| SEQ ID NO: 301 | CCCUGCACAGACACGUUAA AGCC | SEQ ID NO: 577 | CUUUAACGUGUCUGUG CAGGG | 598-621 |
| SEQ ID NO: 302 | CACGCAGUCUAUAAUGCCU UUGU | SEQ ID NO: 578 | AAAGGCAUUAUAGACU GCGUG | 224-247 |
| SEQ ID NO: 303 | UAAUACAACAUGCCUGUUC ACAG | SEQ ID NO: 579 | GUGAACAGGCAUGUUG UAUUA | 993-1016 |
| SEQ ID NO: 305 | UGCACAGACACGUUAAAGC CUUG | SEQ ID NO: 581 | AGGCUUUAACGUGUCU GUGCA | 595-618 |
| SEQ ID NO: 306 | GUUCCUUUGCGCCCUGACU GCAU | SEQ ID NO: 582 | GCAGUCAGGGCGCAAA GGAAC | 790-813 |
| SEQ ID NO: 307 | GUGACAGUCUGUGCGAUCA UCCA | SEQ ID NO: 583 | GAUGAUCGCACAGACU GUCAC | 712-735 |
| SEQ ID NO: 308 | ACCACGCAGUCUAUAAUGC CUUU | SEQ ID NO: 584 | AGGCAUUAUAGACUGC GUGGU | 226-249 |
| SEQ ID NO: 309 | CAGUGACAGUCUGUGCGAU CAUC | SEQ ID NO: 585 | UGAUCGCACAGACUGU CACUG | 714-737 |
| SEQ ID NO: 310 | UUGGGAAUACGGACCACGC AGUC | SEQ ID NO: 586 | CUGCGUGGUCCGUAUU CCCAA | 238-261 |
| SEQ ID NO: 311 | GUCUAUAAUGCCUUUGUAU UGCU | SEQ ID NO: 587 | CAAUACAAAGGCAUUA UAGAC | 218-241 |
| SEQ ID NO: 312 | UGUGCGAUCAUCCAGCUGA UGAC | SEQ ID NO: 588 | CAUCAGCUGGAUGAUC GCACA | 703-726 |
| SEQ ID NO: 313 | ACGGACCACGCAGUCUAUA AUGC | SEQ ID NO: 589 | AUUAUAGACUGCGUGG UCCGU | 230-253 |
| SEQ ID NO: 314 | UGCUAGACGGGUACGGGCA AAAU | SEQ ID NO: 590 | UUUGCCCGUACCCGUC UAGCA | 476-499 |
| SEQ ID NO: 315 | UCAUCCAGCUGAUGACGAU GUGA | SEQ ID NO: 591 | ACAUCGUCAUCAGCUG GAUGA | 696-719 |
| SEQ ID NO: 316 | CGGGUACGGCAAAAUCAA GAGG | SEQ ID NO: 592 | UCUUGAUUUGCCCGU ACCCG | 469-492 |
| SEQ ID NO: 317 | AAUACGGACCACGCAGUCU AUAA | SEQ ID NO: 593 | AUAGACUGCGUGGUCC GUAUU | 233-256 |
| SEQ ID NO: 318 | UCAUAGAUACCGAAGUAGG CGGC | SEQ ID NO: 594 | CGCCUACUUCGGUAUC UAUGA | 637-660 |
| SEQ ID NO: 319 | GGGAAUACGGACCACGCAG UCUA | SEQ ID NO: 595 | GACUGCGUGGUCCGUA UUCCC | 236-259 |
| SEQ ID NO: 320 | GUGCGAUCAUCCAGCUGAU GACG | SEQ ID NO: 596 | UCAUCAGCUGGAUGAU CGCAC | 702-725 |
| SEQ ID NO: 321 | UACGGACCACGCAGUCUAU AAUG | SEQ ID NO: 597 | UUAUAGACUGCGUGGU CCGUA | 231-254 |
| SEQ ID NO: 322 | UGUCAUAGAUACCGAAGUA GGCG | SEQ ID NO: 598 | CCUACUUCGGUAUCUA UGACA | 639-662 |
| SEQ ID NO: 324 | UGCGAUCAUCCAGCUGAUG ACGA | SEQ ID NO: 600 | GUCAUCAGCUGGAUGA UCGCA | 701-724 |
| SEQ ID NO: 325 | CUAGACGGGUACGGGCAAA AUCA | SEQ ID NO: 601 | AUUUUGCCCGUACCCG UCUAG | 474-497 |
| SEQ ID NO: 326 | GAUCCGGAAGCAUUCCCUU UGCA | SEQ ID NO: 602 | CAAAGGGAAUGCUUCC GGAUC | 663-686 |
| SEQ ID NO: 327 | GCACAGACACGUUAAAGCC UUGG | SEQ ID NO: 603 | AAGGCUUUAACGUGUC UGUGC | 594-617 |
| SEQ ID NO: 328 | CCACGCAGUCUAUAAUGCC UUUG | SEQ ID NO: 604 | AAGGCAUUAUAGACUG CGUGG | 225-248 |
| SEQ ID NO: 329 | GACGGGUACGGGCAAAAUC AAGA | SEQ ID NO: 605 | UUGAUUUUGCCCGUAC CCGUC | 471-494 |
| SEQ ID NO: 330 | GAUACCGAAGUAGGCGGCU CGGU | SEQ ID NO: 606 | CGAGCCGCCUACUUCG GUAUC | 632-655 |
| SEQ ID NO: 331 | GUAGGCGGCUCGGUAGAUG AUAA | SEQ ID NO: 607 | AUCAUCUACCGAGCCG CCUAC | 623-646 |
| SEQ ID NO: 332 | AGACGGGUACGGGCAAAAU CAAG | SEQ ID NO: 608 | UGAUUUUGCCCGUACC CGUCU | 472-495 |
| SEQ ID NO: 333 | UAGGCGGCUCGGUAGAUGA UAAU | SEQ ID NO: 609 | UAUCAUCUACCGAGCC GCCUA | 622-645 |
| SEQ ID NO: 334 | AAGUAGGCGGCUCGGUAGA UGAU | SEQ ID NO: 610 | CAUCUACCGAGCCGCC UACUU | 625-648 |
| SEQ ID NO: 335 | CUUUGAAGGCGAAGUUAAG AGCC | SEQ ID NO: 611 | CUCUUAACUUCGCCUU CAAAG | 327-350 |
| SEQ ID NO: 336 | CGAAGUUAAGAGCCUGGGU GGGG | SEQ ID NO: 612 | CCACCCAGGCUCUUAA CUUCG | 318-341 |
| SEQ ID NO: 337 | CCCUGCUCCUUGGGAAUAC GGAC | SEQ ID NO: 613 | CCGUAUUCCCAAGGAG CAGGG | 247-270 |
| SEQ ID NO: 338 | GAAGGACAGAACUCCCUGC UCCU | SEQ ID NO: 614 | GAGCAGGGAGUUCUGU CCUUC | 260-283 |
| SEQ ID NO: 339 | AGAACUCCCUGCUCCUUGG GAAU | SEQ ID NO: 615 | UCCCAAGGAGCAGGGA GUUCU | 253-276 |
| SEQ ID NO: 340 | CCUGCUCCUUGGGAAUACG GACC | SEQ ID NO: 616 | UCCGUAUUCCCAAGGA GCAGG | 246-269 |
| SEQ ID NO: 341 | CAGAAGGACAGAACUCCCU GCUC | SEQ ID NO: 617 | GCAGGGAGUUCUGUCC UUCUG | 262-285 |
| SEQ ID NO: 342 | CUGCUCCUUGGGAAUACGG ACCA | SEQ ID NO: 618 | GUCCGUAUUCCCAAGG AGCAG | 245-268 |
| SEQ ID NO: 343 | AGAAGGACAGAACUCCCUG CUCC | SEQ ID NO: 619 | AGCAGGGAGUUCUGUC CUUCU | 261-284 |
| SEQ ID NO: 344 | AAGGACAGAACUCCCUGCU CCUU | SEQ ID NO: 620 | GGAGCAGGGAGUUCUG UCCUU | 259-282 |
| SEQ ID NO: 345 | GAACUCCCUGCUCCUUGGG AAUA | SEQ ID NO: 621 | UUCCCAAGGAGCAGGG AGUUC | 252-275 |
| SEQ ID NO: 346 | AGGACAGAACUCCCUGCUC CUUG | SEQ ID NO: 622 | AGGAGCAGGGAGUUCU GUCCU | 258-281 |
| SEQ ID NO: 347 | GAAGUUAAGAGCCUGGGUG GGGA | SEQ ID NO: 623 | CCCACCCAGGCUCUUA ACUUC | 317-340 |
| SEQ ID NO: 348 | UCUUUGAAGGCGAAGUUAA GAGC | SEQ ID NO: 624 | UCUUAACUUCGCCUUC AAAGA | 328-351 |
| SEQ ID NO: 349 | UAUUUAUCAGGAGUGACUG AAAU | SEQ ID NO: 625 | UUCAGUCACUCCUGAU AAAUA | 1174-1197 |
| SEQ ID NO: 350 | GUUAUUUAUCAGGAGUGAC UGAA | SEQ ID NO: 626 | CAGUCACUCCUGAUAA AUAAC | 1176-1199 |
| SEQ ID NO: 351 | UUAUUUAUCAGGAGUGACU GAAA | SEQ ID NO: 627 | UCAGUCACUCCUGAUA AAUAA | 1175-1198 |
| SEQ ID NO: 352 | AAAUACAAUUGAGUCCCAU CAUC | SEQ ID NO: 628 | UGAUGGGACUCAAUUG UAUUU | 1148-1171 |
| SEQ ID NO: 353 | AAAAUACAAUUGAGUCCCA UCAU | SEQ ID NO: 629 | GAUGGGACUCAAUUGU AUUUU | 1149-1172 |
| SEQ ID NO: 354 | AUUUGUUAUUUAUCAGGAG UGAC | SEQ ID NO: 630 | CACUCCUGAUAAAUAA CAAAU | 1180-1203 |
| SEQ ID NO: 355 | GAGAAAACUGGUCAGAUGA AUAU | SEQ ID NO: 631 | AUUCAUCUGACCAGUU UUCUC | 1103-1126 |
| SEQ ID NO: 356 | AUAUAAUACAACAUGCCUG UUCA | SEQ ID NO: 632 | AACAGGCAUGUUGUAU UAUAU | 996-1019 |
| SEQ ID NO: 357 | UGGGAUCCGGAAGCAUUCC CUUU | SEQ ID NO: 633 | AGGGAAUGCUUCCGGA UCCCA | 666-689 |
| SEQ ID NO: 358 | UUGGGAUCCGGAAGCAUUC CCUU | SEQ ID NO: 634 | GGGAAUGCUUCCGGAU CCCAA | 667-690 |
| SEQ ID NO: 359 | CAGGCAGUCACCGAGGCCU CGGA | SEQ ID NO: 635 | CGAGGCCUCGGUGACU GCCUG | 533-556 |
| SEQ ID NO: 360 | UAUAUAAUACAACAUGCCU GUUC | SEQ ID NO: 636 | ACAGGCAUGUUGUAUU AUAUA | 997-1020 |
| SEQ ID NO: 361 | AGUCUAUAAUGCCUUUGUA UUGC | SEQ ID NO: 637 | AAUACAAAGGCAUUAU AGACU | 219-242 |
| SEQ ID NO: 362 | UUUAUCAGGAGUGACUGAA AUAA | SEQ ID NO: 638 | AUUUCAGUCACUCCUG AUAAA | 1172-1195 |
| SEQ ID NO: 363 | AAUACAAUUGAGUCCCAUC AUCA | SEQ ID NO: 639 | AUGAUGGGACUCAAUU GUAUU | 1147-1170 |
| SEQ ID NO: 364 | CUUUAAGAGAAAACUGGUC AGAU | SEQ ID NO: 640 | CUGACCAGUUUCUCU UAAAG | 1109-1132 |
| SEQ ID NO: 365 | GCUUUAAGAGAAAACUGGU CAGA | SEQ ID NO: 641 | UGACCAGUUUCUCUU AAAGC | 1110-1133 |
| SEQ ID NO: 366 | AAUGCCUUUGUAUUGCUUA UCUG | SEQ ID NO: 642 | GAUAAGCAAUACAAAG GCAUU | 212-235 |
| SEQ ID NO: 367 | UAAUGCCUUUGUAUUGCUU AUCU | SEQ ID NO: 643 | AUAAGCAAUACAAAGG CAUUA | 213-236 |
| SEQ ID NO: 368 | UACCCUGCACAGACACGUU AAAG | SEQ ID NO: 644 | UUAACGUGUCUGUGCA GGGUA | 600-623 |
| SEQ ID NO: 369 | AUACCCUGCACAGACACGU UAAA | SEQ ID NO: 645 | UAACGUGUCUGUGCAG GGUAU | 601-624 |
| SEQ ID NO: 370 | AUCCAGCUGAUGACGAUGU GAGU | SEQ ID NO: 646 | UCACAUCGUCAUCAGC UGGAU | 694-717 |
| SEQ ID NO: 371 | UACAAUUGAGUCCCAUCAU CAUC | SEQ ID NO: 647 | UGAUGAUGGGACUCAA UUGUA | 1145-1168 |
| SEQ ID NO: 372 | UGGGAAUACGGACCACGCA GUCU | SEQ ID NO: 648 | ACUGCGUGGUCCGUAU UCCCA | 237-260 |
| SEQ ID NO: 373 | UAUAAUACAACAUGCCUGU UCAC | SEQ ID NO: 649 | GAACAGGCAUGUUGUA UUAUA | 995-1018 |
| SEQ ID NO: 374 | UUAUCAGGAGUGACUGAAA UAAA | SEQ ID NO: 650 | UAUUUCAGUCACUCCU GAUAA | 1171-1194 |
| SEQ ID NO: 375 | AUAAUGCCUUUGUAUUGCU UAUC | SEQ ID NO: 651 | UAAGCAAUACAAAGGC AUUAU | 214-237 |
| SEQ ID NO: 376 | CCAGCUGAUGACGAUGUGA GUGU | SEQ ID NO: 652 | ACUCACAUCGUCAUCA GCUGG | 692-715 |
| SEQ ID NO: 377 | CAUAGAUACCGAAGUAGGC GGCU | SEQ ID NO: 653 | CCGCCUACUUCGGUAU CUAUG | 636-659 |
| SEQ ID NO: 378 | CAUCCAGCUGAUGACGAUG UGAG | SEQ ID NO: 654 | CACAUCGUCAUCAGCU GGAUG | 695-718 |
| SEQ ID NO: 379 | GUCAUAGAUACCGAAGUAG GCGG | SEQ ID NO: 655 | GCCUACUUCGGUAUCU AUGAC | 638-661 |
| SEQ ID NO: 380 | ACCCUGCACAGACACGUUA AAGC | SEQ ID NO: 656 | UUUAACGUGUCUGUGC AGGGU | 599-622 |
| SEQ ID NO: 381 | UGCAUCAUCAUGCGGCGGC GAAC | SEQ ID NO: 657 | UCGCCGCCGCAUGAUG AUGCA | 772-795 |
| SEQ ID NO: 382 | CGGACCACGCAGUCUAUAA UGCC | SEQ ID NO: 658 | CAUUAUAGACUGCGUG GUCCG | 229-252 |
| SEQ ID NO: 383 | CUUGGGAAUACGGACCACG CAGU | SEQ ID NO: 659 | UGCGUGGUCCGUAUUC CCAAG | 239-262 |
| SEQ ID NO: 384 | GAAGUAGGCGGCUCGGUAG AUGA | SEQ ID NO: 660 | AUCUACCGAGCCGCCU ACUUC | 626-649 |
| SEQ ID NO: 385 | CUGCUAGACGGGUACGGGC AAAA | SEQ ID NO: 661 | UUGCCCGUACCCGUCU AGCAG | 477-500 |
| SEQ ID NO: 386 | UCCAGCUGAUGACGAUGUG AGUG | SEQ ID NO: 662 | CUCACAUCGUCAUCAG CUGGA | 693-716 |
| SEQ ID NO: 387 | AUACGGACCACGCAGUCUA UAAU | SEQ ID NO: 663 | UAUAGACUGCGUGGUC CGUAU | 232-255 |
| SEQ ID NO: 388 | AACCAGGCAGUCACCGAGG CCUC | SEQ ID NO: 664 | GGCCUCGGUGACUGCC UGGUU | 536-559 |
| SEQ ID NO: 389 | UGACAGUCUGUGCGAUCAU CCAG | SEQ ID NO: 665 | GGAUGAUCGCACAGAC UGUCA | 711-734 |
| SEQ ID NO: 390 | UAGACGGGUACGGGCAAAA UCAA | SEQ ID NO: 666 | GAUUUUGCCCGUACCC GUCUA | 473-496 |
| SEQ ID NO: 391 | GCAGUGACAGUCUGUGCGA UCAU | SEQ ID NO: 667 | GAUCGCACAGACUGUC ACUGC | 715-738 |
| SEQ ID NO: 392 | AGUGACAGUCUGUGCGAUC AUCC | SEQ ID NO: 668 | AUGAUCGCACAGACUG UCACU | 713-736 |
| SEQ ID NO: 393 | GCUAGACGGGUACGGGCAA AAUC | SEQ ID NO: 669 | UUUUGCCCGUACCCGU CUAGC | 475-498 |
| SEQ ID NO: 394 | AGUAGGCGGCUCGGUAGAU GAUA | SEQ ID NO: 670 | UCAUCUACCGAGCCGC CUACU | 624-647 |
| SEQ ID NO: 395 | GAAUACGGACCACGCAGUC UAUA | SEQ ID NO: 671 | UAGACUGCGUGGUCCG UAUUC | 234-257 |
| SEQ ID NO: 396 | CUUUGUAUUGCUUAUCUGC AGUG | SEQ ID NO: 672 | CUGCAGAUAAGCAAUA CAAAG | 207-230 |
| SEQ ID NO: 397 | UUUGUAUUGCUUAUCUGCA GUGA | SEQ ID NO: 673 | ACUGCAGAUAAGCAAU ACAAA | 206-229 |
| SEQ ID NO: 398 | UUGUAUUGCUUAUCUGCAG UGAU | SEQ ID NO: 674 | CACUGCAGAUAAGCAA UACAA | 205-228 |
| SEQ ID NO: 399 | UAUCUGCAGUGAUCUGCUU GCUG | SEQ ID NO: 675 | GCAAGCAGAUCACUGC AGAUA | 195-218 |
| SEQ ID NO: 400 | GUAUUGCUUAUCUGCAGUG AUCU | SEQ ID NO: 676 | AUCACUGCAGAUAAGC AAUAC | 203-226 |
| SEQ ID NO: 401 | UGUAUUGCUUAUCUGCAGU GAUC | SEQ ID NO: 677 | UCACUGCAGAUAAGCA AUACA | 204-227 |
| SEQ ID NO: 402 | UGCUUAUCUGCAGUGAUCU GCUU | SEQ ID NO: 678 | GCAGAUCACUGCAGAU AAGCA | 199-222 |
| SEQ ID NO: 403 | UUGCUUAUCUGCAGUGAUC UGCU | SEQ ID NO: 679 | CAGAUCACUGCAGAUA AGCAA | 200-223 |
| SEQ ID NO: 404 | AUCUGCAGUGAUCUGCUUG CUGG | SEQ ID NO: 680 | AGCAAGCAGAUCACUG CAGAU | 194-217 |
| SEQ ID NO: 405 | UAUUGCUUAUCUGCAGUGA UCUG | SEQ ID NO: 681 | GAUCACUGCAGAUAAG CAAUA | 202-225 |
| SEQ ID NO: 406 | UUCUCCAAAUUUGUUAUUU AUCA | SEQ ID NO: 682 | AUAAAUAACAAAUUUG GAGAA | 1188-1211 |
| SEQ ID NO: 407 | CAAAUUUGUUAUUUAUCAG GAGU | SEQ ID NO: 683 | UCCUGAUAAAUAACAA AUUUG | 1183-1206 |
| SEQ ID NO: 408 | GAGGCCUCGGAAUUCCCUU UCAG | SEQ ID NO: 684 | GAAAGGGAAUUCCGAG GCCUC | 521-544 |
| SEQ ID NO: 409 | AGAUAUGUUAUAUAAUACA ACAU | SEQ ID NO: 685 | GUUGUAUUAUAUAACA UAUCU | 1005-1028 |
| SEQ ID NO: 410 | GCAGUCACCGAGGCCUCGG AAUU | SEQ ID NO: 686 | UUCCGAGGCCUCGGUG ACUGC | 530-553 |
| SEQ ID NO: 411 | CGAGGCCUCGGAAUUCCCU UUCA | SEQ ID NO: 687 | AAAGGGAAUUCCGAGG CCUCG | 522-545 |
| SEQ ID NO: 412 | CAAGAUAUGUUAUAUAAUA CAAC | SEQ ID NO: 688 | UGUAUUAUAUAACAUA UCUUG | 1007-1030 |
| SEQ ID NO: 413 | AGUCACCGAGGCCUCGGAA UUCC | SEQ ID NO: 689 | AAUUCCGAGGCCUCGG UGACU | 528-551 |
| SEQ ID NO: 414 | CACCGAGGCCUCGGAAUUC CCUU | SEQ ID NO: 690 | GGGAAUUCCGAGGCCU CGGUG | 525-548 |
| SEQ ID NO: 415 | CCUCGGAAUUCCCUUUCAG CUCC | SEQ ID NO: 691 | AGCUGAAAGGAAUUC CGAGG | 517-540 |
| SEQ ID NO: 416 | UCGGAAUUCCCUUUCAGCU CCAG | SEQ ID NO: 692 | GGAGCUGAAAGGGAAU UCCGA | 515-538 |
| SEQ ID NO: 417 | GGCCUCGGAAUUCCCUUUC AGCU | SEQ ID NO: 693 | CUGAAAGGGAAUUCCG AGGCC | 519-542 |
| SEQ ID NO: 418 | CUCGGAAUUCCCUUUCAGC UCCA | SEQ ID NO: 694 | GAGCUGAAAGGGAAUU CCGAG | 516-539 |
| SEQ ID NO: 419 | GCCUCGGAAUUCCCUUUCA GCUC | SEQ ID NO: 695 | GCUGAAAGGGAAUUCC GAGGC | 518-541 |
| SEQ ID NO: 420 | AUGUUAUAUAAUACAACAU GCCU | SEQ ID NO: 696 | GCAUGUUGUAUUAUAU AACAU | 1001-1024 |
| SEQ ID NO: 421 | AAGAUAUGUUAUAUAAUAC AACA | SEQ ID NO: 697 | UUGUAUUAUAUAACAU AUCUU | 1006-1029 |
| SEQ ID NO: 422 | UCAAGAUAUGUUAUAUAAU ACAA | SEQ ID NO: 698 | GUAUUAUAUAACAUAU CUUGA | 1008-1031 |
| SEQ ID NO: 423 | UGUUAUAUAAUACAACAUG CCUG | SEQ ID NO: 699 | GGCAUGUUGUAUUAUA UAACA | 1000-1023 |
| SEQ ID NO: 424 | CCGAGGCCUCGGAAUUCCC UUUC | SEQ ID NO: 700 | AAGGGAAUUCCGAGGC CUCGG | 523-546 |
| SEQ ID NO: 425 | GUCACCGAGGCCUCGGAAU UCCC | SEQ ID NO: 701 | GAAUUCCGAGGCCUCG GUGAC | 527-550 |
| SEQ ID NO: 426 | ACCGAGGCCUCGGAAUUCC CUUU | SEQ ID NO: 702 | AGGGAAUUCCGAGGCC UCGGU | 524-547 |
| SEQ ID NO: 427 | UCACCGAGGCCUCGGAAUU CCCU | SEQ ID NO: 703 | GGAAUUCCGAGGCCUC GGUGA | 526-549 |
| SEQ ID NO: 428 | AUUUCAUCAUACAAGACAA GCAC | SEQ ID NO: 704 | GCUUGUCUUGUAUGAU GAAAU | 931-954 |
| SEQ ID NO: 429 | UGUAUUUAUCUUGAAGGC GAAG | SEQ ID NO: 705 | UCGCCUUCAAAGAUAA AUACA | 336-359 |
| SEQ ID NO: 430 | AUUUGUAGAUCUUAACCAG GCAG | SEQ ID NO: 706 | GCCUGGUUAAGAUCUA CAAAU | 549-572 |
| SEQ ID NO: 431 | UUCCCUUUGCAGUGUCAUA GAUA | SEQ ID NO: 707 | UCUAUGACACUGCAAA GGGAA | 651-674 |
| SEQ ID NO: 432 | AUCAUACAAGACAAGCACA AAAG | SEQ ID NO: 708 | UUUGUGCUUGUCUUGU AUGAU | 926-949 |
| SEQ ID NO: 433 | UAGAUCUUAACCAGGCAGU CACC | SEQ ID NO: 709 | UGACUGCCUGGUUAAG AUCUA | 544-567 |
| SEQ ID NO: 434 | AAGCAUUCCCUUUGCAGUG UCAU | SEQ ID NO: 710 | GACACUGCAAAGGGAA UGCUU | 656-679 |
| SEQ ID NO: 435 | UUUGCAGUGUCAUAGAUAC CGAA | SEQ ID NO: 711 | CGGUAUCUAUGACACU GCAAA | 646-669 |
| SEQ ID NO: 436 | AGAGAAAACUGGUCAGAUG AAUA | SEQ ID NO: 712 | UUCAUCUGACCAGUUU UCUCU | 1104-1127 |
| SEQ ID NO: 437 | UGCAGUGUCAUAGAUACCG AAGU | SEQ ID NO: 713 | UUCGGUAUCUAUGACA CUGCA | 644-667 |
| SEQ ID NO: 438 | UCAGAUUUGUAGAUCUUAA CCAG | SEQ ID NO: 714 | GGUUAAGAUCUACAAA UCUGA | 553-576 |
| SEQ ID NO: 440 | GAACAUUGGACCAUGCACC CUUG | SEQ ID NO: 716 | AGGGUGCAUGGUCCAA UGUUC | 885-908 |
| SEQ ID NO: 441 | AAUCCCAUCAGAUUUGUAG AUCU | SEQ ID NO: 717 | AUCUACAAAUCUGAUG GGAUU | 560-583 |
| SEQ ID NO: 442 | GCAGUGUCAUAGAUACCGA AGUA | SEQ ID NO: 718 | CUUCGGUAUCUAUGAC ACUGC | 643-666 |
| SEQ ID NO: 443 | CAAGCACAAAAGCACCACC CAUG | SEQ ID NO: 719 | UGGGUGGUGCUUUGU GCUUG | 915-938 |
| SEQ ID NO: 444 | GCGGCUCGGUAGAUGAUAA UACC | SEQ ID NO: 720 | UAUUAUCAUCUACCGA GCCGC | 619-642 |
| SEQ ID NO: 445 | CUCCUUGGGAAUACGGACC ACGC | SEQ ID NO: 721 | GUGGUCCGUAUUCCCA AGGAG | 242-265 |
| SEQ ID NO: 446 | AUUCCCUUUGCAGUGUCAU AGAU | SEQ ID NO: 722 | CUAUGACACUGCAAAG GGAAU | 652-675 |
| SEQ ID NO: 447 | CAUACAAGACAAGCACAAA AGCA | SEQ ID NO: 723 | CUUUUGUGCUUGUCUU GUAUG | 924-947 |
| SEQ ID NO: 448 | AGACACGUUAAAGCCUUGG UACA | SEQ ID NO: 724 | UACCAAGGCUUUAACG UGUCU | 590-613 |
| SEQ ID NO: 449 | UGAGAACAUUGGACCAUGC ACCC | SEQ ID NO: 725 | GUGCAUGGUCCAAUGU UCUCA | 888-911 |
| SEQ ID NO: 450 | GCUUGUAUUUAUCUUUGAA GGCG | SEQ ID NO: 726 | CCUUCAAAGAUAAAUA CAAGC | 339-362 |
| SEQ ID NO: 451 | GCUCGGUAGAUGAUAAUAC CCUG | SEQ ID NO: 727 | GGGUAUUAUCAUCUAC CGAGC | 616-639 |
| SEQ ID NO: 452 | AGUGUCAUAGAUACCGAAG UAGG | SEQ ID NO: 728 | UACUUCGGUAUCUAUG ACACU | 641-664 |
| SEQ ID NO: 454 | GGUAGAUGAUAAUACCCUG CACA | SEQ ID NO: 730 | UGCAGGGUAUUAUCAU CUACC | 612-635 |
| SEQ ID NO: 455 | CAGGCCCUUAAUCCCAUCA GAUU | SEQ ID NO: 731 | UCUGAUGGGAUUAAGG GCCUG | 569-592 |
| SEQ ID NO: 456 | AGAUGAUAAUACCCUGCAC AGAC | SEQ ID NO: 732 | CUGUGCAGGGUAUUAU CAUCU | 609-632 |
| SEQ ID NO: 457 | UACAGGCCCUUAAUCCCAU CAGA | SEQ ID NO: 733 | UGAUGGGAUUAAGGGC CUGUA | 571-594 |
| SEQ ID NO: 458 | ACAGACACGUUAAAGCCUU GGUA | SEQ ID NO: 734 | CCAAGGCUUUAACGUG UCUGU | 592-615 |
| SEQ ID NO: 459 | AGAAAACUGGUCAGAUGAA UAUU | SEQ ID NO: 735 | UAUUCAUCUGACCAGU UUUCU | 1102-1125 |
| SEQ ID NO: 460 | UGUAGAUCUUAACCAGGCA GUCA | SEQ ID NO: 736 | ACUGCCUGGUUAAGAU CUACA | 546-569 |
| SEQ ID NO: 461 | UCAAAUGGAUAGGAAGUCA ACCC | SEQ ID NO: 737 | GUUGACUUCCUAUCCA UUUGA | 745-768 |
| SEQ ID NO: 462 | CAUUCCCUUUGCAGUGUCA UAGA | SEQ ID NO: 738 | UAUGACACUGCAAAGG GAAUG | 653-676 |
| SEQ ID NO: 463 | CAUCAGAUUUGUAGAUCUU AACC | SEQ ID NO: 739 | UUAAGAUCUACAAAUC UGAUG | 555-578 |
| SEQ ID NO: 464 | CUCGGUAGAUGAUAAUACC CUGC | SEQ ID NO: 740 | AGGGUAUUAUCAUCUA CCGAG | 615-638 |
| SEQ ID NO: 465 | UUUGUAGAUCUUAACCAGG CAGU | SEQ ID NO: 741 | UGCCUGGUUAAGAUCU ACAAA | 548-571 |
| SEQ ID NO: 466 | ACAGGCCCUUAAUCCCAUC AGAU | SEQ ID NO: 742 | CUGAUGGGAUUAAGGG CCUGU | 570-593 |
| SEQ ID NO: 467 | CUUUGCAGUGUCAUAGAUA CCGA | SEQ ID NO: 743 | GGUAUCUAUGACACUG CAAAG | 647-670 |
| SEQ ID NO: 468 | CACAUCAGCUGCUAGACGG GUAC | SEQ ID NO: 744 | ACCCGUCUAGCAGCUG AUGUG | 485-508 |
| SEQ ID NO: 469 | CCCUUUGCAGUGUCAUAGA UACC | SEQ ID NO: 745 | UAUCUAUGACACUGCA AAGGG | 649-672 |
| SEQ ID NO: 470 | CUCCAGCUUUACCCACAUC AGCU | SEQ ID NO: 746 | CUGAUGUGGGUAAAGC UGGAG | 498-521 |
| SEQ ID NO: 471 | UUACCCACAUCAGCUGCUA GACG | SEQ ID NO: 747 | UCUAGCAGCUGAUGUG GGUAA | 490-513 |
| SEQ ID NO: 472 | UUAAAGCCUUGGUACAGGC CCUU | SEQ ID NO: 748 | GGGCCUGUACCAAGGC UUUAA | 583-606 |
| SEQ ID NO: 473 | CAGCUGCUAGACGGGUACG GGCA | SEQ ID NO: 749 | CCCGUACCCGUCUAGC AGCUG | 480-503 |
| SEQ ID NO: 474 | CAGCUUUACCCACAUCAGC UGCU | SEQ ID NO: 750 | CAGCUGAUGUGGGUAA AGCUG | 495-518 |
| SEQ ID NO: 475 | UCAGCUGCUAGACGGGUAC GGGC | SEQ ID NO: 751 | CCGUACCCGUCUAGCA GCUGA | 481-504 |
| SEQ ID NO: 476 | UCCAGCUUUACCCACAUCA GCUG | SEQ ID NO: 752 | GCUGAUGUGGGUAAAG CUGGA | 497-520 |
| SEQ ID NO: 477 | GGCUCGGUAGAUGAUAAUA CCCU | SEQ ID NO: 753 | GGUAUUAUCAUCUACC GAGCC | 617-640 |
| SEQ ID NO: 478 | AGGCGGCUCGGUAGAUGAU AAUA | SEQ ID NO: 754 | UUAUCAUCUACCGAGC CGCCU | 621-644 |
| SEQ ID NO: 479 | CAUCAGCUGCUAGACGGGU ACGG | SEQ ID NO: 755 | GUACCCGUCUAGCAGC UGAUG | 483-506 |
| SEQ ID NO: 480 | GUACAGGCCCUUAAUCCCA UCAG | SEQ ID NO: 756 | GAUGGGAUUAAGGGCC UGUAC | 572-595 |
| SEQ ID NO: 481 | AUGAUAAUACCCUGCACAG ACAC | SEQ ID NO: 757 | GUCUGUGCAGGGUAUU AUCAU | 607-630 |
| SEQ ID NO: 482 | CCUUGGGAAUACGGACCAC GCAG | SEQ ID NO: 758 | GCGUGGUCCGUAUUCC CAAGG | 240-263 |
| SEQ ID NO: 483 | UCCUUGGGAAUACGGACCA CGCA | SEQ ID NO: 759 | CGUGGUCCGUAUUCCC AAGGA | 241-264 |
| SEQ ID NO: 484 | CAGAUUUGUAGAUCUUAAC CAGG | SEQ ID NO: 760 | UGGUUAAGAUCUACAA AUCUG | 552-575 |
| SEQ ID NO: 485 | UGUACUUCUUGAUUUCAUC AUAC | SEQ ID NO: 761 | AUGAUGAAAUCAAGAA GUACA | 942-965 |
| SEQ ID NO: 486 | AUCCCAUCAGAUUUGUAGA UCUU | SEQ ID NO: 762 | GAUCUACAAAUCUGAU GGGAU | 559-582 |
| SEQ ID NO: 487 | GUAGAUGAUAAUACCCUGC ACAG | SEQ ID NO: 763 | GUGCAGGGUAUUAUCA UCUAC | 611-634 |
| SEQ ID NO: 488 | GACACGUUAAAGCCUUGGU ACAG | SEQ ID NO: 764 | GUACCAAGGCUUUAAC GUGUC | 589-612 |
| SEQ ID NO: 489 | AGCUUUACCCACAUCAGCU GCUA | SEQ ID NO: 765 | GCAGCUGAUGUGGGUA AAGCU | 494-517 |
| SEQ ID NO: 490 | AAGCACAAAAGCACCACCC AUGC | SEQ ID NO: 766 | AUGGGUGGUGCUUUUG UGCUU | 914-937 |
| SEQ ID NO: 491 | AUCUUAACCAGGCAGUCAC CGAG | SEQ ID NO: 767 | CGGUGACUGCCUGGUU AAGAU | 541-564 |
| SEQ ID NO: 492 | UUUACCCACAUCAGCUGCU AGAC | SEQ ID NO: 768 | CUAGCAGCUGAUGUGG GUAAA | 491-514 |
| SEQ ID NO: 493 | CUUAAUCCCAUCAGAUUUG UAGA | SEQ ID NO: 769 | UACAAAUCUGAUGGA UUAAG | 563-586 |
| SEQ ID NO: 494 | CAGUGUCAUAGAUACCGAA GUAG | SEQ ID NO: 770 | ACUUCGGUAUCUAUGA CACUG | 642-665 |
| SEQ ID NO: 495 | CCAGCUUUACCCACAUCAG CUGC | SEQ ID NO: 771 | AGCUGAUGUGGGUAAA GCUGG | 496-519 |
| SEQ ID NO: 497 | GAUGAUAAUACCCUGCACA GACA | SEQ ID NO: 773 | UCUGUGCAGGGUAUUA UCAUC | 608-631 |
| SEQ ID NO: 498 | UUUCAUCAUACAAGACAAG CACA | SEQ ID NO: 774 | UGCUUGUCUUGUAUGA UGAAA | 930-953 |
| SEQ ID NO: 499 | CAGACACGUUAAAGCCUUG GUAC | SEQ ID NO: 775 | ACCAAGGCUUUAACGU GUCUG | 591-614 |
| SEQ ID NO: 500 | AGAACAUUGGACCAUGCAC CCUU | SEQ ID NO: 776 | GGGUGCAUGGUCCAAU GUUCU | 886-909 |
| SEQ ID NO: 501 | GUAGAUCUUAACCAGGCAG UCAC | SEQ ID NO: 777 | GACUGCCUGGUUAAGA UCUAC | 545-568 |
| SEQ ID NO: 502 | AGCUCCAGCUUUACCCACA UCAG | SEQ ID NO: 778 | GAUGUGGGUAAAGCUG GAGCU | 500-523 |
| SEQ ID NO: 503 | GCUCCUUGGGAAUACGGAC CACG | SEQ ID NO: 779 | UGGUCCGUAUUCCCAA GGAGC | 243-266 |
| SEQ ID NO: 504 | GAAGCAUUCCCUUUGCAGU GUCA | SEQ ID NO: 780 | ACACUGCAAAGGGAAU GCUUC | 657-680 |
| SEQ ID NO: 505 | CCUUGGUACAGGCCCUUAA UCCC | SEQ ID NO: 781 | GAUUAAGGGCCUGUAC CAAGG | 577-600 |
| SEQ ID NO: 506 | UAGAUGAUAAUACCCUGCA CAGA | SEQ ID NO: 782 | UGUGCAGGGUAUUAUC AUCUA | 610-633 |
| SEQ ID NO: 507 | AAUACCCUGCACAGACACG UUAA | SEQ ID NO: 783 | AACGUGUCUGUGCAGG GUAUU | 602-625 |
| SEQ ID NO: 508 | GUUAAAGCCUUGGUACAGG CCCU | SEQ ID NO: 784 | GGCCUGUACCAAGGCU UUAAC | 584-607 |
| SEQ ID NO: 509 | AGCAUUCCCUUUGCAGUGU CAUA | SEQ ID NO: 785 | UGACACUGCAAAGGGA AUGCU | 655-678 |
| SEQ ID NO: 510 | UCUUAACCAGGCAGUCACC GAGG | SEQ ID NO: 786 | UCGGUGACUGCCUGGU UAAGA | 540-563 |
| SEQ ID NO: 511 | AUACAAGACAAGCACAAAA GCAC | SEQ ID NO: 787 | GCUUUUGUGCUUGUCU UGUAU | 923-946 |
| SEQ ID NO: 512 | GACAAGCACAAAAGCACCA CCCA | SEQ ID NO: 788 | GGUGGUGCUUUUGUGC UUGUC | 917-940 |
| SEQ ID NO: 513 | ACCCACAUCAGCUGCUAGA CGGG | SEQ ID NO: 789 | CGUCUAGCAGCUGAUG UGGGU | 488-511 |
| SEQ ID NO: 514 | UAAAGCCUUGGUACAGGCC CUUA | SEQ ID NO: 790 | AGGGCCUGUACCAAGG CUUUA | 582-605 |
| SEQ ID NO: 515 | UUCAUCAUACAAGACAAGC ACAA | SEQ ID NO: 791 | GUGCUUGUCUUGUAUG AUGAA | 929-952 |
| SEQ ID NO: 516 | GCCCUUAAUCCCAUCAGAU UUGU | SEQ ID NO: 792 | AAAUCUGAUGGGAUUA AGGGC | 566-589 |
| SEQ ID NO: 517 | UUAAUCCCAUCAGAUUUGU AGAU | SEQ ID NO: 793 | CUACAAAUCUGAUGGG AUUAA | 562-585 |
| SEQ ID NO: 518 | UCGGUAGAUGAUAAUACCC UGCA | SEQ ID NO: 794 | CAGGGUAUUAUCAUCU ACCGA | 614-637 |
| SEQ ID NO: 519 | CACGUUAAAGCCUUGGUAC AGGC | SEQ ID NO: 795 | CUGUACCAAGGCUUUA ACGUG | 587-610 |
| SEQ ID NO: 520 | UCCCAUCAGAUUUGUAGAU CUUA | SEQ ID NO: 796 | AGAUCUACAAAUCUGA UGGGA | 558-581 |
| SEQ ID NO: 521 | UCAUACAAGACAAGCACAA AAGC | SEQ ID NO: 797 | UUUUGUGCUUGUCUUG UAUGA | 925-948 |
| SEQ ID NO: 522 | CCUUAAUCCCAUCAGAUUU GUAG | SEQ ID NO: 798 | ACAAAUCUGAUGGGAU UAAGG | 564-587 |
| SEQ ID NO: 523 | ACAUUGGACCAUGCACCCU UGAA | SEQ ID NO: 799 | CAAGGGUGCAUGGUCC AAUGU | 883-906 |
| SEQ ID NO: 524 | CUUGGUACAGGCCCUUAAU CCCA | SEQ ID NO: 800 | GGAUUAAGGGCCUGUA CCAAG | 576-599 |
| SEQ ID NO: 525 | UUGCAGUGUCAUAGAUACC GAAG | SEQ ID NO: 801 | UCGGUAUCUAUGACAC UGCAA | 645-668 |
| SEQ ID NO: 526 | CACAGACACGUUAAAGCCU UGGU | SEQ ID NO: 802 | CAAGGCUUUAACGUGU CUGUG | 593-616 |
| SEQ ID NO: 527 | AGCACAAAAGCACCACCCA UGCC | SEQ ID NO: 803 | CAUGGGUGGUGCUUUU GUGCU | 913-936 |
| SEQ ID NO: 528 | CCAUCAGAUUUGUAGAUCU UAAC | SEQ ID NO: 804 | UAAGAUCUACAAAUCU GAUGG | 556-579 |
| SEQ ID NO: 529 | CCACAUCAGCUGCUAGACG GGUA | SEQ ID NO: 805 | CCCGUCUAGCAGCUGA UGUGG | 486-509 |
| SEQ ID NO: 530 | GCAUUCCCUUUGCAGUGUC AUAG | SEQ ID NO: 806 | AUGACACUGCAAAGGG AAUGC | 654-677 |
| SEQ ID NO: 531 | CUUUACCCACAUCAGCUGC UAGA | SEQ ID NO: 807 | UAGCAGCUGAUGUGGG UAAAG | 492-515 |
| SEQ ID NO: 532 | AUCCGGAAGCAUUCCCUUU GCAG | SEQ ID NO: 808 | GCAAAGGGAAUGCUUC CGGAU | 662-685 |
| SEQ ID NO: 533 | AGCUGCUAGACGGGUACGG GCAA | SEQ ID NO: 809 | GCCCGUACCCGUCUAG CAGCU | 479-502 |
| SEQ ID NO: 534 | CGGCUCGGUAGAUGAUAAU ACCC | SEQ ID NO: 810 | GUAUUAUCAUCUACCG AGCCG | 618-641 |
| SEQ ID NO: 535 | GGGUACGGGCAAAAUCAAG AGGG | SEQ ID NO: 811 | CUCUUGAUUUUGCCCG UACCC | 468-491 |
| SEQ ID NO: 536 | ACAUCAGCUGCUAGACGGG UACG | SEQ ID NO: 812 | UACCCGUCUAGCAGCU GAUGU | 484-507 |
| SEQ ID NO: 537 | CCCUUAAUCCCAUCAGAUU UGUA | SEQ ID NO: 813 | CAAAUCUGAUGGGAUU AAGGG | 565-588 |
| SEQ ID NO: 538 | CGGGCAAAAUCAAGAGGGU ACAC | SEQ ID NO: 814 | GUACCCUCUUGAUUUU GCCCG | 463-486 |
| SEQ ID NO: 539 | ACGUUAAAGCCUUGGUACA GGCC | SEQ ID NO: 815 | CCUGUACCAAGGCUUU AACGU | 586-609 |
| SEQ ID NO: 540 | UGGUACAGGCCCUUAAUCC CAUC | SEQ ID NO: 816 | UGGGAUUAAGGGCCUG UACCA | 574-597 |
| SEQ ID NO: 541 | GGCGGCUCGGUAGAUGAUA AUAC | SEQ ID NO: 817 | AUUAUCAUCUACCGAG CCGCC | 620-643 |
| SEQ ID NO: 542 | CGUUAAAGCCUUGGUACAG GCCC | SEQ ID NO: 818 | GCCUGUACCAAGGCUU UAACG | 585-608 |
| SEQ ID NO: 543 | GGUACAGGCCCUUAAUCCC AUCA | SEQ ID NO: 819 | AUGGGAUUAAGGGCCU GUACC | 573-596 |
| SEQ ID NO: 544 | UAAUACCCUGCACAGACAC GUUA | SEQ ID NO: 820 | ACGUGUCUGUGCAGGG UAUUA | 603-626 |
| SEQ ID NO: 545 | GACCACGCAGUCUAUAAUG CCUU | SEQ ID NO: 821 | GGCAUUAUAGACUGCG UGGUC | 227-250 |
| SEQ ID NO: 546 | GUUAAGAGCCUGGGUGGGG AAGU | SEQ ID NO: 822 | UUCCCCACCCAGGCUC UUAAC | 314-337 |
| SEQ ID NO: 547 | UGGGGAAGUAUCUGAUGAC AUUG | SEQ ID NO: 823 | AUGUCAUCAGAUACUU CCCCA | 300-323 |
| SEQ ID NO: 548 | GGUGGGGAAGUAUCUGAUG ACAU | SEQ ID NO: 824 | GUCAUCAGAUACUUCC CCACC | 302-325 |
| SEQ ID NO: 549 | GUGGGGAAGUAUCUGAUGA CAUU | SEQ ID NO: 825 | UGUCAUCAGAUACUUC CCCAC | 301-324 |
| SEQ ID NO: 550 | GGGGAAGUAUCUGAUGACA UUGG | SEQ ID NO: 826 | AAUGUCAUCAGAUACU UCCCC | 299-322 |
| SEQ ID NO: 551 | UUAAGAGCCUGGGUGGGA AGUA | SEQ ID NO: 827 | CUUCCCCACCCAGGCU CUUAA | 313-336 |
| SEQ ID NO: 552 | CCGAAGUAGGCGGCUCGGU AGAU | SEQ ID NO: 828 | CUACCGAGCCGCCUAC UUCGG | 628-651 |

In a particular embodiment, the invention relates to a nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO:304 | SEQ ID NO:580 |
| SEQ ID NO:323 | SEQ ID NO:599 |
| SEQ ID NO:439 | SEQ ID NO:715 |
| SEQ ID NO:453 | SEQ ID NO:729 |
| SEQ ID NO:496 | SEQ ID NO:772 |

In a particularly preferred embodiment, the invention relates to a nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO:304 | SEQ ID NO:580 |

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 17 contiguous nucleosides differing by 0 or 1 nucleosides from any one of SEQ ID NO:829-1104.

In certain embodiments, the first strand comprises nucleosides 2-18 of any one of the sequences set forth in SEQ ID NO: 829-1104.

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 21 contiguous nucleosides differing by 0 or 1 nucleosides from any one of SEQ ID NO: 829-1104.

In certain embodiments, the first strand comprises nucleosides 2-22 of any one of the sequences set forth in SEQ ID NO: 829-1104.

In certain embodiments, the first strand comprises any one of SEQ ID NO: 829-1104.

The modification pattern of the nucleic acids as set forth in SEQ ID NO: 829-1104 is summarized in Table 3 below:

Table 3 provides the modified first (antisense) sequences, together with the corresponding unmodified first (antisense) sequences for siRNA oligonucleosides according to the present invention as follows.

**Table 3**

| Antisense strand ID | Modified Antisense Strand 5' → 3' | SEQ ID NO (AS - mod) | Underlying Base Sequence 5' → 3' (Shown as an Unmodified Nucleotide Sequence) | SEQ ID NO (AS - unmod) |
|---|---|---|---|---|
| ETX-S00003198 | UmsUfsAmAmGmAfGmAmAmAmAmCm UmGfGmUfCmAfGmAmUmsGmsAm | SEQ ID NO: 856 | UUAAGAGAAAAC UGGUCAGAUGA | SEQ ID NO: 304 |
| ETX-S00003274 | UmsAfsUmCmAmGfGmAmGmUmGmAm CmUfGmAfAmAfUmAmAmsAmsAm | SEQ ID NO: 875 | UAUCAGGAGUGA CUGAAAUAAAA | SEQ ID NO: 323 |
| ETX-S00003738 | UmsCfsCmCmUmUfUmGmCmAmGmUm GmUfCmAfUmAfGmAmUmsAmsCm | SEQ ID NO: 991 | UCCCUUUGCAGU GUCAUAGAUAC | SEQ ID NO: 439 |
| ETX-S00003794 | AmsUfsCmAmGmAfUmUmUmGmUmAm GmAfUmCfUmUfAmAmCmsCmsAm | SEQ ID NO: 1005 | AUCAGAUUUGUA GAUCUUAACCA | SEQ ID NO: 453 |
| ETX-S00003966 | AmsUfsCmAmGmCfUmGmCmUmAmGm AmCfGmGfGmUfAmCmGmsGmsGm | SEQ ID NO: 1048 | AUCAGCUGCUAG ACGGGUACGGG | SEQ ID NO: 496 |
| ETX-S00003090 | CmsCfsCmAmUmUfUmUmCmAmAmCm CmAfGmUfAmAfAmUmAmsGmsUm | SEQ ID NO: 829 | CCCAUUUUCAAC CAGUAAAUAGU | SEQ ID NO: 277 |
| ETX-S00003094 | AmsCfsGmCmAmGfUmCmUmAmUmAm AmUfGmCfCmUfUmUmGmsUmsAm | SEQ ID NO: 830 | ACGCAGUCUAUA AUGCCUUUGUA | SEQ ID NO: 278 |
| ETX-S00003098 | GmsUfsGmUmAmCfUmUmCmUmUmGm AmUfUmUfCmAfUmCmAmsUmsAm | SEQ ID NO: 831 | GUGUACUUCUUG AUUUCAUCAUA | SEQ ID NO: 279 |
| ETX-S00003102 | AmsUfsAmCmAmAfUmUmGmAmGmUm CmCfCmAfUmCfAmUmCmsAmsUm | SEQ ID NO: 832 | AUACAAUUGAGU CCCAUCAUCAU | SEQ ID NO: 280 |
| ETX-S00003106 | GmsGfsAmCmCmAfCmGmCmAmGmUm CmUfAmUfAmAfUmGmCmsCmsUm | SEQ ID NO: 833 | GGACCACGCAGU CUAUAAUGCCU | SEQ ID NO: 281 |
| ETX-S00003110 | UmsUfsGmUmUmAfUmUmUmAmUmCm AmGfGmAfGmUfGmAmCmsUmsGm | SEQ ID NO: 834 | UUGUUAUUUAUC AGGAGUGACUG | SEQ ID NO: 282 |
| ETX-S00003114 | CmsAfsGmUmCmUfAmUmAmAmUmGm CmCfUmUfUmGfUmAmUmsUmsGm | SEQ ID NO: 835 | CAGUCUAUAAUG CCUUUGUAUUG | SEQ ID NO: 283 |
| ETX-S00003118 | AmsUfsAmGmAmUfAmCmCmGmAmAm GmUfAmGfGmCfGmGmCmsUmsCm | SEQ ID NO: 836 | AUAGAUACCGAA GUAGGCGGCUC | SEQ ID NO: 284 |
| ETX-S00003122 | UmsAfsGmAmUmAfCmCmGmAmAmGm UmAfGmGfCmGfGmCmUmsCmsGm | SEQ ID NO: 837 | UAGAUACCGAAG UAGGCGGCUCG | SEQ ID NO: 285 |
| ETX-S00003126 | GmsGfsAmAmUmAfCmGmGmAmCmCm AmCfGmCfAmGfUmCmUmsAmsUm | SEQ ID NO: 838 | GGAAUACGGACC ACGCAGUCUAU | SEQ ID NO: 286 |
| ETX-S00003130 | CmsAfsGmCmUmGfAmUmGmAmCmGm AmUfGmUfGmAfGmUmGmsUmsUm | SEQ ID NO: 839 | CAGCUGAUGACG AUGUGAGUGUU | SEQ ID NO: 287 |
| ETX-S00003134 | AmsUfsUmUmAmUfCmUmUmUmGmAm AmGfGmCfGmAfAmGmUmsUmsAm | SEQ ID NO: 840 | AUUUAUCUUUGA AGGCGAAGUUA | SEQ ID NO: 288 |
| ETX-S00003138 | GmsUfsGmUmCmAfUmAmGmAmUmAm CmCfGmAfAmGfUmAmGmsGmsCm | SEQ ID NO: 841 | GUGUCAUAGAUA CCGAAGUAGGC | SEQ ID NO: 289 |
| ETX-S00003142 | UmsUfsCmCmCmAfUmUmUmUmCmAm AmCfCmAfGmUfAmAmAmsUmsAm | SEQ ID NO: 842 | UUCCCAUUUUCA ACCAGUAAAUA | SEQ ID NO: 290 |
| ETX-S00003146 | GmsCfsUmGmCmUfAmGmAmCmGmGm GmUfAmCfGmGfGmCmAmsAmsAm | SEQ ID NO: 843 | GCUGCUAGACGG GUACGGGCAAA | SEQ ID NO: 291 |
| ETX-S00003150 | CmsAfsGmUmUmCfCmUmUmGmCm GmCfCmCfUmGfAmCmUmsGmsCm | SEQ ID NO: 844 | CAGUUCCUUUGC GCCCUGACUGC | SEQ ID NO: 292 |
| ETX-S00003154 | CmsUfsUmCmCmCfAmUmUmUmUmCm AmAfCmCfAmGfUmAmAmsAmsUm | SEQ ID NO: 845 | CUUCCCAUUUUC AACCAGUAAAU | SEQ ID NO: 293 |
| ETX-S00003158 | AmsGfsAmUmAmCfCmGmAmAmGmUm AmGfGmCfGmGfCmUmCmsGmsGm | SEQ ID NO: 846 | AGAUACCGAAGU AGGCGGCUCGG | SEQ ID NO: 294 |
| ETX-S00003162 | UmsUfsAmAmCmCfAmGmGmCmAmGm UmCfAmCfCmGfAmGmGmsCmsCm | SEQ ID NO: 847 | UUAACCAGGCAG UCACCGAGGCC | SEQ ID NO: 295 |
| ETX-S00003166 | AmsUfsCmAmUmCfCmAmGmCmUmGm AmUfGmAfCmGfAmUmGmsUmsGm | SEQ ID NO: 848 | AUCAUCCAGCUG AUGACGAUGUG | SEQ ID NO: 296 |
| ETX-S00003170 | AmsUfsUmUmAmUfCmAmGmGmAmGm UmGfAmCfUmGfAmAmAmsUmsAm | SEQ ID NO: 849 | AUUUAUCAGGAG UGACUGAAAUA | SEQ ID NO: 297 |
| ETX-S00003174 | GmsAfsCmAmGmUfCmUmGmUmGmCm GmAfUmCfAmUfCmCmAmsGmsCm | SEQ ID NO: 850 | GACAGUCUGUGC GAUCAUCCAGC | SEQ ID NO: 298 |
| ETX-S00003178 | UmsCfsUmAmUmAfAmUmGmCmCmUm UmUfGmUfAmUfUmGmCmsUmsUm | SEQ ID NO: 851 | UCUAUAAUGCCU UUGUAUUGCUU | SEQ ID NO: 299 |
| ETX-S00003182 | UmsAfsAmCmCmAfGmGmCmAmGmUm CmAfCmCfGmAfGmGmCmsCmsUm | SEQ ID NO: 852 | UAACCAGGCAGU CACCGAGGCCU | SEQ ID NO: 300 |
| ETX-S00003186 | CmsCfsCmUmGmCfAmCmAmGmAmCm AmCfGmUfUmAfAmAmGmsCmsCm | SEQ ID NO: 853 | CCCUGCACAGAC ACGUUAAAGCC | SEQ ID NO: 301 |
| ETX-S00003190 | CmsAfsCmGmCmAfGmUmCmUmAmUm AmAfUmGfCmCfUmUmUmsGmsUm | SEQ ID NO: 854 | CACGCAGUCUAU AAUGCCUUUGU | SEQ ID NO: 302 |
| ETX-S00003194 | UmsAfsAmUmAmCfAmAmCmAmUmGm CmCfUmGfUmUfCmAmCmsAmsGm | SEQ ID NO: 855 | UAAUACAACAUG CCUGUUCACAG | SEQ ID NO: 303 |
| ETX-S00003202 | UmsGfsCmAmCmAfGmAmCmAmCmGm UmUfAmAfAmGfCmCmUmsUmsGm | SEQ ID NO: 857 | UGCACAGACACG UUAAAGCCUUG | SEQ ID NO: 305 |
| ETX-S00003206 | GmsUfsUmCmCmUfUmUmGmCmGmCm CmCfUmGfAmCfUmGmCmsAmsUm | SEQ ID NO: 858 | GUUCCUUUGCGC CCUGACUGCAU | SEQ ID NO: 306 |
| ETX-S00003210 | GmsUfsGmAmCmAfGmUmCmUmGmUm GmCfGmAfUmCfAmUmCmsCmsAm | SEQ ID NO: 859 | GUGACAGUCUGU GCGAUCAUCCA | SEQ ID NO: 307 |
| ETX-S00003214 | AmsCfsCmAmCmGfCmAmGmUmCmUm AmUfAmAfUmGfCmCmUmsUmsUm | SEQ ID NO: 860 | ACCACGCAGUCU AUAAUGCCUUU | SEQ ID NO: 308 |
| ETX-S00003218 | CmsAfsGmUmGmAfCmAmGmUmCmUm GmUfGmCfGmAfUmCmAmsUmsCm | SEQ ID NO: 861 | CAGUGACAGUCU GUGCGAUCAUC | SEQ ID NO: 309 |
| ETX-S00003222 | UmsUfsGmGmGmAfAmUmAmCmGmGm AmCfCmAfCmGfCmAmGmsUmsCm | SEQ ID NO: 862 | UUGGGAAUACGG ACCACGCAGUC | SEQ ID NO: 310 |
| ETX-S00003226 | GmsUfsCmUmAmUfAmAmUmGmCmCm UmUfUmGfUmAfUmUmGmsCmsUm | SEQ ID NO: 863 | GUCUAUAAUGCC UUUGUAUUGCU | SEQ ID NO: 311 |
| ETX-S00003230 | UmsGfsUmGmCmGfAmUmCmAmUmCm CmAfGmCfUmGfAmUmGmsAmsCm | SEQ ID NO: 864 | UGUGCGAUCAUC CAGCUGAUGAC | SEQ ID NO: 312 |
| ETX-S00003234 | AmsCfsGmGmAmCfCmAmCmGmCmAm GmUfCmUfAmUfAmAmUmsGmsCm | SEQ ID NO: 865 | ACGGACCACGCA GUCUAUAAUGC | SEQ ID NO: 313 |
| ETX-S00003238 | UmsGfsCmUmAmGfAmCmGmGmGmUm AmCfGmGfGmCfAmAmAmsAmsUm | SEQ ID NO: 866 | UGCUAGACGGU ACGGGCAAAAU | SEQ ID NO: 314 |
| ETX-S00003242 | UmsCfsAmUmCmCfAmGmCmUmGmAm UmGfAmCfGmAfUmGmUmsGmsAm | SEQ ID NO: 867 | UCAUCCAGCUGA UGACGAUGUGA | SEQ ID NO: 315 |
| ETX-S00003246 | CmsGfsGmGmUmAfCmGmGmGmCmAm AmAfAmUfCmAfAmGmAmsGmsGm | SEQ ID NO: 868 | CGGGUACGGGCA AAAUCAAGAGG | SEQ ID NO: 316 |
| ETX-S00003250 | AmsAfsUmAmCmGfGmAmCmCmAmCm GmCfAmGfUmCfUmAmUmsAmsAm | SEQ ID NO: 869 | AAUACGGACCAC GCAGUCUAUAA | SEQ ID NO: 317 |
| ETX-S00003254 | UmsCfsAmUmAmGfAmUmAmCmCmGm AmAfGmUfAmGfGmCmGmsGmsCm | SEQ ID NO: 870 | UCAUAGAUACCG AAGUAGGCGGC | SEQ ID NO: 318 |
| ETX-S00003258 | GmsGfsGmAmAmUfAmCmGmGmAmCm CmAfCmGfCmAfGmUmCmsUmsAm | SEQ ID NO: 871 | GGGAAUACGGAC CACGCAGUCUA | SEQ ID NO: 319 |
| ETX-S00003262 | GmsUfsGmCmGmAfUmCmAmUmCmCm AmGfCmUfGmAfUmGmAmsCmsGm | SEQ ID NO: 872 | GUGCGAUCAUCC AGCUGAUGACG | SEQ ID NO: 320 |
| ETX-S00003266 | UmsAfsCmGmGmAfCmCmAmCmGmCm AmGfUmCfUmAfUmAmAmsUmsGm | SEQ ID NO: 873 | UACGGACCACGC AGUCUAUAAUG | SEQ ID NO: 321 |
| ETX-S00003270 | UmsGfsUmCmAmUfAmGmAmUmAmCm CmGfAmAfGmUfAmGmGmsCmsGm | SEQ ID NO: 874 | UGUCAUAGAUAC CGAAGUAGGCG | SEQ ID NO: 322 |
| ETX-S00003278 | UmsGfsCmGmAmUfCmAmUmCmCmAm GmCfUmGfAmUfGmAmCmsGmsAm | SEQ ID NO: 876 | UGCGAUCAUCCA GCUGAUGACGA | SEQ ID NO: 324 |
| ETX-S00003282 | CmsUfsAmGmAmCfGmGmGmUmAmCm GmGfGmCfAmAfAmAmUmsCmsAm | SEQ ID NO: 877 | CUAGACGGGUAC GGGCAAAAUCA | SEQ ID NO: 325 |
| ETX-S00003286 | GmsAfsUmCmCmGfGmAmAmGmCmAm UmUfCmCfCmUfUmUmGmsCmsAm | SEQ ID NO: 878 | GAUCCGGAAGCA UUCCCUUUGCA | SEQ ID NO: 326 |
| ETX-S00003290 | GmsCfsAmCmAmGfAmCmAmCmGmUm UmAfAmAfGmCfCmUmUmsGmsGm | SEQ ID NO: 879 | GCACAGACACGU UAAAGCCUUGG | SEQ ID NO: 327 |
| ETX-S00003294 | CmsCfsAmCmGmCfAmGmUmCmUmAm UmAfAmUfGmCfCmUmUmsUmsGm | SEQ ID NO: 880 | CCACGCAGUCUA UAAUGCCUUUG | SEQ ID NO: 328 |
| ETX-S00003298 | GmsAfsCmGmGmGfUmAmCmGmGmGm CmAfAmAfAmUfCmAmAmsGmsAm | SEQ ID NO: 881 | GACGGGUACGGG CAAAAUCAAGA | SEQ ID NO: 329 |
| ETX-S00003302 | GmsAfsUmAmCmCfGmAmAmGmUmAm GmGfCmGfGmCfUmCmGmsGmsUm | SEQ ID NO: 882 | GAUACCGAAGUA GGCGGCUCGGU | SEQ ID NO: 330 |
| ETX-S00003306 | GmsUfsAmGmGmCfGmGmCmUmCmGm GmUfAmGfAmUfGmAmUmsAmsAm | SEQ ID NO: 883 | GUAGGCGGCUCG GUAGAUGAUAA | SEQ ID NO: 331 |
| ETX-S00003310 | AmsGfsAmCmGmGfGmUmAmCmGmGm GmCfAmAfAmAfUmCmAmsAmsGm | SEQ ID NO: 884 | AGACGGUACGG GCAAAAUCAAG | SEQ ID NO: 332 |
| ETX-S00003314 | UmsAfsGmGmCmGfGmCmUmCmGmGm UmAfGmAfUmGfAmUmAmsAmsUm | SEQ ID NO: 885 | UAGGCGGCUCGG UAGAUGAUAAU | SEQ ID NO: 333 |
| ETX-S00003318 | AmsAfsGmUmAmGfGmCmGmGmCmUm CmGfGmUfAmGfAmUmGmsAmsUm | SEQ ID NO: 886 | AAGUAGGCGGCU CGGUAGAUGAU | SEQ ID NO: 334 |
| ETX-S00003322 | CmsUfsUmUmGmAfAmGmGmCmGmAm AmGfUmUfAmAfGmAmGmsCmsCm | SEQ ID NO: 887 | CUUUGAAGGCGA AGUUAAGAGCC | SEQ ID NO: 335 |
| ETX-S00003326 | CmsGfsAmAmGmUfUmAmAmGmAmGm CmCfUmGfGmGfUmGmGmsGmsGm | SEQ ID NO: 888 | CGAAGUUAAGAG CCUGGGUGGGG | SEQ ID NO: 336 |
| ETX-S00003330 | CmsCfsCmUmGmCfUmCmCmUmUmGm GmGfAmAfUmAfCmGmGmsAmsCm | SEQ ID NO: 889 | CCCUGCUCCUUG GGAAUACGGAC | SEQ ID NO: 337 |
| ETX-S00003334 | GmsAfsAmGmGmAfCmAmGmAmAmCm UmCfCmCfUmGfCmUmCmsCmsUm | SEQ ID NO: 890 | GAAGGACAGAAC UCCCUGCUCCU | SEQ ID NO: 338 |
| ETX-S00003338 | AmsGfsAmAmCmUfCmCmCmUmGmCm UmCfCmUfUmGfGmGmAmsAmsUm | SEQ ID NO: 891 | AGAACUCCCUGC UCCUUGGGAAU | SEQ ID NO: 339 |
| ETX-S00003342 | CmsCfsUmGmCmUfCmCmUmUmGmGm GmAfAmUfAmCfGmGmAmsCmsCm | SEQ ID NO: 892 | CCUGCUCCUUGG GAAUACGGACC | SEQ ID NO: 340 |
| ETX-S00003346 | CmsAfsGmAmAmGfGmAmCmAmGmAm AmCfUmCfCmCfUmGmCmsUmsCm | SEQ ID NO: 893 | CAGAAGGACAGA ACUCCCUGCUC | SEQ ID NO: 341 |
| ETX-S00003350 | CmsUfsGmCmUmCfCmUmUmGmGmGm AmAfUmAfCmGfGmAmCmsCmsAm | SEQ ID NO: 894 | CUGCUCCUUGGG AAUACGGACCA | SEQ ID NO: 342 |
| ETX-S00003354 | AmsGfsAmAmGmGfAmCmAmGmAmAm CmUfCmCfCmUfGmCmUmsCmsCm | SEQ ID NO: 895 | AGAAGGACAGAA CUCCCUGCUCC | SEQ ID NO: 343 |
| ETX-S00003358 | AmsAfsGmGmAmCfAmGmAmAmCmUm CmCfCmUfGmCfUmCmCmsUmsUm | SEQ ID NO: 896 | AAGGACAGAACU CCCUGCUCCUU | SEQ ID NO: 344 |
| ETX-S00003362 | GmsAfsAmCmUmCfCmCmUmGmCmUm CmCfUmUfGmGfGmAmAmsUmsAm | SEQ ID NO: 897 | GAACUCCCUGCU CCUUGGGAAUA | SEQ ID NO: 345 |
| ETX-S00003366 | AmsGfsGmAmCmAfGmAmAmCmUmCm CmCfUmGfCmUfCmCmUmsUmsGm | SEQ ID NO: 898 | AGGACAGAACUC CCUGCUCCUUG | SEQ ID NO: 346 |
| ETX-S00003370 | GmsAfsAmGmUmUfAmAmGmAmGmCm CmUfGmGfGmUfGmGmGmsGmsAm | SEQ ID NO: 899 | GAAGUUAAGAGC CUGGGUGGGGA | SEQ ID NO: 347 |
| ETX-S00003374 | UmsCfsUmUmUmGfAmAmGmGmCmGm AmAfGmUfUmAfAmGmAmsGmsCm | SEQ ID NO: 900 | UCUUUGAAGGCG AAGUUAAGAGC | SEQ ID NO: 348 |
| ETX-S00003378 | UmsAfsUmUmUmAfUmCmAmGmGmAm GmUfGmAfCmUfGmAmAmsAmsUm | SEQ ID NO: 901 | UAUUUAUCAGGA GUGACUGAAAU | SEQ ID NO: 349 |
| ETX-S00003382 | GmsUfsUmAmUmUfUmAmUmCmAmGm GmAfGmUfGmAfCmUmGmsAmsAm | SEQ ID NO: 902 | GUUAUUUAUCAG GAGUGACUGAA | SEQ ID NO: 350 |
| ETX-S00003386 | UmsUfsAmUmUmUfAmUmCmAmGmGm AmGfUmGfAmCfUmGmAmsAmsAm | SEQ ID NO: 903 | UUAUUUAUCAGG AGUGACUGAAA | SEQ ID NO: 351 |
| ETX-S00003390 | AmsAfsAmUmAmCfAmAmUmUmGmAm GmUfCmCfCmAfUmCmAmsUmsCm | SEQ ID NO: 904 | AAAUACAAUUGA GUCCCAUCAUC | SEQ ID NO: 352 |
| ETX-S00003394 | AmsAfsAmAmUmAfCmAmAmUmUmGm AmGfUmCfCmCfAmUmCmsAmsUm | SEQ ID NO: 905 | AAAAUACAAUUG AGUCCCAUCAU | SEQ ID NO: 353 |
| ETX-S00003398 | AmsUfsUmUmGmUfUmAmUmUmUmAm UmCfAmGfGmAfGmUmGmsAmsCm | SEQ ID NO: 906 | AUUUGUUAUUUA UCAGGAGUGAC | SEQ ID NO: 354 |
| ETX-S00003402 | GmsAfsGmAmAmAfAmCmUmGmGmUm CmAfGmAfUmGfAmAmUmsAmsUm | SEQ ID NO: 907 | GAGAAAACUGGU CAGAUGAAUAU | SEQ ID NO: 355 |
| ETX-S00003406 | AmsUfsAmUmAmAfUmAmCmAmAmCm AmUfGmCfCmUfGmUmUmsCmsAm | SEQ ID NO: 908 | AUAUAAUACAAC AUGCCUGUUCA | SEQ ID NO: 356 |
| ETX-S00003410 | UmsGfsGmGmAmUfCmCmGmGmAmAm GmCfAmUfUmCfCmCmUmsUmsUm | SEQ ID NO: 909 | UGGGAUCCGGAA GCAUUCCCUUU | SEQ ID NO: 357 |
| ETX-S00003414 | UmsUfsGmGmGmAfUmCmCmGmGmAm AmGfCmAfUmUfCmCmCmsUmsUm | SEQ ID NO: 910 | UUGGGAUCCGGA AGCAUUCCCUU | SEQ ID NO: 358 |
| ETX-S00003418 | CmsAfsGmGmCmAfGmUmCmAmCmCm GmAfGmGfCmCfUmCmGmsGmsAm | SEQ ID NO: 911 | CAGGCAGUCACC GAGGCCUCGGA | SEQ ID NO: 359 |
| ETX-S00003422 | UmsAfsUmAmUmAfAmUmAmCmAmAm CmAfUmGfCmCfUmGmUmsUmsCm | SEQ ID NO: 912 | UAUAUAAUACAA CAUGCCUGUUC | SEQ ID NO: 360 |
| ETX-S00003426 | AmsGfsUmCmUmAfUmAmAmUmGmCm CmUfUmUfGmUfAmUmUmsGmsCm | SEQ ID NO: 913 | AGUCUAUAAUGC CUUUGUAUUGC | SEQ ID NO: 361 |
| ETX-S00003430 | UmsUfsUmAmUmCfAmGmGmAmGmUm GmAfCmUfGmAfAmAmUmsAmsAm | SEQ ID NO: 914 | UUUAUCAGGAGU GACUGAAAUAA | SEQ ID NO: 362 |
| ETX-S00003434 | AmsAfsUmAmCmAfAmUmUmGmAmGm UmCfCmCfAmUfCmAmUmsCmsAm | SEQ ID NO: 915 | AAUACAAUUGAG UCCCAUCAUCA | SEQ ID NO: 363 |
| ETX-S00003438 | CmsUfsUmUmAmAfGmAmGmAmAmAm AmCfUmGfGmUfCmAmGmsAmsUm | SEQ ID NO: 916 | CUUUAAGAGAAA ACUGGUCAGAU | SEQ ID NO: 364 |
| ETX-S00003442 | GmsCfsUmUmUmAfAmGmAmGmAmAm AmAfCmUfGmGfUmCmAmsGmsAm | SEQ ID NO: 917 | GCUUUAAGAGAA AACUGGUCAGA | SEQ ID NO: 365 |
| ETX-S00003446 | AmsAfsUmGmCmCfUmUmUmGmUmAm UmUfGmCfUmUfAmUmCmsUmsGm | SEQ ID NO: 918 | AAUGCCUUUGUA UUGCUUAUCUG | SEQ ID NO: 366 |
| ETX-S00003450 | UmsAfsAmUmGmCfCmUmUmUmGmUm AmUfUmGfCmUfUmAmUmsCmsUm | SEQ ID NO: 919 | UAAUGCCUUUGU AUUGCUUAUCU | SEQ ID NO: 367 |
| ETX-S00003454 | UmsAfsCmCmCmUfGmCmAmCmAmGm AmCfAmCfGmUfUmAmAmsAmsGm | SEQ ID NO: 920 | UACCCUGCACAG ACACGUUAAAG | SEQ ID NO: 368 |
| ETX-S00003458 | AmsUfsAmCmCmCfUmGmCmAmCmAm GmAfCmAfCmGfUmUmAmsAmsAm | SEQ ID NO: 921 | AUACCCUGCACA GACACGUUAAA | SEQ ID NO: 369 |
| ETX-S00003462 | AmsUfsCmCmAmGfCmUmGmAmUmGm AmCfGmAfUmGfUmGmAmsGmsUm | SEQ ID NO: 922 | AUCCAGCUGAUG ACGAUGUGAGU | SEQ ID NO: 370 |
| ETX-S00003466 | UmsAfsCmAmAmUfUmGmAmGmUmCm CmCfAmUfCmAfUmCmAmsUmsCm | SEQ ID NO: 923 | UACAAUUGAGUC CCAUCAUCAUC | SEQ ID NO: 371 |
| ETX-S00003470 | UmsGfsGmGmAmAfUmAmCmGmGmAm CmCfAmCfGmCfAmGmUmsCmsUm | SEQ ID NO: 924 | UGGGAAUACGGA CCACGCAGUCU | SEQ ID NO: 372 |
| ETX-S00003474 | UmsAfsUmAmAmUfAmCmAmAmCmAm UmGfCmCfUmGfUmUmCmsAmsCm | SEQ ID NO: 925 | UAUAAUACAACA UGCCUGUUCAC | SEQ ID NO: 373 |
| ETX-S00003478 | UmsUfsAmUmCmAfGmGmAmGmUmGm AmCfUmGfAmAfAmUmAmsAmsAm | SEQ ID NO: 926 | UUAUCAGGAGUG ACUGAAAUAAA | SEQ ID NO: 374 |
| ETX-S00003482 | AmsUfsAmAmUmGfCmCmUmUmUmGm UmAfUmUfGmCfUmUmAmsUmsCm | SEQ ID NO: 927 | AUAAUGCCUUUG UAUUGCUUAUC | SEQ ID NO: 375 |
| ETX-S00003486 | CmsCfsAmGmCmUfGmAmUmGmAmCm GmAfUmGfUmGfAmGmUmsGmsUm | SEQ ID NO: 928 | CCAGCUGAUGAC GAUGUGAGUGU | SEQ ID NO: 376 |
| ETX-S00003490 | CmsAfsUmAmGmAfUmAmCmCmGmAm AmGfUmAfGmGfCmGmGmsCmsUm | SEQ ID NO: 929 | CAUAGAUACCGA AGUAGGCGGCU | SEQ ID NO: 377 |
| ETX-S00003494 | CmsAfsUmCmCmAfGmCmUmGmAmUm GmAfCmGfAmUfGmUmGmsAmsGm | SEQ ID NO: 930 | CAUCCAGCUGAU GACGAUGUGAG | SEQ ID NO: 378 |
| ETX-S00003498 | GmsUfsCmAmUmAfGmAmUmAmCmCm GmAfAmGfUmAfGmGmCmsGmsGm | SEQ ID NO: 931 | GUCAUAGAUACC GAAGUAGGCGG | SEQ ID NO: 379 |
| ETX-S00003502 | AmsCfsCmCmUmGfCmAmCmAmGmAm CmAfCmGfUmUfAmAmAmsGmsCm | SEQ ID NO: 932 | ACCCUGCACAGA CACGUUAAAGC | SEQ ID NO: 380 |
| ETX-S00003506 | UmsGfsCmAmUmCfAmUmCmAmUmGm CmGfGmCfGmGfCmGmAmsAmsCm | SEQ ID NO: 933 | UGCAUCAUCAUG CGGCGGCGAAC | SEQ ID NO: 381 |
| ETX-S00003510 | CmsGfsGmAmCmCfAmCmGmCmAmGm UmCfUmAfUmAfAmUmGmsCmsCm | SEQ ID NO: 934 | CGGACCACGCAG UCUAUAAUGCC | SEQ ID NO: 382 |
| ETX-S00003514 | CmsUfsUmGmGmGfAmAmUmAmCmGm GmAfCmCfAmCfGmCmAmsGmsUm | SEQ ID NO: 935 | CUUGGGAAUACG GACCACGCAGU | SEQ ID NO: 383 |
| ETX-S00003518 | GmsAfsAmGmUmAfGmGmCmGmGmCm UmCfGmGfUmAfGmAmUmsGmsAm | SEQ ID NO: 936 | GAAGUAGGCGGC UCGGUAGAUGA | SEQ ID NO: 384 |
| ETX-S00003522 | CmsUfsGmCmUmAfGmAmCmGmGmGm UmAfCmGfGmGfCmAmAmsAmsAm | SEQ ID NO: 937 | CUGCUAGACGGG UACGGGCAAAA | SEQ ID NO: 385 |
| ETX-S00003526 | UmsCfsCmAmGmCfUmGmAmUmGmAm CmGfAmUfGmUfGmAmGmsUmsGm | SEQ ID NO: 938 | UCCAGCUGAUGA CGAUGUGAGUG | SEQ ID NO: 386 |
| ETX-S00003530 | AmsUfsAmCmGmGfAmCmCmAmCmGm CmAfGmUfCmUfAmUmAmsAmsUm | SEQ ID NO: 939 | AUACGGACCACG CAGUCUAUAAU | SEQ ID NO: 387 |
| ETX-S00003534 | AmsAfsCmCmAmGfGmCmAmGmUmCm AmCfCmGfAmGfGmCmCmsUmsCm | SEQ ID NO: 940 | AACCAGGCAGUC ACCGAGGCCUC | SEQ ID NO: 388 |
| ETX-S00003538 | UmsGfsAmCmAmGfUmCmUmGmUmGm CmGfAmUfCmAfUmCmCmsAmsGm | SEQ ID NO: 941 | UGACAGUCUGUG CGAUCAUCCAG | SEQ ID NO: 389 |
| ETX-S00003542 | UmsAfsGmAmCmGfGmGmUmAmCmGm GmGfCmAfAmAfAmUmCmsAmsAm | SEQ ID NO: 942 | UAGACGGUACG GGCAAAAUCAA | SEQ ID NO: 390 |
| ETX-S00003546 | GmsCfsAmGmUmGfAmCmAmGmUmCm UmGfUmGfCmGfAmUmCmsAmsUm | SEQ ID NO: 943 | GCAGUGACAGUC UGUGCGAUCAU | SEQ ID NO: 391 |
| ETX-S00003550 | AmsGfsUmGmAmCfAmGmUmCmUmGm UmGfCmGfAmUfCmAmUmsCmsCm | SEQ ID NO: 944 | AGUGACAGUCUG UGCGAUCAUCC | SEQ ID NO: 392 |
| ETX-S00003554 | GmsCfsUmAmGmAfCmGmGmGmUmAm CmGfGmGfCmAfAmAmAmsUmsCm | SEQ ID NO: 945 | GCUAGACGGUA CGGGCAAAAUC | SEQ ID NO: 393 |
| ETX-S00003558 | AmsGfsUmAmGmGfCmGmGmCmUmCm GmGfUmAfGmAfUmGmAmsUmsAm | SEQ ID NO: 946 | AGUAGGCGGCUC GGUAGAUGAUA | SEQ ID NO: 394 |
| ETX-S00003562 | GmsAfsAmUmAmCfGmGmAmCmCmAm CmGfCmAfGmUfCmUmAmsUmsAm | SEQ ID NO: 947 | GAAUACGGACCA CGCAGUCUAUA | SEQ ID NO: 395 |
| ETX-S00003566 | CmsUfsUmUmGmUfAmUmUmGmCmUm UmAfUmCfUmGfCmAmGmsUmsGm | SEQ ID NO: 948 | CUUUGUAUUGCU UAUCUGCAGUG | SEQ ID NO: 396 |
| ETX-S00003570 | UmsUfsUmGmUmAfUmUmGmCmUmUm AmUfCmUfGmCfAmGmUmsGmsAm | SEQ ID NO: 949 | UUUGUAUUGCUU AUCUGCAGUGA | SEQ ID NO: 397 |
| ETX-S00003574 | UmsUfsGmUmAmUfUmGmCmUmUmAm UmCfUmGfCmAfGmUmGmsAmsUm | SEQ ID NO: 950 | UUGUAUUGCUUA UCUGCAGUGAU | SEQ ID NO: 398 |
| ETX-S00003578 | UmsAfsUmCmUmGfCmAmGmUmGmAm UmCfUmGfCmUfUmGmCmsUmsGm | SEQ ID NO: 951 | UAUCUGCAGUGA UCUGCUUGCUG | SEQ ID NO: 399 |
| ETX-S00003582 | GmsUfsAmUmUmGfCmUmUmAmUmCm UmGfCmAfGmUfGmAmUmsCmsUm | SEQ ID NO: 952 | GUAUUGCUUAUC UGCAGUGAUCU | SEQ ID NO: 400 |
| ETX-S00003586 | UmsGfsUmAmUmUfGmCmUmUmAmUm CmUfGmCfAmGfUmGmAmsUmsCm | SEQ ID NO: 953 | UGUAUUGCUUAU CUGCAGUGAUC | SEQ ID NO: 401 |
| ETX-S00003590 | UmsGfsCmUmUmAfUmCmUmGmCmAm GmUfGmAfUmCfUmGmCmsUmsUm | SEQ ID NO: 954 | UGCUUAUCUGCA GUGAUCUGCUU | SEQ ID NO: 402 |
| ETX-S00003594 | UmsUfsGmCmUmUfAmUmCmUmGmCm AmGfUmGfAmUfCmUmGmsCmsUm | SEQ ID NO: 955 | UUGCUUAUCUGC AGUGAUCUGCU | SEQ ID NO: 403 |
| ETX-S00003598 | AmsUfsCmUmGmCfAmGmUmGmAmUm CmUfGmCfUmUfGmCmUmsGmsGm | SEQ ID NO: 956 | AUCUGCAGUGAU CUGCUUGCUGG | SEQ ID NO: 404 |
| ETX-S00003602 | UmsAfsUmUmGmCfUmUmAmUmCmUm GmCfAmGfUmGfAmUmCmsUmsGm | SEQ ID NO: 957 | UAUUGCUUAUCU GCAGUGAUCUG | SEQ ID NO: 405 |
| ETX-S00003606 | UmsUfsCmUmCmCfAmAmAmUmUmUm GmUfUmAfUmUfUmAmUmsCmsAm | SEQ ID NO: 958 | UUCUCCAAAUUU GUUAUUUAUCA | SEQ ID NO: 406 |
| ETX-S00003610 | CmsAfsAmAmUmUfUmGmUmUmAmUm UmUfAmUfCmAfGmGmAmsGmsUm | SEQ ID NO: 959 | CAAAUUUGUUAU UUAUCAGGAGU | SEQ ID NO: 407 |
| ETX-S00003614 | GmsAfsGmGmCmCfUmCmGmGmAmAm UmUfCmCfCmUfUmUmCmsAmsGm | SEQ ID NO: 960 | GAGGCCUCGGAA UUCCCUUUCAG | SEQ ID NO: 408 |
| ETX-S00003618 | AmsGfsAmUmAmUfGmUmUmAmUmAm UmAfAmUfAmCfAmAmCmsAmsUm | SEQ ID NO: 961 | AGAUAUGUUAUA UAAUACAACAU | SEQ ID NO: 409 |
| ETX-S00003622 | GmsCfsAmGmUmCfAmCmCmGmAmGm GmCfCmUfCmGfGmAmAmsUmsUm | SEQ ID NO: 962 | GCAGUCACCGAG GCCUCGGAAUU | SEQ ID NO: 410 |
| ETX-S00003626 | CmsGfsAmGmGmCfCmUmCmGmGmAm AmUfUmCfCmCfUmUmUmsCmsAm | SEQ ID NO: 963 | CGAGGCCUCGGA AUUCCCUUUCA | SEQ ID NO: 411 |
| ETX-S00003630 | CmsAfsAmGmAmUfAmUmGmUmUmAm UmAfUmAfAmUfAmCmAmsAmsCm | SEQ ID NO: 964 | CAAGAUAUGUUA UAUAAUACAAC | SEQ ID NO: 412 |
| ETX-S00003634 | AmsGfsUmCmAmCfCmGmAmGmGmCm CmUfCmGfGmAfAmUmUmsCmsCm | SEQ ID NO: 965 | AGUCACCGAGGC CUCGGAAUUCC | SEQ ID NO: 413 |
| ETX-S00003638 | CmsAfsCmCmGmAfGmGmCmCmUmCm GmGfAmAfUmUfCmCmCmsUmsUm | SEQ ID NO: 966 | CACCGAGGCCUC GGAAUUCCCUU | SEQ ID NO: 414 |
| ETX-S00003642 | CmsCfsUmCmGmGfAmAmUmUmCmCm CmUfUmUfCmAfGmCmUmsCmsCm | SEQ ID NO: 967 | CCUCGGAAUUCC CUUUCAGCUCC | SEQ ID NO: 415 |
| ETX-S00003646 | UmsCfsGmGmAmAfUmUmCmCmCmUm UmUfCmAfGmCfUmCmCmsAmsGm | SEQ ID NO: 968 | UCGGAAUUCCCU UUCAGCUCCAG | SEQ ID NO: 416 |
| ETX-S00003650 | GmsGfsCmCmUmCfGmGmAmAmUmUm CmCfCmUfUmUfCmAmGmsCmsUm | SEQ ID NO: 969 | GGCCUCGGAAUU CCCUUUCAGCU | SEQ ID NO: 417 |
| ETX-S00003654 | CmsUfsCmGmGmAfAmUmUmCmCmCm UmUfUmCfAmGfCmUmCmsCmsAm | SEQ ID NO: 970 | CUCGGAAUUCCC UUUCAGCUCCA | SEQ ID NO: 418 |
| ETX-S00003658 | GmsCfsCmUmCmGfGmAmAmUmUmCm CmCfUmUfUmCfAmGmCmsUmsCm | SEQ ID NO: 971 | GCCUCGGAAUUC CCUUUCAGCUC | SEQ ID NO: 419 |
| ETX-S00003662 | AmsUfsGmUmUmAfUmAmUmAmAmUm AmCfAmAfCmAfUmGmCmsCmsUm | SEQ ID NO: 972 | AUGUUAUAUAAU ACAACAUGCCU | SEQ ID NO: 420 |
| ETX-S00003666 | AmsAfsGmAmUmAfUmGmUmUmAmUm AmUfAmAfUmAfCmAmAmsCmsAm | SEQ ID NO: 973 | AAGAUAUGUUAU AUAAUACAACA | SEQ ID NO: 421 |
| ETX-S00003670 | UmsCfsAmAmGmAfUmAmUmGmUmUm AmUfAmUfAmAfUmAmCmsAmsAm | SEQ ID NO: 974 | UCAAGAUAUGUU AUAUAAUACAA | SEQ ID NO: 422 |
| ETX-S00003674 | UmsGfsUmUmAmUfAmUmAmAmUmAm CmAfAmCfAmUfGmCmCmsUmsGm | SEQ ID NO: 975 | UGUUAUAUAAUA CAACAUGCCUG | SEQ ID NO: 423 |
| ETX-S00003678 | CmsCfsGmAmGmGfCmCmUmCmGmGm AmAfUmUfCmCfCmUmUmsUmsCm | SEQ ID NO: 976 | CCGAGGCCUCGG AAUUCCCUUUC | SEQ ID NO: 424 |
| ETX-S00003682 | GmsUfsCmAmCmCfGmAmGmGmCmCm UmCfGmGfAmAfUmUmCmsCmsCm | SEQ ID NO: 977 | GUCACCGAGGCC UCGGAAUUCCC | SEQ ID NO: 425 |
| ETX-S00003686 | AmsCfsCmGmAmGfGmCmCmUmCmGm GmAfAmUfUmCfCmCmUmsUmsUm | SEQ ID NO: 978 | ACCGAGGCCUCG GAAUUCCCUUU | SEQ ID NO: 426 |
| ETX-S00003690 | UmsCfsAmCmCmGfAmGmGmCmCmUm CmGfGmAfAmUfUmCmCmsCmsUm | SEQ ID NO: 979 | UCACCGAGGCCU CGGAAUUCCCU | SEQ ID NO: 427 |
| ETX-S00003694 | AmsUfsUmUmCmAfUmCmAmUmAmCm AmAfGmAfCmAfAmGmCmsAmsCm | SEQ ID NO: 980 | AUUUCAUCAUAC AAGACAAGCAC | SEQ ID NO: 428 |
| ETX-S00003698 | UmsGfsUmAmUmUfUmAmUmCmUmUm UmGfAmAfGmGfCmGmAmsAmsGm | SEQ ID NO: 981 | UGUAUUUAUCUU UGAAGGCGAAG | SEQ ID NO: 429 |
| ETX-S00003702 | AmsUfsUmUmGmUfAmGmAmUmCmUm UmAfAmCfCmAfGmGmCmsAmsGm | SEQ ID NO: 982 | AUUUGUAGAUCU UAACCAGGCAG | SEQ ID NO: 430 |
| ETX-S00003706 | UmsUfsCmCmCmUfUmUmGmCmAmGm UmGfUmCfAmUfAmGmAmsUmsAm | SEQ ID NO: 983 | UUCCCUUUGCAG UGUCAUAGAUA | SEQ ID NO: 431 |
| ETX-S00003710 | AmsUfsCmAmUmAfCmAmAmGmAmCm AmAfGmCfAmCfAmAmAmsAmsGm | SEQ ID NO: 984 | AUCAUACAAGAC AAGCACAAAAG | SEQ ID NO: 432 |
| ETX-S00003714 | UmsAfsGmAmUmCfUmUmAmAmCmCm AmGfGmCfAmGfUmCmAmsCmsCm | SEQ ID NO: 985 | UAGAUCUUAACC AGGCAGUCACC | SEQ ID NO: 433 |
| ETX-S00003718 | AmsAfsGmCmAmUfUmCmCmCmUmUm UmGfCmAfGmUfGmUmCmsAmsUm | SEQ ID NO: 986 | AAGCAUUCCCUU UGCAGUGUCAU | SEQ ID NO: 434 |
| ETX-S00003722 | UmsUfsUmGmCmAfGmUmGmUmCmAm UmAfGmAfUmAfCmCmGmsAmsAm | SEQ ID NO: 987 | UUUGCAGUGUCA UAGAUACCGAA | SEQ ID NO: 435 |
| ETX-S00003726 | AmsGfsAmGmAmAfAmAmCmUmGmGm UmCfAmGfAmUfGmAmAmsUmsAm | SEQ ID NO: 988 | AGAGAAAACUGG UCAGAUGAAUA | SEQ ID NO: 436 |
| ETX-S00003730 | UmsGfsCmAmGmUfGmUmCmAmUmAm GmAfUmAfCmCfGmAmAmsGmsUm | SEQ ID NO: 989 | UGCAGUGUCAUA GAUACCGAAGU | SEQ ID NO: 437 |
| ETX-S00003734 | UmsCfsAmGmAmUfUmUmGmUmAmGm AmUfCmUfUmAfAmCmCmsAmsGm | SEQ ID NO: 990 | UCAGAUUUGUAG AUCUUAACCAG | SEQ ID NO: 438 |
| ETX-S00003742 | GmsAfsAmCmAmUfUmGmGmAmCmCm AmUfGmCfAmCfCmCmUmsUmsGm | SEQ ID NO: 992 | GAACAUUGGACC AUGCACCCUUG | SEQ ID NO: 440 |
| ETX-S00003746 | AmsAfsUmCmCmCfAmUmCmAmGmAm UmUfUmGfUmAfGmAmUmsCmsUm | SEQ ID NO: 993 | AAUCCCAUCAGA UUUGUAGAUCU | SEQ ID NO: 441 |
| ETX-S00003750 | GmsCfsAmGmUmGfUmCmAmUmAmGm AmUfAmCfCmGfAmAmGmsUmsAm | SEQ ID NO: 994 | GCAGUGUCAUAG AUACCGAAGUA | SEQ ID NO: 442 |
| ETX-S00003754 | CmsAfsAmGmCmAfCmAmAmAmAmGm CmAfCmCfAmCfCmCmAmsUmsGm | SEQ ID NO: 995 | CAAGCACAAAAG CACCACCCAUG | SEQ ID NO: 443 |
| ETX-S00003758 | GmsCfsGmGmCmUfCmGmGmUmAmGm AmUfGmAfUmAfAmUmAmsCmsCm | SEQ ID NO: 996 | GCGGCUCGGUAG AUGAUAAUACC | SEQ ID NO: 444 |
| ETX-S00003762 | CmsUfsCmCmUmUfGmGmGmAmAmUm AmCfGmGfAmCfCmAmCmsGmsCm | SEQ ID NO: 997 | CUCCUUGGGAAU ACGGACCACGC | SEQ ID NO: 445 |
| ETX-S00003766 | AmsUfsUmCmCmCfUmUmUmGmCmAm GmUfGmUfCmAfUmAmGmsAmsUm | SEQ ID NO: 998 | AUUCCCUUUGCA GUGUCAUAGAU | SEQ ID NO: 446 |
| ETX-S00003770 | CmsAfsUmAmCmAfAmGmAmCmAmAm GmCfAmCfAmAfAmAmGmsCmsAm | SEQ ID NO: 999 | CAUACAAGACAA GCACAAAAGCA | SEQ ID NO: 447 |
| ETX-S00003774 | AmsGfsAmCmAmCfGmUmUmAmAmAm GmCfCmUfUmGfGmUmAmsCmsAm | SEQ ID NO: 1000 | AGACACGUUAAA GCCUUGGUACA | SEQ ID NO: 448 |
| ETX-S00003778 | UmsGfsAmGmAmAfCmAmUmUmGmGm AmCfCmAfUmGfCmAmCmsCmsCm | SEQ ID NO: 1001 | UGAGAACAUUGG ACCAUGCACCC | SEQ ID NO: 449 |
| ETX-S00003782 | GmsCfsUmUmGmUfAmUmUmUmAmUm CmUfUmUfGmAfAmGmGmsCmsGm | SEQ ID NO: 1002 | GCUUGUAUUUAU CUUUGAAGGCG | SEQ ID NO: 450 |
| ETX-S00003786 | GmsCfsUmCmGmGfUmAmGmAmUmGm AmUfAmAfUmAfCmCmCmsUmsGm | SEQ ID NO: 1003 | GCUCGGUAGAUG AUAAUACCCUG | SEQ ID NO: 451 |
| ETX-S00003790 | AmsGfsUmGmUmCfAmUmAmGmAmUm AmCfCmGfAmAfGmUmAmsGmsGm | SEQ ID NO: 1004 | AGUGUCAUAGAU ACCGAAGUAGG | SEQ ID NO: 452 |
| ETX-S00003798 | GmsGfsUmAmGmAfUmGmAmUmAmAm UmAfCmCfCmUfGmCmAmsCmsAm | SEQ ID NO: 1006 | GGUAGAUGAUAA UACCCUGCACA | SEQ ID NO: 454 |
| ETX-S00003802 | CmsAfsGmGmCmCfCmUmUmAmAmUm CmCfCmAfUmCfAmGmAmsUmsUm | SEQ ID NO: 1007 | CAGGCCCUUAAU CCCAUCAGAUU | SEQ ID NO: 455 |
| ETX-S00003806 | AmsGfsAmUmGmAfUmAmAmUmAmCm CmCfUmGfCmAfCmAmGmsAmsCm | SEQ ID NO: 1008 | AGAUGAUAAUAC CCUGCACAGAC | SEQ ID NO: 456 |
| ETX-S00003810 | UmsAfsCmAmGmGfCmCmCmUmUmAm AmUfCmCfCmAfUmCmAmsGmsAm | SEQ ID NO: 1009 | UACAGGCCCUUA AUCCCAUCAGA | SEQ ID NO: 457 |
| ETX-S00003814 | AmsCfsAmGmAmCfAmCmGmUmUmAm AmAfGmCfCmUfUmGmGmsUmsAm | SEQ ID NO: 1010 | ACAGACACGUUA AAGCCUUGGUA | SEQ ID NO: 458 |
| ETX-S00003818 | AmsGfsAmAmAmAfCmUmGmGmUmCm AmGfAmUfGmAfAmUmAmsUmsUm | SEQ ID NO: 1011 | AGAAAACUGGUC AGAUGAAUAUU | SEQ ID NO: 459 |
| ETX-S00003822 | UmsGfsUmAmGmAfUmCmUmUmAmAm CmCfAmGfGmCfAmGmUmsCmsAm | SEQ ID NO: 1012 | UGUAGAUCUUAA CCAGGCAGUCA | SEQ ID NO: 460 |
| ETX-S00003826 | UmsCfsAmAmAmUfGmGmAmUmAmGm GmAfAmGfUmCfAmAmCmsCmsCm | SEQ ID NO: 1013 | UCAAAUGGAUAG GAAGUCAACCC | SEQ ID NO: 461 |
| ETX-S00003830 | CmsAfsUmUmCmCfCmUmUmUmGmCm AmGfUmGfUmCfAmUmAmsGmsAm | SEQ ID NO: 1014 | CAUUCCCUUUGC AGUGUCAUAGA | SEQ ID NO: 462 |
| ETX-S00003834 | CmsAfsUmCmAmGfAmUmUmUmGmUm AmGfAmUfCmUfUmAmAmsCmsCm | SEQ ID NO: 1015 | CAUCAGAUUUGU AGAUCUUAACC | SEQ ID NO: 463 |
| ETX-S00003838 | CmsUfsCmGmGmUfAmGmAmUmGmAm UmAfAmUfAmCfCmCmUmsGmsCm | SEQ ID NO: 1016 | CUCGGUAGAUGA UAAUACCCUGC | SEQ ID NO: 464 |
| ETX-S00003842 | UmsUfsUmGmUmAfGmAmUmCmUmUm AmAfCmCfAmGfGmCmAmsGmsUm | SEQ ID NO: 1017 | UUUGUAGAUCUU AACCAGGCAGU | SEQ ID NO: 465 |
| ETX-S00003846 | AmsCfsAmGmGmCfCmCmUmUmAmAm UmCfCmCfAmUfCmAmGmsAmsUm | SEQ ID NO: 1018 | ACAGGCCCUUAA UCCCAUCAGAU | SEQ ID NO: 466 |
| ETX-S00003850 | CmsUfsUmUmGmCfAmGmUmGmUmCm AmUfAmGfAmUfAmCmCmsGmsAm | SEQ ID NO: 1019 | CUUUGCAGUGUC AUAGAUACCGA | SEQ ID NO: 467 |
| ETX-S00003854 | CmsAfsCmAmUmCfAmGmCmUmGmCm UmAfGmAfCmGfGmGmUmsAmsCm | SEQ ID NO: 1020 | CACAUCAGCUGC UAGACGGGUAC | SEQ ID NO: 468 |
| ETX-S00003858 | CmsCfsCmUmUmUfGmCmAmGmUmGm UmCfAmUfAmGfAmUmAmsCmsCm | SEQ ID NO: 1021 | CCCUUUGCAGUG UCAUAGAUACC | SEQ ID NO: 469 |
| ETX-S00003862 | CmsUfsCmCmAmGfCmUmUmUmAmCm CmCfAmCfAmUfCmAmGmsCmsUm | SEQ ID NO: 1022 | CUCCAGCUUUAC CCACAUCAGCU | SEQ ID NO: 470 |
| ETX-S00003866 | UmsUfsAmCmCmCfAmCmAmUmCmAm GmCfUmGfCmUfAmGmAmsCmsGm | SEQ ID NO: 1023 | UUACCCACAUCA GCUGCUAGACG | SEQ ID NO: 471 |
| ETX-S00003870 | UmsUfsAmAmAmGfCmCmUmUmGmGm UmAfCmAfGmGfCmCmCmsUmsUm | SEQ ID NO: 1024 | UUAAAGCCUUGG UACAGGCCCUU | SEQ ID NO: 472 |
| ETX-S00003874 | CmsAfsGmCmUmGfCmUmAmGmAmCm GmGfGmUfAmCfGmGmGmsCmsAm | SEQ ID NO: 1025 | CAGCUGCUAGAC GGGUACGGGCA | SEQ ID NO: 473 |
| ETX-S00003878 | CmsAfsGmCmUmUfUmAmCmCmCmAm CmAfUmCfAmGfCmUmGmsCmsUm | SEQ ID NO: 1026 | CAGCUUUACCCA CAUCAGCUGCU | SEQ ID NO: 474 |
| ETX-S00003882 | UmsCfsAmGmCmUfGmCmUmAmGmAm CmGfGmGfUmAfCmGmGmsGmsCm | SEQ ID NO: 1027 | UCAGCUGCUAGA CGGGUACGGGC | SEQ ID NO: 475 |
| ETX-S00003886 | UmsCfsCmAmGmCfUmUmUmAmCmCm CmAfCmAfUmCfAmGmCmsUmsGm | SEQ ID NO: 1028 | UCCAGCUUUACC CACAUCAGCUG | SEQ ID NO: 476 |
| ETX-S00003890 | GmsGfsCmUmCmGfGmUmAmGmAmUm GmAfUmAfAmUfAmCmCmsCmsUm | SEQ ID NO: 1029 | GGCUCGGUAGAU GAUAAUACCCU | SEQ ID NO: 477 |
| ETX-S00003894 | AmsGfsGmCmGmGfCmUmCmGmGmUm AmGfAmUfGmAfUmAmAmsUmsAm | SEQ ID NO: 1030 | AGGCGGCUCGGU AGAUGAUAAUA | SEQ ID NO: 478 |
| ETX-S00003898 | CmsAfsUmCmAmGfCmUmGmCmUmAm GmAfCmGfGmGfUmAmCmsGmsGm | SEQ ID NO: 1031 | CAUCAGCUGCUA GACGGGUACGG | SEQ ID NO: 479 |
| ETX-S00003902 | GmsUfsAmCmAmGfGmCmCmCmUmUm AmAfUmCfCmCfAmUmCmsAmsGm | SEQ ID NO: 1032 | GUACAGGCCCUU AAUCCCAUCAG | SEQ ID NO: 480 |
| ETX-S00003906 | AmsUfsGmAmUmAfAmUmAmCmCmCm UmGfCmAfCmAfGmAmCmsAmsCm | SEQ ID NO: 1033 | AUGAUAAUACCC UGCACAGACAC | SEQ ID NO: 481 |
| ETX-S00003910 | CmsCfsUmUmGmGfGmAmAmUmAmCm GmGfAmCfCmAfCmGmCmsAmsGm | SEQ ID NO: 1034 | CCUUGGGAAUAC GGACCACGCAG | SEQ ID NO: 482 |
| ETX-S00003914 | UmsCfsCmUmUmGfGmGmAmAmUmAm CmGfGmAfCmCfAmCmGmsCmsAm | SEQ ID NO: 1035 | UCCUUGGGAAUA CGGACCACGCA | SEQ ID NO: 483 |
| ETX-S00003918 | CmsAfsGmAmUmUfUmGmUmAmGmAm UmCfUmUfAmAfCmCmAmsGmsGm | SEQ ID NO: 1036 | CAGAUUUGUAGA UCUUAACCAGG | SEQ ID NO: 484 |
| ETX-S00003922 | UmsGfsUmAmCmUfUmCmUmUmGmAm UmUfUmCfAmUfCmAmUmsAmsCm | SEQ ID NO: 1037 | UGUACUUCUUGA UUUCAUCAUAC | SEQ ID NO: 485 |
| ETX-S00003926 | AmsUfsCmCmCmAfUmCmAmGmAmUm UmUfGmUfAmGfAmUmCmsUmsUm | SEQ ID NO: 1038 | AUCCCAUCAGAU UUGUAGAUCUU | SEQ ID NO: 486 |
| ETX-S00003930 | GmsUfsAmGmAmUfGmAmUmAmAmUm AmCfCmCfUmGfCmAmCmsAmsGm | SEQ ID NO: 1039 | GUAGAUGAUAAU ACCCUGCACAG | SEQ ID NO: 487 |
| ETX-S00003934 | GmsAfsCmAmCmGfUmUmAmAmAmGm CmCfUmUfGmGfUmAmCmsAmsGm | SEQ ID NO: 1040 | GACACGUUAAAG CCUUGGUACAG | SEQ ID NO: 488 |
| ETX-S00003938 | AmsGfsCmUmUmUfAmCmCmCmAmCm AmUfCmAfGmCfUmGmCmsUmsAm | SEQ ID NO: 1041 | AGCUUUACCCAC AUCAGCUGCUA | SEQ ID NO: 489 |
| ETX-S00003942 | AmsAfsGmCmAmCfAmAmAmAmGmCm AmCfCmAfCmCfCmAmUmsGmsCm | SEQ ID NO: 1042 | AAGCACAAAAGC ACCACCCAUGC | SEQ ID NO: 490 |
| ETX-S00003946 | AmsUfsCmUmUmAfAmCmCmAmGmGm CmAfGmUfCmAfCmCmGmsAmsGm | SEQ ID NO: 1043 | AUCUUAACCAGG CAGUCACCGAG | SEQ ID NO: 491 |
| ETX-S00003950 | UmsUfsUmAmCmCfCmAmCmAmUmCm AmGfCmUfGmCfUmAmGmsAmsCm | SEQ ID NO: 1044 | UUUACCCACAUC AGCUGCUAGAC | SEQ ID NO: 492 |
| ETX-S00003954 | CmsUfsUmAmAmUfCmCmCmAmUmCm AmGfAmUfUmUfGmUmAmsGmsAm | SEQ ID NO: 1045 | CUUAAUCCCAUC AGAUUUGUAGA | SEQ ID NO: 493 |
| ETX-S00003958 | CmsAfsGmUmGmUfCmAmUmAmGmAm UmAfCmCfGmAfAmGmUmsAmsGm | SEQ ID NO: 1046 | CAGUGUCAUAGA UACCGAAGUAG | SEQ ID NO: 494 |
| ETX-S00003962 | CmsCfsAmGmCmUfUmUmAmCmCmCm AmCfAmUfCmAfGmCmUmsGmsCm | SEQ ID NO: 1047 | CCAGCUUUACCC ACAUCAGCUGC | SEQ ID NO: 495 |
| ETX-S00003970 | GmsAfsUmGmAmUfAmAmUmAmCmCm CmUfGmCfAmCfAmGmAmsCmsAm | SEQ ID NO: 1049 | GAUGAUAAUACC CUGCACAGACA | SEQ ID NO: 497 |
| ETX-S00003974 | UmsUfsUmCmAmUfCmAmUmAmCmAm AmGfAmCfAmAfGmCmAmsCmsAm | SEQ ID NO: 1050 | UUUCAUCAUACA AGACAAGCACA | SEQ ID NO: 498 |
| ETX-S00003978 | CmsAfsGmAmCmAfCmGmUmUmAmAm AmGfCmCfUmUfGmGmUmsAmsCm | SEQ ID NO: 1051 | CAGACACGUUAA AGCCUUGGUAC | SEQ ID NO: 499 |
| ETX-S00003982 | AmsGfsAmAmCmAfUmUmGmGmAmCm CmAfUmGfCmAfCmCmCmsUmsUm | SEQ ID NO: 1052 | AGAACAUUGGAC CAUGCACCCUU | SEQ ID NO: 500 |
| ETX-S00003986 | GmsUfsAmGmAmUfCmUmUmAmAmCm CmAfGmGfCmAfGmUmCmsAmsCm | SEQ ID NO: 1053 | GUAGAUCUUAAC CAGGCAGUCAC | SEQ ID NO: 501 |
| ETX-S00003990 | AmsGfsCmUmCmCfAmGmCmUmUmUm AmCfCmCfAmCfAmUmCmsAmsGm | SEQ ID NO: 1054 | AGCUCCAGCUUU ACCCACAUCAG | SEQ ID NO: 502 |
| ETX-S00003994 | GmsCfsUmCmCmUfUmGmGmGmAmAm UmAfCmGfGmAfCmCmAmsCmsGm | SEQ ID NO: 1055 | GCUCCUUGGGAA UACGGACCACG | SEQ ID NO: 503 |
| ETX-S00003998 | GmsAfsAmGmCmAfUmUmCmCmCmUm UmUfGmCfAmGfUmGmUmsCmsAm | SEQ ID NO: 1056 | GAAGCAUUCCCU UUGCAGUGUCA | SEQ ID NO: 504 |
| ETX-S00004002 | CmsCfsUmUmGmGfUmAmCmAmGmGm CmCfCmUfUmAfAmUmCmsCmsCm | SEQ ID NO: 1057 | CCUUGGUACAGG CCCUUAAUCCC | SEQ ID NO: 505 |
| ETX-S00004006 | UmsAfsGmAmUmGfAmUmAmAmUmAm CmCfCmUfGmCfAmCmAmsGmsAm | SEQ ID NO: 1058 | UAGAUGAUAAUA CCCUGCACAGA | SEQ ID NO: 506 |
| ETX-S00004010 | AmsAfsUmAmCmCfCmUmGmCmAmCm AmGfAmCfAmCfGmUmUmsAmsAm | SEQ ID NO: 1059 | AAUACCCUGCAC AGACACGUUAA | SEQ ID NO: 507 |
| ETX-S00004014 | GmsUfsUmAmAmAfGmCmCmUmUmGm GmUfAmCfAmGfGmCmCmsCmsUm | SEQ ID NO: 1060 | GUUAAAGCCUUG GUACAGGCCCU | SEQ ID NO: 508 |
| ETX-S00004018 | AmsGfsCmAmUmUfCmCmCmUmUmUm GmCfAmGfUmGfUmCmAmsUmsAm | SEQ ID NO: 1061 | AGCAUUCCCUUU GCAGUGUCAUA | SEQ ID NO: 509 |
| ETX-S00004022 | UmsCfsUmUmAmAfCmCmAmGmGmCm AmGfUmCfAmCfCmGmAmsGmsGm | SEQ ID NO: 1062 | UCUUAACCAGGC AGUCACCGAGG | SEQ ID NO: 510 |
| ETX-S00004026 | AmsUfsAmCmAmAfGmAmCmAmAmGm CmAfCmAfAmAfAmGmCmsAmsCm | SEQ ID NO: 1063 | AUACAAGACAAG CACAAAAGCAC | SEQ ID NO: 511 |
| ETX-S00004030 | GmsAfsCmAmAmGfCmAmCmAmAmAm AmGfCmAfCmCfAmCmCmsCmsAm | SEQ ID NO: 1064 | GACAAGCACAAA AGCACCACCCA | SEQ ID NO: 512 |
| ETX-S00004034 | AmsCfsCmCmAmCfAmUmCmAmGmCm UmGfCmUfAmGfAmCmGmsGmsGm | SEQ ID NO: 1065 | ACCCACAUCAGC UGCUAGACGGG | SEQ ID NO: 513 |
| ETX-S00004038 | UmsAfsAmAmGmCfCmUmUmGmGmUm AmCfAmGfGmCfCmCmUmsUmsAm | SEQ ID NO: 1066 | UAAAGCCUUGGU ACAGGCCCUUA | SEQ ID NO: 514 |
| ETX-S00004042 | UmsUfsCmAmUmCfAmUmAmCmAmAm GmAfCmAfAmGfCmAmCmsAmsAm | SEQ ID NO: 1067 | UUCAUCAUACAA GACAAGCACAA | SEQ ID NO: 515 |
| ETX-S00004046 | GmsCfsCmCmUmUfAmAmUmCmCmCm AmUfCmAfGmAfUmUmUmsGmsUm | SEQ ID NO: 1068 | GCCCUUAAUCCC AUCAGAUUUGU | SEQ ID NO: 516 |
| ETX-S00004050 | UmsUfsAmAmUmCfCmCmAmUmCmAm GmAfUmUfUmGfUmAmGmsAmsUm | SEQ ID NO: 1069 | UUAAUCCCAUCA GAUUUGUAGAU | SEQ ID NO: 517 |
| ETX-S00004054 | UmsCfsGmGmUmAfGmAmUmGmAmUm AmAfUmAfCmCfCmUmGmsCmsAm | SEQ ID NO: 1070 | UCGGUAGAUGAU AAUACCCUGCA | SEQ ID NO: 518 |
| ETX-S00004058 | CmsAfsCmGmUmUfAmAmAmGmCmCm UmUfGmGfUmAfCmAmGmsGmsCm | SEQ ID NO: 1071 | CACGUUAAAGCC UUGGUACAGGC | SEQ ID NO: 519 |
| ETX-S00004062 | UmsCfsCmCmAmUfCmAmGmAmUmUm UmGfUmAfGmAfUmCmUmsUmsAm | SEQ ID NO: 1072 | UCCCAUCAGAUU UGUAGAUCUUA | SEQ ID NO: 520 |
| ETX-S00004066 | UmsCfsAmUmAmCfAmAmGmAmCmAm AmGfCmAfCmAfAmAmAmsGmsCm | SEQ ID NO: 1073 | UCAUACAAGACA AGCACAAAAGC | SEQ ID NO: 521 |
| ETX-S00004070 | CmsCfsUmUmAmAfUmCmCmCmAmUm CmAfGmAfUmUfUmGmUmsAmsGm | SEQ ID NO: 1074 | CCUUAAUCCCAU CAGAUUUGUAG | SEQ ID NO: 522 |
| ETX-S00004074 | AmsCfsAmUmUmGfGmAmCmCmAmUm GmCfAmCfCmCfUmUmGmsAmsAm | SEQ ID NO: 1075 | ACAUUGGACCAU GCACCCUUGAA | SEQ ID NO: 523 |
| ETX-S00004078 | CmsUfsUmGmGmUfAmCmAmGmGmCm CmCfUmUfAmAfUmCmCmsCmsAm | SEQ ID NO: 1076 | CUUGGUACAGGC CCUUAAUCCCA | SEQ ID NO: 524 |
| ETX-S00004082 | UmsUfsGmCmAmGfUmGmUmCmAmUm AmGfAmUfAmCfCmGmAmsAmsGm | SEQ ID NO: 1077 | UUGCAGUGUCAU AGAUACCGAAG | SEQ ID NO: 525 |
| ETX-S00004086 | CmsAfsCmAmGmAfCmAmCmGmUmUm AmAfAmGfCmCfUmUmGmsGmsUm | SEQ ID NO: 1078 | CACAGACACGUU AAAGCCUUGGU | SEQ ID NO: 526 |
| ETX-S00004090 | AmsGfsCmAmCmAfAmAmAmGmCmAm CmCfAmCfCmCfAmUmGmsCmsCm | SEQ ID NO: 1079 | AGCACAAAAGCA CCACCCAUGCC | SEQ ID NO: 527 |
| ETX-S00004094 | CmsCfsAmUmCmAfGmAmUmUmUmGm UmAfGmAfUmCfUmUmAmsAmsCm | SEQ ID NO: 1080 | CCAUCAGAUUUG UAGAUCUUAAC | SEQ ID NO: 528 |
| ETX-S00004098 | CmsCfsAmCmAmUfCmAmGmCmUmGm CmUfAmGfAmCfGmGmGmsUmsAm | SEQ ID NO: 1081 | CCACAUCAGCUG CUAGACGGGUA | SEQ ID NO: 529 |
| ETX-S00004102 | GmsCfsAmUmUmCfCmCmUmUmUmGm CmAfGmUfGmUfCmAmUmsAmsGm | SEQ ID NO: 1082 | GCAUUCCCUUUG CAGUGUCAUAG | SEQ ID NO: 530 |
| ETX-S00004106 | CmsUfsUmUmAmCfCmCmAmCmAmUm CmAfGmCfUmGfCmUmAmsGmsAm | SEQ ID NO: 1083 | CUUUACCCACAU CAGCUGCUAGA | SEQ ID NO: 531 |
| ETX-S00004110 | AmsUfsCmCmGmGfAmAmGmCmAmUm UmCfCmCfUmUfUmGmCmsAmsGm | SEQ ID NO: 1084 | AUCCGGAAGCAU UCCCUUUGCAG | SEQ ID NO: 532 |
| ETX-S00004114 | AmsGfsCmUmGmCfUmAmGmAmCmGm GmGfUmAfCmGfGmGmCmsAmsAm | SEQ ID NO: 1085 | AGCUGCUAGACG GGUACGGGCAA | SEQ ID NO: 533 |
| ETX-S00004118 | CmsGfsGmCmUmCfGmGmUmAmGmAm UmGfAmUfAmAfUmAmCmsCmsCm | SEQ ID NO: 1086 | CGGCUCGGUAGA UGAUAAUACCC | SEQ ID NO: 534 |
| ETX-S00004122 | GmsGfsGmUmAmCfGmGmGmCmAmAm AmAfUmCfAmAfGmAmGmsGmsGm | SEQ ID NO: 1087 | GGGUACGGGCAA AAUCAAGAGGG | SEQ ID NO: 535 |
| ETX-S00004126 | AmsCfsAmUmCmAfGmCmUmGmCmUm AmGfAmCfGmGfGmUmAmsCmsGm | SEQ ID NO: 1088 | ACAUCAGCUGCU AGACGGGUACG | SEQ ID NO: 536 |
| ETX-S00004130 | CmsCfsCmUmUmAfAmUmCmCmCmAm UmCfAmGfAmUfUmUmGmsUmsAm | SEQ ID NO: 1089 | CCCUUAAUCCCA UCAGAUUUGUA | SEQ ID NO: 537 |
| ETX-S00004134 | CmsGfsGmGmCmAfAmAmAmUmCmAm AmGfAmGfGmGfUmAmCmsAmsCm | SEQ ID NO: 1090 | CGGGCAAAAUCA AGAGGGUACAC | SEQ ID NO: 538 |
| ETX-S00004138 | AmsCfsGmUmUmAfAmAmGmCmCmUm UmGfGmUfAmCfAmGmGmsCmsCm | SEQ ID NO: 1091 | ACGUUAAAGCCU UGGUACAGGCC | SEQ ID NO: 539 |
| ETX-S00004142 | UmsGfsGmUmAmCfAmGmGmCmCmCm UmUfAmAfUmCfCmCmAmsUmsCm | SEQ ID NO: 1092 | UGGUACAGGCCC UUAAUCCCAUC | SEQ ID NO: 540 |
| ETX-S00004146 | GmsGfsCmGmGmCfUmCmGmGmUmAm GmAfUmGfAmUfAmAmUmsAmsCm | SEQ ID NO: 1093 | GGCGGCUCGGUA GAUGAUAAUAC | SEQ ID NO: 541 |
| ETX-S00004150 | CmsGfsUmUmAmAfAmGmCmCmUmUm GmGfUmAfCmAfGmGmCmsCmsCm | SEQ ID NO: 1094 | CGUUAAAGCCUU GGUACAGGCCC | SEQ ID NO: 542 |
| ETX-S00004154 | GmsGfsUmAmCmAfGmGmCmCmCmUm UmAfAmUfCmCfCmAmUmsCmsAm | SEQ ID NO: 1095 | GGUACAGGCCCU UAAUCCCAUCA | SEQ ID NO: 543 |
| ETX-S00004158 | UmsAfsAmUmAmCfCmCmUmGmCmAm CmAfGmAfCmAfCmGmUmsUmsAm | SEQ ID NO: 1096 | UAAUACCCUGCA CAGACACGUUA | SEQ ID NO: 544 |
| ETX-S00004162 | GmsAfsCmCmAmCfGmCmAmGmUmCm UmAfUmAfAmUfGmCmCmsUmsUm | SEQ ID NO: 1097 | GACCACGCAGUC UAUAAUGCCUU | SEQ ID NO: 545 |
| ETX-S00004166 | GmsUfsUmAmAmGfAmGmCmCmUmGm GmGfUmGfGmGfGmAmAmsGmsUm | SEQ ID NO: 1098 | GUUAAGAGCCUG GGUGGGGAAGU | SEQ ID NO: 546 |
| ETX-S00004170 | UmsGfsGmGmGmAfAmGmUmAmUmCm UmGfAmUfGmAfCmAmUmsUmsGm | SEQ ID NO: 1099 | UGGGGAAGUAUC UGAUGACAUUG | SEQ ID NO: 547 |
| ETX-S00004174 | GmsGfsUmGmGmGfGmAmAmGmUmAm UmCfUmGfAmUfGmAmCmsAmsUm | SEQ ID NO: 1100 | GGUGGGGAAGUA UCUGAUGACAU | SEQ ID NO: 548 |
| ETX-S00004178 | GmsUfsGmGmGmGfAmAmGmUmAmUm CmUfGmAfUmGfAmCmAmsUmsUm | SEQ ID NO: 1101 | GUGGGGAAGUAU CUGAUGACAUU | SEQ ID NO: 549 |
| ETX-S00004182 | GmsGfsGmGmAmAfGmUmAmUmCmUm GmAfUmGfAmCfAmUmUmsGmsGm | SEQ ID NO: 1102 | GGGGAAGUAUCU GAUGACAUUGG | SEQ ID NO: 550 |
| ETX-S00004186 | UmsUfsAmAmGmAfGmCmCmUmGmGm GmUfGmGfGmGfAmAmGmsUmsAm | SEQ ID NO: 1103 | UUAAGAGCCUGG GUGGGGAAGUA | SEQ ID NO: 551 |
| ETX-S00004190 | CmsCfsGmAmAmGfUmAmGmGmCmGm GmCfUmCfGmGfUmAmGmsAmsUm | SEQ ID NO: 1104 | CCGAAGUAGGCG GCUCGGUAGAU | SEQ ID NO: 552 |

In certain embodiments, the second strand comprises a nucleoside sequence of at least 17 contiguous nucleosides differing by 0 or 1 nucleosides from any one of SEQ ID NO: 1105-1380; wherein the second strand has a region of at least 85% complementarity over the 17 contiguous nucleosides to the first strand.

In certain embodiments, the second strand comprises a nucleoside sequence of at least 19 contiguous nucleosides differing by 0 or 1 nucleosides from any one of SEQ ID NO: 1105-1380; wherein the second strand has a region of at least 85% complementarity over the 19 contiguous nucleosides to the first strand.

In certain embodiments, the second strand comprises a nucleoside sequence of at least 21 contiguous nucleosides differing by 0 or 1 nucleosides from any one of SEQ ID NO: 1105-1380; wherein the second strand has a region of at least 85% complementarity over the 21 contiguous nucleosides to the first strand.

In certain embodiments, the second strand comprises any one of SEQ ID NO: 1105-1380.

The modification pattern of the nucleic acids as set forth in SEQ ID NO: 1105-1380 is summarized in Table 4 below.

Table 4 provides the modified second (sense) sequences, together with the corresponding unmodified second (sense) sequences for siRNA oligonucleosides according to the present invention as follows.

**Table 4**

| Sense strand ID | Modified Sense Strand | SEQ ID NO (SS - mod) | Underlying Base Sequence | SEQ ID NO (SS - unmod) |
|---|---|---|---|---|
| | 5' → 3' | | 5' → 3' | |
| | | | (Shown as an Unmodified Nucleotide Sequence) | |
| ETX-S00003197 | iaiaAmsUmsCmUmGmAmCmCmAfGfUfU mUmUmCmUmCmUmUmAmAm | SEQ ID NO: 1132 | AUCUGACCAGUUU UCUCUUAA | SEQ ID NO: 580 |
| ETX-S00003273 | iaiaUmsUmsAmUmUmUmCmAmGfUfCfA mCmUmCmCmUmGmAmUmAm | SEQ ID NO: 1151 | UUAUUUCAGUCAC UCCUGAUA | SEQ ID NO: 599 |
| ETX-S00003737 | iaiaAmsUmsCmUmAmUmGmAmCfAfCfU mGmCmAmAmAmGmGmGmAm | SEQ ID NO: 1267 | AUCUAUGACACUG CAAAGGGA | SEQ ID NO: 715 |
| ETX-S00003793 | iaiaGmsUmsUmAmAmGmAmUmCfUfAfC mAmAmAmUmCmUmGmAmUm | SEQ ID NO: 1281 | GUUAAGAUCUACA AAUCUGAU | SEQ ID NO: 729 |
| ETX-S00003965 | iaiaCmsGmsUmAmCmCmCmGmUfCfUfA mGmCmAmGmCmUmGmAmUm | SEQ ID NO: 1324 | CGUACCCGUCUAG CAGCUGAU | SEQ ID NO: 772 |
| ETX-S00003089 | iaiaUmsAmsUmUmUmAmCmUmGfGfUfU mGmAmAmAmAmUmGmGmGm | SEQ ID NO: 1105 | UAUUUACUGGUUG AAAAUGGG | SEQ ID NO: 553 |
| ETX-S00003093 | iaiaCmsAmsAmAmGmGmCmAmUfUfAfU mAmGmAmCmUmGmCmGmUm | SEQ ID NO: 1106 | CAAAGGCAUUAUA GACUGCGU | SEQ ID NO: 554 |
| ETX-S00003097 | iaiaUmsGmsAmUmGmAmAmAmUfCfAfA mGmAmAmGmUmAmCmAmCm | SEQ ID NO: 1107 | UGAUGAAAUCAAG AAGUACAC | SEQ ID NO: 555 |
| ETX-S00003101 | iaiaGmsAmsUmGmAmUmGmGmGfAfCfU mCmAmAmUmUmGmUmAmUm | SEQ ID NO: 1108 | GAUGAUGGGACUC AAUUGUAU | SEQ ID NO: 556 |
| ETX-S00003105 | iaiaGmsCmsAmUmUmAmUmAmGfAfCfU mGmCmGmUmGmGmUmCmCm | SEQ ID NO: 1109 | GCAUUAUAGACUG CGUGGUCC | SEQ ID NO: 557 |
| ETX-S00003109 | iaiaGmsUmsCmAmCmUmCmCmUfGfAfU mAmAmAmUmAmAmCmAmAm | SEQ ID NO: 1110 | GUCACUCCUGAUA AAUAACAA | SEQ ID NO: 558 |
| ETX-S00003113 | iaiaAmsUmsAmCmAmAmAmGmGfCfAfU mUmAmUmAmGmAmCmUmGm | SEQ ID NO: 1111 | AUACAAAGGCAUU AUAGACUG | SEQ ID NO: 559 |
| ETX-S00003117 | iaiaGmsCmsCmGmCmCmUmAmCfUfUfC mGmGmUmAmUmCmUmAmUm | SEQ ID NO: 1112 | GCCGCCUACUUCG GUAUCUAU | SEQ ID NO: 560 |
| ETX-S00003121 | iaiaAmsGmsCmCmGmCmCmUmAfCfUfU mCmGmGmUmAmUmCmUmAm | SEQ ID NO: 1113 | AGCCGCCUACUUC GGUAUCUA | SEQ ID NO: 561 |
| ETX-S00003125 | iaiaAmsGmsAmCmUmGmCmGmUfGfGfU mCmCmGmUmAmUmUmCmCm | SEQ ID NO: 1114 | AGACUGCGUGGUC CGUAUUCC | SEQ ID NO: 562 |
| ETX-S00003129 | iaiaCmsAmsCmUmCmAmCmAmUfCfGfU mCmAmUmCmAmGmCmUmGm | SEQ ID NO: 1115 | CACUCACAUCGUC AUCAGCUG | SEQ ID NO: 563 |
| ETX-S00003133 | iaiaAmsCmsUmUmCmGmCmCmUfUfCfA mAmAmGmAmUmAmAmAmUm | SEQ ID NO: 1116 | ACUUCGCCUUCAA AGAUAAAU | SEQ ID NO: 564 |
| ETX-S00003137 | iaiaCmsUmsAmCmUmUmCmGmGfUfAfU mCmUmAmUmGmAmCmAmCm | SEQ ID NO: 1117 | CUACUUCGGUAUC UAUGACAC | SEQ ID NO: 565 |
| ETX-S00003141 | iaiaUmsUmsUmAmCmUmGmGmUfUfGfA mAmAmAmUmGmGmGmAmAm | SEQ ID NO: 1118 | UUUACUGGUUGAA AAUGGGAA | SEQ ID NO: 566 |
| ETX-S00003145 | iaiaUmsGmsCmCmCmGmUmAmCfCfCfG mUmCmUmAmGmCmAmGmCm | SEQ ID NO: 1119 | UGCCCGUACCCGU CUAGCAGC | SEQ ID NO: 567 |
| ETX-S00003149 | iaiaAmsGmsUmCmAmGmGmGmCfGfCfA mAmAmGmGmAmAmCmUmGm | SEQ ID NO: 1120 | AGUCAGGGCGCAA AGGAACUG | SEQ ID NO: 568 |
| ETX-S00003153 | iaiaUmsUmsAmCmUmGmGmUmUfGfAfA mAmAmUmGmGmGmAmAmGm | SEQ ID NO: 1121 | UUACUGGUUGAAA AUGGGAAG | SEQ ID NO: 569 |
| ETX-S00003157 | iaiaGmsAmsGmCmCmGmCmCmUfAfCfU mUmCmGmGmUmAmUmCmUm | SEQ ID NO: 1122 | GAGCCGCCUACUU CGGUAUCU | SEQ ID NO: 570 |
| ETX-S00003161 | iaiaCmsCmsUmCmGmGmUmGmAfCfUfG mCmCmUmGmGmUmUmAmAm | SEQ ID NO: 1123 | CCUCGGUGACUGC CUGGUUAA | SEQ ID NO: 571 |
| ETX-S00003165 | iaiaCmsAmsUmCmGmUmCmAmUfCfAfG mCmUmGmGmAmUmGmAmUm | SEQ ID NO: 1124 | CAUCGUCAUCAGC UGGAUGAU | SEQ ID NO: 572 |
| ETX-S00003169 | iaiaUmsUmsUmCmAmGmUmCmAfCfUfC mCmUmGmAmUmAmAmAmUm | SEQ ID NO: 1125 | UUUCAGUCACUCC UGAUAAAU | SEQ ID NO: 573 |
| ETX-S00003173 | iaiaUmsGmsGmAmUmGmAmUmCfGfCfA mCmAmGmAmCmUmGmUmCm | SEQ ID NO: 1126 | UGGAUGAUCGCAC AGACUGUC | SEQ ID NO: 574 |
| ETX-S00003177 | iaiaGmsCmsAmAmUmAmCmAmAfAfGfG mCmAmUmUmAmUmAmGmAm | SEQ ID NO: 1127 | GCAAUACAAAGGC AUUAUAGA | SEQ ID NO: 575 |
| ETX-S00003181 | iaiaGmsCmsCmUmCmGmGmUmGfAfCfU mGmCmCmUmGmGmUmUmAm | SEQ ID NO: 1128 | GCCUCGGUGACUG CCUGGUUA | SEQ ID NO: 576 |
| ETX-S00003185 | iaiaCmsUmsUmUmAmAmCmGmUfGfUfC mUmGmUmGmCmAmGmGmGm | SEQ ID NO: 1129 | CUUUAACGUGUCU GUGCAGGG | SEQ ID NO: 577 |
| ETX-S00003189 | iaiaAmsAmsAmGmGmCmAmUmUfAfUfA mGmAmCmUmGmCmGmUmGm | SEQ ID NO: 1130 | AAAGGCAUUAUAG ACUGCGUG | SEQ ID NO: 578 |
| ETX-S00003193 | iaiaGmsUmsGmAmAmCmAmGmGfCfAfU mGmUmUmGmUmAmUmUmAm | SEQ ID NO: 1131 | GUGAACAGGCAUG UUGUAUUA | SEQ ID NO: 579 |
| ETX-S00003201 | iaiaAmsGmsGmCmUmUmUmAmAfCfGfU mGmUmCmUmGmUmGmCmAm | SEQ ID NO: 1133 | AGGCUUUAACGUG UCUGUGCA | SEQ ID NO: 581 |
| ETX-S00003205 | iaiaGmsCmsAmGmUmCmAmGmGfGfCfG mCmAmAmAmGmGmAmAmCm | SEQ ID NO: 1134 | GCAGUCAGGGCGC AAAGGAAC | SEQ ID NO: 582 |
| ETX-S00003209 | iaiaGmsAmsUmGmAmUmCmGmCfAfCfA mGmAmCmUmGmUmCmAmCm | SEQ ID NO: 1135 | GAUGAUCGCACAG ACUGUCAC | SEQ ID NO: 583 |
| ETX-S00003213 | iaiaAmsGmsGmCmAmUmUmAmUfAfGfA mCmUmGmCmGmUmGmGmUm | SEQ ID NO: 1136 | AGGCAUUAUAGAC UGCGUGGU | SEQ ID NO: 584 |
| ETX-S00003217 | iaiaUmsGmsAmUmCmGmCmAmCfAfGfA mCmUmGmUmCmAmCmUmGm | SEQ ID NO: 1137 | UGAUCGCACAGAC UGUCACUG | SEQ ID NO: 585 |
| ETX-S00003221 | iaiaCmsUmsGmCmGmUmGmGmUfCfCfG mUmAmUmUmCmCmCmAmAm | SEQ ID NO: 1138 | CUGCGUGGUCCGU AUUCCCAA | SEQ ID NO: 586 |
| ETX-S00003225 | iaiaCmsAmsAmUmAmCmAmAmAfGfGfC mAmUmUmAmUmAmGmAmCm | SEQ ID NO: 1139 | CAAUACAAAGGCA UUAUAGAC | SEQ ID NO: 587 |
| ETX-S00003229 | iaiaCmsAmsUmCmAmGmCmUmGfGfAfU mGmAmUmCmGmCmAmCmAm | SEQ ID NO: 1140 | CAUCAGCUGGAUG AUCGCACA | SEQ ID NO: 588 |
| ETX-S00003233 | iaiaAmsUmsUmAmUmAmGmAmCfUfGfC mGmUmGmGmUmCmCmGmUm | SEQ ID NO: 1141 | AUUAUAGACUGCG UGGUCCGU | SEQ ID NO: 589 |
| ETX-S00003237 | iaiaUmsUmsUmGmCmCmCmGmUfAfCfC mCmGmUmCmUmAmGmCmAm | SEQ ID NO: 1142 | UUUGCCCGUACCC GUCUAGCA | SEQ ID NO: 590 |
| ETX-S00003241 | iaiaAmsCmsAmUmCmGmUmCmAfUfCfA mGmCmUmGmGmAmUmGmAm | SEQ ID NO: 1143 | ACAUCGUCAUCAG CUGGAUGA | SEQ ID NO: 591 |
| ETX-S00003245 | iaiaUmsCmsUmUmGmAmUmUmUfUfGfC mCmCmGmUmAmCmCmCmGm | SEQ ID NO: 1144 | UCUUGAUUUGCC CGUACCCG | SEQ ID NO: 592 |
| ETX-S00003249 | iaiaAmsUmsAmGmAmCmUmGmCfGfUfG mGmUmCmCmGmUmAmUmUm | SEQ ID NO: 1145 | AUAGACUGCGUGG UCCGUAUU | SEQ ID NO: 593 |
| ETX-S00003253 | iaiaCmsGmsCmCmUmAmCmUmUfCfGfG mUmAmUmCmUmAmUmGmAm | SEQ ID NO: 1146 | CGCCUACUUCGGU AUCUAUGA | SEQ ID NO: 594 |
| ETX-S00003257 | iaiaGmsAmsCmUmGmCmGmUmGfGfUfC mCmGmUmAmUmUmCmCmCm | SEQ ID NO: 1147 | GACUGCGUGGUCC GUAUUCCC | SEQ ID NO: 595 |
| ETX-S00003261 | iaiaUmsCmsAmUmCmAmGmCmUfGfGfA mUmGmAmUmCmGmCmAmCm | SEQ ID NO: 1148 | UCAUCAGCUGGAU GAUCGCAC | SEQ ID NO: 596 |
| ETX-S00003265 | iaiaUmsUmsAmUmAmGmAmCmUfGfCfG mUmGmGmUmCmCmGmUmAm | SEQ ID NO: 1149 | UUAUAGACUGCGU GGUCCGUA | SEQ ID NO: 597 |
| ETX-S00003269 | iaiaCmsCmsUmAmCmUmUmCmGfGfUfA mUmCmUmAmUmGmAmCmAm | SEQ ID NO: 1150 | CCUACUUCGGUAU CUAUGACA | SEQ ID NO: 598 |
| ETX-S00003277 | iaiaGmsUmsCmAmUmCmAmGmCfUfGfG mAmUmGmAmUmCmGmCmAm | SEQ ID NO: 1152 | GUCAUCAGCUGGA UGAUCGCA | SEQ ID NO: 600 |
| ETX-S00003281 | iaiaAmsUmsUmUmUmGmCmCmCfGfUfA mCmCmCmGmUmCmUmAmGm | SEQ ID NO: 1153 | AUUUUGCCCGUAC CCGUCUAG | SEQ ID NO: 601 |
| ETX-S00003285 | iaiaCmsAmsAmAmGmGmGmAmAfUfGfC mUmUmCmCmGmGmAmUmCm | SEQ ID NO: 1154 | CAAAGGGAAUGCU UCCGGAUC | SEQ ID NO: 602 |
| ETX-S00003289 | iaiaAmsAmsGmGmCmUmUmUmAfAfCfG mUmGmUmCmUmGmUmGmCm | SEQ ID NO: 1155 | AAGGCUUUAACGU GUCUGUGC | SEQ ID NO: 603 |
| ETX-S00003293 | iaiaAmsAmsGmGmCmAmUmUmAfUfAfG mAmCmUmGmCmGmUmGmGm | SEQ ID NO: 1156 | AAGGCAUUAUAGA CUGCGUGG | SEQ ID NO: 604 |
| ETX-S00003297 | iaiaUmsUmsGmAmUmUmUmUmGfCfCfC mGmUmAmCmCmCmGmUmCm | SEQ ID NO: 1157 | UUGAUUUGCCCG UACCCGUC | SEQ ID NO: 605 |
| ETX-S00003301 | iaiaCmsGmsAmGmCmCmGmCmCfUfAfC mUmUmCmGmGmUmAmUmCm | SEQ ID NO: 1158 | CGAGCCGCCUACU UCGGUAUC | SEQ ID NO: 606 |
| ETX-S00003305 | iaiaAmsUmsCmAmUmCmUmAmCfCfGfA mGmCmCmGmCmCmUmAmCm | SEQ ID NO: 1159 | AUCAUCUACCGAG CCGCCUAC | SEQ ID NO: 607 |
| ETX-S00003309 | iaiaUmsGmsAmUmUmUmUmGmCfCfCfG mUmAmCmCmCmGmUmCmUm | SEQ ID NO: 1160 | UGAUUUGCCCGU ACCCGUCU | SEQ ID NO: 608 |
| ETX-S00003313 | iaiaUmsAmsUmCmAmUmCmUmAfCfCfG mAmGmCmCmGmCmCmUmAm | SEQ ID NO: 1161 | UAUCAUCUACCGA GCCGCCUA | SEQ ID NO: 609 |
| ETX-S00003317 | iaiaCmsAmsUmCmUmAmCmCmGfAfGfC mCmGmCmCmUmAmCmUmUm | SEQ ID NO: 1162 | CAUCUACCGAGCC GCCUACUU | SEQ ID NO: 610 |
| ETX-S00003321 | iaiaCmsUmsCmUmUmAmAmCmUfUfCfG mCmCmUmUmCmAmAmAmGm | SEQ ID NO: 1163 | CUCUUAACUUCGC CUUCAAAG | SEQ ID NO: 611 |
| ETX-S00003325 | iaiaCmsCmsAmCmCmCmAmGmGfCfUfC mUmUmAmAmCmUmUmCmGm | SEQ ID NO: 1164 | CCACCCAGGCUCU UAACUUCG | SEQ ID NO: 612 |
| ETX-S00003329 | iaiaCmsCmsGmUmAmUmUmCmCfCfAfA mGmGmAmGmCmAmGmGmGm | SEQ ID NO: 1165 | CCGUAUUCCCAAG GAGCAGGG | SEQ ID NO: 613 |
| ETX-S00003333 | iaiaGmsAmsGmCmAmGmGmGmAfGfUfU mCmUmGmUmCmCmUmUmCm | SEQ ID NO: 1166 | GAGCAGGGAGUUC UGUCCUUC | SEQ ID NO: 614 |
| ETX-S00003337 | iaiaUmsCmsCmCmAmAmGmGmAfGfCfA mGmGmGmAmGmUmUmCmUm | SEQ ID NO: 1167 | UCCCAAGGAGCAG GGAGUUCU | SEQ ID NO: 615 |
| ETX-S00003341 | iaiaUmsCmsCmGmUmAmUmUmCfCfCfA mAmGmGmAmGmCmAmGmGm | SEQ ID NO: 1168 | UCCGUAUUCCCAA GGAGCAGG | SEQ ID NO: 616 |
| ETX-S00003345 | iaiaGmsCmsAmGmGmGmAmGmUfUfCfU mGmUmCmCmUmUmCmUmGm | SEQ ID NO: 1169 | GCAGGGAGUUCUG UCCUUCUG | SEQ ID NO: 617 |
| ETX-S00003349 | iaiaGmsUmsCmCmGmUmAmUmUfCfCfC mAmAmGmGmAmGmCmAmGm | SEQ ID NO: 1170 | GUCCGUAUUCCCA AGGAGCAG | SEQ ID NO: 618 |
| ETX-S00003353 | iaiaAmsGmsCmAmGmGmGmAmGfUfUfC mUmGmUmCmCmUmUmCmUm | SEQ ID NO: 1171 | AGCAGGGAGUUCU GUCCUUCU | SEQ ID NO: 619 |
| ETX-S00003357 | iaiaGmsGmsAmGmCmAmGmGmGfAfGfU mUmCmUmGmUmCmCmUmUm | SEQ ID NO: 1172 | GGAGCAGGGAGUU CUGUCCUU | SEQ ID NO: 620 |
| ETX-S00003361 | iaiaUmsUmsCmCmCmAmAmGmGfAfGfC mAmGmGmGmAmGmUmUmCm | SEQ ID NO: 1173 | UUCCCAAGGAGCA GGGAGUUC | SEQ ID NO: 621 |
| ETX-S00003365 | iaiaAmsGmsGmAmGmCmAmGmGfGfAfG mUmUmCmUmGmUmCmCmUm | SEQ ID NO: 1174 | AGGAGCAGGGAGU UCUGUCCU | SEQ ID NO: 622 |
| ETX-S00003369 | iaiaCmsCmsCmAmCmCmCmAmGfGfCfU mCmUmUmAmAmCmUmUmCm | SEQ ID NO: 1175 | CCCACCCAGGCUC UUAACUUC | SEQ ID NO: 623 |
| ETX-S00003373 | iaiaUmsCmsUmUmAmAmCmUmUfCfGfC mCmUmUmCmAmAmAmGmAm | SEQ ID NO: 1176 | UCUUAACUUCGCC UUCAAAGA | SEQ ID NO: 624 |
| ETX-S00003377 | iaiaUmsUmsCmAmGmUmCmAmCfUfCfC mUmGmAmUmAmAmAmUmAm | SEQ ID NO: 1177 | UUCAGUCACUCCU GAUAAAUA | SEQ ID NO: 625 |
| ETX-S00003381 | iaiaCmsAmsGmUmCmAmCmUmCfCfUfG mAmUmAmAmAmUmAmAmCm | SEQ ID NO: 1178 | CAGUCACUCCUGA UAAAUAAC | SEQ ID NO: 626 |
| ETX-S00003385 | iaiaUmsCmsAmGmUmCmAmCmUfCfCfU mGmAmUmAmAmAmUmAmAm | SEQ ID NO: 1179 | UCAGUCACUCCUG AUAAAUAA | SEQ ID NO: 627 |
| ETX-S00003389 | iaiaUmsGmsAmUmGmGmGmAmCfUfCfA mAmUmUmGmUmAmUmUmUm | SEQ ID NO: 1180 | UGAUGGGACUCAA UUGUAUUU | SEQ ID NO: 628 |
| ETX-S00003393 | iaiaGmsAmsUmGmGmGmAmCmUfCfAfA mUmUmGmUmAmUmUmUmUm | SEQ ID NO: 1181 | GAUGGGACUCAAU UGUAUUUU | SEQ ID NO: 629 |
| ETX-S00003397 | iaiaCmsAmsCmUmCmCmUmGmAfUfAfA mAmUmAmAmCmAmAmAmUm | SEQ ID NO: 1182 | CACUCCUGAUAAA UAACAAAU | SEQ ID NO: 630 |
| ETX-S00003401 | iaiaAmsUmsUmCmAmUmCmUmGfAfCfC mAmGmUmUmUmUmCmUmCm | SEQ ID NO: 1183 | AUUCAUCUGACCA GUUUUCUC | SEQ ID NO: 631 |
| ETX-S00003405 | iaiaAmsAmsCmAmGmGmCmAmUfGfUfU mGmUmAmUmUmAmUmAmUm | SEQ ID NO: 1184 | AACAGGCAUGUUG UAUUAUAU | SEQ ID NO: 632 |
| ETX-S00003409 | iaiaAmsGmsGmGmAmAmUmGmCfUfUfC mCmGmGmAmUmCmCmCmAm | SEQ ID NO: 1185 | AGGGAAUGCUUCC GGAUCCCA | SEQ ID NO: 633 |
| ETX-S00003413 | iaiaGmsGmsGmAmAmUmGmCmUfUfCfC mGmGmAmUmCmCmCmAmAm | SEQ ID NO: 1186 | GGGAAUGCUUCCG GAUCCCAA | SEQ ID NO: 634 |
| ETX-S00003417 | iaiaCmsGmsAmGmGmCmCmUmCfGfGfU mGmAmCmUmGmCmCmUmGm | SEQ ID NO: 1187 | CGAGGCCUCGGUG ACUGCCUG | SEQ ID NO: 635 |
| ETX-S00003421 | iaiaAmsCmsAmGmGmCmAmUmGfUfUfG mUmAmUmUmAmUmAmUmAm | SEQ ID NO: 1188 | ACAGGCAUGUUGU AUUAUAUA | SEQ ID NO: 636 |
| ETX-S00003425 | iaiaAmsAmsUmAmCmAmAmAmGfGfCfA mUmUmAmUmAmGmAmCmUm | SEQ ID NO: 1189 | AAUACAAAGGCAU UAUAGACU | SEQ ID NO: 637 |
| ETX-S00003429 | iaiaAmsUmsUmUmCmAmGmUmCfAfCfU mCmCmUmGmAmUmAmAmAm | SEQ ID NO: 1190 | AUUUCAGUCACUC CUGAUAAA | SEQ ID NO: 638 |
| ETX-S00003433 | iaiaAmsUmsGmAmUmGmGmGmAfCfUfC mAmAmUmUmGmUmAmUmUm | SEQ ID NO: 1191 | AUGAUGGGACUCA AUUGUAUU | SEQ ID NO: 639 |
| ETX-S00003437 | iaiaCmsUmsGmAmCmCmAmGmUfUfUfU mCmUmCmUmUmAmAmAmGm | SEQ ID NO: 1192 | CUGACCAGUUUC UCUUAAAG | SEQ ID NO: 640 |
| ETX-S00003441 | iaiaUmsGmsAmCmCmAmGmUmUfUfUfC mUmCmUmUmAmAmAmGmCm | SEQ ID NO: 1193 | UGACCAGUUUCU CUUAAAGC | SEQ ID NO: 641 |
| ETX-S00003445 | iaiaGmsAmsUmAmAmGmCmAmAfUfAfC mAmAmAmGmGmCmAmUmUm | SEQ ID NO: 1194 | GAUAAGCAAUACA AAGGCAUU | SEQ ID NO: 642 |
| ETX-S00003449 | iaiaAmsUmsAmAmGmCmAmAmUfAfCfA mAmAmGmGmCmAmUmUmAm | SEQ ID NO: 1195 | AUAAGCAAUACAA AGGCAUUA | SEQ ID NO: 643 |
| ETX-S00003453 | iaiaUmsUmsAmAmCmGmUmGmUfCfUfG mUmGmCmAmGmGmGmUmAm | SEQ ID NO: 1196 | UUAACGUGUCUGU GCAGGGUA | SEQ ID NO: 644 |
| ETX-S00003457 | iaiaUmsAmsAmCmGmUmGmUmCfUfGfU mGmCmAmGmGmGmUmAmUm | SEQ ID NO: 1197 | UAACGUGUCUGUG CAGGGUAU | SEQ ID NO: 645 |
| ETX-S00003461 | iaiaUmsCmsAmCmAmUmCmGmUfCfAfU mCmAmGmCmUmGmGmAmUm | SEQ ID NO: 1198 | UCACAUCGUCAUC AGCUGGAU | SEQ ID NO: 646 |
| ETX-S00003465 | iaiaUmsGmsAmUmGmAmUmGmGfGfAfC mUmCmAmAmUmUmGmUmAm | SEQ ID NO: 1199 | UGAUGAUGGGACU CAAUUGUA | SEQ ID NO: 647 |
| ETX-S00003469 | iaiaAmsCmsUmGmCmGmUmGmGfUfCfC mGmUmAmUmUmCmCmCmAm | SEQ ID NO: 1200 | ACUGCGUGGUCCG UAUUCCCA | SEQ ID NO: 648 |
| ETX-S00003473 | iaiaGmsAmsAmCmAmGmGmCmAfUfGfU mUmGmUmAmUmUmAmUmAm | SEQ ID NO: 1201 | GAACAGGCAUGUU GUAUUAUA | SEQ ID NO: 649 |
| ETX-S00003477 | iaiaUmsAmsUmUmUmCmAmGmUfCfAfC mUmCmCmUmGmAmUmAmAm | SEQ ID NO: 1202 | UAUUUCAGUCACU CCUGAUAA | SEQ ID NO: 650 |
| ETX-S00003481 | iaiaUmsAmsAmGmCmAmAmUmAfCfAfA mAmGmGmCmAmUmUmAmUm | SEQ ID NO: 1203 | UAAGCAAUACAAA GGCAUUAU | SEQ ID NO: 651 |
| ETX-S00003485 | iaiaAmsCmsUmCmAmCmAmUmCfGfUfC mAmUmCmAmGmCmUmGmGm | SEQ ID NO: 1204 | ACUCACAUCGUCA UCAGCUGG | SEQ ID NO: 652 |
| ETX-S00003489 | iaiaCmsCmsGmCmCmUmAmCmUfUfCfG mGmUmAmUmCmUmAmUmGm | SEQ ID NO: 1205 | CCGCCUACUUCGG UAUCUAUG | SEQ ID NO: 653 |
| ETX-S00003493 | iaiaCmsAmsCmAmUmCmGmUmCfAfUfC mAmGmCmUmGmGmAmUmGm | SEQ ID NO: 1206 | CACAUCGUCAUCA GCUGGAUG | SEQ ID NO: 654 |
| ETX-S00003497 | iaiaGmsCmsCmUmAmCmUmUmCfGfGfU mAmUmCmUmAmUmGmAmCm | SEQ ID NO: 1207 | GCCUACUUCGGUA UCUAUGAC | SEQ ID NO: 655 |
| ETX-S00003501 | iaiaUmsUmsUmAmAmCmGmUmGfUfCfU mGmUmGmCmAmGmGmGmUm | SEQ ID NO: 1208 | UUUAACGUGUCUG UGCAGGGU | SEQ ID NO: 656 |
| ETX-S00003505 | iaiaUmsCmsGmCmCmGmCmCmGfCfAfU mGmAmUmGmAmUmGmCmAm | SEQ ID NO: 1209 | UCGCCGCCGCAUG AUGAUGCA | SEQ ID NO: 657 |
| ETX-S00003509 | iaiaCmsAmsUmUmAmUmAmGmAfCfUfG mCmGmUmGmGmUmCmCmGm | SEQ ID NO: 1210 | CAUUAUAGACUGC GUGGUCCG | SEQ ID NO: 658 |
| ETX-S00003513 | iaiaUmsGmsCmGmUmGmGmUmCfCfGfU mAmUmUmCmCmCmAmAmGm | SEQ ID NO: 1211 | UGCGUGGUCCGUA UUCCCAAG | SEQ ID NO: 659 |
| ETX-S00003517 | iaiaAmsUmsCmUmAmCmCmGmAfGfCfC mGmCmCmUmAmCmUmUmCm | SEQ ID NO: 1212 | AUCUACCGAGCCG CCUACUUC | SEQ ID NO: 660 |
| ETX-S00003521 | iaiaUmsUmsGmCmCmCmGmUmAfCfCfC mGmUmCmUmAmGmCmAmGm | SEQ ID NO: 1213 | UUGCCCGUACCCG UCUAGCAG | SEQ ID NO: 661 |
| ETX-S00003525 | iaiaCmsUmsCmAmCmAmUmCmGfUfCfA mUmCmAmGmCmUmGmGmAm | SEQ ID NO: 1214 | CUCACAUCGUCAU CAGCUGGA | SEQ ID NO: 662 |
| ETX-S00003529 | iaiaUmsAmsUmAmGmAmCmUmGfCfGfU mGmGmUmCmCmGmUmAmUm | SEQ ID NO: 1215 | UAUAGACUGCGUG GUCCGUAU | SEQ ID NO: 663 |
| ETX-S00003533 | iaiaGmsGmsCmCmUmCmGmGmUfGfAfC mUmGmCmCmUmGmGmUmUm | SEQ ID NO: 1216 | GGCCUCGGUGACU GCCUGGUU | SEQ ID NO: 664 |
| ETX-S00003537 | iaiaGmsGmsAmUmGmAmUmCmGfCfAfC mAmGmAmCmUmGmUmCmAm | SEQ ID NO: 1217 | GGAUGAUCGCACA GACUGUCA | SEQ ID NO: 665 |
| ETX-S00003541 | iaiaGmsAmsUmUmUmUmGmCmCfCfGfU mAmCmCmCmGmUmCmUmAm | SEQ ID NO: 1218 | GAUUUUGCCCGUA CCCGUCUA | SEQ ID NO: 666 |
| ETX-S00003545 | iaiaGmsAmsUmCmGmCmAmCmAfGfAfC mUmGmUmCmAmCmUmGmCm | SEQ ID NO: 1219 | GAUCGCACAGACU GUCACUGC | SEQ ID NO: 667 |
| ETX-S00003549 | iaiaAmsUmsGmAmUmCmGmCmAfCfAfG mAmCmUmGmUmCmAmCmUm | SEQ ID NO: 1220 | AUGAUCGCACAGA CUGUCACU | SEQ ID NO: 668 |
| ETX-S00003553 | iaiaUmsUmsUmUmGmCmCmCmGfUfAfC mCmCmGmUmCmUmAmGmCm | SEQ ID NO: 1221 | UUUUGCCCGUACC CGUCUAGC | SEQ ID NO: 669 |
| ETX-S00003557 | iaiaUmsCmsAmUmCmUmAmCmCfGfAfG mCmCmGmCmCmUmAmCmUm | SEQ ID NO: 1222 | UCAUCUACCGAGC CGCCUACU | SEQ ID NO: 670 |
| ETX-S00003561 | iaiaUmsAmsGmAmCmUmGmCmGfUfGfG mUmCmCmGmUmAmUmUmCm | SEQ ID NO: 1223 | UAGACUGCGUGGU CCGUAUUC | SEQ ID NO: 671 |
| ETX-S00003565 | iaiaCmsUmsGmCmAmGmAmUmAfAfGfC mAmAmUmAmCmAmAmAmGm | SEQ ID NO: 1224 | CUGCAGAUAAGCA AUACAAAG | SEQ ID NO: 672 |
| ETX-S00003569 | iaiaAmsCmsUmGmCmAmGmAmUfAfAfG mCmAmAmUmAmCmAmAmAm | SEQ ID NO: 1225 | ACUGCAGAUAAGC AAUACAAA | SEQ ID NO: 673 |
| ETX-S00003573 | iaiaCmsAmsCmUmGmCmAmGmAfUfAfA mGmCmAmAmUmAmCmAmAm | SEQ ID NO: 1226 | CACUGCAGAUAAG CAAUACAA | SEQ ID NO: 674 |
| ETX-S00003577 | iaiaGmsCmsAmAmGmCmAmGmAfUfCfA mCmUmGmCmAmGmAmUmAm | SEQ ID NO: 1227 | GCAAGCAGAUCAC UGCAGAUA | SEQ ID NO: 675 |
| ETX-S00003581 | iaiaAmsUmsCmAmCmUmGmCmAfGfAfU mAmAmGmCmAmAmUmAmCm | SEQ ID NO: 1228 | AUCACUGCAGAUA AGCAAUAC | SEQ ID NO: 676 |
| ETX-S00003585 | iaiaUmsCmsAmCmUmGmCmAmGfAfUfA mAmGmCmAmAmUmAmCmAm | SEQ ID NO: 1229 | UCACUGCAGAUAA GCAAUACA | SEQ ID NO: 677 |
| ETX-S00003589 | iaiaGmsCmsAmGmAmUmCmAmCfUfGfC mAmGmAmUmAmAmGmCmAm | SEQ ID NO: 1230 | GCAGAUCACUGCA GAUAAGCA | SEQ ID NO: 678 |
| ETX-S00003593 | iaiaCmsAmsGmAmUmCmAmCmUfGfCfA mGmAmUmAmAmGmCmAmAm | SEQ ID NO: 1231 | CAGAUCACUGCAG AUAAGCAA | SEQ ID NO: 679 |
| ETX-S00003597 | iaiaAmsGmsCmAmAmGmCmAmGfAfUfC mAmCmUmGmCmAmGmAmUm | SEQ ID NO: 1232 | AGCAAGCAGAUCA CUGCAGAU | SEQ ID NO: 680 |
| ETX-S00003601 | iaiaGmsAmsUmCmAmCmUmGmCfAfGfA mUmAmAmGmCmAmAmUmAm | SEQ ID NO: 1233 | GAUCACUGCAGAU AAGCAAUA | SEQ ID NO: 681 |
| ETX-S00003605 | iaiaAmsUmsAmAmAmUmAmAmCfAfAfA mUmUmUmGmGmAmGmAmAm | SEQ ID NO: 1234 | AUAAAUAACAAAU UUGGAGAA | SEQ ID NO: 682 |
| ETX-S00003609 | iaiaUmsCmsCmUmGmAmUmAmAfAfUfA mAmCmAmAmAmUmUmUmGm | SEQ ID NO: 1235 | UCCUGAUAAAUAA CAAAUUUG | SEQ ID NO: 683 |
| ETX-S00003613 | iaiaGmsAmsAmAmGmGmGmAmAfUfUfC mCmGmAmGmGmCmCmUmCm | SEQ ID NO: 1236 | GAAAGGGAAUUCC GAGGCCUC | SEQ ID NO: 684 |
| ETX-S00003617 | iaiaGmsUmsUmGmUmAmUmUmAfUfAf UmAmAmCmAmUmAmUmCmUm | SEQ ID NO: 1237 | GUUGUAUUAUAUA ACAUAUCU | SEQ ID NO: 685 |
| ETX-S00003621 | iaiaUmsUmsCmCmGmAmGmGmCfCfUfC mGmGmUmGmAmCmUmGmCm | SEQ ID NO: 1238 | UUCCGAGGCCUCG GUGACUGC | SEQ ID NO: 686 |
| ETX-S00003625 | iaiaAmsAmsAmGmGmGmAmAmUfUfCfC mGmAmGmGmCmCmUmCmGm | SEQ ID NO: 1239 | AAAGGGAAUUCCG AGGCCUCG | SEQ ID NO: 687 |
| ETX-S00003629 | iaiaUmsGmsUmAmUmUmAmUmAfUfAf AmCmAmUmAmUmCmUmUmGm | SEQ ID NO: 1240 | UGUAUUAUAUAAC AUAUCUUG | SEQ ID NO: 688 |
| ETX-S00003633 | iaiaAmsAmsUmUmCmCmGmAmGfGfCfC mUmCmGmGmUmGmAmCmUm | SEQ ID NO: 1241 | AAUUCCGAGGCCU CGGUGACU | SEQ ID NO: 689 |
| ETX-S00003637 | iaiaGmsGmsGmAmAmUmUmCmCfGfAfG mGmCmCmUmCmGmGmUmGm | SEQ ID NO: 1242 | GGGAAUUCCGAGG CCUCGGUG | SEQ ID NO: 690 |
| ETX-S00003641 | iaiaAmsGmsCmUmGmAmAmAmGfGfGfA mAmUmUmCmCmGmAmGmGm | SEQ ID NO: 1243 | AGCUGAAAGGGAA UUCCGAGG | SEQ ID NO: 691 |
| ETX-S00003645 | iaiaGmsGmsAmGmCmUmGmAmAfAfGfG mGmAmAmUmUmCmCmGmAm | SEQ ID NO: 1244 | GGAGCUGAAAGGG AAUUCCGA | SEQ ID NO: 692 |
| ETX-S00003649 | iaiaCmsUmsGmAmAmAmGmGmGfAfAfU mUmCmCmGmAmGmGmCmCm | SEQ ID NO: 1245 | CUGAAAGGGAAUU CCGAGGCC | SEQ ID NO: 693 |
| ETX-S00003653 | iaiaGmsAmsGmCmUmGmAmAmAfGfGfG mAmAmUmUmCmCmGmAmGm | SEQ ID NO: 1246 | GAGCUGAAAGGGA AUUCCGAG | SEQ ID NO: 694 |
| ETX-S00003657 | iaiaGmsCmsUmGmAmAmAmGmGfGfAfA mUmUmCmCmGmAmGmGmCm | SEQ ID NO: 1247 | GCUGAAAGGGAAU UCCGAGGC | SEQ ID NO: 695 |
| ETX-S00003661 | iaiaGmsCmsAmUmGmUmUmGmUfAfUfU mAmUmAmUmAmAmCmAmUm | SEQ ID NO: 1248 | GCAUGUUGUAUUA UAUAACAU | SEQ ID NO: 696 |
| ETX-S00003665 | iaiaUmsUmsGmUmAmUmUmAmUfAfUf AmAmCmAmUmAmUmCmUmUm | SEQ ID NO: 1249 | UUGUAUUAUAUAA CAUAUCUU | SEQ ID NO: 697 |
| ETX-S00003669 | iaiaGmsUmsAmUmUmAmUmAmUfAfAfC mAmUmAmUmCmUmUmGmAm | SEQ ID NO: 1250 | GUAUUAUAUAACA UAUCUUGA | SEQ ID NO: 698 |
| ETX-S00003673 | iaiaGmsGmsCmAmUmGmUmGfUfAfU mUmAmUmAmUmAmAmCmAm | SEQ ID NO: 1251 | GGCAUGUUGUAUU AUAUAACA | SEQ ID NO: 699 |
| ETX-S00003677 | iaiaAmsAmsGmGmGmAmAmUmUfCfCfG mAmGmGmCmCmUmCmGmGm | SEQ ID NO: 1252 | AAGGGAAUUCCGA GGCCUCGG | SEQ ID NO: 700 |
| ETX-S00003681 | iaiaGmsAmsAmUmUmCmCmGmAfGfGfC mCmUmCmGmGmUmGmAmCm | SEQ ID NO: 1253 | GAAUUCCGAGGCC UCGGUGAC | SEQ ID NO: 701 |
| ETX-S00003685 | iaiaAmsGmsGmGmAmAmUmUmCfCfGfA mGmGmCmCmUmCmGmGmUm | SEQ ID NO: 1254 | AGGGAAUUCCGAG GCCUCGGU | SEQ ID NO: 702 |
| ETX-S00003689 | iaiaGmsGmsAmAmUmUmCmCmGfAfGfG mCmCmUmCmGmGmUmGmAm | SEQ ID NO: 1255 | GGAAUUCCGAGGC CUCGGUGA | SEQ ID NO: 703 |
| ETX-S00003693 | iaiaGmsCmsUmUmGmUmCmUmUfGfUfA mUmGmAmUmGmAmAmAmUm | SEQ ID NO: 1256 | GCUUGUCUUGUAU GAUGAAAU | SEQ ID NO: 704 |
| ETX-S00003697 | iaiaUmsCmsGmCmCmUmUmCmAfAfAfG mAmUmAmAmAmUmAmCmAm | SEQ ID NO: 1257 | UCGCCUUCAAAGA UAAAUACA | SEQ ID NO: 705 |
| ETX-S00003701 | iaiaGmsCmsCmUmGmGmUmUmAfAfGfA mUmCmUmAmCmAmAmAmUm | SEQ ID NO: 1258 | GCCUGGUUAAGAU CUACAAAU | SEQ ID NO: 706 |
| ETX-S00003705 | iaiaUmsCmsUmAmUmGmAmCmAfCfUfG mCmAmAmAmGmGmGmAmAm | SEQ ID NO: 1259 | UCUAUGACACUGC AAAGGGAA | SEQ ID NO: 707 |
| ETX-S00003709 | iaiaUmsUmsUmGmUmGmCmUmUfGfUfC mUmUmGmUmAmUmGmAmUm | SEQ ID NO: 1260 | UUUGUGCUUGUCU UGUAUGAU | SEQ ID NO: 708 |
| ETX-S00003713 | iaiaUmsGmsAmCmUmGmCmCmUfGfGfU mUmAmAmGmAmUmCmUmAm | SEQ ID NO: 1261 | UGACUGCCUGGUU AAGAUCUA | SEQ ID NO: 709 |
| ETX-S00003717 | iaiaGmsAmsCmAmCmUmGmCmAfAfAfG mGmGmAmAmUmGmCmUmUm | SEQ ID NO: 1262 | GACACUGCAAAGG GAAUGCUU | SEQ ID NO: 710 |
| ETX-S00003721 | iaiaCmsGmsGmUmAmUmCmUmAfUfGfA mCmAmCmUmGmCmAmAmAm | SEQ ID NO: 1263 | CGGUAUCUAUGAC ACUGCAAA | SEQ ID NO: 711 |
| ETX-S00003725 | iaiaUmsUmsCmAmUmCmUmGmAfCfCfA mGmUmUmUmUmCmUmCmUm | SEQ ID NO: 1264 | UUCAUCUGACCAG UUUUCUCU | SEQ ID NO: 712 |
| ETX-S00003729 | iaiaUmsUmsCmGmGmUmAmUmCfUfAfU mGmAmCmAmCmUmGmCmAm | SEQ ID NO: 1265 | UUCGGUAUCUAUG ACACUGCA | SEQ ID NO: 713 |
| ETX-S00003733 | iaiaGmsGmsUmUmAmAmGmAmUfCfUfA mCmAmAmAmUmCmUmGmAm | SEQ ID NO: 1266 | GGUUAAGAUCUAC AAAUCUGA | SEQ ID NO: 714 |
| ETX-S00003741 | iaiaAmsGmsGmGmUmGmCmAmUfGfGfU mCmCmAmAmUmGmUmUmCm | SEQ ID NO: 1268 | AGGGUGCAUGGUC CAAUGUUC | SEQ ID NO: 716 |
| ETX-S00003745 | iaiaAmsUmsCmUmAmCmAmAmAfUfCfU mGmAmUmGmGmGmAmUmUm | SEQ ID NO: 1269 | AUCUACAAAUCUG AUGGGAUU | SEQ ID NO: 717 |
| ETX-S00003749 | iaiaCmsUmsUmCmGmGmUmAmUfCfUfA mUmGmAmCmAmCmUmGmCm | SEQ ID NO: 1270 | CUUCGGUAUCUAU GACACUGC | SEQ ID NO: 718 |
| ETX-S00003753 | iaiaUmsGmsGmGmUmGmGmUmGfCfUfU mUmUmGmUmGmCmUmUmGm | SEQ ID NO: 1271 | UGGGUGGUGCUUU UGUGCUUG | SEQ ID NO: 719 |
| ETX-S00003757 | iaiaUmsAmsUmUmAmUmCmAmUfCfUfA mCmCmGmAmGmCmCmGmCm | SEQ ID NO: 1272 | UAUUAUCAUCUAC CGAGCCGC | SEQ ID NO: 720 |
| ETX-S00003761 | iaiaGmsUmsGmGmUmCmCmGmUfAfUfU mCmCmCmAmAmGmGmAmGm | SEQ ID NO: 1273 | GUGGUCCGUAUUC CCAAGGAG | SEQ ID NO: 721 |
| ETX-S00003765 | iaiaCmsUmsAmUmGmAmCmAmCfUfGfC mAmAmAmGmGmGmAmAmUm | SEQ ID NO: 1274 | CUAUGACACUGCA AAGGGAAU | SEQ ID NO: 722 |
| ETX-S00003769 | iaiaCmsUmsUmUmUmGmUmGmCfUfUfG mUmCmUmUmGmUmAmUmGm | SEQ ID NO: 1275 | CUUUUGUGCUUGU CUUGUAUG | SEQ ID NO: 723 |
| ETX-S00003773 | iaiaUmsAmsCmCmAmAmGmGmCfUfUfU mAmAmCmGmUmGmUmCmUm | SEQ ID NO: 1276 | UACCAAGGCUUUA ACGUGUCU | SEQ ID NO: 724 |
| ETX-S00003777 | iaiaGmsUmsGmCmAmUmGmGmUfCfCfA mAmUmGmUmUmCmUmCmAm | SEQ ID NO: 1277 | GUGCAUGGUCCAA UGUUCUCA | SEQ ID NO: 725 |
| ETX-S00003781 | iaiaCmsCmsUmUmCmAmAmAmGfAfUfA mAmAmUmAmCmAmAmGmCm | SEQ ID NO: 1278 | CCUUCAAAGAUAA AUACAAGC | SEQ ID NO: 726 |
| ETX-S00003785 | iaiaGmsGmsGmUmAmUmUmAmUfCfAfU mCmUmAmCmCmGmAmGmCm | SEQ ID NO: 1279 | GGGUAUUAUCAUC UACCGAGC | SEQ ID NO: 727 |
| ETX-S00003789 | iaiaUmsAmsCmUmUmCmGmGmUfAfUfC mUmAmUmGmAmCmAmCmUm | SEQ ID NO: 1280 | UACUUCGGUAUCU AUGACACU | SEQ ID NO: 728 |
| ETX-S00003797 | iaiaUmsGmsCmAmGmGmGmUmAfUfUfA mUmCmAmUmCmUmAmCmCm | SEQ ID NO: 1282 | UGCAGGGUAUUAU CAUCUACC | SEQ ID NO: 730 |
| ETX-S00003801 | iaiaUmsCmsUmGmAmUmGmGmGfAfUfU mAmAmGmGmGmCmCmUmGm | SEQ ID NO: 1283 | UCUGAUGGGAUUA AGGGCCUG | SEQ ID NO: 731 |
| ETX-S00003805 | iaiaCmsUmsGmUmGmCmAmGmGfGfUfA mUmUmAmUmCmAmUmCmUm | SEQ ID NO: 1284 | CUGUGCAGGGUAU UAUCAUCU | SEQ ID NO: 732 |
| ETX-S00003809 | iaiaUmsGmsAmUmGmGmGmAmUfUfAf AmGmGmGmCmCmUmGmUmAm | SEQ ID NO: 1285 | UGAUGGGAUUAAG GGCCUGUA | SEQ ID NO: 733 |
| ETX-S00003813 | iaiaCmsCmsAmAmGmGmCmUmUfUfAfA mCmGmUmGmUmCmUmGmUm | SEQ ID NO: 1286 | CCAAGGCUUUAAC GUGUCUGU | SEQ ID NO: 734 |
| ETX-S00003817 | iaiaUmsAmsUmUmCmAmUmCmUfGfAfC mCmAmGmUmUmUmUmCmUm | SEQ ID NO: 1287 | UAUUCAUCUGACC AGUUUUCU | SEQ ID NO: 735 |
| ETX-S00003821 | iaiaAmsCmsUmGmCmCmUmGmGfUfUfA mAmGmAmUmCmUmAmCmAm | SEQ ID NO: 1288 | ACUGCCUGGUUAA GAUCUACA | SEQ ID NO: 736 |
| ETX-S00003825 | iaiaGmsUmsUmGmAmCmUmUmCfCfUfA mUmCmCmAmUmUmUmGmAm | SEQ ID NO: 1289 | GUUGACUUCCUAU CCAUUUGA | SEQ ID NO: 737 |
| ETX-S00003829 | iaiaUmsAmsUmGmAmCmAmCmUfGfCfA mAmAmGmGmGmAmAmUmGm | SEQ ID NO: 1290 | UAUGACACUGCAA AGGGAAUG | SEQ ID NO: 738 |
| ETX-S00003833 | iaiaUmsUmsAmAmGmAmUmCmUfAfCfA mAmAmUmCmUmGmAmUmGm | SEQ ID NO: 1291 | UUAAGAUCUACAA AUCUGAUG | SEQ ID NO: 739 |
| ETX-S00003837 | iaiaAmsGmsGmGmUmAmUmUmAfUfCfA mUmCmUmAmCmCmGmAmGm | SEQ ID NO: 1292 | AGGGUAUUAUCAU CUACCGAG | SEQ ID NO: 740 |
| ETX-S00003841 | iaiaUmsGmsCmCmUmGmGmUmUfAfAfG mAmUmCmUmAmCmAmAmAm | SEQ ID NO: 1293 | UGCCUGGUUAAGA UCUACAAA | SEQ ID NO: 741 |
| ETX-S00003845 | iaiaCmsUmsGmAmUmGmGmGmAfUfUfA mAmGmGmGmCmCmUmGmUm | SEQ ID NO: 1294 | CUGAUGGGAUUAA GGGCCUGU | SEQ ID NO: 742 |
| ETX-S00003849 | iaiaGmsGmsUmAmUmCmUmAmUfGfAfC mAmCmUmGmCmAmAmAmGm | SEQ ID NO: 1295 | GGUAUCUAUGACA CUGCAAAG | SEQ ID NO: 743 |
| ETX-S00003853 | iaiaAmsCmsCmCmGmUmCmUmAfGfCfA mGmCmUmGmAmUmGmUmGm | SEQ ID NO: 1296 | ACCCGUCUAGCAG CUGAUGUG | SEQ ID NO: 744 |
| ETX-S00003857 | iaiaUmsAmsUmCmUmAmUmGmAfCfAfC mUmGmCmAmAmAmGmGmGm | SEQ ID NO: 1297 | UAUCUAUGACACU GCAAAGGG | SEQ ID NO: 745 |
| ETX-S00003861 | iaiaCmsUmsGmAmUmGmUmGmGfGfUfA mAmAmGmCmUmGmGmAmGm | SEQ ID NO: 1298 | CUGAUGUGGGUAA AGCUGGAG | SEQ ID NO: 746 |
| ETX-S00003865 | iaiaUmsCmsUmAmGmCmAmGmCfUfGfA mUmGmUmGmGmGmUmAmAm | SEQ ID NO: 1299 | UCUAGCAGCUGAU GUGGGUAA | SEQ ID NO: 747 |
| ETX-S00003869 | iaiaGmsGmsGmCmCmUmGmUmAfCfCfA mAmGmGmCmUmUmUmAmAm | SEQ ID NO: 1300 | GGGCCUGUACCAA GGCUUUAA | SEQ ID NO: 748 |
| ETX-S00003873 | iaiaCmsCmsCmGmUmAmCmCmCfGfUfC mUmAmGmCmAmGmCmUmGm | SEQ ID NO: 1301 | CCCGUACCCGUCU AGCAGCUG | SEQ ID NO: 749 |
| ETX-S00003877 | iaiaCmsAmsGmCmUmGmAmUmGfUfGfG mGmUmAmAmAmGmCmUmGm | SEQ ID NO: 1302 | CAGCUGAUGUGGG UAAAGCUG | SEQ ID NO: 750 |
| ETX-S00003881 | iaiaCmsCmsGmUmAmCmCmCmGfUfCfU mAmGmCmAmGmCmUmGmAm | SEQ ID NO: 1303 | CCGUACCCGUCUA GCAGCUGA | SEQ ID NO: 751 |
| ETX-S00003885 | iaiaGmsCmsUmGmAmUmGmUmGfGfGfU mAmAmAmGmCmUmGmGmAm | SEQ ID NO: 1304 | GCUGAUGUGGGUA AAGCUGGA | SEQ ID NO: 752 |
| ETX-S00003889 | iaiaGmsGmsUmAmUmUmAmUmCfAfUfC mUmAmCmCmGmAmGmCmCm | SEQ ID NO: 1305 | GGUAUUAUCAUCU ACCGAGCC | SEQ ID NO: 753 |
| ETX-S00003893 | iaiaUmsUmsAmUmCmAmUmCmUfAfCfC mGmAmGmCmCmGmCmCmUm | SEQ ID NO: 1306 | UUAUCAUCUACCG AGCCGCCU | SEQ ID NO: 754 |
| ETX-S00003897 | iaiaGmsUmsAmCmCmCmGmUmCfUfAfG mCmAmGmCmUmGmAmUmGm | SEQ ID NO: 1307 | GUACCCGUCUAGC AGCUGAUG | SEQ ID NO: 755 |
| ETX-S00003901 | iaiaGmsAmsUmGmGmGmAmUmUfAfAf GmGmGmCmCmUmGmUmAmCm | SEQ ID NO: 1308 | GAUGGGAUUAAGG GCCUGUAC | SEQ ID NO: 756 |
| ETX-S00003905 | iaiaGmsUmsCmUmGmUmGmCmAfGfGfG mUmAmUmUmAmUmCmAmUm | SEQ ID NO: 1309 | GUCUGUGCAGGGU AUUAUCAU | SEQ ID NO: 757 |
| ETX-S00003909 | iaiaGmsCmsGmUmGmGmUmCmCfGfUfA mUmUmCmCmCmAmAmGmGm | SEQ ID NO: 1310 | GCGUGGUCCGUAU UCCCAAGG | SEQ ID NO: 758 |
| ETX-S00003913 | iaiaCmsGmsUmGmGmUmCmCmGfUfAfU mUmCmCmCmAmAmGmGmAm | SEQ ID NO: 1311 | CGUGGUCCGUAUU CCCAAGGA | SEQ ID NO: 759 |
| ETX-S00003917 | iaiaUmsGmsGmUmUmAmAmGmAfUfCfU mAmCmAmAmAmUmCmUmGm | SEQ ID NO: 1312 | UGGUUAAGAUCUA CAAAUCUG | SEQ ID NO: 760 |
| ETX-S00003921 | iaiaAmsUmsGmAmUmGmAmAmAfUfCfA mAmGmAmAmGmUmAmCmAm | SEQ ID NO: 1313 | AUGAUGAAAUCAA GAAGUACA | SEQ ID NO: 761 |
| ETX-S00003925 | iaiaGmsAmsUmCmUmAmCmAmAfAfUfC mUmGmAmUmGmGmGmAmUm | SEQ ID NO: 1314 | GAUCUACAAAUCU GAUGGGAU | SEQ ID NO: 762 |
| ETX-S00003929 | iaiaGmsUmsGmCmAmGmGmGmUfAfUfU mAmUmCmAmUmCmUmAmCm | SEQ ID NO: 1315 | GUGCAGGGUAUUA UCAUCUAC | SEQ ID NO: 763 |
| ETX-S00003933 | iaiaGmsUmsAmCmCmAmAmGmGfCfUfU mUmAmAmCmGmUmGmUmCm | SEQ ID NO: 1316 | GUACCAAGGCUUU AACGUGUC | SEQ ID NO: 764 |
| ETX-S00003937 | iaiaGmsCmsAmGmCmUmGmAmUfGfUfG mGmGmUmAmAmAmGmCmUm | SEQ ID NO: 1317 | GCAGCUGAUGUGG GUAAAGCU | SEQ ID NO: 765 |
| ETX-S00003941 | iaiaAmsUmsGmGmGmUmGmGmUfGfCfU mUmUmUmGmUmGmCmUmUm | SEQ ID NO: 1318 | AUGGGUGGUGCUU UUGUGCUU | SEQ ID NO: 766 |
| ETX-S00003945 | iaiaCmsGmsGmUmGmAmCmUmGfCfCfU mGmGmUmUmAmAmGmAmUm | SEQ ID NO: 1319 | CGGUGACUGCCUG GUUAAGAU | SEQ ID NO: 767 |
| ETX-S00003949 | iaiaCmsUmsAmGmCmAmGmCmUfGfAfU mGmUmGmGmGmUmAmAmAm | SEQ ID NO: 1320 | CUAGCAGCUGAUG UGGGUAAA | SEQ ID NO: 768 |
| ETX-S00003953 | iaiaUmsAmsCmAmAmAmUmCmUfGfAfU mGmGmGmAmUmUmAmAmGm | SEQ ID NO: 1321 | UACAAAUCUGAUG GGAUUAAG | SEQ ID NO: 769 |
| ETX-S00003957 | iaiaAmsCmsUmUmCmGmGmUmAfUfCfU mAmUmGmAmCmAmCmUmGm | SEQ ID NO: 1322 | ACUUCGGUAUCUA UGACACUG | SEQ ID NO: 770 |
| ETX-S00003961 | iaiaAmsGmsCmUmGmAmUmGmUfGfGfG mUmAmAmAmGmCmUmGmGm | SEQ ID NO: 1323 | AGCUGAUGUGGGU AAAGCUGG | SEQ ID NO: 771 |
| ETX-S00003969 | iaiaUmsCmsUmGmUmGmCmAmGfGfGfU mAmUmUmAmUmCmAmUmCm | SEQ ID NO: 1325 | UCUGUGCAGGGUA UUAUCAUC | SEQ ID NO: 773 |
| ETX-S00003973 | iaiaUmsGmsCmUmUmGmUmCmUfUfGfU mAmUmGmAmUmGmAmAmAm | SEQ ID NO: 1326 | UGCUUGUCUUGUA UGAUGAAA | SEQ ID NO: 774 |
| ETX-S00003977 | iaiaAmsCmsCmAmAmGmGmCmUfUfUfA mAmCmGmUmGmUmCmUmGm | SEQ ID NO: 1327 | ACCAAGGCUUUAA CGUGUCUG | SEQ ID NO: 775 |
| ETX-S00003981 | iaiaGmsGmsGmUmGmCmAmUmGfGfUfC mCmAmAmUmGmUmUmCmUm | SEQ ID NO: 1328 | GGGUGCAUGGUCC AAUGUUCU | SEQ ID NO: 776 |
| ETX-S00003985 | iaiaGmsAmsCmUmGmCmCmUmGfGfUfU mAmAmGmAmUmCmUmAmCm | SEQ ID NO: 1329 | GACUGCCUGGUUA AGAUCUAC | SEQ ID NO: 777 |
| ETX-S00003989 | iaiaGmsAmsUmGmUmGmGmGmUfAfAf AmGmCmUmGmGmAmGmCmUm | SEQ ID NO: 1330 | GAUGUGGGUAAAG CUGGAGCU | SEQ ID NO: 778 |
| ETX-S00003993 | iaiaUmsGmsGmUmCmCmGmUmAfUfUfC mCmCmAmAmGmGmAmGmCm | SEQ ID NO: 1331 | UGGUCCGUAUUCC CAAGGAGC | SEQ ID NO: 779 |
| ETX-S00003997 | iaiaAmsCmsAmCmUmGmCmAmAfAfGfG mGmAmAmUmGmCmUmUmCm | SEQ ID NO: 1332 | ACACUGCAAAGGG AAUGCUUC | SEQ ID NO: 780 |
| ETX-S00004001 | iaiaGmsAmsUmUmAmAmGmGmGfCfCfU mGmUmAmCmCmAmAmGmGm | SEQ ID NO: 1333 | GAUUAAGGGCCUG UACCAAGG | SEQ ID NO: 781 |
| ETX-S00004005 | iaiaUmsGmsUmGmCmAmGmGmGfUfAfU mUmAmUmCmAmUmCmUmAm | SEQ ID NO: 1334 | UGUGCAGGGUAUU AUCAUCUA | SEQ ID NO: 782 |
| ETX-S00004009 | iaiaAmsAmsCmGmUmGmUmCmUfGfUfG mCmAmGmGmGmUmAmUmUm | SEQ ID NO: 1335 | AACGUGUCUGUGC AGGGUAUU | SEQ ID NO: 783 |
| ETX-S00004013 | iaiaGmsGmsCmCmUmGmUmAmCfCfAfA mGmGmCmUmUmUmAmAmCm | SEQ ID NO: 1336 | GGCCUGUACCAAG GCUUUAAC | SEQ ID NO: 784 |
| ETX-S00004017 | iaiaUmsGmsAmCmAmCmUmGmCfAfAfA mGmGmGmAmAmUmGmCmUm | SEQ ID NO: 1337 | UGACACUGCAAAG GGAAUGCU | SEQ ID NO: 785 |
| ETX-S00004021 | iaiaUmsCmsGmGmUmGmAmCmUfGfCfC mUmGmGmUmUmAmAmGmAm | SEQ ID NO: 1338 | UCGGUGACUGCCU GGUUAAGA | SEQ ID NO: 786 |
| ETX-S00004025 | iaiaGmsCmsUmUmUmUmGmUmGfCfUfU mGmUmCmUmUmGmUmAmUm | SEQ ID NO: 1339 | GCUUUUGUGCUUG UCUUGUAU | SEQ ID NO: 787 |
| ETX-S00004029 | iaiaGmsGmsUmGmGmUmGmCmUfUfUfU mGmUmGmCmUmUmGmUmCm | SEQ ID NO: 1340 | GGUGGUGCUUUUG UGCUUGUC | SEQ ID NO: 788 |
| ETX-S00004033 | iaiaCmsGmsUmCmUmAmGmCmAfGfCfU mGmAmUmGmUmGmGmGmUm | SEQ ID NO: 1341 | CGUCUAGCAGCUG AUGUGGGU | SEQ ID NO: 789 |
| ETX-S00004037 | iaiaAmsGmsGmGmCmCmUmGmUfAfCfC mAmAmGmGmCmUmUmUmAm | SEQ ID NO: 1342 | AGGGCCUGUACCA AGGCUUUA | SEQ ID NO: 790 |
| ETX-S00004041 | iaiaGmsUmsGmCmUmUmGmUmCfUfUfG mUmAmUmGmAmUmGmAmAm | SEQ ID NO: 1343 | GUGCUUGUCUUGU AUGAUGAA | SEQ ID NO: 791 |
| ETX-S00004045 | iaiaAmsAmsAmUmCmUmGmAmUfGfGfG mAmUmUmAmAmGmGmGmCm | SEQ ID NO: 1344 | AAAUCUGAUGGGA UUAAGGGC | SEQ ID NO: 792 |
| ETX-S00004049 | iaiaCmsUmsAmCmAmAmAmUmCfUfGfA mUmGmGmGmAmUmUmAmAm | SEQ ID NO: 1345 | CUACAAAUCUGAU GGGAUUAA | SEQ ID NO: 793 |
| ETX-S00004053 | iaiaCmsAmsGmGmGmUmAmUmUfAfUfC mAmUmCmUmAmCmCmGmAm | SEQ ID NO: 1346 | CAGGGUAUUAUCA UCUACCGA | SEQ ID NO: 794 |
| ETX-S00004057 | iaiaCmsUmsGmUmAmCmCmAmAfGfGfC mUmUmUmAmAmCmGmUmGm | SEQ ID NO: 1347 | CUGUACCAAGGCU UUAACGUG | SEQ ID NO: 795 |
| ETX-S00004061 | iaiaAmsGmsAmUmCmUmAmCmAfAfAfU mCmUmGmAmUmGmGmGmAm | SEQ ID NO: 1348 | AGAUCUACAAAUC UGAUGGGA | SEQ ID NO: 796 |
| ETX-S00004065 | iaiaUmsUmsUmUmGmUmGmCmUfUfGfU mCmUmUmGmUmAmUmGmAm | SEQ ID NO: 1349 | UUUUGUGCUUGUC UUGUAUGA | SEQ ID NO: 797 |
| ETX-S00004069 | iaiaAmsCmsAmAmAmUmCmUmGfAfUfG mGmGmAmUmUmAmAmGmGm | SEQ ID NO: 1350 | ACAAAUCUGAUGG GAUUAAGG | SEQ ID NO: 798 |
| ETX-S00004073 | iaiaCmsAmsAmGmGmGmUmGmCfAfUfG mGmUmCmCmAmAmUmGmUm | SEQ ID NO: 1351 | CAAGGGUGCAUGG UCCAAUGU | SEQ ID NO: 799 |
| ETX-S00004077 | iaiaGmsGmsAmUmUmAmAmGmGfGfCfC mUmGmUmAmCmCmAmAmGm | SEQ ID NO: 1352 | GGAUUAAGGGCCU GUACCAAG | SEQ ID NO: 800 |
| ETX-S00004081 | iaiaUmsCmsGmGmUmAmUmCmUfAfUfG mAmCmAmCmUmGmCmAmAm | SEQ ID NO: 1353 | UCGGUAUCUAUGA CACUGCAA | SEQ ID NO: 801 |
| ETX-S00004085 | iaiaCmsAmsAmGmGmCmUmUmUfAfAfC mGmUmGmUmCmUmGmUmGm | SEQ ID NO: 1354 | CAAGGCUUUAACG UGUCUGUG | SEQ ID NO: 802 |
| ETX-S00004089 | iaiaCmsAmsUmGmGmGmUmGmGfUfGfC mUmUmUmUmGmUmGmCmUm | SEQ ID NO: 1355 | CAUGGGUGGUGCU UUUGUGCU | SEQ ID NO: 803 |
| ETX-S00004093 | iaiaUmsAmsAmGmAmUmCmUmAfCfAfA mAmUmCmUmGmAmUmGmGm | SEQ ID NO: 1356 | UAAGAUCUACAAA UCUGAUGG | SEQ ID NO: 804 |
| ETX-S00004097 | iaiaCmsCmsCmGmUmCmUmAmGfCfAfG mCmUmGmAmUmGmUmGmGm | SEQ ID NO: 1357 | CCCGUCUAGCAGC UGAUGUGG | SEQ ID NO: 805 |
| ETX-S00004101 | iaiaAmsUmsGmAmCmAmCmUmGfCfAfA mAmGmGmGmAmAmUmGmCm | SEQ ID NO: 1358 | AUGACACUGCAAA GGGAAUGC | SEQ ID NO: 806 |
| ETX-S00004105 | iaiaUmsAmsGmCmAmGmCmUmGfAfUfG mUmGmGmGmUmAmAmAmGm | SEQ ID NO: 1359 | UAGCAGCUGAUGU GGGUAAAG | SEQ ID NO: 807 |
| ETX-S00004109 | iaiaGmsCmsAmAmAmGmGmGmAfAfUfG mCmUmUmCmCmGmGmAmUm | SEQ ID NO: 1360 | GCAAAGGAAUGC UUCCGGAU | SEQ ID NO: 808 |
| ETX-S00004113 | iaiaGmsCmsCmCmGmUmAmCmCfCfGfU mCmUmAmGmCmAmGmCmUm | SEQ ID NO: 1361 | GCCCGUACCCGUC UAGCAGCU | SEQ ID NO: 809 |
| ETX-S00004117 | iaiaGmsUmsAmUmUmAmUmCmAfUfCfU mAmCmCmGmAmGmCmCmGm | SEQ ID NO: 1362 | GUAUUAUCAUCUA CCGAGCCG | SEQ ID NO: 810 |
| ETX-S00004121 | iaiaCmsUmsCmUmUmGmAmUmUfUfUfG mCmCmCmGmUmAmCmCmCm | SEQ ID NO: 1363 | CUCUUGAUUUGC CCGUACCC | SEQ ID NO: 811 |
| ETX-S00004125 | iaiaUmsAmsCmCmCmGmUmCmUfAfGfC mAmGmCmUmGmAmUmGmUm | SEQ ID NO: 1364 | UACCCGUCUAGCA GCUGAUGU | SEQ ID NO: 812 |
| ETX-S00004129 | iaiaCmsAmsAmAmUmCmUmGmAfUfGfG mGmAmUmUmAmAmGmGmGm | SEQ ID NO: 1365 | CAAAUCUGAUGGG AUUAAGGG | SEQ ID NO: 813 |
| ETX-S00004133 | iaiaGmsUmsAmCmCmCmUmCmUfUfGfA mUmUmUmUmGmCmCmCmGm | SEQ ID NO: 1366 | GUACCCUCUUGAU UUUGCCCG | SEQ ID NO: 814 |
| ETX-S00004137 | iaiaCmsCmsUmGmUmAmCmCmAfAfGfG mCmUmUmUmAmAmCmGmUm | SEQ ID NO: 1367 | CCUGUACCAAGGC UUUAACGU | SEQ ID NO: 815 |
| ETX-S00004141 | iaiaUmsGmsGmGmAmUmUmAmAfGfGf GmCmCmUmGmUmAmCmCmAm | SEQ ID NO: 1368 | UGGGAUUAAGGGC CUGUACCA | SEQ ID NO: 816 |
| ETX-S00004145 | iaiaAmsUmsUmAmUmCmAmUmCfUfAfC mCmGmAmGmCmCmGmCmCm | SEQ ID NO: 1369 | AUUAUCAUCUACC GAGCCGCC | SEQ ID NO: 817 |
| ETX-S00004149 | iaiaGmsCmsCmUmGmUmAmCmCfAfAfG mGmCmUmUmUmAmAmCmGm | SEQ ID NO: 1370 | GCCUGUACCAAGG CUUUAACG | SEQ ID NO: 818 |
| ETX-S00004153 | iaiaAmsUmsGmGmGmAmUmUmAfAfGf GmGmCmCmUmGmUmAmCmCm | SEQ ID NO: 1371 | AUGGGAUUAAGGG CCUGUACC | SEQ ID NO: 819 |
| ETX-S00004157 | iaiaAmsCmsGmUmGmUmCmUmGfUfGfC mAmGmGmGmUmAmUmUmAm | SEQ ID NO: 1372 | ACGUGUCUGUGCA GGGUAUUA | SEQ ID NO: 820 |
| ETX-S00004161 | iaiaGmsGmsCmAmUmUmAmUmAfGfAfC mUmGmCmGmUmGmGmUmCm | SEQ ID NO: 1373 | GGCAUUAUAGACU GCGUGGUC | SEQ ID NO: 821 |
| ETX-S00004165 | iaiaUmsUmsCmCmCmCmAmCmCfCfAfG mGmCmUmCmUmUmAmAmCm | SEQ ID NO: 1374 | UUCCCCACCCAGG CUCUUAAC | SEQ ID NO: 822 |
| ETX-S00004169 | iaiaAmsUmsGmUmCmAmUmCmAfGfAfU mAmCmUmUmCmCmCmCmAm | SEQ ID NO: 1375 | AUGUCAUCAGAUA CUUCCCCA | SEQ ID NO: 823 |
| ETX-S00004173 | iaiaGmsUmsCmAmUmCmAmGmAfUfAfC mUmUmCmCmCmCmAmCmCm | SEQ ID NO: 1376 | GUCAUCAGAUACU UCCCCACC | SEQ ID NO: 824 |
| ETX-S00004177 | iaiaUmsGmsUmCmAmUmCmAmGfAfUfA mCmUmUmCmCmCmCmAmCm | SEQ ID NO: 1377 | UGUCAUCAGAUAC UUCCCCAC | SEQ ID NO: 825 |
| ETX-S00004181 | iaiaAmsAmsUmGmUmCmAmUmCfAfGfA mUmAmCmUmUmCmCmCmCm | SEQ ID NO: 1378 | AAUGUCAUCAGAU ACUUCCCC | SEQ ID NO: 826 |
| ETX-S00004185 | iaiaCmsUmsUmCmCmCmCmAmCfCfCfA mGmGmCmUmCmUmUmAmAm | SEQ ID NO: 1379 | CUUCCCCACCCAG GCUCUUAA | SEQ ID NO: 827 |
| ETX-S00004189 | iaiaCmsUmsAmCmCmGmAmGmCfCfGfC mCmUmAmCmUmUmCmGmGm | SEQ ID NO: 1380 | CUACCGAGCCGCC UACUUCGG | SEQ ID NO: 828 |

As used herein, and in particular in Tables 3 and 4, the following abbreviations are used for modified nucleosides:
A - adenosine
C - cytidine
G - guanosine
T - thymidine
m - 2'-O-methyl
f - 2'fluro
s - phosphorothioate bond
o - thermally destabilised nucleoside
ia - inverted abasic nucleoside

Am stands for 2'-O-methyl-adenosine, Cm stands for 2'-O-methyl-cytidine, Gm stands for 2'-O-methyl-guanosine, Um stands for 2'-O-methyl-uridine, Af stands for 2'-Fluoro-adenosine, Cf stands for 2'-Fluoro-cytidine, Gf stands for 2'-Fluoro-guanosine and Uf stands for 2'-Fluoro-uridine.

Furthermore, the letter "s" is used as abbreviation for a phosphorothioate linkage between two consecutive (modified) nucleosides. For example, the abbreviation "AmsAm" is used for two consecutive 2'-O-methyl-adenosine nucleosides that are linked via a 3'5' phosphorothioate linkage. No abbreviation is used for nucleosides that are linked via a standard 3'5' phosphodiester linkage. For example, the abbreviation "AmAm" is used for two consecutive 2'-O-methyl-adenosine nucleosides that are linked via a 3'5' phosphodiester linkage.

Some of the modified second strand sequences as illustrated above in Table 4 include the preferred 5' iaia motif. However, it should also be understood that the scope of these modified second strand sequences additionally includes the Me / F modified second strand in the absence of the 5'iaia motif.

In certain embodiments, the nucleic acid comprises a first strand that comprises, consists of, or consists essentially of a (modified) nucleoside sequence differing by 0 or 1 nucleosides from any one of SEQ ID NO: 829-1104;
and a second strand that comprises, consists of, or consists essentially of a (modified) nucleoside sequence differing by 0 or 1 nucleosides from any one of SEQ ID NO:1105-1380.

Preferred combinations of complementary modified antisense (first) and sense (second) strands are listed below in Table 5.

Table 5 identifies duplexes with Duplex IDs referencing the modified antisense and sense IDs from previous Tables 3 and 4.

**Table 5**

| Duplex ID | First (Antisense) strand ID | Second (Sense) strand ID |
|---|---|---|
| ETX-M00001351 | ETX-S00003090 | ETX-S00003089 |
| ETX-M00001352 | ETX-S00003094 | ETX-S00003093 |
| ETX-M00001353 | ETX-S00003098 | ETX-S00003097 |
| ETX-M00001354 | ETX-S00003102 | ETX-S00003101 |
| ETX-M00001355 | ETX-S00003106 | ETX-S00003105 |
| ETX-M00001356 | ETX-S00003110 | ETX-S00003109 |
| ETX-M00001357 | ETX-S00003114 | ETX-S00003113 |
| ETX-M00001358 | ETX-S00003118 | ETX-S00003117 |
| ETX-M00001359 | ETX-S00003122 | ETX-S00003121 |
| ETX-M00001360 | ETX-S00003126 | ETX-S00003125 |
| ETX-M00001361 | ETX-S00003130 | ETX-S00003129 |
| ETX-M00001362 | ETX-S00003134 | ETX-S00003133 |
| ETX-M00001363 | ETX-S00003138 | ETX-S00003137 |
| ETX-M00001364 | ETX-S00003142 | ETX-S00003141 |
| ETX-M00001365 | ETX-S00003146 | ETX-S00003145 |
| ETX-M00001366 | ETX-S00003150 | ETX-S00003149 |
| ETX-M00001367 | ETX-S00003154 | ETX-S00003153 |
| ETX-M00001368 | ETX-S00003158 | ETX-S00003157 |
| ETX-M00001369 | ETX-S00003162 | ETX-S00003161 |
| ETX-M00001370 | ETX-S00003166 | ETX-S00003165 |
| ETX-M00001371 | ETX-S00003170 | ETX-S00003169 |
| ETX-M00001372 | ETX-S00003174 | ETX-S00003173 |
| ETX-M00001373 | ETX-S00003178 | ETX-S00003177 |
| ETX-M00001374 | ETX-S00003182 | ETX-S00003181 |
| ETX-M00001375 | ETX-S00003186 | ETX-S00003185 |
| ETX-M00001376 | ETX-S00003190 | ETX-S00003189 |
| ETX-M00001377 | ETX-S00003194 | ETX-S00003193 |
| ETX-M00001378 | ETX-S00003198 | ETX-S00003197 |
| ETX-M00001379 | ETX-S00003202 | ETX-S00003201 |
| ETX-M00001380 | ETX-S00003206 | ETX-S00003205 |
| ETX-M00001381 | ETX-S00003210 | ETX-S00003209 |
| ETX-M00001382 | ETX-S00003214 | ETX-S00003213 |
| ETX-M00001383 | ETX-S00003218 | ETX-S00003217 |
| ETX-M00001384 | ETX-S00003222 | ETX-S00003221 |
| ETX-M00001385 | ETX-S00003226 | ETX-S00003225 |
| ETX-M00001386 | ETX-S00003230 | ETX-S00003229 |
| ETX-M00001387 | ETX-S00003234 | ETX-S00003233 |
| ETX-M00001388 | ETX-S00003238 | ETX-S00003237 |
| ETX-M00001389 | ETX-S00003242 | ETX-S00003241 |
| ETX-M00001390 | ETX-S00003246 | ETX-S00003245 |
| ETX-M00001391 | ETX-S00003250 | ETX-S00003249 |
| ETX-M00001392 | ETX-S00003254 | ETX-S00003253 |
| ETX-M00001393 | ETX-S00003258 | ETX-S00003257 |
| ETX-M00001394 | ETX-S00003262 | ETX-S00003261 |
| ETX-M00001395 | ETX-S00003266 | ETX-S00003265 |
| ETX-M00001396 | ETX-S00003270 | ETX-S00003269 |
| ETX-M00001397 | ETX-S00003274 | ETX-S00003273 |
| ETX-M00001398 | ETX-S00003278 | ETX-S00003277 |
| ETX-M00001399 | ETX-S00003282 | ETX-S00003281 |
| ETX-M00001400 | ETX-S00003286 | ETX-S00003285 |
| ETX-M00001401 | ETX-S00003290 | ETX-S00003289 |
| ETX-M00001402 | ETX-S00003294 | ETX-S00003293 |
| ETX-M00001403 | ETX-S00003298 | ETX-S00003297 |
| ETX-M00001404 | ETX-S00003302 | ETX-S00003301 |
| ETX-M00001405 | ETX-S00003306 | ETX-S00003305 |
| ETX-M00001406 | ETX-S00003310 | ETX-S00003309 |
| ETX-M00001407 | ETX-S00003314 | ETX-S00003313 |
| ETX-M00001408 | ETX-S00003318 | ETX-S00003317 |
| ETX-M00001409 | ETX-S00003322 | ETX-S00003321 |
| ETX-M00001410 | ETX-S00003326 | ETX-S00003325 |
| ETX-M00001411 | ETX-S00003330 | ETX-S00003329 |
| ETX-M00001412 | ETX-S00003334 | ETX-S00003333 |
| ETX-M00001413 | ETX-S00003338 | ETX-S00003337 |
| ETX-M00001414 | ETX-S00003342 | ETX-S00003341 |
| ETX-M00001415 | ETX-S00003346 | ETX-S00003345 |
| ETX-M00001416 | ETX-S00003350 | ETX-S00003349 |
| ETX-M00001417 | ETX-S00003354 | ETX-S00003353 |
| ETX-M00001418 | ETX-S00003358 | ETX-S00003357 |
| ETX-M00001419 | ETX-S00003362 | ETX-S00003361 |
| ETX-M00001420 | ETX-S00003366 | ETX-S00003365 |
| ETX-M00001421 | ETX-S00003370 | ETX-S00003369 |
| ETX-M00001422 | ETX-S00003374 | ETX-S00003373 |
| ETX-M00001423 | ETX-S00003378 | ETX-S00003377 |
| ETX-M00001424 | ETX-S00003382 | ETX-S00003381 |
| ETX-M00001425 | ETX-S00003386 | ETX-S00003385 |
| ETX-M00001426 | ETX-S00003390 | ETX-S00003389 |
| ETX-M00001427 | ETX-S00003394 | ETX-S00003393 |
| ETX-M00001428 | ETX-S00003398 | ETX-S00003397 |
| ETX-M00001429 | ETX-S00003402 | ETX-S00003401 |
| ETX-M00001430 | ETX-S00003406 | ETX-S00003405 |
| ETX-M00001431 | ETX-S00003410 | ETX-S00003409 |
| ETX-M00001432 | ETX-S00003414 | ETX-S00003413 |
| ETX-M00001433 | ETX-S00003418 | ETX-S00003417 |
| ETX-M00001434 | ETX-S00003422 | ETX-S00003421 |
| ETX-M00001435 | ETX-S00003426 | ETX-S00003425 |
| ETX-M00001436 | ETX-S00003430 | ETX-S00003429 |
| ETX-M00001437 | ETX-S00003434 | ETX-S00003433 |
| ETX-M00001438 | ETX-S00003438 | ETX-S00003437 |
| ETX-M00001439 | ETX-S00003442 | ETX-S00003441 |
| ETX-M00001440 | ETX-S00003446 | ETX-S00003445 |
| ETX-M00001441 | ETX-S00003450 | ETX-S00003449 |
| ETX-M00001442 | ETX-S00003454 | ETX-S00003453 |
| ETX-M00001443 | ETX-S00003458 | ETX-S00003457 |
| ETX-M00001444 | ETX-S00003462 | ETX-S00003461 |
| ETX-M00001445 | ETX-S00003466 | ETX-S00003465 |
| ETX-M00001446 | ETX-S00003470 | ETX-S00003469 |
| ETX-M00001447 | ETX-S00003474 | ETX-S00003473 |
| ETX-M00001448 | ETX-S00003478 | ETX-S00003477 |
| ETX-M00001449 | ETX-S00003482 | ETX-S00003481 |
| ETX-M00001450 | ETX-S00003486 | ETX-S00003485 |
| ETX-M00001451 | ETX-S00003490 | ETX-S00003489 |
| ETX-M00001452 | ETX-S00003494 | ETX-S00003493 |
| ETX-M00001453 | ETX-S00003498 | ETX-S00003497 |
| ETX-M00001454 | ETX-S00003502 | ETX-S00003501 |
| ETX-M00001455 | ETX-S00003506 | ETX-S00003505 |
| ETX-M00001456 | ETX-S00003510 | ETX-S00003509 |
| ETX-M00001457 | ETX-S00003514 | ETX-S00003513 |
| ETX-M00001458 | ETX-S00003518 | ETX-S00003517 |
| ETX-M00001459 | ETX-S00003522 | ETX-S00003521 |
| ETX-M00001460 | ETX-S00003526 | ETX-S00003525 |
| ETX-M00001461 | ETX-S00003530 | ETX-S00003529 |
| ETX-M00001462 | ETX-S00003534 | ETX-S00003533 |
| ETX-M00001463 | ETX-S00003538 | ETX-S00003537 |
| ETX-M00001464 | ETX-S00003542 | ETX-S00003541 |
| ETX-M00001465 | ETX-S00003546 | ETX-S00003545 |
| ETX-M00001466 | ETX-S00003550 | ETX-S00003549 |
| ETX-M00001467 | ETX-S00003554 | ETX-S00003553 |
| ETX-M00001468 | ETX-S00003558 | ETX-S00003557 |
| ETX-M00001469 | ETX-S00003562 | ETX-S00003561 |
| ETX-M00001470 | ETX-S00003566 | ETX-S00003565 |
| ETX-M00001471 | ETX-S00003570 | ETX-S00003569 |
| ETX-M00001472 | ETX-S00003574 | ETX-S00003573 |
| ETX-M00001473 | ETX-S00003578 | ETX-S00003577 |
| ETX-M00001474 | ETX-S00003582 | ETX-S00003581 |
| ETX-M00001475 | ETX-S00003586 | ETX-S00003585 |
| ETX-M00001476 | ETX-S00003590 | ETX-S00003589 |
| ETX-M00001477 | ETX-S00003594 | ETX-S00003593 |
| ETX-M00001478 | ETX-S00003598 | ETX-S00003597 |
| ETX-M00001479 | ETX-S00003602 | ETX-S00003601 |
| ETX-M00001480 | ETX-S00003606 | ETX-S00003605 |
| ETX-M00001481 | ETX-S00003610 | ETX-S00003609 |
| ETX-M00001482 | ETX-S00003614 | ETX-S00003613 |
| ETX-M00001483 | ETX-S00003618 | ETX-S00003617 |
| ETX-M00001484 | ETX-S00003622 | ETX-S00003621 |
| ETX-M00001485 | ETX-S00003626 | ETX-S00003625 |
| ETX-M00001486 | ETX-S00003630 | ETX-S00003629 |
| ETX-M00001487 | ETX-S00003634 | ETX-S00003633 |
| ETX-M00001488 | ETX-S00003638 | ETX-S00003637 |
| ETX-M00001489 | ETX-S00003642 | ETX-S00003641 |
| ETX-M00001490 | ETX-S00003646 | ETX-S00003645 |
| ETX-M00001491 | ETX-S00003650 | ETX-S00003649 |
| ETX-M00001492 | ETX-S00003654 | ETX-S00003653 |
| ETX-M00001493 | ETX-S00003658 | ETX-S00003657 |
| ETX-M00001494 | ETX-S00003662 | ETX-S00003661 |
| ETX-M00001495 | ETX-S00003666 | ETX-S00003665 |
| ETX-M00001496 | ETX-S00003670 | ETX-S00003669 |
| ETX-M00001497 | ETX-S00003674 | ETX-S00003673 |
| ETX-M00001498 | ETX-S00003678 | ETX-S00003677 |
| ETX-M00001499 | ETX-S00003682 | ETX-S00003681 |
| ETX-M00001500 | ETX-S00003686 | ETX-S00003685 |
| ETX-M00001501 | ETX-S00003690 | ETX-S00003689 |
| ETX-M00001502 | ETX-S00003694 | ETX-S00003693 |
| ETX-M00001503 | ETX-S00003698 | ETX-S00003697 |
| ETX-M00001504 | ETX-S00003702 | ETX-S00003701 |
| ETX-M00001505 | ETX-S00003706 | ETX-S00003705 |
| ETX-M00001506 | ETX-S00003710 | ETX-S00003709 |
| ETX-M00001507 | ETX-S00003714 | ETX-S00003713 |
| ETX-M00001508 | ETX-S00003718 | ETX-S00003717 |
| ETX-M00001509 | ETX-S00003722 | ETX-S00003721 |
| ETX-M00001510 | ETX-S00003726 | ETX-S00003725 |
| ETX-M00001511 | ETX-S00003730 | ETX-S00003729 |
| ETX-M00001512 | ETX-S00003734 | ETX-S00003733 |
| ETX-M00001513 | ETX-S00003738 | ETX-S00003737 |
| ETX-M00001514 | ETX-S00003742 | ETX-S00003741 |
| ETX-M00001515 | ETX-S00003746 | ETX-S00003745 |
| ETX-M00001516 | ETX-S00003750 | ETX-S00003749 |
| ETX-M00001517 | ETX-S00003754 | ETX-S00003753 |
| ETX-M00001518 | ETX-S00003758 | ETX-S00003757 |
| ETX-M00001519 | ETX-S00003762 | ETX-S00003761 |
| ETX-M00001520 | ETX-S00003766 | ETX-S00003765 |
| ETX-M00001521 | ETX-S00003770 | ETX-S00003769 |
| ETX-M00001522 | ETX-S00003774 | ETX-S00003773 |
| ETX-M00001523 | ETX-S00003778 | ETX-S00003777 |
| ETX-M00001524 | ETX-S00003782 | ETX-S00003781 |
| ETX-M00001525 | ETX-S00003786 | ETX-S00003785 |
| ETX-M00001526 | ETX-S00003790 | ETX-S00003789 |
| ETX-M00001527 | ETX-S00003794 | ETX-S00003793 |
| ETX-M00001528 | ETX-S00003798 | ETX-S00003797 |
| ETX-M00001529 | ETX-S00003802 | ETX-S00003801 |
| ETX-M00001530 | ETX-S00003806 | ETX-S00003805 |
| ETX-M00001531 | ETX-S00003810 | ETX-S00003809 |
| ETX-M00001532 | ETX-S00003814 | ETX-S00003813 |
| ETX-M00001533 | ETX-S00003818 | ETX-S00003817 |
| ETX-M00001534 | ETX-S00003822 | ETX-S00003821 |
| ETX-M00001535 | ETX-S00003826 | ETX-S00003825 |
| ETX-M00001536 | ETX-S00003830 | ETX-S00003829 |
| ETX-M00001537 | ETX-S00003834 | ETX-S00003833 |
| ETX-M00001538 | ETX-S00003838 | ETX-S00003837 |
| ETX-M00001539 | ETX-S00003842 | ETX-S00003841 |
| ETX-M00001540 | ETX-S00003846 | ETX-S00003845 |
| ETX-M00001541 | ETX-S00003850 | ETX-S00003849 |
| ETX-M00001542 | ETX-S00003854 | ETX-S00003853 |
| ETX-M00001543 | ETX-S00003858 | ETX-S00003857 |
| ETX-M00001544 | ETX-S00003862 | ETX-S00003861 |
| ETX-M00001545 | ETX-S00003866 | ETX-S00003865 |
| ETX-M00001546 | ETX-S00003870 | ETX-S00003869 |
| ETX-M00001547 | ETX-S00003874 | ETX-S00003873 |
| ETX-M00001548 | ETX-S00003878 | ETX-S00003877 |
| ETX-M00001549 | ETX-S00003882 | ETX-S00003881 |
| ETX-M00001550 | ETX-S00003886 | ETX-S00003885 |
| ETX-M00001551 | ETX-S00003890 | ETX-S00003889 |
| ETX-M00001552 | ETX-S00003894 | ETX-S00003893 |
| ETX-M00001553 | ETX-S00003898 | ETX-S00003897 |
| ETX-M00001554 | ETX-S00003902 | ETX-S00003901 |
| ETX-M00001555 | ETX-S00003906 | ETX-S00003905 |
| ETX-M00001556 | ETX-S00003910 | ETX-S00003909 |
| ETX-M00001557 | ETX-S00003914 | ETX-S00003913 |
| ETX-M00001558 | ETX-S00003918 | ETX-S00003917 |
| ETX-M00001559 | ETX-S00003922 | ETX-S00003921 |
| ETX-M00001560 | ETX-S00003926 | ETX-S00003925 |
| ETX-M00001561 | ETX-S00003930 | ETX-S00003929 |
| ETX-M00001562 | ETX-S00003934 | ETX-S00003933 |
| ETX-M00001563 | ETX-S00003938 | ETX-S00003937 |
| ETX-M00001564 | ETX-S00003942 | ETX-S00003941 |
| ETX-M00001565 | ETX-S00003946 | ETX-S00003945 |
| ETX-M00001566 | ETX-S00003950 | ETX-S00003949 |
| ETX-M00001567 | ETX-S00003954 | ETX-S00003953 |
| ETX-M00001568 | ETX-S00003958 | ETX-S00003957 |
| ETX-M00001569 | ETX-S00003962 | ETX-S00003961 |
| ETX-M00001570 | ETX-S00003966 | ETX-S00003965 |
| ETX-M00001571 | ETX-S00003970 | ETX-S00003969 |
| ETX-M00001572 | ETX-S00003974 | ETX-S00003973 |
| ETX-M00001573 | ETX-S00003978 | ETX-S00003977 |
| ETX-M00001574 | ETX-S00003982 | ETX-S00003981 |
| ETX-M00001575 | ETX-S00003986 | ETX-S00003985 |
| ETX-M00001576 | ETX-S00003990 | ETX-S00003989 |
| ETX-M00001577 | ETX-S00003994 | ETX-S00003993 |
| ETX-M00001578 | ETX-S00003998 | ETX-S00003997 |
| ETX-M00001579 | ETX-S00004002 | ETX-S00004001 |
| ETX-M00001580 | ETX-S00004006 | ETX-S00004005 |
| ETX-M00001581 | ETX-S00004010 | ETX-S00004009 |
| ETX-M00001582 | ETX-S00004014 | ETX-S00004013 |
| ETX-M00001583 | ETX-S00004018 | ETX-S00004017 |
| ETX-M00001584 | ETX-S00004022 | ETX-S00004021 |
| ETX-M00001585 | ETX-S00004026 | ETX-S00004025 |
| ETX-M00001586 | ETX-S00004030 | ETX-S00004029 |
| ETX-M00001587 | ETX-S00004034 | ETX-S00004033 |
| ETX-M00001588 | ETX-S00004038 | ETX-S00004037 |
| ETX-M00001589 | ETX-S00004042 | ETX-S00004041 |
| ETX-M00001590 | ETX-S00004046 | ETX-S00004045 |
| ETX-M00001591 | ETX-S00004050 | ETX-S00004049 |
| ETX-M00001592 | ETX-S00004054 | ETX-S00004053 |
| ETX-M00001593 | ETX-S00004058 | ETX-S00004057 |
| ETX-M00001594 | ETX-S00004062 | ETX-S00004061 |
| ETX-M00001595 | ETX-S00004066 | ETX-S00004065 |
| ETX-M00001596 | ETX-S00004070 | ETX-S00004069 |
| ETX-M00001597 | ETX-S00004074 | ETX-S00004073 |
| ETX-M00001598 | ETX-S00004078 | ETX-S00004077 |
| ETX-M00001599 | ETX-S00004082 | ETX-S00004081 |
| ETX-M00001600 | ETX-S00004086 | ETX-S00004085 |
| ETX-M00001601 | ETX-S00004090 | ETX-S00004089 |
| ETX-M00001602 | ETX-S00004094 | ETX-S00004093 |
| ETX-M00001603 | ETX-S00004098 | ETX-S00004097 |
| ETX-M00001604 | ETX-S00004102 | ETX-S00004101 |
| ETX-M00001605 | ETX-S00004106 | ETX-S00004105 |
| ETX-M00001606 | ETX-S00004110 | ETX-S00004109 |
| ETX-M00001607 | ETX-S00004114 | ETX-S00004113 |
| ETX-M00001608 | ETX-S00004118 | ETX-S00004117 |
| ETX-M00001609 | ETX-S00004122 | ETX-S00004121 |
| ETX-M00001610 | ETX-S00004126 | ETX-S00004125 |
| ETX-M00001611 | ETX-S00004130 | ETX-S00004129 |
| ETX-M00001612 | ETX-S00004134 | ETX-S00004133 |
| ETX-M00001613 | ETX-S00004138 | ETX-S00004137 |
| ETX-M00001614 | ETX-S00004142 | ETX-S00004141 |
| ETX-M00001615 | ETX-S00004146 | ETX-S00004145 |
| ETX-M00001616 | ETX-S00004150 | ETX-S00004149 |
| ETX-M00001617 | ETX-S00004154 | ETX-S00004153 |
| ETX-M00001618 | ETX-S00004158 | ETX-S00004157 |
| ETX-M00001619 | ETX-S00004162 | ETX-S00004161 |
| ETX-M00001620 | ETX-S00004166 | ETX-S00004165 |
| ETX-M00001621 | ETX-S00004170 | ETX-S00004169 |
| ETX-M00001622 | ETX-S00004174 | ETX-S00004173 |
| ETX-M00001623 | ETX-S00004178 | ETX-S00004177 |
| ETX-M00001624 | ETX-S00004182 | ETX-S00004181 |
| ETX-M00001625 | ETX-S00004186 | ETX-S00004185 |
| ETX-M00001626 | ETX-S00004190 | ETX-S00004189 |

For duplexes of Table 5:
ETX-M00001351 - ETX-M00001626 preferably have a duplex structure according to Figure 8b.

In a particularly preferred embodiment, the invention relates to a nucleic acid comprising first and second strands that comprise, consist of, or consist essentially of a nucleoside sequence differing by 0 or 1 nucleosides from any one of the following first and second sequences:

| Duplex ID | Modified first strand | Modified second strand |
|---|---|---|
| ETX-M00001378 | ETX-S00003198 (SEQ ID NO: 856) | ETX-S00003197 (SEQ ID NO: 1132) |
| ETX-M00001397 | ETX-S00003274 (SEQ ID NO: 875) | ETX-S00003273 (SEQ ID NO: 1151) |
| ETX-M00001513 | ETX-S00003738 (SEQ ID NO: 991) | ETX-S00003737 (SEQ ID NO: 1267) |
| ETX-M00001527 | ETX-S00003794 (SEQ ID NO: 1005) | ETX-S00003793 (SEQ ID NO: 1281) |
| ETX-M00001570 | ETX-S00003966 (SEQ ID NO: 1048) | ETX-S00003965 (SEQ ID NO: 1324) |

In an even more preferred embodiment, the invention relates to a nucleic acid comprising first and second strands that comprise, consist of, or consist essentially of a nucleoside sequence differing by 0 or 1 nucleosides from any one of the following first and second sequences:

| Duplex ID | Modified first strand | Modified second strand |
|---|---|---|
| ETX-M00001378 | ETX-S00003198 (SEQ ID NO: 856) | ETX-S00003197 (SEQ ID NO: 1132) |

In case of ambiguity between the sequences in this specification and the sequences in the attached sequence listing, the sequences provided herein are considered to be the correct sequences.

### ABASIC NUCLEOTIDES

In certain embodiments, there are 1, e.g. 2, e.g. 3, e.g. 4 or more abasic nucleosides present in nucleic acids according to the invention. Abasic nucleosides are modified nucleosides because they lack the base normally seen at position 1 of the sugar moiety. Typically, there will be a hydrogen at position 1 of the sugar moiety of the abasic nucleosides present in a nucleic acid according to the present invention.

The abasic nucleosides are in the terminal region of the second strand, preferably located within the terminal 5 nucleosides of the end of the strand. The terminal region may be the terminal 5 nucleosides, which includes abasic nucleosides.

The second strand may comprise, as preferred features (which are all specifically contemplated in combination unless mutually exclusive):
2, or more than 2, abasic nucleosides in a terminal region of the second strand; and/or
2, or more than 2, abasic nucleosides in either the 5' or 3' terminal region of the second strand; and/or
2, or more than 2, abasic nucleosides in either the 5' or 3' terminal region of the second strand, wherein the abasic nucleosides are present in an overhang as herein described; and/or
2, or more than 2, consecutive abasic nucleosides in a terminal region of the second strand, wherein preferably one such abasic nucleosides is a terminal nucleosides; and/or
2, or more than 2, consecutive abasic nucleosides in either the 5' or 3' terminal region of the second strand, wherein preferably one such abasic nucleosides is a terminal nucleosides in either the 5' or 3' terminal region of the second strand; and/or
a reversed internucleoside linkage connects at least one abasic nucleoside to an adjacent basic nucleoside in a terminal region of the second strand; and/or
a reversed internucleoside linkage connects at least one abasic nucleoside to an adjacent basic nucleoside in either the 5' or 3' terminal region of the second strand; and/or
an abasic nucleoside as the penultimate nucleoside which is connected via the reversed linkage to the nucleoside which is not the terminal nucleoside (called the antepenultimate nucleoside herein); and/or
abasic nucleosides as the 2 terminal nucleosides connected via a 5'-3' linkage when reading the strand in the direction towards the terminus comprising the terminal nucleosides;
abasic nucleosides as the 2 terminal nucleosides connected via a 3'-5' linkage when reading the strand in the direction towards the terminus comprising the terminal nucleosides;
abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
abasic nucleosides as the terminal 2 positions, wherein the penultimate nucleoside is connected via the reversed linkage to the antepenultimate nucleoside, and wherein either
   (1) the reversed linkage is a 5-5' reversed linkage and the linkage between the terminal and penultimate abasic nucleosides is 3'5' when reading towards the terminus comprising the terminal and penultimate abasic nucleosides; or
   (2) the reversed linkage is a 3-3' reversed linkage and the linkage between the terminal and penultimate abasic nucleosides is 5'3' when reading towards the terminus comprising the terminal and penultimate abasic nucleosides.

Preferably there is an abasic nucleoside at the terminus of the second strand.

Preferably there are 2 or at least 2 abasic nucleosides in the terminal region of the second strand, preferably at the terminal and penultimate positions.

Preferably 2 or more abasic nucleosides are consecutive, for example all abasic nucleosides may be consecutive. For example, the terminal 1 or terminal 2 or terminal 3 or terminal 4 nucelotides may be abasic nucleosides.

An abasic nucleoside may also be linked to an adjacent nucleoside through a 5'-3' phosphodiester linkage or reversed linkage unless there is only 1 abasic nucleoside at the terminus, in which case it will have a reversed linkage to the adjacent nucleoside.

A reversed linkage (which may also be referred to as an inverted linkage, which is also seen in the art), comprises either a 5'-5', a 3-'3', a 3'-2' or a 2'-3' phosphodiester linkage between the adjacent sugar moieties of the nucleosides.

Abasic nucleosides which are not terminal will have 2 phosphodiester bonds, one with each adjacent nucleoside, and these may be a reversed linkage or may be a 5'-3 phosphodiester bond or may be one of each.

A preferred embodiment comprises 2 abasic nucleosides at the terminal and penultimate positions of the second strand, and wherein the reversed internucleoside linkage is located between the penultimate (abasic) nucleoside and the antepenultimate nucleoside.

Preferably there are 2 abasic nucleosides at the terminal and penultimate positions of the second strand and the penultimate nucleoside is linked to the antepenultimate nucleoside through a reversed internucleoside linkage and is linked to the terminal nucleoside through a 5'-3' or 3'-5' phosphodiester linkage (reading in the direction of the terminus of the molecule).

Preferably a nucleic acid according to the present invention comprises one or more abasic nucleosides, optionally wherein the one or more abasic nucleosides are in a terminal region of the second strand, and/or wherein at least one abasic nucleoside is linked to an adjacent basic nucleoside through a reversed internucleoside linkage.

Different preferred features are as follows:
The reversed internucleoside linkage is a 3'-3' reversed linkage. The reversed internucleoside linkage is at a terminal region which is distal to the 5' terminal phosphate of the second strand.

The reversed internucleoside linkage is a 5'-5' reversed linkage. The reversed internucleoside linkage is at a terminal region which is distal to the 3' terminal hydroxide of the second strand.

In certain embodiments, the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein: (a) said penultimate abasic nucleoside is connected to an adjacent first basic nucleoside in an adjacent 5' near terminal region through a reversed internucleoside linkage; and (b) the reversed linkage is a 5-5' reversed linkage; and (c) the linkage between the terminal and penultimate abasic nucleosides is 3'5' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 3' terminal region of the second strand.

Alternatively the second strand comprises 2 consecutive abasic nucleosides preferably in an overhang in the 3' terminal region of the second strand, wherein one such abasic nucleoside is a terminal nucleoside at the 3' terminal region of the second strand and the other abasic nucleoside is a penultimate nucleoside at the 3' terminal region of the second strand, wherein: (a) said penultimate abasic nucleoside is connected to an adjacent first basic nucleoside in an adjacent 3' near terminal region through a reversed internucleoside linkage; and (b) the reversed linkage is a 3-3' reversed linkage; and (c) the linkage between the terminal and penultimate abasic nucleosides is 5'-3' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 3' near terminal region of the second strand, wherein a first phosphorothioate internucleoside linkage is present between said adjacent first basic nucleoside of (a) and an adjacent second basic nucleoside in said 3' near terminal region of the second strand, and a second phosphorothioate internucleoside linkage is present between said adjacent second basic nucleoside and an adjacent third basic nucleoside in said 3' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 5' terminal region of the second strand.

Examples of the structures are as follows (where the specific RNA nucleosides shown are not limiting and could be any RNA nucleoside):
A A 3'-3' reversed bond (and also showing the 5'-3 direction of the last phosphodiester bond between the two abasic molecules reading towards the terminus of the molecule) 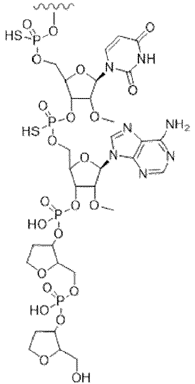
B Illustrating a 5'-5' reversed bond (and also showing the 3'-5' direction of the last phosphodiester bond between the two abasic molecules reading towards the terminus of the molecule)

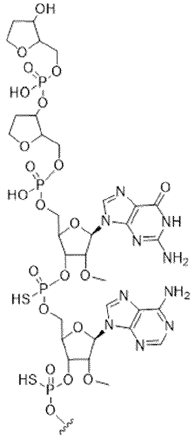

The abasic nucleoside or abasic nucleosides present in the nucleic acid are provided in the presence of a reversed internucleoside linkage or linkages, namely a 5'-5' or a 3'-3' reversed internucleoside linkage. A reversed linkage occurs as a result of a change of orientation of an adjacent nucleoside sugar, such that the sugar will have a 3' - 5' orientation as opposed to the conventional 5' - 3' orientation (with reference to the numbering of ring atoms on the nucleoside sugars). The abasic nucleoside or nucleosides as present in the nucleic acids of the invention preferably include such inverted nucleoside sugars.

In the case of a terminal nucleoside having an inverted orientation, then this will result in an "inverted" end configuration for the overall nucleic acid. Whilst certain structures drawn and referenced herein are represented using conventional 5' - 3' direction (with reference to the numbering of ring atoms on the nucleoside sugars), it will be appreciated that the presence of a terminal nucleoside having a change of orientation and a proximal 3'-3' reversed linkage, will result in a nucleic acid having an overall 5'- 5' end structure (i.e. the conventional 3' end nucleoside becomes a 5' end nucleoside). Alternatively, it will be appreciated that the presence of a terminal nucleoside having a change of orientation and a proximal 5'-5' reversed linkage will result in a nucleic acid with an overall 3'- 3' end structure.

The proximal 3'-3' or 5'-5' reversed linkage as herein described, may comprise the reversed linkage being directly adjacent / attached to a terminal nucleoside having an inverted orientation, such as a single terminal nucleoside having an inverted orientation. Alternatively, the proximal 3'-3' or 5'-5' reversed linkage as herein described, may comprise the reversed linkage being adjacent 2, or more than 2, nucleosides having an inverted orientation, such as 2, or more than 2, terminal region nucleosides having an inverted orientation, such as the terminal and penultimate nucleosides. In this way, the reversed linkage may be attached to a penultimate nucleoside having an inverted orientation. While a skilled addressee will appreciate that inverted orientations as described above can result in nucleic acid molecules having overall 3' - 3' or 5'-5' end structures as described herein, it will also be appreciated that with the presence of one or more additional reversed linkages and / or nucleosides having an inverted orientation, then the overall nucleic acid may have 3' - 5' end structures corresponding to the conventionally positioned 5' / 3' ends.

In one aspect the nucleic acid may have a 3'-3' reversed linkage, and the terminal sugar moiety may comprise a 5' OH rather than a 5' phosphate group at the 5' position of that terminal sugar.

A skilled person would therefore clearly understand that 5'-5', 3'-3' and 3'-5' (reading in the direction of that terminus) end variants of the more conventional 5'-3' structures (with reference to the numbering of ring atoms on the end nucleoside sugars) drawn herein are included in the scope of the disclosure, where a reversed linkage or linkages is / are present.

In the situation of e.g. a reversed internucleoside linkage and / or one or more nucleosides having an inverted orientation creating an inverted end, and where the relative position of a linkage (e.g. to a linker) or the location of an internal feature (such as a modified nucleoside) is defined relative to the 5' or 3' end of the nucleic acid, then the 5' or 3' end is the conventional 5' or 3' end which would have existed had a reversed linkage not been in place, and wherein the conventional 5' or 3' end is determined by consideration of the directionality of the majority of the internal nucleoside linkages and / or nucleoside orientation within the nucleic acid. It is possible to tell from these internal bonds and / or nucleoside orientation which ends of the nucleic acid would constitute the conventional 5' and 3' ends (with reference to the numbering of ring atoms on the end nucleoside sugars) of the molecule absent the reversed linkage.

For example, in the structure shown below there are abasic residues in the first 2 positions located at the "5'" end. Where the terminal nucleoside has an inverted orientation then the "5'" end indicated in the diagram below, which is the conventional 5' end, can in fact comprise a 3' OH in view of the inverted nucleoside at the terminal position. Nevertheless the majority of the molecule will comprise conventional internucleoside linkages that run from the 3' OH of the sugar to the 5' phosphate of the next sugar, when reading in the standard 5' [PO4] to 3' [OH] direction of a nucleic acid molecule (with reference to the numbering of ring atoms on the nucleoside sugars), which can be used to determine the conventional 5' and 3' ends that would be found absent the inverted end configuration.
A 5' A-A-Me-Me-Me-Me-Me-Me-F-Me-F-F-F-Me-Me-Me-Me-Me-Me-Me-Me-Me-Me 3'

The reversed bond is preferably located at the end of the nucleic acid e.g. RNA which is distal to a ligand moiety, such as a GalNAc containing portion, of the molecule.

GalNAc-siRNA constructs with a 5'-GalNAc on the sense strand can have a reversed linkage on the opposite end of the sense strand.

GalNAc-siRNA constructs with a 3'-GalNAc on the sense strand can have a reversed linkage on the opposite end of the sense strand.

In some embodiments, the second (sense) strand of the nucleic acid according to the invention comprises 2 consecutive abasic nucleosides in the 5' terminal region as shown in the following 5' terminal motif 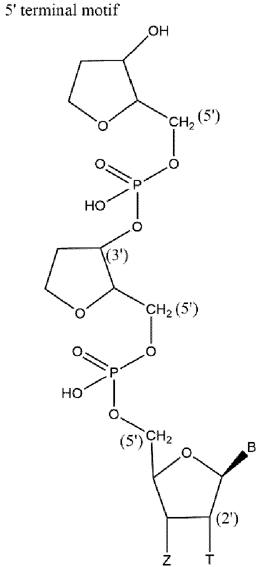 wherein:
B represents a nucleoside base,
T represent H, OH or a 2' ribose modification,
Z represents the remaining nucleosides of said second strand.

In some embodiments, the second (sense) strand of the nucleic acid according to the invention comprises 2 consecutive abasic nucleosides in the 5' terminal region as shown in the following 5' terminal motif 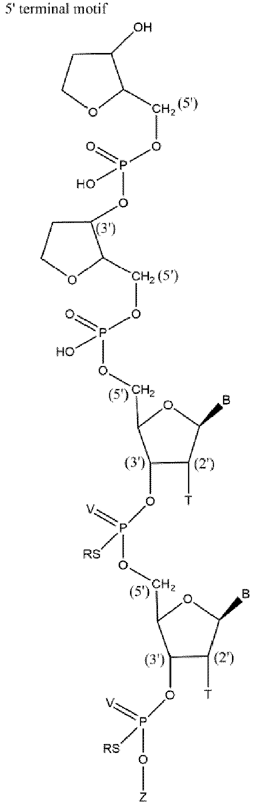 wherein:
B represents a nucleoside base,
T represents H, OH or a 2' ribose modification (preferably a 2' ribose modification, more preferably a 2'Me or 2'F ribose modification),
V represents O or S (preferably O),
R represents H or C₁₋₄ alkyl (preferably H),
Z represents the remaining nucleosides of said second strand,
more preferably the following 5' terminal motif 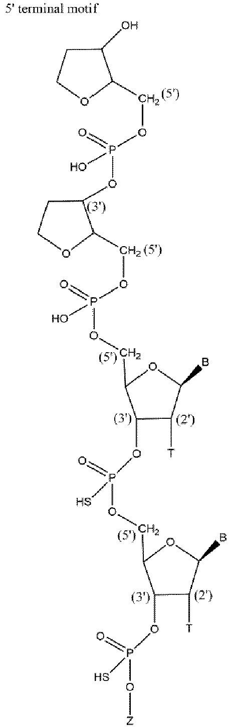
wherein:
   B represents a nucleoside base,
   T represents a 2' ribose modification (preferably a 2'Me or 2'F ribose modification),
   Z represents the remaining nucleosides of said second strand.

The reversed bond is preferably located at the end of the nucleic acid eg RNA which is distal to a ligand moiety, such as a GalNAc containing portion, of the molecule.

GalNAc-siRNA constructs with a 5'-GalNAc on the sense strand can have a reversed linkage on the opposite end of the sense strand.

GalNAc-siRNA constructs with a 3'-GalNAc on the sense strand can have a reversed linkage on the opposite end of the sense strand.

In a preferred embodiment, the second (sense) strand of the nucleic acid according to the invention comprises 2 consecutive abasic nucleosides in the 5' terminal region as shown in the following 5' terminal motif 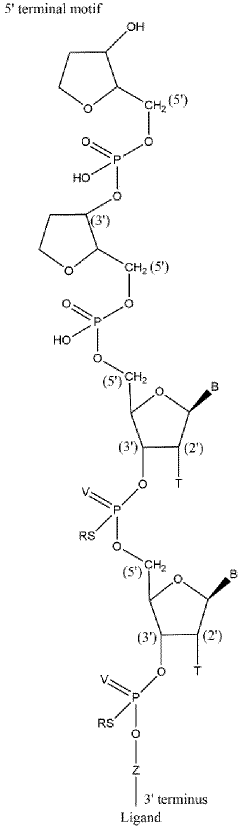 wherein:
B represents a nucleoside base,
T represent H, OH or a 2' ribose modification (preferably a 2' ribose modification, more preferably a 2'Me or 2'F ribose modification),
V represent O or S (preferably O),
R represent H or C₁₋₄ alkyl (preferably H),
Z comprises 11 to 26 contiguous nucleosides, preferably 15 to 21 contiguous nucleosides, and more preferably 19 contiguous nucleosides, more preferably the following 5' terminal motif 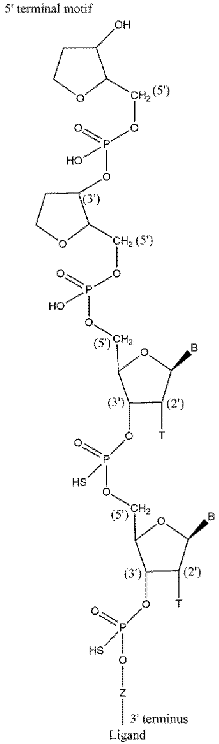
wherein:
   B represents a nucleoside base,
   T represents a 2' ribose modification (preferably a 2'Me or 2'F ribose modification),
   Z comprises 19 contiguous nucleosides.

### NUCLEIC ACID LENGTHS

In one aspect the i) the first strand of the nucleic acid has a length in the range of 17 to 30 nucleosides, preferably 19 to 25 nucleosides, more preferably 19 or 23 nucleosides; and / or ii) the second strand of the nucleic acid has a length in the range of 17 to 30 nucleosides, preferably 19 to 25 nucleosides, more preferably 19 or 21 nucleosides.

Typically, the duplex region of the nucleic acid is between 17 and 30 nucleosides in length, more preferably is 19 or 21 nucleosides in length. Similarly, the region of complementarity between the first strand and the portion of RNA transcribed from the SLC25A5 gene is between 17 and 30 nucleosides in length.

Generally, the duplex structure of the nucleic acid e.g. an iRNA is about 15 to 30 base pairs in length, e.g., 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19- 23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs in length. Ranges and lengths intermediate to the above recited ranges and lengths are also contemplated to be part of the invention.

Similarly, the region of complementarity of an antisense sequence to a target sequence and/or the region of complementarity of an antisense sequence to a sense sequence is about 15 to 30 nucleosides in length, e.g., 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20- 24,20-23, 20-22, 20-21, 21-30, 21-29, 21-28, 21-27, 21-26, 21-25, 21-24, 21-23, or 21-22 nucleosides in length. Ranges and lengths intermediate to the above recited ranges and lengths are also contemplated to be part of the invention.

In certain preferred embodiments, the region of complementarity of an antisense sequence to a target sequence and/or the region of complementarity of an antisense sequence to a sense sequence is at least 17 nucleosides in length. For example, the region of complementarity between the antisense strand and the target is 19 to 21 nucleosides in length, for example, the region of complementarity is 21 nucleosides in length.

In preferred embodiments, each strand is no more than 30 nucleosides in length.

In certain preferred embodiments, the duplex structure of the nucleic acid e.g. an siRNA is 19 or 21 base pairs in length. In particularly preferred embodiment, the duplex may have one of the following structures:
e.g., ETX-M00001351 - ETX-M00001626 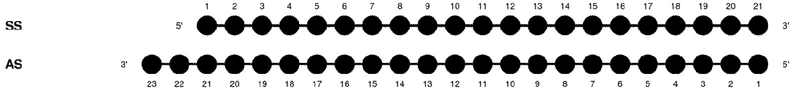
1 2 3 4 5 6 7 8 9 10 11 12 13 14 15 16 17 18 19 20 21
SS 5' 3'
AS 3' 5'
23 22 21 20 19 18 17 16 15 14 13 12 11 10 9 8 7 6 5 4 3 2 1
 or

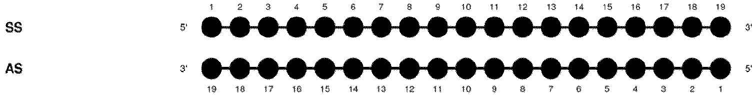
SS 5' 3'
1 2 3 4 5 6 7 8 9 10 11 12 13 14 15 16 17 18 19
AS 3' 5'
19 18 17 16 15 14 13 12 11 10 9 8 7 6 5 4 3 2 1

A nucleic acid e.g. a dsRNA as described herein can further include one or more single-stranded nucleoside overhangs e.g., 1-4, 2-4, 1-3, 2-3, 1, 2, 3, or 4 nucleosides. A nucleoside overhang can comprise or consist of a nucleoside/nucleoside analog, including a deoxynucleoside/nucleoside. The overhang(s) can be on the sense strand, the antisense strand, or any combination thereof. Furthermore, the nucleoside(s) of an overhang can be present on the 5'-end, 3'- end, or both ends of an antisense or sense strand of a nucleic acid e.g. a dsRNA.

In certain preferred embodiments, at least one strand comprises a 3' overhang of at least 1 nucleoside, e.g. , at least one strand comprises a 3' overhang of at least 2 nucleosides. The overhang is suitably on the antisense/ guide strand and/or the sense / passenger strand.

### NUCLEIC ACID MODIFICATIONS

In certain embodiments, the nucleic acid e.g. an RNA of the invention e.g., a dsiRNA, does not comprise further modifications, e.g., chemical modifications or conjugations known in the art and described herein.

In other preferred embodiments, the nucleic acid e.g. RNA of the invention, e.g., a dsiRNA, is further chemically modified to enhance stability or other beneficial characteristics.

In certain embodiments of the invention, substantially all of the nucleosides are modified.

The nucleic acids featured in the invention can be synthesized or modified by methods well established in the art, such as those described in "Current protocols in nucleic acid chemistry," Beaucage, S.L. et al. (Edrs.), John Wiley & Sons, Inc., New York, NY, USA, which is hereby incorporated herein by reference.

Modifications include, for example, end modifications, e.g., 5'-end modifications (phosphorylation, conjugation, inverted linkages) or 3 '-end modifications (conjugation, DNA nucleosides within an RNA, or RNA nucleosides within a DNA, inverted linkages, etc.); base modifications, e.g., replacement with stabilizing bases, destabilizing bases, or bases that base pair with an expanded repertoire of partners, conjugated bases; sugar modifications (e.g. , at the 2'-position or 4'- position) or replacement of the sugar; or backbone modifications, including modification or replacement of the phosphodiester linkages.

Specific examples of nucleic acids such as siRNA compounds useful in the embodiments described herein include, but are not limited to RNAs containing modified backbones or no natural internucleoside linkages. Nucleic acids such as RNAs having modified backbones include, among others, those that do not have a phosphorus atom in the backbone. For the purposes of this specification, and as sometimes referenced in the art, modified nucleic acids e.g. RNAs that do not have a phosphorus atom in their internucleoside backbone can also be considered to be oligonucleosides. In some embodiments, a modified nucleic acid e.g. an siRNA will have a phosphorus atom in its internucleoside backbone.

Modified nucleic acid e.g. RNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5'-linked analogs of these, and those having inverted polarity wherein the adjacent pairs of nucleoside units are linked 5'-3' or 5'-2'. Various salts, mixed salts and free acid forms are also included.

Modified nucleic acids e.g. RNAs can also contain one or more substituted sugar moieties. The nucleic acids e.g. siRNAs, e.g., dsiRNAs, featured herein can include one of the following at the 2'-position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted. 2' O- methyl and 2'-F are preferred modifications.

In certain preferred embodiments, the nucleic acid comprises at least one modified nucleoside.

The nucleic acid of the invention may comprise one or more modified nucleosides on the first strand and/or the second strand.

In some embodiments, substantially all of the nucleosides of the sense strand and all of the nucleosides of the antisense strand comprise a modification.

In some embodiments, all of the nucleosides of the sense strand and substantially all of the nucleosides of the antisense strand comprise a modification.

In some embodiments, all of the nucleosides of the sense strand and all of the nucleosides of the antisense strand comprise a modification.

In one embodiment, at least one of the modified nucleosides is selected from the group consisting of a deoxy- nucleoside, a 3 '-terminal deoxy-thymine (dT) nucleoside, a 2'-O-methyl modified nucleoside (also called herein 2'-Me, where Me is a methoxy) , a 2'-fluoro modified nucleoside, a 2'-deoxy- modified nucleoside, a locked nucleoside, an unlocked nucleoside, a conformationally restricted nucleoside, a constrained ethyl nucleoside, an abasic nucleoside, a 2' -amino- modified nucleoside, a 2'-O-allyl- modified nucleoside, 2' -C-alkyl- modified nucleoside, 2'-hydroxly-modified nucleoside, a 2'- methoxyethyl modified nucleoside, a 2'-O-alkyl-modified nucleoside, a morpholino nucleoside, a phosphoramidate, a non-natural base comprising nucleoside, a tetrahydropyran modified nucleoside, a 1 ,5-anhydrohexitol modified nucleoside, a cyclohexenyl modified nucleoside, a nucleoside comprising a phosphorothioate group, a nucleoside comprising a methylphosphonate group, a nucleoside comprising a 5 '-phosphate, and a nucleoside comprising a 5 '-phosphate mimic. In another embodiment, the modified nucleosides comprise a short sequence of 3 '-terminal deoxy-thymine nucleosides (dT).

Modifications on the nucleosides may preferably be selected from the group including, but not limited to, LNA, HNA, CeNA, 2 -methoxyethyl, 2'-O-alkyl, 2-O-allyl, 2'-C-allyl, 2'-fluoro, 2'-deoxy, 2'-hydroxyl, and combinations thereof. In another embodiment, the modifications on the nucleosides are 2 -O-methyl ("2-Me") or 2'-fluoro modifications.

One preferred modification is a modification at the 2'-OH group of the ribose sugar, optionally selected from 2'-Me or 2'-F modifications.

In certain embodiments, the nucleic acid e.g. RNAi agent further comprises at least one phosphorothioate or methylphosphonate internucleoside linkage. For example the phosphorothioate or methylphosphonate internucleoside linkage can be at the 3 '-terminus or in the terminal region of one strand, i.e. , the sense strand or the antisense strand; or at the ends of both strands, the sense strand and the antisense strand.

In certain embodiments, the phosphorothioate or methylphosphonate internucleoside linkage is at the 5 'terminus or in the terminal region of one strand, i.e. , the sense strand or the antisense strand; or at the ends of both strands, the sense strand and the antisense strand.

In certain embodiments, a phosphorothioate or a methylphosphonate internucleoside linkage is at both the 5'- and 3 '-terminus or in the terminal region of one strand, i.e. , the sense strand or the antisense strand; or at the ends of both strands, the sense strand and the antisense strand.

Any nucleic acid may comprise one or more phosphorothioate (PS) modifications within the nucleic acid, such as at least two PS internucleoside bonds at the ends of a strand.

At least one of the oligoribonucleoside strands preferably comprises at least two consecutive phosphorothioate modifications in the last 3 nucleosides of the oligonucleoside.

The invention therefore also relates to: A nucleic acid disclosed herein which comprises phosphorothioate internucleoside linkages respectively between at least two or three consecutive positions, such as in a 5' and/or 3' terminal region and/or near terminal region of the second strand, whereby said near terminal region is preferably adjacent said terminal region wherein said one or more abasic nucleosides of said second strand is / are located.

A nucleic acid disclosed herein which comprises phosphorothioate internucleoside linkages respectively between at least two or three consecutive positions in a 5' and / or 3' terminal region of the first strand, whereby preferably the terminal position at the 5' and / or 3' terminal region of said first strand is attached to its adjacent position by a phosphorothioate internucleoside linkage.

The nucleic acid strand may be an RNA comprising a phosphorothioate internucleoside linkage between the three nucleosides contiguous with 2 terminally located abasic nucleosides.

A preferred nucleic acid is a double stranded RNA comprising 2 adjacent abasic nucleosides at the 5' terminus of the second strand and a ligand moiety comprising one or more GalNAc ligand moieties at the opposite 3' end of the second strand. Further preferred, the same nucleic acid may also comprise a phosphorothioate bond between nucelotides at positions 3-4 and 4-5 of the second strand, reading from the position 1 of the second strand.

Position 1 of the first or the second strand is the nucleoside which is the closest to the end of the nucleic acid (ignoring any abasic nucleosides) and that is joined to an adjacent nucleoside (at Position 2) via a 3' to 5' internal bond, with reference to the bonds between the sugar moieties of the backbone, and reading in a direction away from that end of the molecule.

It can therefore be seen that "position 1 of the sense strand" is the 5' most nucleoside (not including abasic nucleosides) at the conventional 5' end of the sense strand. Typically, the nucleoside at this position 1 of the sense strand will be equivalent to the 5' nucleoside of the selected target nucleic acid sequence, and more generally the sense strand will have equivalent nucleosides to those of the target nucleic acid sequence starting from this position 1 of the sense strand, whilst also allowing for acceptable mismatches between the sequences.

As used herein, "position 1 of the antisense strand" is the 5' most nucleoside (not including abasic nucleosides) at the conventional 5' end of the antisense strand. As hereinbefore described, there will be a region of complementarity between the sense and antisense strands, and in this way the antisense strand will also have a region of complementarity to the target nucleic acid sequence as referred to above.

Preferred modifications that can be used with sequences according to the present invention can be as follows:
Modification 1:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNfNfNfNfNfNmNmNmNmNmNmNmNfNmNm (5' to 3')
Modification 2:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNfNfNmNfNfNfNfNmNmNmNmNmNmNmNmNm (5' to 3')
Modification 3:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNfNmNfNfNfNfNmNmNmNmNmNmNmNmNm (5' to 3')
Modification 4:
   First strand modification:
      NmsNfsNmNfNmNfNmNmNmNmNmNmNmNfNmNfNmNmNmNmNmsNmsNm (5' to 3')
Second strand modification:
   iaiaNmsNmsNmNmNmNmNfNmNfNfNfNfNmNmNmNmNmNmNmNmNm (5' to 3')
Modification 5:
   First strand modification:
      NmsNfsNmNmNmNfNmNmNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 6:
   First strand modification:
      NmsNfsNmNmNmNfNmNmNmNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 7:
   First strand modification:
      NmsNfsNmNmNmNyNmNmNmNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 8:
   First strand modification:
      NmsNfsNmNmNmNyNmNfNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 9:
   First strand modification:
      NmsNfsNmNmNmNfNmNmNmNmNmNmNmNfNmNfNmNmNmNfNmsNmsNm (5' to 3')
   Second strand modification:
      iaiaNmsNmsNmNmNmNmNmNmNfNfNfNmNmNmNmNmNmNmNmNmNm (5' to 3')
Modification 10:
   First strand modification:
      NmsNfsNmNfNmNfNmNmNmNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 11:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 12:
   First strand modification:
      NmsNfsNmNmNmNfNmNfNfNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 13:
   First strand modification:
      NmsNfsNmNmNmNfNmNfNfNmNmNmNmNfNmNfNmNmNmNfNmsNmsNm (5' to 3')
   Second strand modification:
      iaiaNmsNmsNmNmNmNmNmNmNfNfNfNmNmNmNmNmNmNmNmNmNm (5' to 3')
wherein in each of the above modifications:
ia represents an inverted abasic nucleoside;
Nm represents a 2'Me ribose modified nucleoside;
Nf represents a 2'F ribose modified nucleoside;
Ny represents a nucleoside with a thermally destabilizing modification, preferably wherein the destabilizing modification is selected from a modified unlocked nucleic acid (UNA) and a glycol nucleic acid (GNA), more preferably a glycol nucleic acid, most preferably an (S)-glycol nucleic acid;
s represents a phosphorothioate internucleoside bond.

A particularly preferred modification that can be used with sequences according to the present invention can be:
Modification 6:
First strand modification:
   NmsNfsNmNmNmNfNmNmNmNmNmNmNmNfNmNfNmNfNmNmNmsNmsNm (5' to 3')
Second strand modification:
   iaiaNmsNmsNmNmNmNmNmNmNfNfNfNmNmNmNmNmNmNmNmNmNm (5' to 3')
wherein in each of the above modifications:
ia represents an inverted abasic nucleoside;
Nm represents a 2'Me ribose modified nucleoside; and
s represents a phosphorothioate internucleoside bond.

### CONJUGATION OF NUCLEIC ACID TO LIGAND

Another modification of a nucleic acid e.g. RNA e.g. an siRNA of the invention involves linking the nucleic acid e.g. the siRNA to one or more ligand moieties e.g. to enhance the activity, cellular distribution, or cellular uptake of the nucleic acid e.g. siRNA e.g., into a cell.

In certain embodiments, the inhibitor according to the invention is conjugated to a ligand moiety that enables and/or facilitates targeting of hepatocytes. In certain embodiments, targeting of hepatocytes is achieved using N-acetylgalactosamine (GalNAc) conjugates as described in more detail herein below. That is, in certain embodiments, the inhibitor according to the invention is an siRNA-GalNAc conjugate.

In some embodiments, the ligand moiety described can be attached to a nucleic acid e.g. an siRNA oligonucleoside, via a linker that can be cleavable or non-cleavable. The term "linker" or "linking group" means an organic moiety that connects two parts of a compound, e.g., covalently attaches two parts of a compound.

The ligand can be attached to the 3' or 5' end of the sense strand.

The ligand is preferably conjugated to 3' end of the sense strand of the nucleic acid e.g. an siRNA agent.

The invention therefore relates in a further aspect to a conjugate for inhibiting expression of a target e.g. a target gene, in a cell, said conjugate comprising a nucleic acid portion and one or more ligand moieties, said nucleic acid portion comprising a nucleic acid as disclosed herein.

In one aspect the second strand of the nucleic acid is conjugated directly or indirectly (e.g. via a linker) to the one or more ligand moiety(s), wherein said ligand moiety is typically present at a terminal region of the second strand, preferably at the 3' terminal region thereof.

In certain embodiments, the ligand moiety comprises a GalNAc or GalNAc derivative attached to the nucleic acid e.g. dsiRNA through a linker.

Therefore, the invention relates to a conjugate wherein the ligand moiety comprises
i) one or more GalNAc ligands; and/or
ii) one or more GalNAc ligand derivatives; and/or
iii) one or more GalNAc ligands conjugated to said nucleic acid through a linker.

Said GalNAc ligand may be conjugated directly or indirectly to the 5' or 3' terminal region of the second strand of the nucleic acid, preferably at the 3' terminal region thereof.

GalNAc ligands are well known in the art and described in, inter alia, EP3775207A1.

In some embodiments, the ligand moiety comprises one or more ligands.

In some embodiments, the ligand moiety comprises one or more carbohydrate ligands.

In some embodiments, the one or more carbohydrates can be a monosaccharide, disaccharide, trisaccharide, tetrasaccharide, oligosaccharide and / or polysaccharide.

In some embodiments, the one or more carbohydrates comprise one or more galactose moieties, one or more lactose moieties, one or more N-AcetylGalactosamine moieties, and / or one or more mannose moieties.

In some embodiments, the one or more carbohydrates comprise one or more N-AcetylGalactosamine moieties.

In some embodiments, the compounds as described anywhere herein comprise two or three N-AcetylGalactosamine moieties.

In some embodiments, the one or more ligands are attached in a linear configuration, or in a branched configuration, for example each configuration being respectively attached to a branch point in an overall linker.

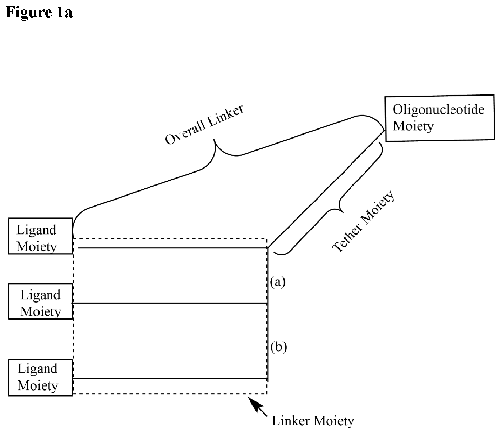

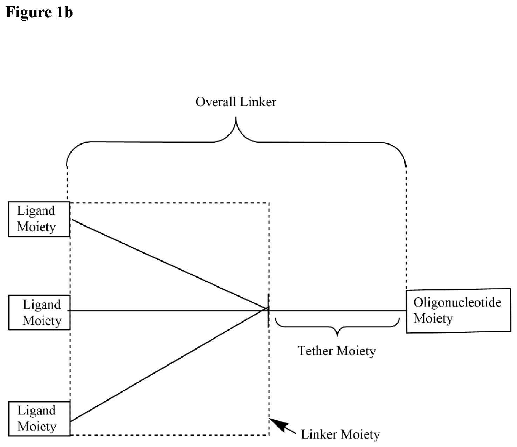

Exemplary linear configurations and Exemplary branched configurations are shown in Figures 1a and 1b:
In Fig 1a, (linear), (a) and / or (b) can typically represent connecting bonds or groups, such as phosphate or phosphorothioate groups.

In Fig 1b, (branched), in some embodiments, the one or more ligands are attached as a biantennary or triantennary branched configuration. Typically, a triantennary branched configuration can be preferred, such as an N-AcetylGalactosamine triantennary branched configuration.

### Linker

Exemplary compounds of the invention comprise a 'linker moiety', such as that as depicted in Formula (I), that is part of an overall 'linker'. 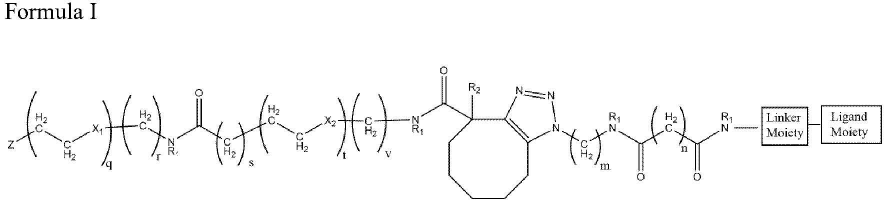 wherein:
R₁ at each occurrence is independently selected from the group consisting of hydrogen, methyl and ethyl;
R₂ is selected from the group consisting of hydrogen, hydroxy, -OC₁₋₃alkyl, -C(=O)OC₁₋₃alkyl, halo and nitro;
X₁ and X₂ at each occurrence are independently selected from the group consisting of methylene, oxygen and sulfur;
m is an integer of from 1 to 6;
n is an integer of from 1 to 10;
q, r, s, t, v are independently integers from 0 to 4, with the proviso that:
   (i) q and r cannot both be 0 at the same time; and
   (ii) s, t and v cannot all be 0 at the same time;
Z is an oligonucleoside moiety.

As will be further understood in the art, exemplary compounds of the invention comprise an overall linker that is located between the oligonucleoside moiety and the ligand moiety of these compounds. The overall linker, thereby 'links' the oligonucleoside moiety and the ligand moiety to each other.

The overall linker is often notionally envisaged as comprising one or more linker building blocks. For example, there is a linker portion that is depicted as the 'linker moiety' as represented in Formula (I) positioned adjacent the ligand moiety and attaching the ligand moiety, typically via a branch point, directly or indirectly to the oligonucleoside moiety. The linker moiety as depicted in Formula (I) can also often be referred to as the 'ligand arm or arms' of the overall linker. There can also, but not always, be a further linker portion between the oligonucleoside moiety and the branch point, that is often referred to as the 'tether moiety' of the overall linker, 'tethering' the oligonucleoside moiety to the remainder of the conjugated compound. Such 'ligand arms' and / or 'linker moieties' and / or 'tether moieties' can be envisaged by reference to the linear and / or branched configurations as set out above.

As can be seen from the claims, and the reminder of the patent specification, the scope of the present invention extends to linear or branched configurations, and with no limitation as to the number of individual ligands that might be present. Furthermore, the addressee will also be aware that there are many structures that could be used as the linker moiety, based on the state of the art and the expertise of an oligonucleoside chemist.

The remainder of the overall linker (other than the linker moiety) as set out in the claims, and the remainder of the patent specification, is shown by its chemical constituents in Formula (I), which the inventors consider to be particularly unique to the current invention. In more general terms, however, these chemical constituents could be described as a 'tether moiety' as hereinbefore described, wherein the 'tether moiety' is that portion of the overall linker which comprises the group of atoms between Z, namely the oligonucleoside moiety, and the linker moiety as depicted in Formula (I).

### Tether moiety of Formula I

In relation to Formula (I), the 'tether moiety' comprises the group of atoms between Z, namely the oligonucleoside moiety, and the linker moiety.

In some embodiments, R₁ is hydrogen at each occurrence. In some embodiments, R₁ is methyl. In some embodiments, R₁ is ethyl.

In some embodiments, R₂ is hydroxy. In some embodiments, R₂ is halo. In some embodiments, R₂ is fluoro. In some embodiments, R₂ is chloro. In some embodiments, R₂ is bromo. In some embodiments, R₂ is iodo. In some embodiments, R₂ is nitro.

In some embodiments, X₁ is methylene. In some embodiments, X₁ is oxygen. In some embodiments, X₁ is sulfur.

In some embodiments, X₂ is methylene. In some embodiments, X₂ is oxygen. In some embodiments, X₂ is sulfur.

In some embodiments, m = 3.

In some embodiments, n = 6.

In some embodiments, X₁ is oxygen and X₂ is methylene. In some embodiments, both X₁ and X₂ are methylene.

In some embodiments, q = 1, r = 2, s = 1, t = 1, v = 1. In some embodiments, q = 1, r = 3, s = 1, t = 1, v = 1.

In some embodiments, R₁ is hydrogen at each occurrence, n = 6, m = 3, R₂ is fluoro, X₂ is methylene, v = 1, t = 1, s = 1, X₁ is methylene, q = 1 and r = 2.

Thus, in some embodiments, exemplary compounds of the invention comprise the following structure:

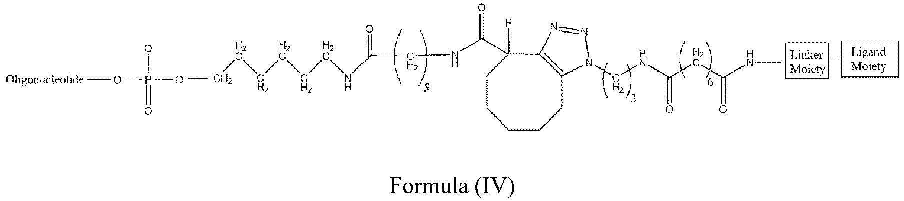

In some embodiments, R₁ is hydrogen at each occurrence, n = 6, m = 3, R₂ is fluoro, X₂ is methylene, v = 1, t = 1, s = 1, X₁ is oxygen, q = 1 and r = 2.

Thus, in some embodiments, exemplary compounds of the invention comprise the following structure:

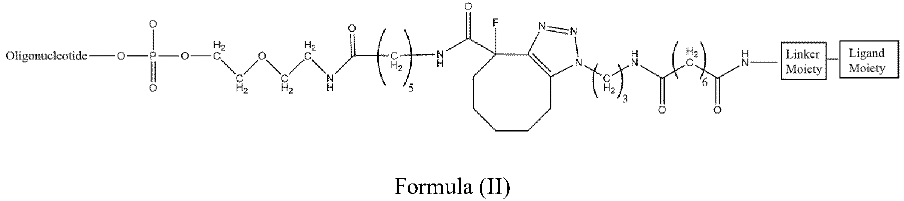

### Alternative tether moieties

During the synthesis of compounds of the present invention, alternative tether moiety structures may arise. In some embodiments, alternative tether moieties have a change of one or more atoms in the tether moiety of the overall linker compared to tether moieties described anywhere herein.

In some embodiments, the alternative tether moiety is a compound of Formula (I) as described anywhere herein, wherein R₂ is hydroxy.

In some embodiments, R₁ is hydrogen at each occurrence, n = 6, m = 3, R₂ is hydroxy, X₂ is methylene, v = 1, t = 1, s = 1, X₁ is methylene, q = 1 and r = 2.

Thus, in some embodiments, compounds of the invention comprise the following structure:

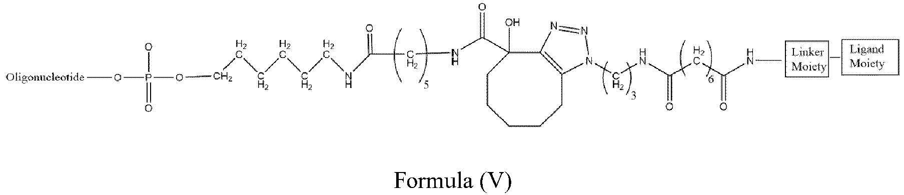

In some embodiments, R₁ is hydrogen at each occurrence, n = 6, m = 3, R₂ is hydroxy, X₂ is methylene, v = 1, t = 1, s = 1, X₁ is oxygen, q = 1 and r = 2.

Thus, in some embodiments, compounds of the invention comprise the following structure:

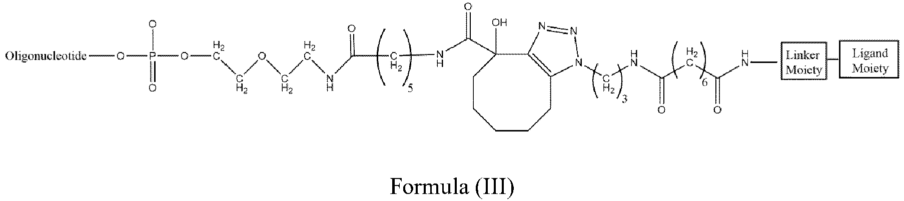

### Linker moiety

In relation to Formula (I), the 'linker moiety' as depicted in Formula (I) comprises the group of atoms located between the tether moiety as described anywhere herein, and the ligand moiety as described anywhere herein.

In some embodiments: 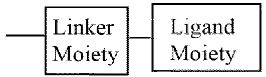 as depicted in Formula (I) as described anywhere herein is any of Formulae (VIa), (VIb) or (VIc), preferably Formula (VIa): 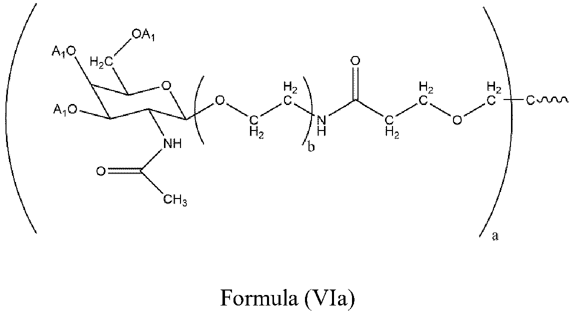 wherein:
A_{I} is hydrogen, or a suitable hydroxy protecting group;
a is an integer of 2 or 3; and
b is an integer of 2 to 5; or 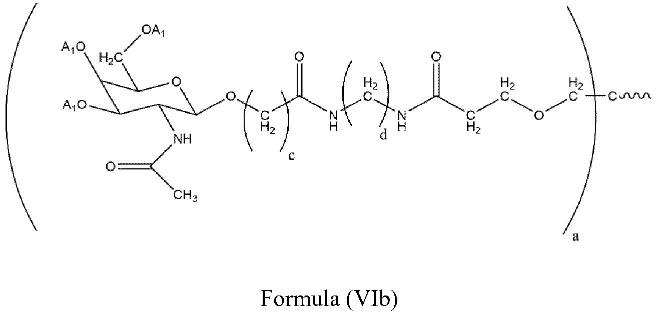
wherein:
   A_{I} is hydrogen, or a suitable hydroxy protecting group;
   a is an integer of 2 or 3; and
   c and d are independently integers of 1 to 6; or 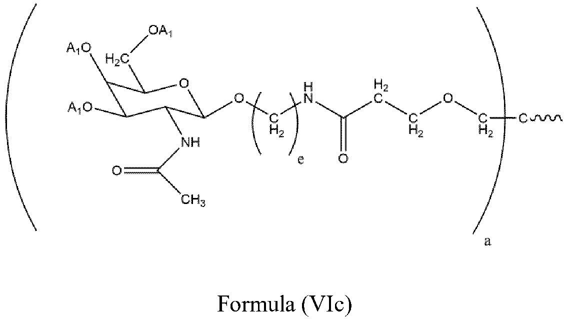
   wherein:
      A_{I} is hydrogen, or a suitable hydroxy protecting group;
      a is an integer of 2 or 3; and
      e is an integer of 2 to 10.

In some embodiments, the moiety: 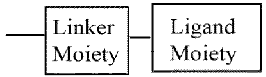 as depicted in Formula (I) is Formula (VIa): 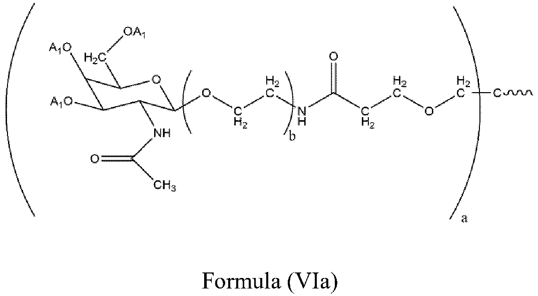 wherein:
A_{I} is hydrogen, or a suitable hydroxy protecting group;
a is 3; and
b is an integer of 3.

In some embodiments, the moiety: 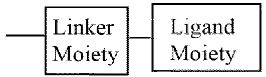 as depicted in Formula (I) as described anywhere herein is Formula (VII): 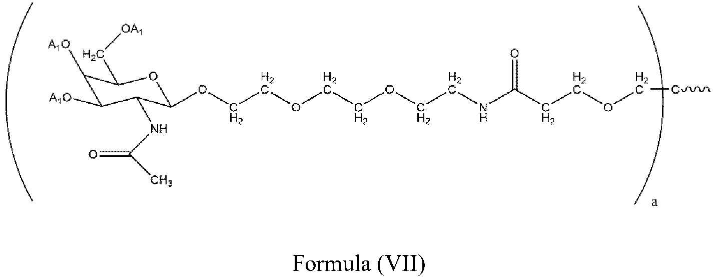 wherein:
A_{I} is hydrogen;
a is an integer of 2 or 3, preferably 3.

Other exemplary compounds of the invention comprise a 'linker moiety', as depicted in Formula (I*), that is part of an overall 'linker'.

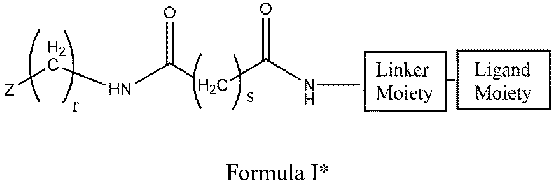

Where:
r and s are independently an integer selected from 1 to 16; and
Z is an oligonucleoside moiety.

As will be further understood in the art, exemplary compounds of the invention comprise an overall linker that is located between the oligonucleoside moiety and the ligand moiety of these compounds. The overall linker, thereby 'links' the oligonucleoside moiety and the ligand moiety to each other.

The overall linker is often notionally envisaged as comprising one or more linker building blocks. For example, there is a linker portion that is depicted as the 'linker moiety' as represented in Formula (I*) positioned adjacent the ligand moiety and attaching the ligand moiety, typically via a branch point, directly or indirectly to the oligonucleoside moiety. The linker moiety as depicted in Formula (I*) can also often be referred to as the 'ligand arm or arms' of the overall linker. There can also, but not always, be a further linker portion between the oligonucleoside moiety and the branch point, that is often referred to as the 'tether moiety' of the overall linker, 'tethering' the oligonucleoside moiety to the remainder of the conjugated compound. Such 'ligand arms' and / or 'linker moieties' and / or 'tether moieties' can be envisaged by reference to the linear and / or branched configurations as set out above.

As can be seen from the claims, and the reminder of the patent specification, the scope of the present invention extends to linear or branched configurations, and with no limitation as to the number of individual ligands that might be present. Furthermore, the addressee will also be aware that there are many structures that could be used as the linker moiety, based on the state of the art and the expertise of an oligonucleoside chemist.

The remainder of the overall linker (other than the linker moiety) as set out in the claims, and the remainder of the patent specification, is shown by its chemical constituents in Formula (I), which the inventors consider to be particularly unique to the current invention. In more general terms, however, these chemical constituents could be described as a 'tether moiety' as hereinbefore described, wherein the 'tether moiety' is that portion of the overall linker which comprises the group of atoms between Z, namely the oligonucleoside moiety, and the linker moiety as depicted in Formula (I).

### Tether moiety

In relation to Formula (I*), the 'tether moiety' comprises the group of atoms between Z, namely the oligonucleoside moiety, and the linker moiety.

In some embodiments, s is an integer selected from 4 to 12. In some embodiments, s is 6.

In some embodiments, r is an integer selected from 4 to 14. In some embodiments, r is 6. In some embodiments, r is 12.

In some embodiments, r is 12 and s is 6.

Thus, in some embodiments, exemplary compounds of the invention comprise the following structure:

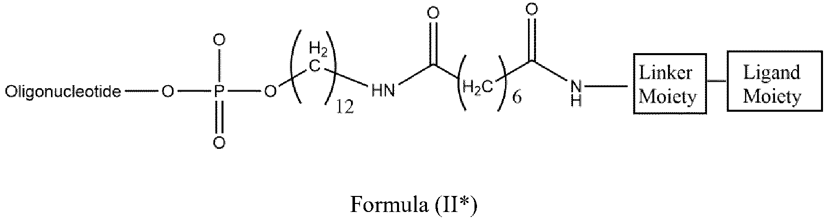

In some embodiments, r is 6 and s is 6.

Thus, in some embodiments, exemplary compounds of the invention comprise the following structure:

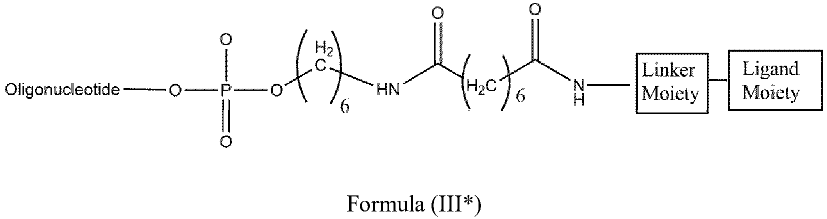

### Linker moiety

In relation to Formula (I*), the 'linker moiety' as depicted in Formula (I) comprises the group of atoms located between the tether moiety as described anywhere herein, and the ligand moiety as described anywhere herein.

In some embodiments, the moiety: 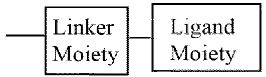 as depicted in Formula (I*) as described anywhere herein is any of Formulae (IV*), (V*) or (VI*), preferably Formula (IV*): 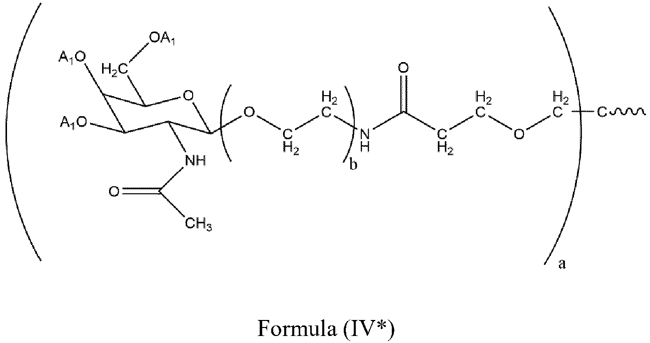 wherein:
A_{I} is hydrogen, or a suitable hydroxy protecting group;
a is an integer of 2 or 3; and
b is an integer of 2 to 5; or 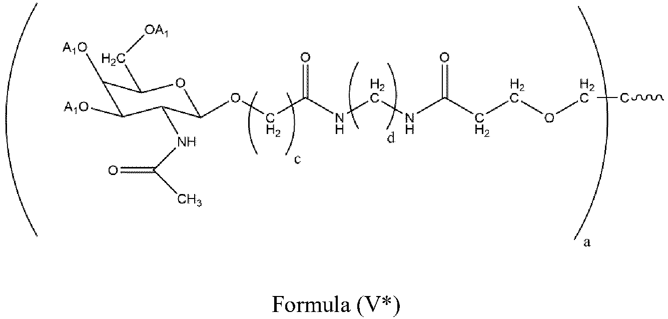
wherein:
   A_{I} is hydrogen, or a suitable hydroxy protecting group;
   a is an integer of 2 or 3; and
   c and d are independently integers of 1 to 6; or 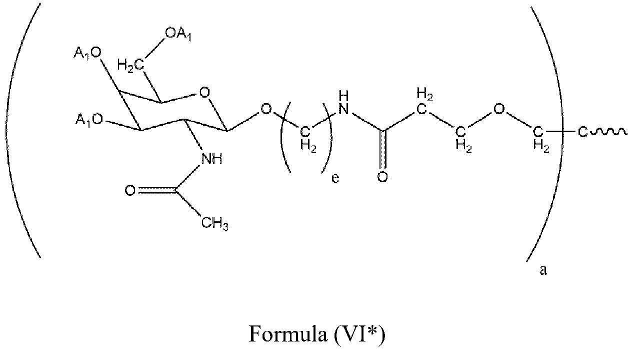
   wherein:
      A_{I} is hydrogen, or a suitable hydroxy protecting group;
      a is an integer of 2 or 3; and
      e is an integer of 2 to 10.

In some embodiments, the moiety: 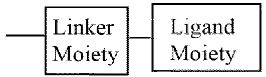 as depicted in Formula (I) is Formula (VIa*): 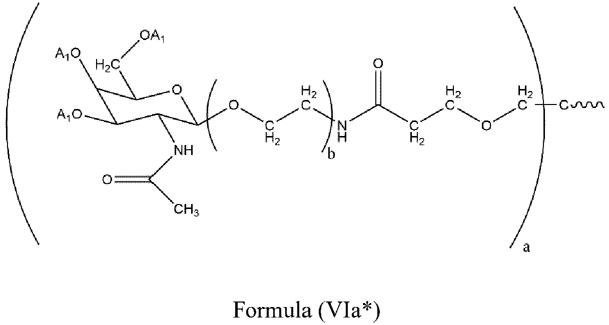 wherein:
A_{I} is hydrogen, or a suitable hydroxy protecting group;
a is 3; and
b is an integer of 3.

In some embodiments, the moiety: 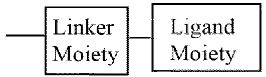 as depicted in Formula (I) as described anywhere herein is Formula (VII*): 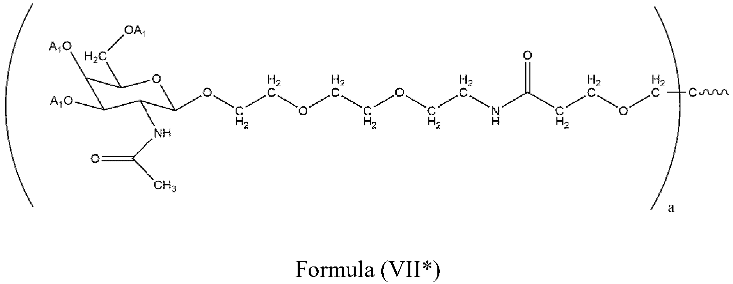 wherein:
A_{I} is hydrogen;
a is an integer of 2 or 3.

In some embodiments, a = 2. In some embodiments, a = 3. In some embodiments, b = 3.

In some embodiments, the GalNAc ligand is comprised in any one of the linkers shown in Figures 2 to 5 or Figure 6 (Formula XI), wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in the linker shown in Figure 4, wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in the linker shown in Figure 6 (Formula XI), wherein the "oligonucleotide" may be any nucleic acid disclosed herein. Accordingly, the "oligonucleotide" may comprise other bonds than a phosphodiester bond, such as one or more phosphorothioate bonds. Preferably, the nucleic acid according to the invention is a double stranded oligonucleoside as defined herein and the linker is conjugated to the second strand, more preferably to the 3' terminal region of the second strand, via a phosphodiester bond.

In some embodiments, the GalNAc ligand is comprised in any one of the linkers shown in Figures 2 to 5 or Figure 6 (Formula XI), wherein the "oligonucleotide" represents a nucleic acid according to the invention, wherein the nucleic acid according to the invention comprises a modified or unmodified second strand comprising or consisting of any one of SEQ ID NO:553 to SEQ ID NO:828, preferably wherein the linker is conjugated to the 3' terminal region of the second strand, i.e., to the 3' terminal region of any one of SEQ ID NO:553 to SEQ ID NO:828, via a phosphodiester bond.

In some embodiments, the GalNAc ligand is comprised in the linker shown in Figure 4, wherein the "oligonucleotide" represents a nucleic acid according to the invention, wherein the nucleic acid according to the invention comprises a modified or unmodified second strand comprising or consisting of any one of SEQ ID NO:553 to SEQ ID NO:828, preferably wherein the linker is conjugated to the 3' terminal region of the second strand, i.e., to the 3' terminal region of any one of SEQ ID NO:553 to SEQ ID NO:828, via a phosphodiester bond.

In some embodiments, the GalNAc ligand is comprised in the linker shown in Figure 6 (Formula XI), wherein the "oligonucleotide" represents a nucleic acid according to the invention, wherein the nucleic acid according to the invention comprises a modified or unmodified second strand comprising or consisting of any one of SEQ ID NO:553 to SEQ ID NO:828, preferably wherein the linker is conjugated to the 3' terminal region of the second strand, i.e., to the 3' terminal region of any one SEQ ID NO:553 to SEQ ID NO:828, via a phosphodiester bond.

In some embodiments, the GalNAc ligand is comprised in any one of the linkers shown in Figures 2 to 5 or Figure 6 (Formula XI), wherein the "oligonucleotide" represents a nucleic acid according to the invention, wherein the nucleic acid according to the invention comprises a modified or unmodified second strand comprising or consisting of any one of SEQ ID NO:580, SEQ ID NO:599, SEQ ID NO:715, SEQ ID NO:729 or SEQ ID NO:772, preferably wherein the linker is conjugated to the 3' terminal region of the second strand, i.e., to the 3' terminal region of any one of SEQ ID NO:580, SEQ ID NO:599, SEQ ID NO:715, SEQ ID NO:729 and SEQ ID NO:772, via a phosphodiester bond.

In some embodiments, the GalNAc ligand is comprised in the linker shown in Figure 4, wherein the "oligonucleotide" represents a nucleic acid according to the invention, wherein the nucleic acid according to the invention comprises a modified or unmodified second strand comprising or consisting of any one of SEQ ID NO:580, SEQ ID NO:599, SEQ ID NO:715, SEQ ID NO:729 and SEQ ID NO:772, preferably wherein the linker is conjugated to the 3' terminal region of the second strand, i.e., to the 3' terminal region of any one of SEQ ID NO:580, SEQ ID NO:599, SEQ ID NO:715, SEQ ID NO:729 and SEQ ID NO:772, via a phosphodiester bond.

In some embodiments, the GalNAc ligand is comprised in the linker shown in Figure 5 (Formula XI), wherein the "oligonucleotide" represents a nucleic acid according to the invention, wherein the nucleic acid according to the invention comprises a modified or unmodified second strand comprising or consisting of any one of SEQ ID NO:580, SEQ ID NO:599, SEQ ID NO:715, SEQ ID NO:729 and SEQ ID NO:772, preferably wherein the linker is conjugated to the 3' terminal region of the second strand, i.e., to the 3' terminal region of any one SEQ ID NO:580, SEQ ID NO:599, SEQ ID NO:715, SEQ ID NO:729 and SEQ ID NO:772, via a phosphodiester bond.

In some embodiments, the GalNAc ligand is comprised in any one of the linkers shown in Figures 2 to 5 or Figure 6 (Formula XI), wherein the "oligonucleotide" represents a nucleic acid according to the invention, wherein the nucleic acid according to the invention comprises a modified second strand comprising or consisting of any one of SEQ ID NO:1105 to SEQ ID NO:1380, preferably wherein the linker is conjugated to the 3' terminal region of the second strand, i.e., to the 3' terminal region of any one of SEQ ID NO:1105 to SEQ ID NO:1380, via a phosphodiester bond.

In some embodiments, the GalNAc ligand is comprised in the linker shown in Figure 4, wherein the "oligonucleotide" represents a nucleic acid according to the invention, wherein the nucleic acid according to the invention comprises a modified second strand comprising or consisting of any one of SEQ ID NO:1105 to SEQ ID NO:1380, preferably wherein the linker is conjugated to the 3' terminal region of the second strand, i.e., to the 3' terminal region of any one of SEQ ID NO:1105 to SEQ ID NO:1380, via a phosphodiester bond.

In some embodiments, the GalNAc ligand is comprised in the linker shown in Figure 6 (Formula XI), wherein the "oligonucleotide" represents a nucleic acid according to the invention, wherein the nucleic acid according to the invention comprises a modified second strand comprising or consisting of any one of SEQ ID NO:1105 to SEQ ID NO:1380, preferably wherein the linker is conjugated to the 3' terminal region of the second strand, i.e., to the 3' terminal region of any one of SEQ ID NO:1105 to SEQ ID NO:1380, via a phosphodiester bond.

In some embodiments, the GalNAc ligand is comprised in any one of the linkers shown in Figures 2 to 5 or Figure 6 (Formula XI), wherein the "oligonucleotide" represents a nucleic acid according to the invention, wherein the nucleic acid according to the invention comprises a modified second strand comprising or consisting of any one of SEQ ID NO:1132, SEQ ID NO:1151, SEQ ID NO:1267, SEQ ID NO:1281 and SEQ ID NO:1324, preferably wherein the linker is conjugated to the 3' terminal region of the second strand, i.e., to the 3' terminal region of any one of SEQ ID NO:1132, SEQ ID NO:1151, SEQ ID NO:1267, SEQ ID NO:1281 and SEQ ID NO:1324, via a phosphodiester bond.

In some embodiments, the GalNAc ligand is comprised in the linker shown in Figure 4, wherein the "oligonucleotide" represents a nucleic acid according to the invention, wherein the nucleic acid according to the invention comprises a modified second strand comprising or consisting of any one of SEQ ID NO:1132, SEQ ID NO:1151, SEQ ID NO:1267, SEQ ID NO:1281 and SEQ ID NO:1324, preferably wherein the linker is conjugated to the 3' terminal region of the second strand, i.e., to the 3' terminal region of any one of SEQ ID NO:1132, SEQ ID NO:1151, SEQ ID NO:1267, SEQ ID NO:1281 and SEQ ID NO:1324, via a phosphodiester bond.

In some embodiments, the GalNAc ligand is comprised in the linker shown in Figure 6 (Formula XI), wherein the "oligonucleotide" represents a nucleic acid according to the invention, wherein the nucleic acid according to the invention comprises a modified second strand comprising or consisting of any one of SEQ ID NO:1132, SEQ ID NO:1151, SEQ ID NO:1267, SEQ ID NO:1281 and SEQ ID NO:1324, preferably wherein the linker is conjugated to the 3' terminal region of the second strand, i.e., to the 3' terminal region of any one of SEQ ID NO:1132, SEQ ID NO:1151, SEQ ID NO:1267, SEQ ID NO:1281 and SEQ ID NO:1324, via a phosphodiester bond.

In some embodiments, the GalNAc ligand is comprised in the linker shown in Figures 2 to 5 or Figure 6 (Formula XI), wherein the "oligonucleotide" represents a nucleic acid according to the invention, wherein the nucleic acid according to the invention comprises a modified second strand comprising or consisting of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, wherein the second strand has the following structure 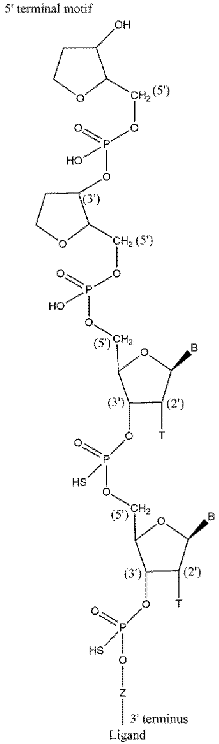 wherein:
T represents a 2'Me ribose modification,
B represents the nucleoside bases of the first two basic nucleosides in the 5' terminal region of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, and
Z represents the remaining 19 contiguous basic nucleosides of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, respectively.

In some embodiments, the GalNAc ligand is comprised in the linker shown in Figure 6 (Formula XI), wherein the "oligonucleotide" represents a nucleic acid according to the invention, wherein the nucleic acid according to the invention comprises a modified second strand comprising or consisting of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, wherein the second strand has the following structure 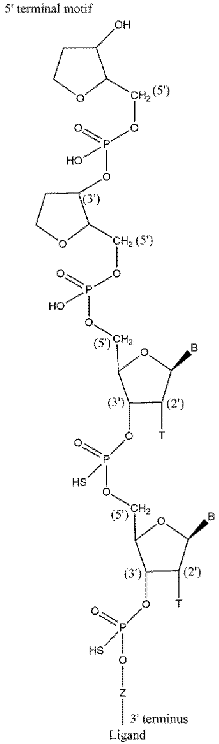 wherein:
T represents a 2'Me ribose modification,
B represents the nucleoside bases of the first two basic nucleosides in the 5' terminal region of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, and
Z represents the remaining 19 contiguous basic nucleosides of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, respectively.

In some embodiments, the GalNAc ligand is comprised in the linker shown in Figure 4, wherein the "oligonucleotide" represents a nucleic acid according to the invention, wherein the nucleic acid according to the invention comprises a modified second strand comprising or consisting of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, wherein the second strand has the following structure 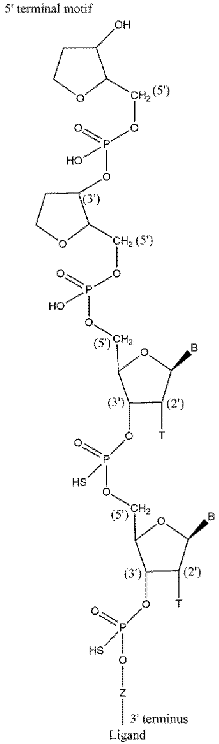 wherein:
T represents a 2'Me ribose modification,
B represents the nucleoside bases of the first two basic nucleosides in the 5' terminal region of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, and
Z represents the remaining 19 contiguous basic nucleosides of any one of SEQ ID NO:1105 to SEQ ID NO:1380, preferably any one of SEQ ID NO:1132, SEQ ID NO:1151, SEQ ID NO:1267, SEQ ID NO:1281 and SEQ ID NO:1324, respectively.

### VECTOR AND CELL

In one aspect, the invention provides a cell containing a nucleic acid, such as inhibitory RNA [RNAi] as described herein.

In one aspect, the invention provides a cell comprising a vector as described herein.

In one aspect the invention provides a vector comprising an oligonucleotide inhibitor, e.g.an iRNA e.g. siRNA.

### PHARMACEUTICALLY ACCEPTABLE COMPOSITIONS

In one aspect, the invention provides a pharmaceutical composition for inhibiting expression of a target gene, the composition comprising an inhibitor such as an oligomer such as a nucleic acid as disclosed herein.

The pharmaceutically acceptable composition may comprise an excipient and or carrier.

Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen- free water; (17) isotonic saline; (18) Ringer's solution; (19) ethyl alcohol; (20) pH buffered solutions; (21) polyesters, polycarbonates and/or poly anhydrides; (22) bulking agents, such as polypeptides and amino acids (23) serum component, such as serum albumin, HDL and LDL; and (22) other non-toxic compatible substances employed in pharmaceutical formulations.

Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g. , magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g. , starch, sodium starch glycolate, etc.); and wetting agents (e.g., sodium lauryl sulphate, etc).

Pharmaceutically acceptable organic or inorganic excipients suitable for non-parenteral administration which do not deleteriously react with nucleic acids can also be used to formulate the compositions of the present invention. Suitable pharmaceutically acceptable excipients include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone, and the like.

Formulations for topical administration of nucleic acids can include sterile and non-sterile aqueous solutions, non-aqueous solutions in common solvents such as alcohols, or solutions of the nucleic acids in liquid or solid oil bases. The solutions can also contain buffers, diluents and other suitable additives. Pharmaceutically acceptable organic or inorganic excipients suitable for non- parenteral administration which do not deleteriously react with nucleic acids can be used.

In one embodiment, the nucleic acid or composition is administered in an unbuffered solution. In certain embodiments, the unbuffered solution is saline or water. In other embodiments, the nucleic acid e.g. RNAi agent is administered in a buffered solution. In such embodiments, the buffer solution can comprise acetate, citrate, prolamine, carbonate, or phosphate, or any combination thereof. For example, the buffer solution can be phosphate buffered saline (PBS).

### DOSAGES

The pharmaceutical compositions of the invention may be administered in dosages sufficient to inhibit expression of a gene or modify the expression or function of a target. In general, where the composition comprising a nucleic acid, a suitable dose of a nucleic acid e.g. an siRNA of the invention will be in the range of about 0.001 to about 200.0 milligrams per kilogram body weight of the recipient per day, generally in the range of about 1 to 50 mg per kilogram body weight per day. Typically, a suitable dose of a nucleic acid e.g. an siRNA of the invention will be in the range of about 0.1 mg/kg to about 5.0 mg/kg, e.g., about 0.3 mg/kg and about 3.0 mg/kg.

A repeat-dose regimen may include administration of a therapeutic amount of a nucleic acid e.g. siRNA on a regular basis, such as every other day or once a year. In certain embodiments, the nucleic acid e.g. siRNA is administered about once per month to about once per quarter (i.e., about once every three months).

In various embodiments, the nucleic acid e.g. siRNA agent is administered at a dose of about 0.01 mg/kg to about 10 mg/kg or about 0.5 mg/kg to about 50 mg/kg. In some embodiments, the nucleic acid e.g. siRNA agent is administered at a dose of about 10 mg/kg to about 30 mg/kg. In certain embodiments, the nucleic acid e.g. siRNA agent is administered at a dose selected from about 0.5 mg/kg 1 mg/kg, 1.5 mg/kg, 3 mg/kg, 5 mg/kg, 10 mg/kg, and 30 mg/kg. In certain embodiments, the nucleic acid e.g. siRNA agent is administered about once per week, once per month, once every other two months, or once a quarter (i.e., once every three months) at a dose of about 0.1 mg/kg to about 5.0 mg/kg. In certain embodiments, the nucleic acid e.g. siRNA agent is administered to the subject once a week. In certain embodiments, the nucleic acid e.g. siRNA agent is administered to the subject once a month. In certain embodiments, the nucleic acid e.g. siRNA agent is administered once per quarter (i.e., every three months).

After an initial treatment regimen, the treatments can be administered on a less frequent basis. For example, after administration weekly or biweekly for three months, administration can be repeated once per month, for six months, or a year; or longer.

The pharmaceutical composition can be administered once daily, or administered as two, three, or more sub-doses at appropriate intervals throughout the day or even using continuous infusion or delivery through a controlled release formulation. In that case, the nucleic acid e.g. siRNA contained in each sub-dose must be correspondingly smaller in order to achieve the total daily dosage. The dosage unit can also be compounded for delivery over several days, e.g., using a conventional sustained release formulation which provides sustained release of the nucleic acid e.g. siRNA over a several day period. Sustained release formulations are well known in the art and are particularly useful for delivery of agents at a particular site, such as could be used with the agents of the present invention. In this embodiment, the dosage unit contains a corresponding multiple of the daily dose.

In other embodiments, a single dose of the pharmaceutical compositions can be long lasting, such that subsequent doses are administered at not more than 3, 4, or 5 day intervals, or at not more than 1, 2, 3, or 4 week intervals. In some embodiments of the invention, a single dose of the pharmaceutical compositions of the invention is administered once per week. In other embodiments of the invention, a single dose of the pharmaceutical compositions of the invention is administered bimonthly. In certain embodiments, the siRNA is administered about once per month to about once per quarter (i.e., about once every three months), or even every 6 months or 12 months.

Estimates of effective dosages and *in vivo* half-lives for the individual nucleic acid e.g. siRNAs encompassed by the invention can be made using conventional methodologies or on the basis of *in vivo* testing using an appropriate animal model, as known in the art.

The pharmaceutical compositions of the present invention can be administered in a number of ways depending upon whether local or systemic treatment is desired and upon the area to be treated. Administration can be topical (e.g., by a transdermal patch), pulmonary, e.g., by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal, or intramuscular injection or infusion; subdermal, e.g., via an implanted device; or intracranial, e.g., by intraparenchymal, intrathecal or intraventricular administration. In certain preferred embodiments, the compositions are administered by intravenous infusion or injection. In certain embodiments, the compositions are administered by subcutaneous injection.

In one embodiment, the nucleic acid e.g. siRNA agent is administered to the subject subcutaneously.

The inhibitor e.g. nucleic acid e.g. siRNA can be delivered in a manner to target a particular tissue (e.g. in particular liver cells).

### METHODS FOR INHIBITING GENE EXPRESSION OR INHIBITION OF TARGET EXPRESSION OR FUNCTION IN VITRO

The present invention also provides methods of inhibiting expression of SLC25A5 gene in a cell. The methods include contacting a cell with a nucleic acid of the invention e.g. siRNA agent, such as double stranded siRNA agent, in an amount effective to inhibit expression of the SLC25A5 gene in the cell, thereby inhibiting expression of the SLC25A5 gene in the cell. It is to be noted that a nucleic acid "for inhibiting the expression of SLC25A5" is a nucleic acid that is capable of inhibiting SLC25A5 expression, preferably as described herein below.

Contacting of a cell with the nucleic acid e.g. an siRNA, such as a double stranded siRNA agent, may be done in vitro or in vivo. Contacting a cell in vivo with nucleic acid e.g. includes contacting a cell or group of cells within a subject, e.g., a human subject, with the nucleic acid e.g. siRNA. Combinations of in vitro and in vivo methods of contacting a cell are also possible.

Contacting a cell may be direct or indirect, as discussed above. Furthermore, contacting a cell may be accomplished via a targeting ligand moiety, including any ligand moiety described herein or known in the art. In preferred embodiments, the targeting ligand moiety is a carbohydrate moiety, e.g. a GalNAc3 ligand, or any other ligand moiety that directs the siRNA agent to a site of interest.

The term "inhibiting," as used herein, is used interchangeably with "reducing," "silencing," "downregulating", "suppressing", and other similar terms, and includes any level of inhibition.

In some embodiments of the methods of the invention, expression or activity of a gene or an inhibition target is inhibited by at least 30%, 35%, 40%, 45%, 50%, 55%, 60%, 65%, 70%, 75%, 80%, 85%, 90%, or 95%, or to below the level of detection of the assay, preferably when determined by qPCR as described herein and/or when the siRNA is introduced into the target cell by transfection. In certain embodiments, the methods include a clinically relevant inhibition of expression of a target gene e.g. as demonstrated by a clinically relevant outcome after treatment of a subject with an agent to reduce the expression of the gene and/ or activity of the target.

In some embodiments, when transfected into the cells, the nucleic acid of the invention inhibits expression of the SLC25A5 gene with an IC50 value below a defined threshold value. In some embodiments, the threshold value may be 2500 pM, 2000 pM, 1900 pM, 1800 pM, 1700 pM, 1600 pM, 1500 pM, 1400 pM, 1300 pM, 1200 pM, 1100 pM, 1000 pM, 900 pM, 800 pM, 700 pM, 600 pM, 500 pM, 400 pM, 300 pM, 200 pM or 100 pM, preferably when determined by qPCR, more preferably by reverse transcriptase (RT)-qPCR, as described herein.

In a preferred embodiment, when transfected into the cells, the nucleic acid of the invention inhibits expression of the SLC25A5 gene with an IC50 value lower than 2500 pM. In a more preferred embodiment, when transfected into the cells, the nucleic acid of the invention inhibits expression of the SLC25A5 gene with an IC50 value lower than 1000 pM. In an even more preferred embodiment, when transfected into the cells, the nucleic acid of the invention inhibits expression of the SLC25A5 gene with an IC50 value lower than 500 pM. In a most preferred embodiment, when transfected into the cells, the nucleic acid of the invention inhibits expression of the SLC25A5 gene with an IC50 value lower than 100 pM.

Inhibition of the SLC25A5 gene may be quantified by the following method:
Huh7 cells (human hepatocyte-derived cell line, obtained from JCRB Cell Bank) may be maintained in Dulbecco's Modified Eagle Medium (DMEM) supplemented with 10% FBS and 1% non-essential amino acids at 37°C in an atmosphere of 5% CO₂. Cells may then be transfected with siRNA duplexes targeting SLC25A5 mRNA or a negative control siRNA (siRNA-control; sense strand 5'-GCCTGTACCAAGGCTTTAA-3' (SEQ ID NO: 1383), antisense strand 5'-TTAAAGCCTTGGTACAGGC-3' (SEQ ID NO: 1382)) using six 10-fold serial dilutions over a final duplex concentration range of 3 nM to 0.03 pM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a RNeasy 96 Kit (Qiagen). Each duplex may be tested by transfection in duplicate wells in a single experiment.

cDNA synthesis may be performed using FastKing RT (with gDNase) Kit (Tiangen). Real-time quantitative PCR (qPCR) may be performed on an ABI Prism 7900HT or ABI QuantStudio 7 with primers specific for human SLC25A5 (forward: ACTGACATCATGTACACAGGCAC (SEQ ID NO:1384), reverse: ACCCATGCCTCTGAGAACATT (SEQ ID NO:1385)) and human GAPDH (forward: GAAGGTGAAGGTCGGAGTC (SEQ ID NO:1386), reverse: GAAGATGGTGATGGGATTTC (SEQ ID NO:1387)) using a SensiFAST SYBR Hi-ROX kit (Meridian).

qPCR may be performed in duplicate on cDNA derived from each well and the mean cycle threshold (Ct) calculated. Relative SLC25A5 expression may be calculated from mean Ct values using the comparative Ct (ΔΔCt) method, normalised to GAPDH and relative to untreated cells. Maximum percent inhibition of SLC25A5 expression and IC50 values may be calculated using a four parameter (variable slope) model using GraphPad Prism 9.

In some embodiments, when transfected into the cells, the nucleic acid of the invention inhibits expression of the SLC25A5 gene with an pEC50 value lower than 5, 6, 7, 8, 9 or 10, preferably when determined by qPCR, more preferably by reverse transcriptase (RT)-qPCR, as described herein.

For that, Huh7 cells (human hepatocyte-derived cell line, obtained from JCRB Cell Bank) may be maintained in Dulbecco's Modified Eagle Medium (DMEM) supplemented with 10% FBS and 1% non-essential amino acids at 37°C, 5% CO₂, 95% humidity. Cells may be transfected with siRNA duplexes targeting either SLC25A5 mRNA or a negative control siRNA (siRNA-control; sense strand 5'-GCCTGTACCAAGGCTTTAA-3' (SEQ ID NO:1383), antisense strand 5'-TTAAAGCCTTGGTACAGGC-3' (SEQ ID NO:1382)) in a 6-point, log dose response curve to give final in assay concentrations of 3nM to 0.03pM. Transfection may be carried out by diluting Lipofectamine RNAiMAX (ThermoFisher) in Opti-MEM (ThermoFisher) medium at a ratio of 48.5:1.5. This solution may be added to an equal volume of siRNA, diluted to the required concentration in phosphate buffered saline. The lipofectamine RNAiMAX and siRNA mixture may be incubated at room temperature for 15 minutes before 20 µL may be added to wells of a 96 well plate. Huh7 cells may be dissociated from flasks using trypsin and resuspended at a density of 200,000 cells/mL. 100 µL of Huh7 cell suspension may be added to each well of the siRNA-containing 96-well plates. Cells may be incubated for 24 hours at 37°C, 5% CO₂,95% humidity. Each siRNA may be tested in triplicate wells and on two separate days for a total of six replicates.

Intracellular RNA may be isolated using an Rneasy kit (Qiagen) according to the manufacturer's instructions. cDNA synthesis may be performed using a FastKing RT kit, with gDNase (Tiangen). Target cDNA may be the quantified by qPCR on an ABI Prism 7900HT or ABI QuantStudio 7 with primers specific for human SLC25A5 (forward:
ACTGACATCATGTACACAGGCAC (SEQ ID NO:1384), reverse:
ACCCATGCCTCTGAGAACATT (SEQ ID NO:1385)) and human GAPDH (forward:
GAAGGTGAAGGTCGGAGTC (SEQ ID NO:1386), reverse:
GAAGATGGTGATGGGATTTC (SEQ ID NO:1387)) using a SensiFAST SYBR Hi-ROX kit (Meridian).

qPCR may be performed in duplicate on cDNA derived from each well and the mean Ct calculated. Relative SLC25A5 expression may be calculated from mean Ct values using the comparative Ct (ΔΔCt) method, normalised to GAPDH and relative to untreated cells. Maximum percent inhibition of SLC25A5 expression and pEC50 values (-log₁₀ of the EC50) may be calculated using a four parameter (variable slope) model using NumPy (Python).

Alternatively or in addition, inhibition of expression of the SLC25A5 gene may be characterized by a reduction of mean relative expression of the SLC25A5 gene.

In some embodiments, when cells are transfected with 0.1 nM of the nucleic acid of the invention, the mean relative expression of SLC25A5 is below 1, 0.9, 0.8, 0.7, 0.6, 0.5, or 0.4, preferably when determined by qPCR, more preferably by reverse transcriptase (RT)-qPCR, as described herein.

In some embodiments, when cells are transfected with 5 nM of the nucleic acid of the invention, the mean relative expression of SLC25A5 is below 1, 0.9, 0.8, 0.7, 0.6, 0.5, 0.4 or 0.3, preferably when determined by qPCR, more preferably by reverse transcriptase (RT)-qPCR, as described herein.

Mean relative expression of the SLC25A5 gene may be quantified by the following method:
Huh7 cells (human hepatocyte-derived cell line, obtained from JCRB Cell Bank) may be maintained in Dulbecco's Modified Eagle Medium (DMEM) supplemented with 10% FBS at 37°C in at atmosphere of 5% CO₂. Cells may be transfected with siRNA duplexes targeting SLC25A5 mRNA or a negative control siRNA (siRNA-control; sense strand 5'-GCCTGTACCAAGGCTTTAA-3' (SEQ ID NO:1383), antisense strand 5'-TTAAAGCCTTGGTACAGGC-3' (SEQ ID NO:1382)) at a final duplex concentration of 5 nM and 0.1 nM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a Rneasy 96 Kit (Qiagen). Each duplex may be tested by transfection in duplicate wells in two independent experiments.

cDNA synthesis may be performed using FastKing RT (with gDNase) Kit (Tiangen). Real-time quantitative PCR (qPCR) may be performed on an ABI Prism 7900HT or ABI QuantStudio 7 with primers specific for human SLC25A5 (forward: ACTGACATCATGTACACAGGCAC (SEQ ID NO:1384), reverse: ACCCATGCCTCTGAGAACATT (SEQ ID NO:1385)) and human GAPDH (forward: GAAGGTGAAGGTCGGAGTC (SEQ ID NO:1386), reverse: GAAGATGGTGATGGGATTTC (SEQ ID NO:1387)) using a SensiFAST SYBR Hi-ROX kit (Meridian) .

qPCR may be performed in duplicate on cDNA derived from each well and the mean Ct calculated. Relative SLC25A5 expression may be calculated from mean Ct values using the comparative Ct (ΔΔCt) method, normalised to GAPDH and relative to untreated cells.

Inhibition of the expression of SLC25A5 gene may be manifested by a reduction of the amount of mRNA of the target SLC25A5 gene in comparison to a suitable control.

In other embodiments, inhibition of the expression of SLC25A5 gene may be assessed in terms of a reduction of a parameter that is functionally linked to gene expression, e.g , protein expression or signaling pathways.

### METHODS OF TREATING OR PREVENTING DISEASES ASSOCIATED WITH GENE EXPRESSION/ EXPRESSION OF FUNCTION OF A TARGET

The present invention also provides methods of using nucleic acid e.g. an siRNA of the invention or a composition containing nucleic acid e.g. an siRNA of the invention to reduce or inhibit gene expression in a cell or reduce expression or function of a target. The methods include contacting the cell with a nucleic acid e.g. dsiRNA of the invention and maintaining the cell for a time sufficient to obtain degradation of the mRNA transcript of a gene, thereby inhibiting expression of the gene in the cell. Reduction in gene expression or function of a target can be assessed by any methods known in the art. In a preferred embodiment, the gene is *SLC25A5*.

In the methods of the invention the cell may be contacted *in vitro* or *in vivo*, i.e., the cell may be within a subject.

A cell suitable for treatment using the methods of the invention may be any cell that expresses a gene of interest associated with a metabolic disease or disorder, such as a metabolic disease or disorder associated with non-alcoholic fatty liver disease (NAFLD) and/or obesity and/or a disease or disorder associated with adipogenesis and/or adipogenesis.

The in vivo methods of the invention may include administering to a subject a composition containing a nucleic acid of the invention e.g. an siRNA, where the nucleic acid e.g. siRNA includes a nucleoside sequence that is complementary to at least a part of an RNA transcript of SLC25A5 gene of the mammal to be treated.

The present invention further provides methods of treatment of a subject in need thereof. The treatment methods of the invention include administering a nucleic acid such as an siRNA of the invention to a subject, e.g., a subject that would benefit from a reduction or inhibition of the expression of a gene and/or expression and/or function of a target, in a therapeutically effective amount e.g. a nucleic acid such as an siRNA targeting a gene or a pharmaceutical composition comprising the nucleic acid targeting a gene.

A nucleic acid e.g. siRNA of the invention may be administered as a "free" nucleic acid or "free" siRNA, administered in the absence of a pharmaceutical composition. The naked nucleic acid may be in a suitable buffer solution. The buffer solution may comprise acetate, citrate, prolamine, carbonate, or phosphate, or any combination thereof. In one embodiment, the buffer solution is phosphate buffered saline (PBS). The pH and osmolarity of the buffer solution can be adjusted such that it is suitable for administering to a subject.

Alternatively, a nucleic acid e.g. siRNA of the invention may be administered as a pharmaceutical composition, such as a dsiRNA liposomal formulation.

In one embodiment, the method includes administering a composition featured herein such that expression of the target gene is decreased, such as for about 1, 2, 3, 4, 5, 6, 7, 8, 12, 16, 18, 24 hours, 28, 32, or about 36 hours. In one embodiment, expression of the target gene is decreased for an extended duration, e.g., at least about two, three, four days or more, e.g., about one week, two weeks, three weeks, or four weeks or longer, e.g., about 1 month, 2 months, or 3 months.

Subjects can be administered a therapeutic amount of nucleic acid e.g. siRNA, such as about 0.01 mg/kg to about 200 mg/kg.

The nucleic acid e.g. siRNA can be administered by intravenous infusion over a period of time, on a regular basis. In certain embodiments, after an initial treatment regimen, the treatments can be administered on a less frequent basis. Administration of the siRNA can reduce gene product levels of a target gene , e.g., in a cell or tissue of the patient by at least about 20%, 25%, 30%, 35%, 40%, 45%, 50%, 55%, 60%, 65%, 70%, 75%, 80%, 85%, 90%, or 95%, or below the level of detection of the assay method used. In certain embodiments, administration results in clinical stabilization or preferably clinically relevant reduction of at least one sign or symptom of a gene-associated disorder.

Alternatively, the nucleic acid e.g. siRNA can be administered subcutaneously, i.e., by subcutaneous injection. One or more injections may be used to deliver the desired daily dose of nucleic acid e.g. s iRNA to a subject. The injections may be repeated over a period of time. The administration may be repeated on a regular basis. In certain embodiments, after an initial treatment regimen, the treatments can be administered on a less frequent basis. A repeat-dose regimen may include administration of a therapeutic amount of nucleic acid on a regular basis, such as every other day or to once a year. In certain embodiments, the nucleic acid is administered about once per month to about once per quarter (i.e., about once every three months).

### COMBINATION THERAPIES

The inhibitor of the present invention may be combined with other therapeutic agents for use in therapy, in particular for use in treatment of any of the metabolic diseases or disorders disclosed herein.

In certain embodiments, the inhibitor of the present invention, such as any of the siRNA molecules disclosed herein, may be combined with a GLP-1 agonist, including, without limitation, GLP-1/GIP dual agonists, GLP-1/FGF21 dual agonists, GLP-1/GCGR dual agonists and GLP-1/GIP/GCGR triple agonists, and/or a THR-beta agonist.

That is, in certain embodiments, the inhibitor of the invention, in particular the siRNA molecules disclosed herein, may be combined with a GLP-1 agonist for the treatment of metabolic diseases or disorders. In a particular embodiment, the inhibitor of the invention, in particular the siRNA molecules disclosed herein, may be combined with a GLP-1 agonist for the treatment of fatty liver diseases, in particular NAFLD and/or NASH.

The term "GLP-1 agonist" as used herein refers to a compound, which fully or partially activates the human GLP-1 receptor. The term is thus equal to the term "GLP-1 receptor agonist" used in other documents. The term GLP-1 agonist as well as the specific GLP-1 agonists described herein also encompass salt forms thereof.

It follows that the GLP-1 agonist should display "GLP-1 activity" which refers to the ability of the compound, i.e. a GLP-1 analogue or a compound comprising a GLP-1 analogue, to bind to the GLP-1 receptor and initiate a signal transduction pathway resulting in insulinotropic action or other physiological effects as is known in the art. In some embodiments the "GLP-1 agonist" binds to a GLP-1 receptor, e.g., with an affinity constant (K_{D}) or activate the receptor with a potency (EC₅o) of below 1 mM, e.g. below 100 nM as measured by methods known in the art (see e.g. WO 98/08871) and exhibits insulinotropic activity, where insulinotropic activity may be measured in vivo or in vitro assays known to those of ordinary skill in the art. For example, the GLP-1 agonist may be administered to an animal with increased blood glucose (e.g. obtained using an Intravenous Glucose Tolerance Test (IVGTT). A person skilled in the art will be able to determine a suitable glucose dosage and a suitable blood sampling regime, e.g. depending on the species of the animal, for the IVGTT) and measure the plasma insulin concentration overtime. Suitable assays have been described in such as WO 2015/155151.

The term half maximal effective concentration (EC₅o) generally refers to the concentration which induces a response halfway between the baseline and maximum, by reference to the dose response curve. EC₅o is used as a measure of the potency of a compound and represents the concentration where 50% of its maximal effect is observed. Due to the albumin binding effects of GLP-1 agonists comprising a substituent as described herein, it is important to pay attention to if the assay includes human serum albumin or not.

The in vitro potency of the GLP-1 agonist may be determined as described in WO 2015/155151 , example 29 without Human Serum Albumin (HSA), and the EC₅o determined. The lower the EC₅o value, the better the potency. In one embodiment, the potency (EC50) as determined (without HSA) is 5-1000 pM, such as 10-750 pM, 10-500 pM or 10-200 pM. In one embodiment the EC50 (without HSA) is at most 500 pM, such as at most 300 pM, such as at most 200 pM.

In one embodiment the EC50 (without HSA) is comparable to human GLP-1 (7-37).

In one embodiment the EC50 (without HSA) is at most 50 pM. In a further such embodiment the EC50 is at most 40 pM, such as at most 30 pM such as at most 20 pM, such as at most 10 pM. In one embodiment the EC50 is around 10 pM

Also, or alternatively, the binding of the GLP-1 agonist to albumin may be measured using the in vitro potency assay of Example 29 of WO 2015/155151 including HSA. An increase of the in vitro potency, EC₅o value, in the presence of serum albumin reflects the affinity to serum albumin.

In one embodiment the potency (EC50) as determined (with 1 % HSA) is 5-1000 pM, such as 100-750 pM, 200-500 pM or 100-400 pM. In one embodiment the EC50 (with 1 % HSA) is at most 750 pM, such as at most 500 pM, such as at most 400 pM, such as at most 300 or such as at most 250 pM.

If desired, the fold variation in relation to a known GLP-1 receptor agonist may be calculated as EC50(test analogue)/EC50(known analogue), and if this ratio is such as 0.5- 1.5, or 0.8-1.2 the potencies are considered to be equivalent.

In one embodiment the potency, EC50 (without HSA), is equivalent to the potency of liraglutide. In one embodiment the potency, EC50 (without HSA), is equivalent to the potency of semaglutide.

In some embodiments the GLP-1 agonist is a GLP-1 analogue, optionally comprising "one substituent". The term "analogue" as used herein referring to a GLP-1 peptide (hereafter "peptide") means a peptide wherein at least one amino acid residue of the peptide has been substituted with another amino acid residue and/or wherein at least one amino acid residue has been deleted from the peptide and/or wherein at least one amino acid residue has been added to the peptide and/or wherein at least one amino acid residue of the peptide has been modified. Such addition or deletion of amino acid residues may take place at the N- terminal of the peptide and/or at the C-terminal of the peptide.

In some embodiments the term "GLP-1 analogue" or "analogue of GLP-1" as used herein refers to a peptide, or a compound, which is a variant of the human Glucagon-Like Peptide-1 (GLP-1 (7-37)). GLP-1 (7-37) has the sequence HAEGTFTSDV SSYLEGQAAKEFIAWLVKGRG (SEQ ID NO: 1388). In some embodiments the term "variant" refers to a compound which comprises one or more amino acid substitutions, deletions, additions and/or insertions.

In one embodiment the GLP-1 agonist exhibits at least 60%, 65%, 70%, 80% or 90% sequence identity to GLP-1 (7-37) over the entire length of GLP-1 (7-37).

In general, the term GLP-1 agonist is meant to encompass the GLP-1 agonist and any pharmaceutically acceptable salt, amide, or ester thereof. In some embodiments the composition comprises the GLP-1 agonist or a pharmaceutically acceptable salt, amide, or ester thereof. In some embodiments the composition comprises the GLP-1 agonist and one or more pharmaceutically acceptable counter ions.

In certain embodiments, the inhibitor of the present invention, in particular the siRNA molecules of the present invention, may be administered with a GLP-1 agonist selected from one or more of the GLP-1 agonists disclosed in WO93/19175, WO96/29342, WO98/08871, WO99/43707, WO99/43706, WO99/43341 , WO99/43708, WO2005/027978, WO2005/058954, WO2005/058958, WO2006/005667, WO2006/037810, WO2006/037811 , WO2006/097537, WO2006/097538, WO2008/023050, WO2009/030738, WO2009/030771, WO2009/030774 and WO2021/219710, which are incorporated herein by reference in their entirety.

In certain embodiments, the inhibitor of the present invention, in particular the siRNA molecules of the present invention, may be administered with a GLP-1 agonist selected from the group consisting of: Dulaglutide (Trulicity^{®}), Exenatide (Byetta^{®}), Exenatide extended-release (Bydureon^{®}), Liraglutide (Victoza^{®}), Lixisenatide (Adlyxin^{®}), Semaglutide injection (Ozempic^{®}), and Semaglutide tablets (Rybelsus^{®}).

In some embodiments, the GLP-1 agonist is semaglutide having a formula of N- epsilon26-[2-(2-{2-[2-(2-{2-[(S)-4-carboxy-4-(17-carboxy-heptadecanoylamino)butyrylamino]ethoxy}ethoxy) acetylamino]ethoxy}ethoxy)acetyl] [Aib8,Arg34]GLP-1 (7-37).

The term "GLP-1 agonist" as used herein also encompasses dual or triple agonists that can activate more than one receptor. These dual or triple agonists may be molecules that have the ability to activate the GLP-1 receptor and at least one further receptor. In some embodiments, the dual or triple agonist may be a chimeric molecule comprising a first portion that activates the GLP-1 receptor and further portions that activate further receptors. In certain embodiments, the GLP-1 agonist is a dual or triple agonist that, in addition to the GLP-1 receptor, further activates one or more of: a glucose-dependent insulinotropic polypeptide (GIP) receptor, a Fibroblast growth factor 21 (FGF21) receptor, and a glucagon receptor (GCGR).

In certain embodiments, the inhibitor of the invention, in particular the siRNA molecules disclosed herein, may be combined with a GLP-1/GIP dual agonist for the treatment of metabolic diseases or disorders. In a particular embodiment, the inhibitor of the invention, in particular the siRNA molecules disclosed herein, may be combined with a GLP-1/GIP dual agonist for the treatment of fatty liver diseases, in particular NAFLD and/or NASH.

The term "GLP-1/GIP dual agonist" as used in the context of the present invention refers to a substance or ligand that can activate the GLP-1 receptor and the glucose-dependent insulinotropic polypeptide (GIP) receptor. GLP-1/GIP receptor co-agonists and their potential medical uses are described in several patent applications such as WO 2010/011439, WO 2013/164483, WO 2014/192284, WO 2015/067715, WO 2015/022420, WO 2015/086728, WO 2015/086729, WO 2016/111971, WO 2020/023386, US 9745360, US 2014/162945, US 2014/0357552, WO 2021/150673, WO 2021/260530, WO 2022/018185, and WO 2022/079639, which are fully incorporated herein by reference.

In certain embodiments, the inhibitor of the invention, in particular the siRNA molecules disclosed herein, may be combined with the GLP-1/GIP dual agonist tirzepatide (Mounjaro^{®}) for the treatment of metabolic diseases or disorders. In a particular embodiment, the inhibitor of the invention, in particular the siRNA molecules disclosed herein, may be combined with the GLP-1/GIP dual agonist tirzepatide (Mounjaro^{®}) for the treatment of fatty liver diseases, in particular NAFLD and/or NASH.

As used herein, "tirzepatide" means a GLP-1/GIP dual agonist peptide as described in U.S. Pat. No. 9,474,780 and described by CAS Registry Number: 2023788-19-2. Tirzepatide is described in Example 1 of U.S. Pat. No. 9,474,780, with the following sequence:
YX₁EGTFTSDYSIX₂LDKIAQKAFVQWLMGGPSSGAPPPS (SEQ ID NO: 1389)
wherein X₁ is α-amino isobutyric acid (Aib); X₂ is Aib; K at position 20 is chemically modified through conjugation to the epsilon-amino group of the K side-chain with (2-[2-(2-Amino-ethoxy)-ethoxy]-acetyl)₂-(γGlu)₁-CO-(CH₂)₁₈-CO₂H; and the C-terminal amino acid is amidated as a C-terminal primary amide.

In certain embodiments, the inhibitor of the invention, in particular the siRNA molecules disclosed herein, may be combined with a GLP-1/ FGF21 dual agonist for the treatment of metabolic diseases or disorders. In a particular embodiment, the inhibitor of the invention, in particular the siRNA molecules disclosed herein, may be combined with a GLP-1/FGF21 dual agonist for the treatment of fatty liver diseases, in particular NAFLD and/or NASH.

The term "GLP-1/FGF21 dual agonist" as used in the context of the present invention refers to a substance or ligand that can activate the GLP-1 receptor and the Fibroblast growth factor 21 (FGF21) receptor. Preferably, the GLP-1/FGF21 dual agonist is a chimeric molecule comprising a GLP-1 agonist as defined herein above and the molecule FGF21 or a functionally active variant or analogue thereof. In certain embodiments, the GLP-1/FGF21 dual agonist may further comprise an antibody Fc region.

GLP-1/ FGF21 receptor co-agonists and their potential medical uses are described in several patent applications such as WO 2010/142665, WO2014/037373, WO 2018/115401, WO 2018/166461, WO 2019/243557, and WO 2022/002408, which are fully incorporated herein by reference.

In certain embodiments, the inhibitor of the invention, in particular the siRNA molecules disclosed herein, may be combined with a GLP-1/ GCGR dual agonist for the treatment of metabolic diseases or disorders. In a particular embodiment, the inhibitor of the invention, in particular the siRNA molecules disclosed herein, may be combined with a GLP-1/GCGR dual agonist for the treatment of fatty liver diseases, in particular NAFLD and/or NASH.

The term "GLP-1/ GCGR dual agonist" as used in the context of the present invention refers to a substance or ligand that can activate the GLP-1 receptor and the glucagon receptor (GCGR). GLP-1/GCGR receptor co-agonists and their potential medical uses are described in several patent applications such as, WO 2008/101017, WO 2009/155258, WO 2011/075393, WO 2011/160630, WO 2014/056872, WO 2014/091316, WO 2015/086733, WO 2017/181452, WO 2018/100174, WO 2019/030268, WO 2019/060660 and WO 2023/006923, which are fully incorporated herein by reference.

In certain embodiments, the inhibitor of the invention, in particular the siRNA molecules disclosed herein, may be combined with the GLP-1/ GCGR dual agonist Survodutide (BI 456906) for the treatment of metabolic diseases or disorders. In a particular embodiment, the inhibitor of the invention, in particular the siRNA molecules disclosed herein, may be combined with the GLP-1/ GCGR dual agonist Survodutide (BI 456906) for the treatment of fatty liver diseases, in particular NAFLD and/or NASH.

In certain embodiments, the inhibitor of the invention, in particular the siRNA molecules disclosed herein, may be combined with a GLP-1/GIP/GCGR triple agonist for the treatment of metabolic diseases or disorders. In a particular embodiment, the inhibitor of the invention, in particular the siRNA molecules disclosed herein, may be combined with a GLP-1/GIP/GCGR dual agonist for the treatment of fatty liver diseases, in particular NAFLD and/or NASH.

The term " GLP-1/GIP/GCGR triple agonist" as used in the context of the present invention refers to a substance or ligand that can activate the GLP-1 receptor, the GIP receptor and the glucagon receptor (GCGR). GLP-1/GIP/GCGR receptor co-agonists and their potential medical uses are described in several patent applications such as, WO 2014/096150, WO 2015/067716, WO 2019/125292, WO 2022/090447, and WO 2022/268029, which are fully incorporated herein by reference.

In certain embodiments, the inhibitor of the invention, in particular the siRNA molecules disclosed herein, may be combined with Retatrutid (LY-3437943) for the treatment of metabolic diseases or disorders. In a particular embodiment, the inhibitor of the invention, in particular the siRNA molecules disclosed herein, may be combined with Retatrutid (LY-3437943) for the treatment of fatty liver diseases, in particular NAFLD and/or NASH.

The person skilled in the art is aware of ways to formulate GLP-1 agonists, including dual and triple agonists, for any suitable route of administration. In certain embodiments, the GLP-1 agonist may be administered orally or by injection, e.g., by subcutaneous injection.

In certain embodiments, the inhibitor of the invention, in particular the siRNA molecules disclosed herein, may be combined with a THR-beta agonist for the treatment of metabolic diseases or disorders. In a particular embodiment, the inhibitor of the invention, in particular the siRNA molecules disclosed herein, may be combined with a THR-beta agonist for the treatment of fatty liver diseases, in particular NAFLD and/or NASH.

The term "THR-beta agonist" as used herein refers to a compound, which fully or partially activates the human thyroid hormone receptor-β. The term THR-beta agonist as well as the specific THR-beta agonists described herein also encompass salt forms thereof.

Various THR-beta agonists have been described in the art and have been summarized, inter alia, by Zucchi (Thyroid Hormone Analogues: An Update, Thyroid. August 2020; 30(8): 1099-1105), which is incorporated herein by reference in its entirety.

In certain embodiments, the THR-beta agonist that is to be administered in combination with the inhibitor of the invention is MGL-3196 (resmetirom), KB-2115 (eprotirome), GC-1 (sobetirome) or MB07344/VK2809, as, for example, described by Zucchi.

In certain embodiments, the THR-beta agonist is MGL-3196 (resmetirom) or any of the compounds disclosed in WO 2014/043706, which is incorporated herein by reference in its entirety. Resmetirom is a thyroid hormone receptor (THR) β-selective agonist. Disclosures related to resmetirom can further be found in US Patent No. 9,266,861, US Patent Application Serial No. 16/343,065, and PCT Application Serial No. PCT/US2019/040276, the contents of each of which are incorporated herein by reference in their entireties.

In certain embodiments, the THR-beta agonist is KB-2115 (eprotirome) or any of the compounds disclosed in WO 2007/110226 or WO 2009/077147, which are incorporated herein by reference in their entirety.

The person skilled in the art is aware of ways to formulate THR-beta agonists for any suitable route of administration.

In certain embodiments, the inhibitor of the invention, in particular the siRNA molecules disclosed herein, may be combined with a GLP-1 agonist, such as any one of the GLP-1 agonists disclosed herein above, including dual or triple agonists, and a THR-beta agonist, such as any one of the THR-beta agonists disclosed herein above, for the treatment of metabolic diseases or disorders. In a particular embodiment, the inhibitor of the invention, in particular the siRNA molecules disclosed herein, may be combined with a GLP-1 agonist, such as any one of the GLP-1 agonists disclosed herein above, including dual or triple agonists, and a THR-beta agonist, such as any one of the THR-beta agonists disclosed herein above, for the treatment of fatty liver diseases, in particular NAFLD and/or NASH.

The inhibitor of the invention, in particular the siRNA molecules disclosed herein, may alternatively, or in addition, be combined and/or co-administered with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin); preferably for the treatment of any of the metabolic diseases or disorders disclosed herein, more preferably for the treatment of NAFLD.

In certain embodiments, the inhibitor of the invention, in particular the siRNA molecules disclosed herein, may be used for the treatment of any of the metabolic diseases or disorders disclosed herein, more preferably for the treatment of NAFLD, in combination with any of the molecules disclosed in Fig.1 of Nathani and Bansal (Gastroenterol Hepatol (NY). 2023 Jul; 19(7): 371-381; incorporated by reference), in particular one or more of: a THR-beta agonist (such as resmetirom, VK2809 or TERN-501), a PPAR agonist (such as Lanifibranor, Saroglitazar, or Elafibranor), a GLP-1 agonist (such as Liraglutide, Semaglutide or Tirzepatide), a CCR2/5 inhibitor (such as Cenicriviroc), an ASK1 inhibitor (such as Selonsertib), an ACC inhibitor (such as Firsocostat or PF-05221304), an SCD inhibitor (such as Aramchol), an FGF21 analogue (such as Efruxifermin or Pegbelfermin), a Galectin-3 inhibitor (such as Belapectin), a LOXL2 inhibitor (such as Simtuzumab), an FXR agonists (such as Obeticholic acid, Tropifexor, Cilofexor, EDP-305, or MET409), and/or an FGF19 analogue (such as Aldafermina).

In certain embodiments, the inhibitor of the invention, in particular the siRNA molecules disclosed herein, may be used for the treatment of any of the metabolic diseases or disorders disclosed herein, more preferably for the treatment of NAFLD, in combination with any of the molecules disclosed in Batchuluun et al. (Nat Rev Drug Discov, 2022, 21(4):283-305. doi: 10.1038/s41573-021-00367-2.; incorporated by reference), in particular one or more of: a citrate/isocitrate carrier (CIC) inhibitor (such as Benzenetricarboxylate, CPTI-1 or CPTI-2), an ATP-citrate lyase (ACLY) inhibitor (such as Bempedoic acid, Hydroxycitrate, BMS-303141, Emodin derivates, Furan carboxylate derivates, MEDICA 16, SB-204990, or NDI-091143), an acetyl-CoA carboxylase (ACC) inhibitor (such as Firsocostat, PF-05221304, PF-05175157, MK-4074, A-908292, Carboxamide derivative-1k, CP-640186, Monocyclic derivate-1q, ND-654, ND-646, Olefin derivate-2e, (S)-9c, Soraphen A, TOFA, or WZ66), and/or a fatty acid synthase (FAS) inhibitor (such as Orlistat, TVB-2640, FT-4101, BI-99179, Cerulenin, C75, Fasnall, GSK2194069, IPI-9119, MP-ML-24-N1, or TVB-3166).

A combination therapy, or a "combination" contemplated herein includes the co-administration of an inhibitor according to the invention, in particular an siRNA according to the invention, with one or more additional therapeutic agent, preferably one or more of the additional therapeutic agents disclosed herein above. The inhibitor according to the invention may be administered prior to, after, or at the same time as the one or more additional therapeutic agent.

In certain embodiments, the inhibitor according to the invention, in particular the siRNA according to the invention, may be co-administered with a GLP-1 agonist, including GLP-1/GIP dual agonists, GLP-1/FGF21 dual agonists, GLP-1/GCGR dual agonists and GLP-1/GIP/GCGR triple agonists, and/or a THR-beta agonist, wherein the inhibitor according to the invention may be administered prior to, after, or at the same time as the GLP-1 agonist and/or the THR-beta agonist.

In an exemplary embodiment, the co-administration includes administering an siRNA according to the invention and any of the GLP-1 agonists, including GLP-1/GIP dual agonists, GLP-1/FGF21 dual agonists, GLP-1/GCGR dual agonists and GLP-1/GIP/GCGR triple agonists, disclosed herein or incorporated herein by reference. In another exemplary embodiment, the co-administration includes administering an siRNA according to the invention and the GLP-1 agonist semaglutide. In another exemplary embodiment, the co-administration includes administering an siRNA according to the invention and the GLP-1/GIP dual agonist terzapetide.

In yet another exemplary embodiment, the co-administration includes administering an siRNA according to the invention and any of the THR-beta agonists disclosed herein or incorporated herein by reference. In another exemplary embodiment, the co-administration includes administering an siRNA according to the invention and the THR-beta agonist resmetirom.

In another exemplary embodiment, the co-administration includes administering an siRNA according to the invention, the GLP-1 agonists semaglutide or terzapetide, and the THR-beta agonist resmetirom.

The combination therapy of the disclosure comprising the inhibitor according to the invention and at least one more additional therapeutic agent may be administered via any route. In some embodiments, the inhibitor according to the invention and the at least one more additional therapeutic agent may be delivered orally, subcutaneously, intravenously, intranasally, transdermally, intraperitoneally, intramuscularly, intrapulmonary, vaginally, rectally, or intraocularly. In exemplary embodiments, the inhibitor according to the invention may be administered intravenously (IV) and/or subcutaneously, and the GLP-1 agonist may be administered subcutaneously.

In one aspect the present invention may be applied in the compounds, processes, compositions or uses of the following Sentences numbered 1-101 wherein reference to any Formula in the Sentences 1-101 refers only to those Formulas that are defined within Sentences 1-101. These formulae are reproduced in Figure 6. Specifically, an oligonucleoside moiety as represented by Z in any of the following sentences can comprise a nucleic acid for inhibiting expression of SLC25A5 as defined in any of the claims hereinafter.
1. A compound comprising the following structure: 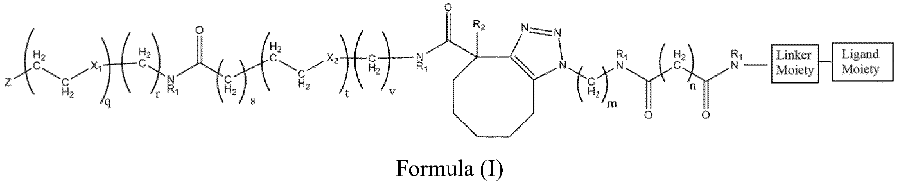 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
2. A compound according to Sentence 1, wherein R₁ is hydrogen at each occurrence.
3. A compound according to Sentence 1, wherein R₁ is methyl.
4. A compound according to Sentence 1, wherein R₁ is ethyl.
5. A compound according to any of Sentences 1 to 4, wherein R₂ is hydroxy.
6. A compound according to any of Sentences 1 to 4, wherein R₂ is halo.
7. A compound according to Sentence 6, wherein R₂ is fluoro.
8. A compound according to Sentence 6, wherein R₂ is chloro.
9. A compound according to Sentence 6, wherein R₂ is bromo.
10. A compound according to Sentence 6, wherein R₂ is iodo.
11. A compound according to Sentence 6, wherein R₂ is nitro.
12. A compound according to any of Sentences 1 to 11, wherein X₁ is methylene.
13. A compound according to any of Sentences 1 to 11, wherein X₁ is oxygen.
14. A compound according to any of Sentences 1 to 11, wherein X₁ is sulfur.
15. A compound according to any of Sentences 1 to 14, wherein X₂ is methylene.
16. A compound according to any of Sentences 1 to 15, wherein X₂ is oxygen.
17. A compound according to any of Sentences 1 to 16, wherein X₂ is sulfur.
18. A compound according to any of Sentences 1 to 17, wherein m = 3.
19. A compound according to any of Sentences 1 to 18, wherein n = 6.
20. A compound according to Sentences 13 and 15, wherein X₁ is oxygen and X₂ is methylene, and preferably wherein:
   q = 1,
   r = 2,
   s = 1,
   t = 1,
   v = 1.
21. A compound according to Sentences 12 and 15, wherein both X₁ and X₂ are methylene, and preferably wherein:
   q = 1,
   r = 3,
   s = 1,
   t = 1,
   v = 1.
22. A compound according to any of Sentences 1 to 21, wherein Z is: 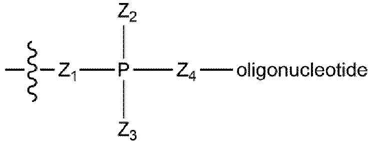 wherein:
   Z₁, Z₂, Z₃, Z₄ are independently at each occurrence oxygen or sulfur; and
   one the bonds between P and Z₂, and P and Z₃ is a single bond and the other bond is a double bond.
23. A compound according to Sentence 22, wherein said oligonucleoside is an RNA compound capable of modulating, preferably inhibiting, expression of a target gene.
24. A compound according to Sentence 23, wherein said RNA compound comprises an RNA duplex comprising first and second strands, wherein the first strand is at least partially complementary to an RNA sequence of a target gene, and the second strand is at least partially complementary to said first strand, and wherein each of the first and second strands have 5' and 3' ends.
25. A compound according to Sentence 24, wherein the RNA compound is attached at the 5' end of its second strand to the adjacent phosphate.
26. A compound according to Sentence 24, wherein the RNA compound is attached at the 3' end of its second strand to the adjacent phosphate.
27. A compound of Formula (II): 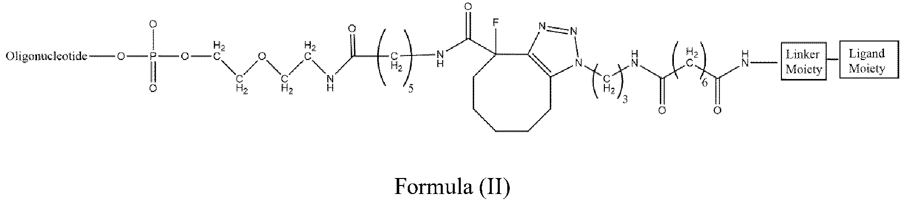
28. A compound of Formula (III): 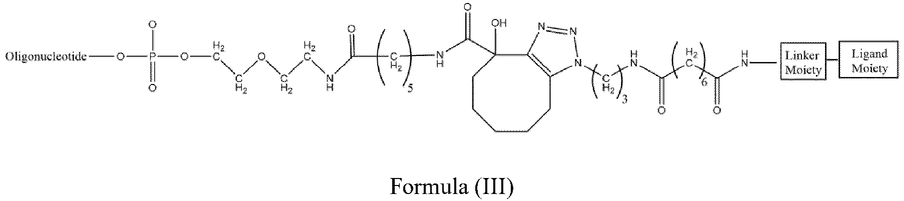
29. A compound according to Sentence 27 or 28, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
30. A composition comprising a compound of Formula (II) as defined in Sentence 27, and a compound of Formula (III) as defined in Sentence 28, optionally dependent on Sentence 29.
31. A composition according to Sentence 30, wherein said compound of Formula (III) as defined in Sentence 28 is present in an amount in the range of 10 to 15% by weight of said composition.
32. A compound of Formula (IV): 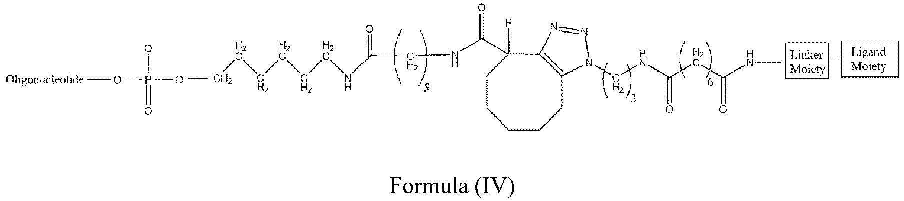
33. A compound of Formula (V): 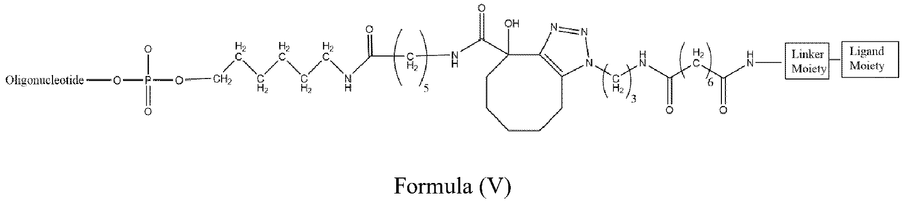
34. A compound according to Sentence 32 or 33, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
35. A composition comprising a compound of Formula (IV) as defined in Sentence 32, and a compound of Formula (V) as defined in Sentence 33, optionally dependent on Sentence 34.
36. A composition according to Sentence 35, wherein said compound of Formula (V) as defined in Sentence 33 is present in an amount in the range of 10 to 15% by weight of said composition.
37. A compound as defined in any of Sentences 1 to 29, or 32 to 34, wherein the oligonucleoside comprises an RNA duplex which further comprises one or more riboses modified at the 2' position, preferably a plurality of riboses modified at the 2' position.
38. A compound according to Sentence 37, wherein the modifications are chosen from 2'-O-methyl, 2'-deoxy-fluoro, and 2'-deoxy.
39. A compound according to any of Sentences 1 to 29, or 32 to 34, or 37 to 38, wherein the oligonucleoside further comprises one or more degradation protective moieties at one or more ends.
40. A compound according to Sentence 39, wherein said one or more degradation protective moieties are not present at the end of the oligonucleoside strand that carries the ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the strand that carries the ligand moieties.
41. A compound according to any of Sentences 1 to 29, or 32 to 34, or 37 to 40, wherein said ligand moiety as depicted in Formula (I) in Sentence 1 comprises one or more ligands.
42. A compound according to Sentence 41, wherein said ligand moiety as depicted in Formula (I) in Sentence 1 comprises one or more carbohydrate ligands.
43. A compound according to Sentence 42, wherein said one or more carbohydrates can be a monosaccharide, disaccharide, trisaccharide, tetrasaccharide, oligosaccharide or polysaccharide.
44. A compound according to Sentence 43, wherein said one or more carbohydrates comprise one or more galactose moieties, one or more lactose moieties, one or more N-AcetylGalactosamine moieties, and / or one or more mannose moieties.
45. A compound according to Sentence 44, wherein said one or more carbohydrates comprise one or more N-Acetyl-Galactosamine moieties.
46. A compound according to Sentence 45, which comprises two or three N-AcetylGalactosamine moieties.
47. A compound according to any of Sentences 41 to 46, wherein said one or more ligands are attached in a linear configuration, or in a branched configuration.
48. A compound according to Sentence 47, wherein said one or more ligands are attached as a biantennary or triantennary branched configuration.
49. A compound according to Sentences 46 to 48, wherein said moiety: 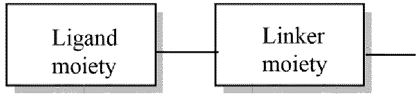 as depicted in Formula (I) in Sentence 1 is any of Formulae (VIa), (VIb) or (VIc), preferably Formula (VIa): 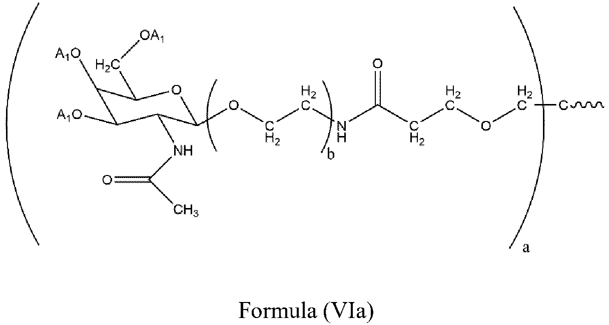 wherein:
   A_{I} is hydrogen, or a suitable hydroxy protecting group;
   a is an integer of 2 or 3; and
   b is an integer of 2 to 5; or 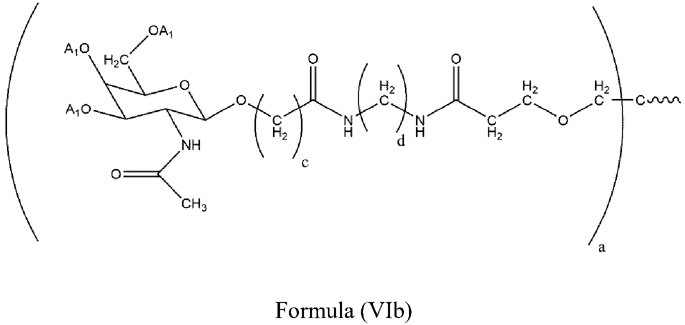
   wherein:
      A_{I} is hydrogen, or a suitable hydroxy protecting group;
      a is an integer of 2 or 3; and
      c and d are independently integers of 1 to 6; or 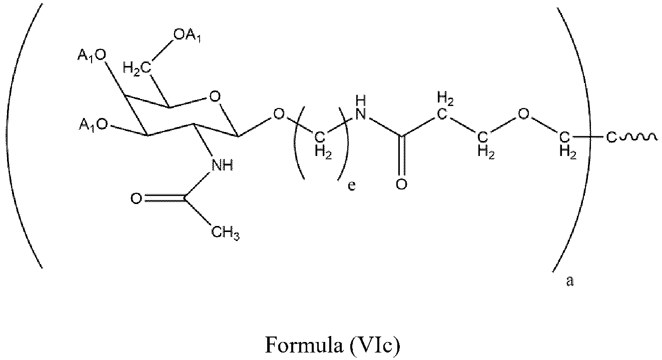
      wherein:
         A_{I} is hydrogen, or a suitable hydroxy protecting group;
         a is an integer of 2 or 3; and
         e is an integer of 2 to 10.
50. A compound according to Sentences 46 to 48, wherein said moiety: 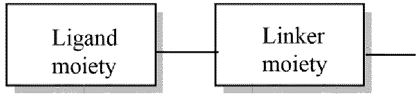 as depicted in Formula (I) in Sentence 1 is Formula (VII): 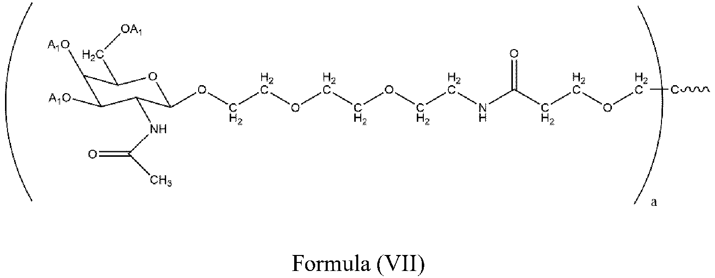 wherein:
   A_{I} is hydrogen;
   a is an integer of 2 or 3.
51. A compound according to Sentence 49 or 50, wherein a = 2.
52. A compound according to Sentence 49 or 50, wherein a = 3.
53. A compound according to Sentence 49, wherein b = 3.
54. A compound of Formula (VIII): 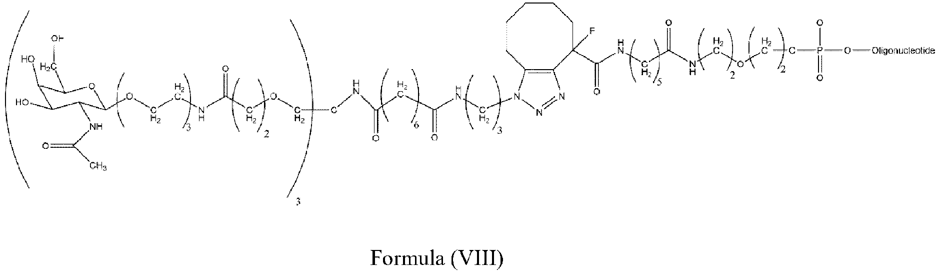
55. A compound of Formula (IX): 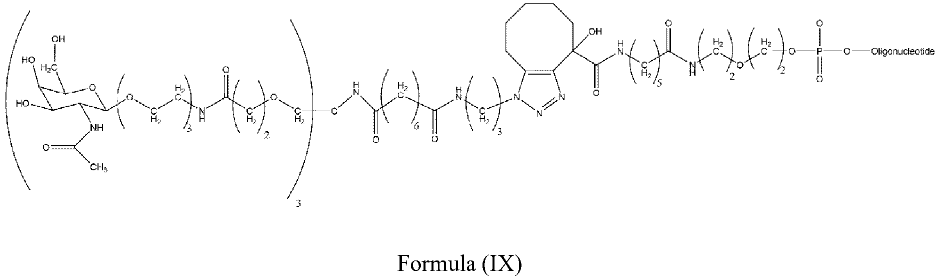
56. A compound according to Sentence 54 or 55, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
57. A composition comprising a compound of Formula (VIII) as defined in Sentence 54, and a compound of Formula (IX) as defined in Sentence 55, optionally dependent on Sentence 56.
58. A composition according to Sentence 57, wherein said compound of Formula (IX) as defined in Sentence 55 is present in an amount in the range of 10 to 15% by weight of said composition.
59. A compound of Formula (X): 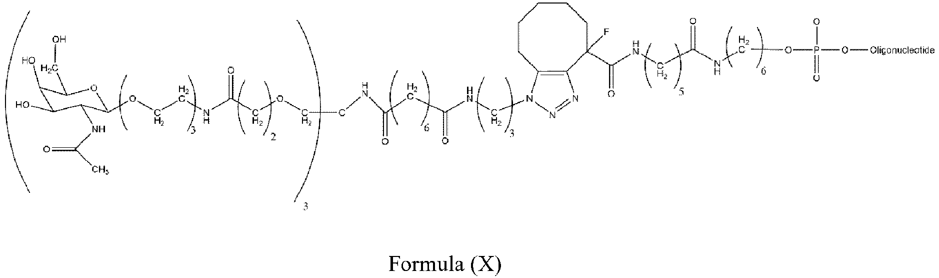
60. A compound of Formula (XI): 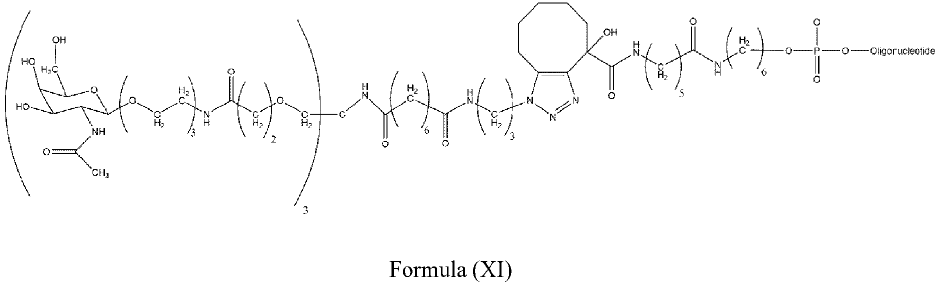
61. A compound according to Sentence 59 or 60, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
62. A composition comprising a compound of Formula (X) as defined in Sentence 59, and a compound of Formula (XI) as defined in Sentence 60, optionally dependent on Sentence 61.
63. A composition according to Sentence 62, wherein said compound of Formula (XI) as defined in Sentence 60 is present in an amount in the range of 10 to 15% by weight of said composition.
64. A compound as defined in any of Sentences 54 to 63, wherein the oligonucleoside comprises an RNA duplex which further comprises one or more riboses modified at the 2' position, preferably a plurality of riboses modified at the 2' position.
65. A compound according to Sentence 64, wherein the modifications are chosen from 2'-O-methyl, 2'-deoxy-fluoro, and 2'-deoxy.
66. A compound according to any of Sentences 54 to 65, wherein the oligonucleoside further comprises one or more degradation protective moieties at one or more ends.
67. A compound according to Sentence 66, wherein said one or more degradation protective moieties are not present at the end of the oligonucleoside strand that carries the ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the strand that carries the ligand moieties, as shown in any of Formulae (VIII), (IX), (X) or (XI) in any of Sentences 54, 55, 59 or 60.
68. A process of preparing a compound according to any of Sentences 1 to 29, 32 to 34, 37 to 56, 59 to 61, and 64 to 67, and / or a composition according to any of Sentences 30, 31, 35, 36, 57, 58, 62, 63, which comprises reacting compounds of Formulae (XII) and (XIII): 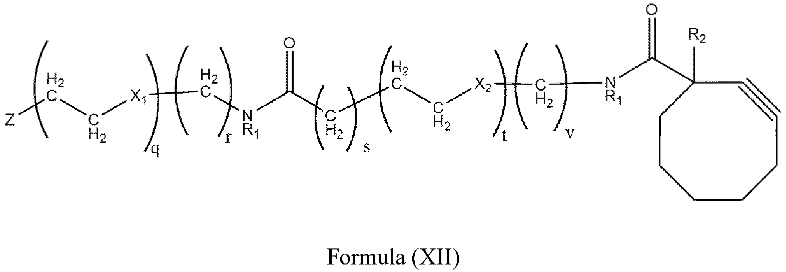 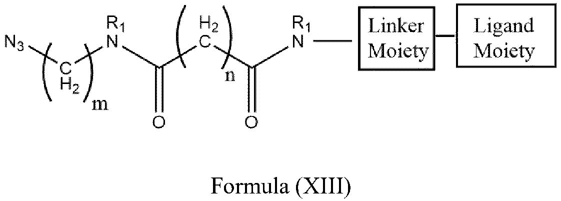 herein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety;
   and where appropriate carrying out deprotection of the ligand and / or annealing of a second strand for the oligonucleoside moiety.
69. A process according to Sentence 68, wherein a compound of Formula (XII) is prepared by reacting compounds of Formulae (XIV) and (XV): 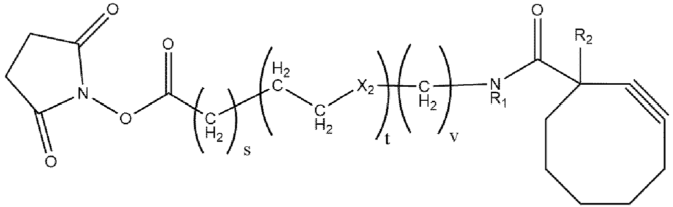 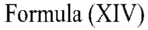 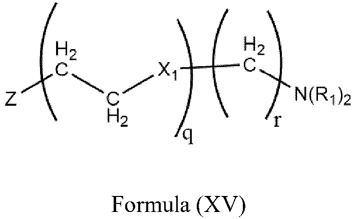
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
70. A process according to Sentence 68, to prepare a compound according to any of Sentences 20, 25, 27, 29, 54, 56, and / or a composition according to any of Sentences 30, 31, 57, 58, wherein:
   compound of Formula (XII) is Formula (XIIa): 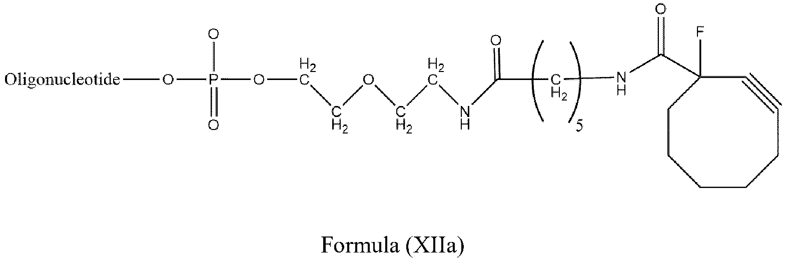
   and compound of Formula (XIII) is Formula (XIIIa): 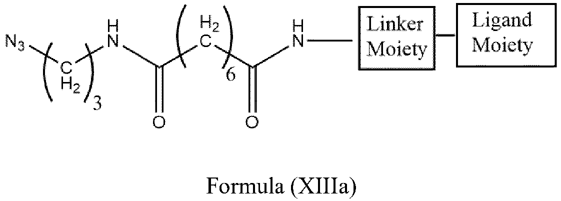
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
71. A process according to Sentence 68, to prepare a compound according to any of Sentences 20, 25, 28, 29, 55, 56, and / or a composition according to any of Sentences 30, 31, 57, 58, wherein:
   compound of Formula (XII) is Formula (XIIb): 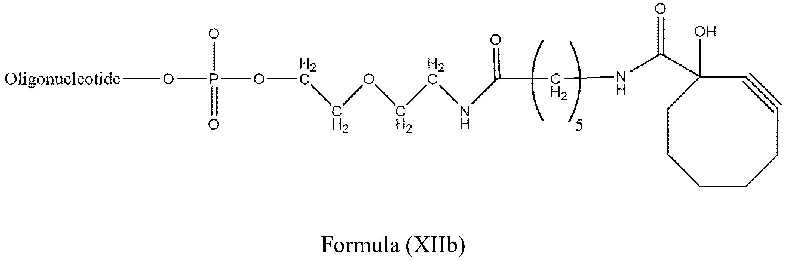
   and compound of Formula (XIII) is Formula (XIIIa): 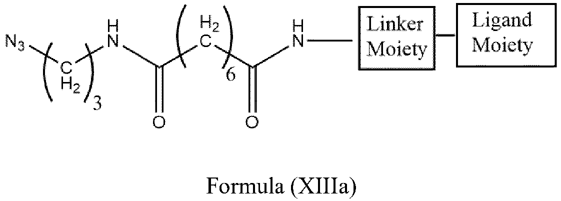
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
72. A process according to Sentence 68, to prepare a compound according to any of Sentences 21, 26, 32, 34, 59, 61, and / or a composition according to any of Sentences 35, 36, 62, 63, wherein:
   compound of Formula (XII) is Formula (XIIc): 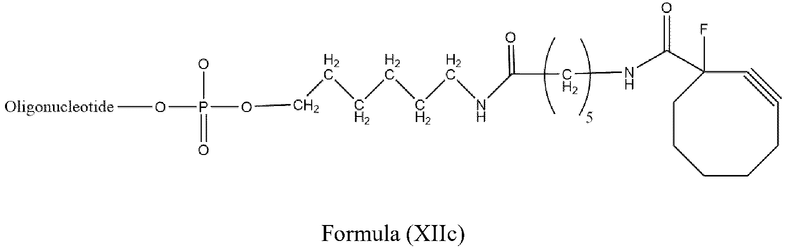
   and compound of Formula (XIII) is Formula (XIIIa): 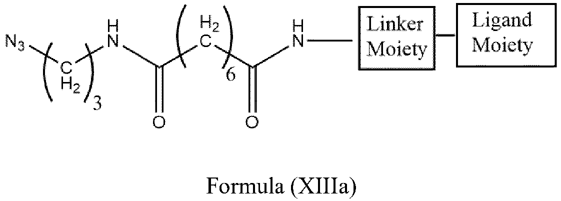
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
73. A process according to Sentence 68, to prepare a compound according to any of Sentences 21, 26, 33, 34, 60, 61, and / or a composition according to any of Sentences 35, 36, 62, 63, wherein:
   compound of Formula (XII) is Formula (XIId): 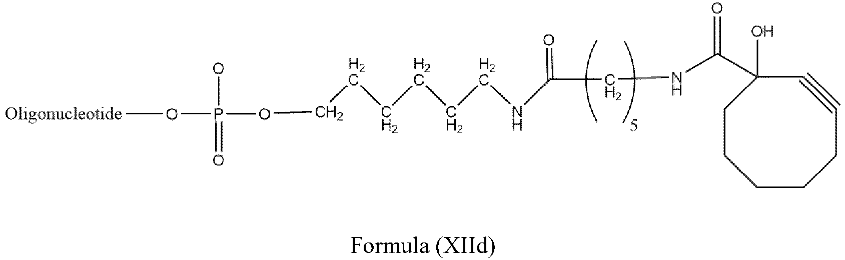
   and compound of Formula (XIII) is Formula (XIIIa): 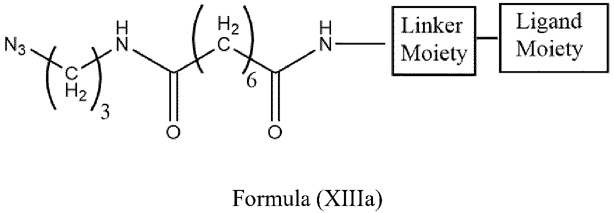
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
74. A process according to any of Sentences 70 to 73, wherein:
   compound of Formula (XIIIa) is Formula (XIIIb): 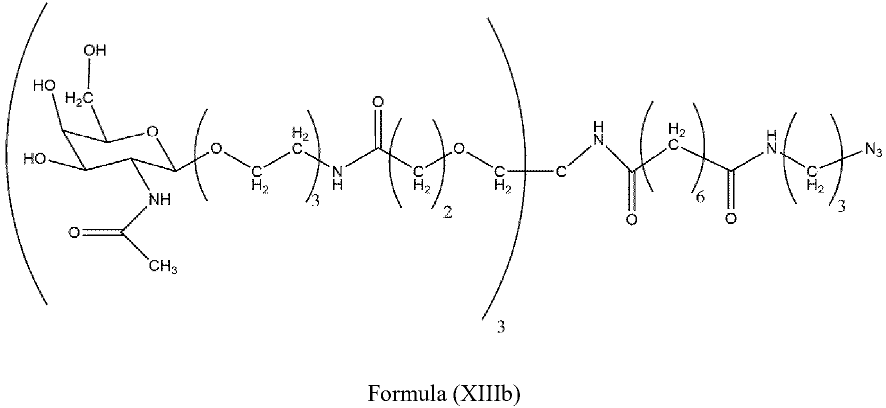
75. A process according to Sentences 69, as dependent on Sentences 70 to 73, wherein:
   compound of Formula (XIV) is either Formula (XIVa) or Formula (XIVb): 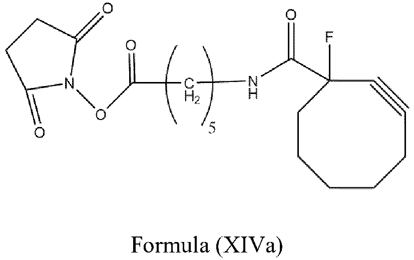 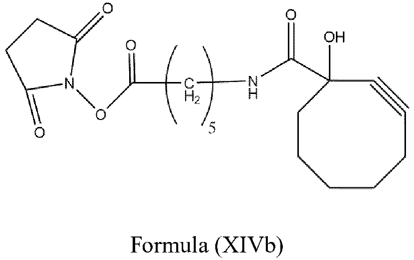
   and compound of Formula (XV) is either Formula (XVa) or Formula (XIVb): 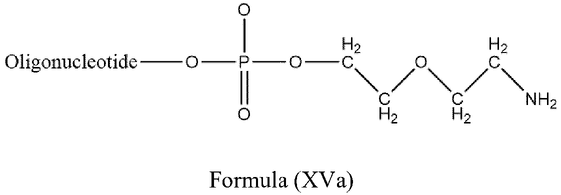 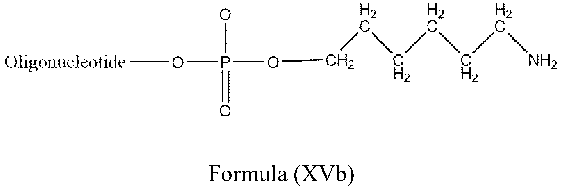
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein (i) said RNA duplex is attached at the 5' end of its second strand to the adjacent phosphate in Formula (XVa), or (ii) said RNA duplex is attached at the 3' end of its second strand to the adjacent phosphate in Formula (XVb).
76. A compound of Formula (XII): 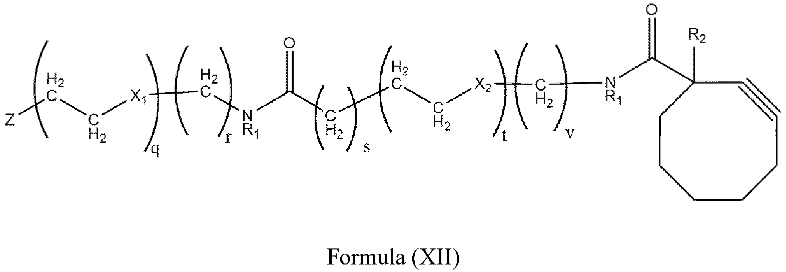 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
77. A compound of Formula (XIIa): 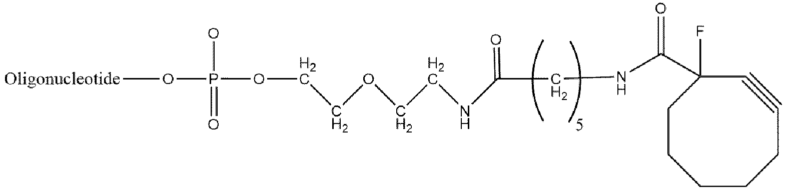 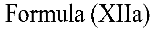
78. A compound of Formula (XIIb): 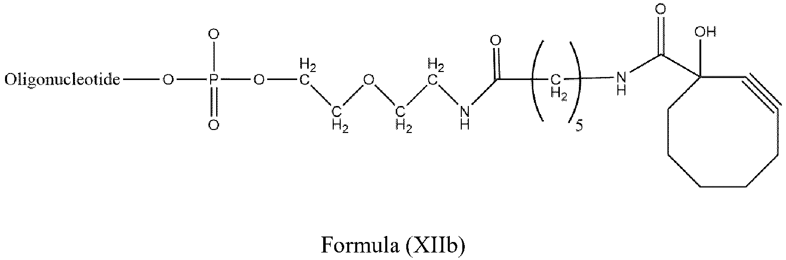
79. A compound of Formula (XIIc): 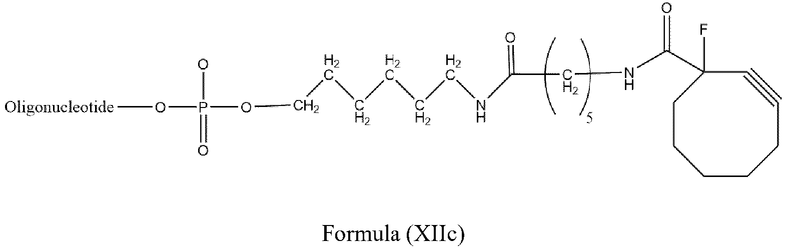
80. A compound of Formula (XIId): 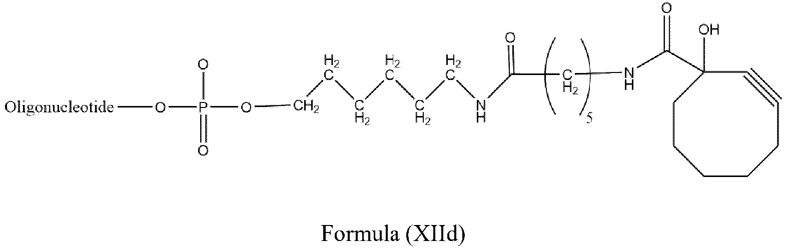
81. A compound of Formula (XIII): 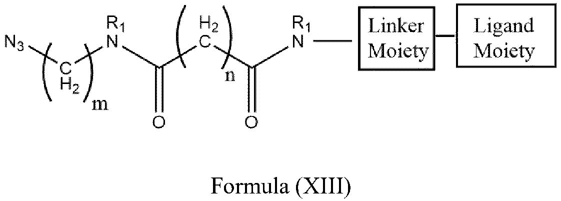 wherein:
   R₁ at each occurrence is independently selected from the group consisting of hydrogen, methyl and ethyl;
   m is an integer of from 1 to 6;
   n is an integer of from 1 to 10.
82. A compound of Formula (XIIIa): 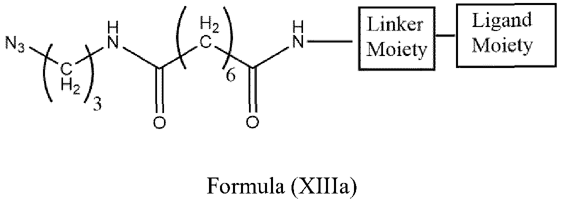
83. A compound of Formula (XIIIb): 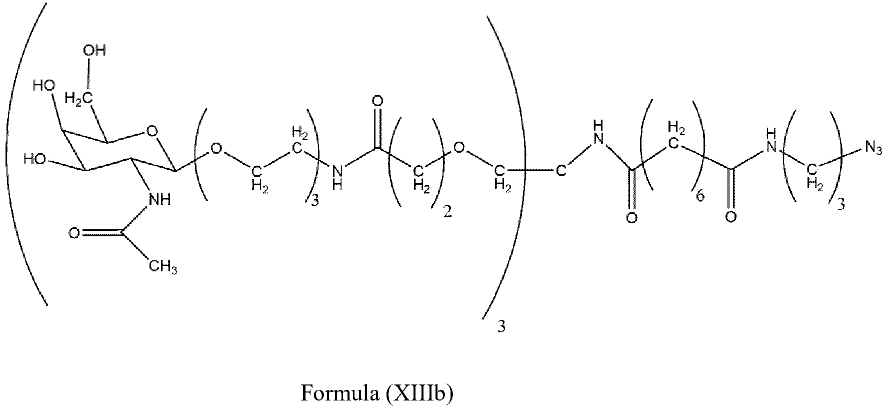
84. A compound of Formula (XIV): 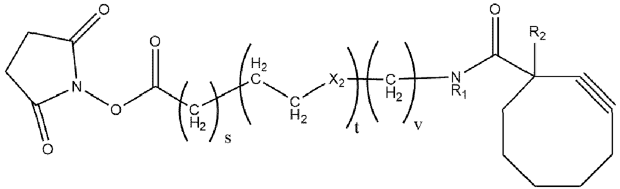 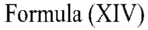 wherein:
   R₁ is selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₂ is selected from the group consisting of methylene, oxygen and sulfur;
   s, t, v are independently integers from 0 to 4, with the proviso that s, t and v cannot all be 0 at the same time.
85. A compound of Formula (XIVa): 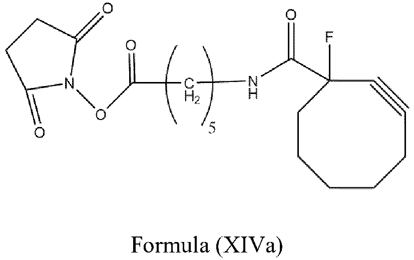
86. A compound of Formula (XIVb): 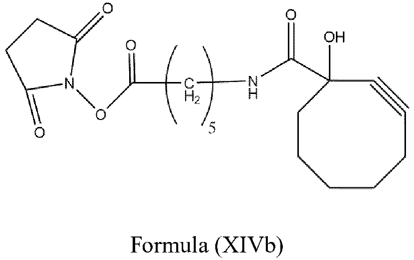
87. A compound of Formula (XV): 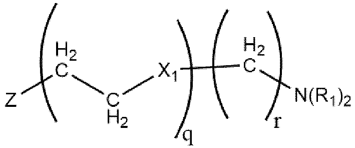 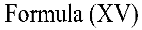 wherein:
   R₁ at each occurrence is independently selected from the group consisting of hydrogen, methyl and ethyl;
   X₁ is selected from the group consisting of methylene, oxygen and sulfur;
   q and r are independently integers from 0 to 4, with the proviso that q and r cannot both be 0 at the same time;
   Z is an oligonucleoside moiety.
88. A compound of Formula (XVa): 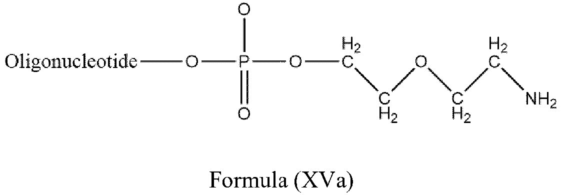
89. A compound of Formula (XVb): 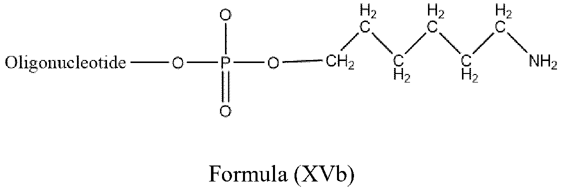
90. Use of a compound according to any of Sentences 76, 81 to 84, 87, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63.
91. Use of a compound according to Sentence 85, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63, wherein R₂ = F.
92. Use of a compound according to Sentence 86, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63, wherein R₂ = OH.
93. Use of a compound according to Sentence 77, for the preparation of a compound according to any of Sentences 20, 25, 27, 29, 54, 56, and / or a composition according to any of Sentences 30, 31, 57, 58.
94. Use of a compound according to Sentence 78, for the preparation of a compound according to any of Sentences 20, 25, 28, 29, 55, 56, and / or a composition according to any of Sentences 30, 31, 57, 58.
95. Use of a compound according to Sentence 79, for the preparation of a compound according to any of Sentences 21, 26, 32, 34, 59, 61, and / or a composition according to any of Sentences 35, 36, 62, 63.
96. Use of a compound according to Sentence 80, for the preparation of a compound according to any of Sentences 21, 26, 33, 34, 60, 61, and / or a composition according to any of Sentences 35, 36, 62, 63.
97. Use of a compound according to Sentence 88, for the preparation of a compound according to any of Sentences 20, 25, 27 to 29, 54 to 56, and / or a composition according to any of Sentences 30, 31, 57, 58.
98. Use of a compound according to Sentence 89, for the preparation of a compound according to any of Sentences 21, 26, 32 to 34, 59 to 61, and / or a composition according to any of Sentences 35, 36, 62, 63.
99. A compound or composition obtained, or obtainable by a process according to any of Sentences 68 to 75.
100. A pharmaceutical composition comprising of a compound according to any of Sentences 1 to 29, 32 to 34, 37 to 56, 59 to 61, and 64 to 67, and / or a composition according to any of Sentences 30, 31, 35, 36, 57, 58, 62 and 63, together with a pharmaceutically acceptable carrier, diluent or excipient.
101. A compound according to any of Sentences 1 to 29, 32 to 34, 37 to 56, 59 to 61, and 64 to 67, and / or a composition according to any of Sentences 30, 31, 35, 36, 57, 58, 62 and 63, for use in therapy.

In another aspect the present invention may be applied in the compounds, processes, compositions or uses of the following Clauses numbered 1-56 wherein reference to any Formula in the Clauses refers only to those Formulas that are defined within Clause 1-56. These formulae are reproduced in Figure 7. Specifically, an oligonucleoside moiety as represented by Z in any of the following clauses can comprise a nucleic acid for inhibiting expression of SLC25A5 as defined in any of the claims hereinafter.
1. A compound comprising the following structure: 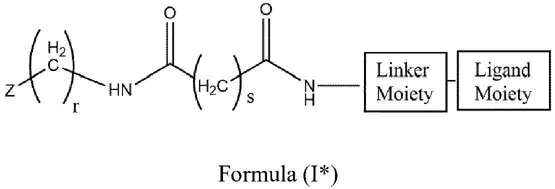 wherein:
   r and s are independently an integer selected from 1 to 16; and
   Z is an oligonucleoside moiety.
2. A compound according to Clause 1, wherein s is an integer selected from 4 to 12.
3. A compound according to Clause 2, wherein s is 6.
4. A compound according to any of Clauses 1 to 3, wherein r is an integer selected from 4 to 14.
5. A compound according to Clause 4, wherein r is 6.
6. A compound according to Clause 4, wherein r is 12.
7. A compound according to Clause 5, which is dependent on Clause 3.
8. A compound according to Clause 6, which is dependent on Clause 3.
9. A compound according to any of Clauses 1 to 8, wherein Z is: 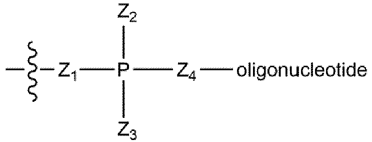 wherein:
   Z₁, Z₂, Z₃, Z₄ are independently at each occurrence oxygen or sulfur; and
   one the bonds between P and Z₂, and P and Z₃ is a single bond and the other bond is a double bond.
10. A compound according to any of Clauses 1 to 9, wherein said oligonucleoside is an RNA compound capable of modulating, preferably inhibiting, expression of a target gene.
11. A compound according to any of Clause 10, wherein said RNA compound comprises an RNA duplex comprising first and second strands, wherein the first strand is at least partially complementary to an RNA sequence of a target gene, and the second strand is at least partially complementary to said first strand, and wherein each of the first and second strands have 5' and 3' ends.
12. A compound according to Clause 11, preferably also dependent on Clauses 3 and 6, wherein the RNA compound is attached at the 5' end of its second strand to the adjacent phosphate.
13. A compound according to Clause 11, preferably also dependent on Clauses 3 and 5, wherein the RNA compound is attached at the 3' end of its second strand to the adjacent phosphate.
14. A compound of Formula (II*), preferably dependent on Clause 12: 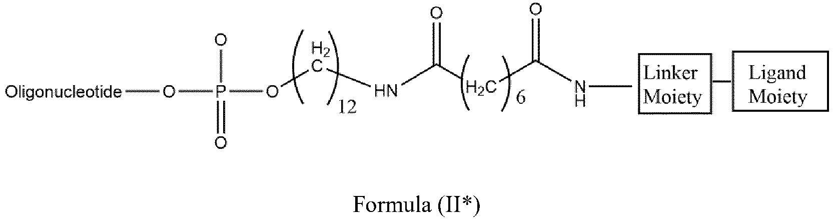
15. A compound of Formula (III*), preferably dependent on Clause 13: 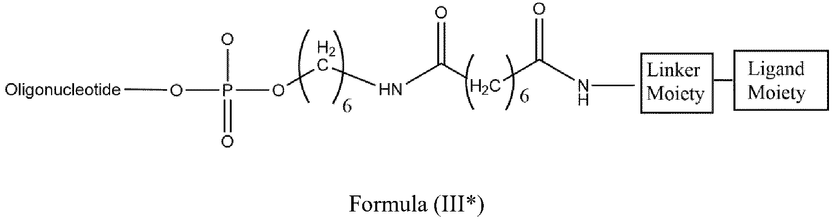
16. A compound as defined in any of Clauses 1 to 15, wherein the oligonucleoside comprises an RNA duplex which further comprises one or more riboses modified at the 2' position, preferably a plurality of riboses modified at the 2' position.
17. A compound according to Clause 16, wherein the modifications are chosen from 2'-O-methyl, 2'-deoxy-fluoro, and 2'-deoxy.
18. A compound according to any of Clauses 1 to 17, wherein the oligonucleoside further comprises one or more degradation protective moieties at one or more ends.
19. A compound according to Clause 18, wherein said one or more degradation protective moieties are not present at the end of the oligonucleoside strand that carries the linker / ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the same strand to the end that carries the linker / ligand moieties.
20. A compound according to any of Clauses 1 to 19, wherein said ligand moiety as depicted in Formula (I*) in Clause 1 comprises one or more ligands.
21. A compound according to Clause 20, wherein said ligand moiety as depicted in Formula (I*) in Clause 1 comprises one or more carbohydrate ligands.
22. A compound according to Clause 21, wherein said one or more carbohydrates can be a monosaccharide, disaccharide, trisaccharide, tetrasaccharide, oligosaccharide or polysaccharide.
23. A compound according to Clause 22, wherein said one or more carbohydrates comprise one or more galactose moieties, one or more lactose moieties, one or more N-AcetylGalactosamine moieties, and / or one or more mannose moieties.
24. A compound according to Clause 23, wherein said one or more carbohydrates comprise one or more N-Acetyl-Galactosamine moieties.
25. A compound according to Clause 24, which comprises two or three N-AcetylGalactosamine moieties.
26. A compound according to any of the preceding Clauses, wherein said one or more ligands are attached in a linear configuration, or in a branched configuration.
27. A compound according to Clause 26, wherein said one or more ligands are attached as a biantennary or triantennary branched configuration.
28. A compound according to Clauses 20 to 27, wherein said moiety: 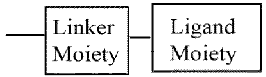 as depicted in Formula (I*) in Clause 1 is any of Formulae (IV*), (V*) or (VI*), preferably Formula (IV*): 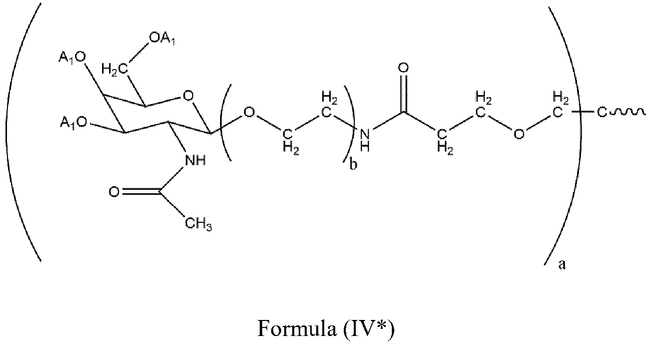 wherein:
   A_{I} is hydrogen, or a suitable hydroxy protecting group;
   a is an integer of 2 or 3; and
   b is an integer of 2 to 5; or 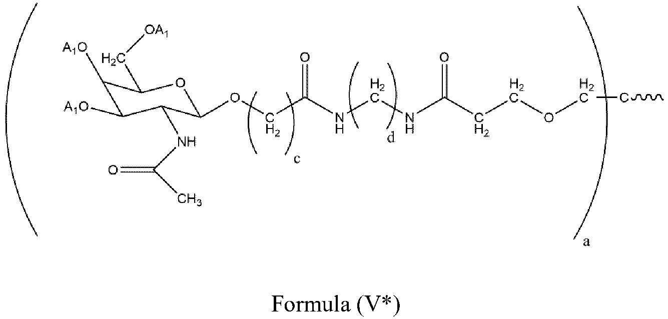
   wherein:
      A_{I} is hydrogen, or a suitable hydroxy protecting group;
      a is an integer of 2 or 3; and
      c and d are independently integers of 1 to 6; or 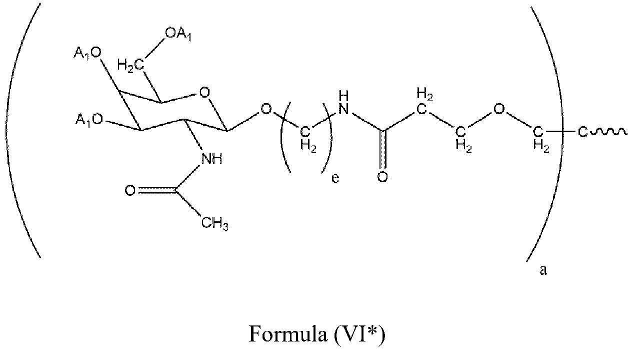
      wherein:
         A_{I} is hydrogen, or a suitable hydroxy protecting group;
         a is an integer of 2 or 3; and
         e is an integer of 2 to 10.
29. A compound according to any of Clauses 1 to 28, wherein said moiety: 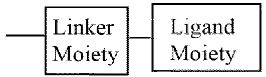 as depicted in Formula (I*) in Clause 1 is Formula (VII*): 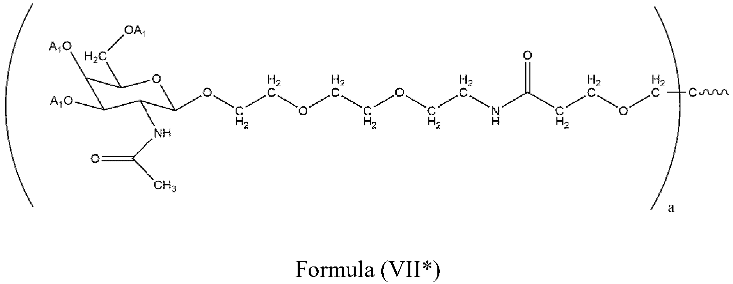 wherein:
   A_{I} is hydrogen;
   a is an integer of 2 or 3.
30. A compound according to Clause 28 or 29, wherein a = 2.
31. A compound according to Clause 28 or 29, wherein a = 3.
32. A compound according to Clause 28, wherein b = 3.
33. A compound of Formula (VIII*): 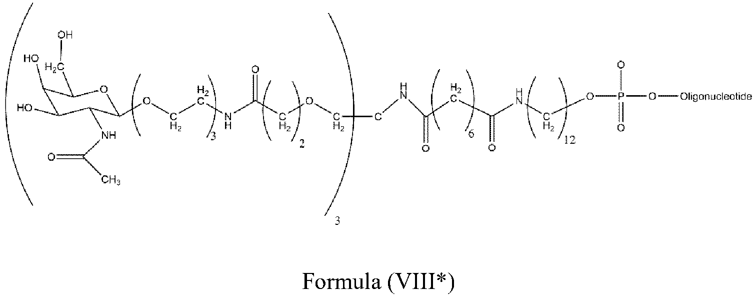
34. A compound of Formula (IX*): 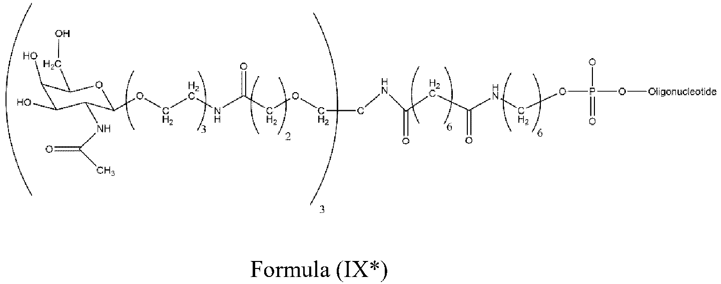
35. A compound according to Clause 33 or 34, wherein the oligonucleoside comprises an RNA duplex which further comprises one or more riboses modified at the 2' position, preferably a plurality of riboses modified at the 2' position.
36. A compound according to Clause 35, wherein the modifications are chosen from 2'-O-methyl, 2'-deoxy-fluoro, and 2'-deoxy.
37. A compound according to any of Clauses 33 to 36, wherein the oligonucleoside further comprises one or more degradation protective moieties at one or more ends.
38. A compound according to Clause 37, wherein said one or more degradation protective moieties are not present at the end of the oligonucleoside strand that carries the linker / ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the same strand to the end that carries the linker / ligand moieties.
39. A compound according to Clause 33, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
40. A compound according to Clause 34, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
41. A process of preparing a compound according to any of Clauses 1 to 40, which comprises reacting compounds of Formulae (X*) and (XI*): 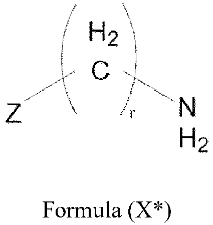 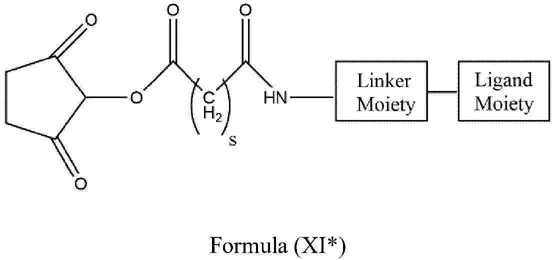 wherein:
   r and s are independently an integer selected from 1 to 16; and
   Z is an oligonucleoside moiety;
   and where appropriate carrying out deprotection of the ligand and / or annealing of a second strand for the oligonucleoside.
42. A process according to Clause 41, to prepare a compound according to any of Clauses 6, 8 to 14, 16 to 33, and 35 to 40, wherein:
   compound of Formula (X*) is Formula (Xa*): 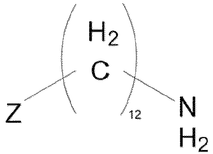 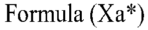
   and compound of Formula (XI*) is Formula (XIa*): 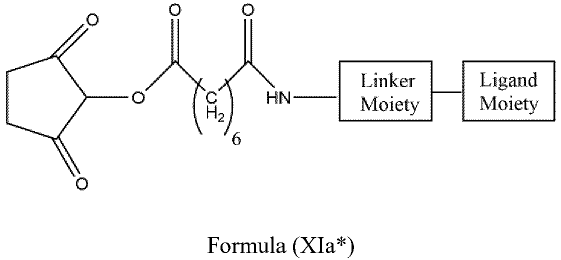
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
43. A process according to Clause 41, to prepare a compound according to any of Clauses 5, 7, 9 to 13, 15 to 32, and 34 to 40, wherein:
   compound of Formula (X*) is Formula (Xb*): 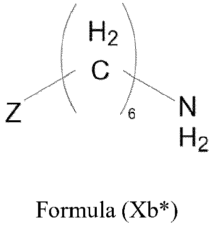
   and compound of Formula (XI*) is Formula (XIa*): 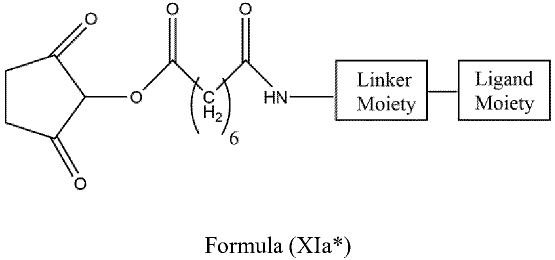
   wherein the oligonucleoside comprises an RNA duplex comprising first and second strands,
   wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
44. A process according to Clauses 42 or 43, wherein:
   compound of Formula (XIa*) is Formula (XIb*): 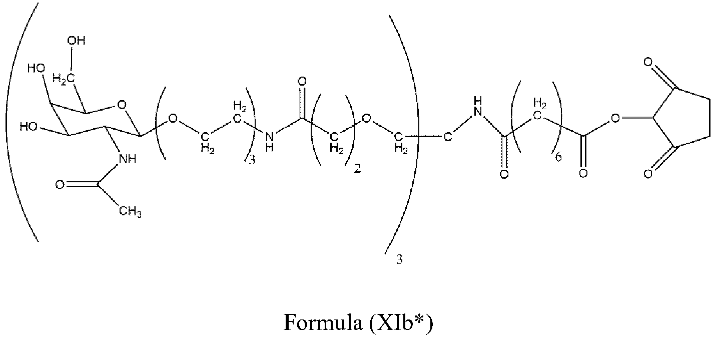
45. A compound of Formula (X*): 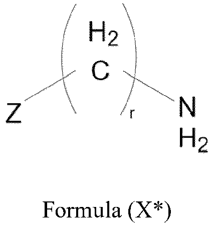 wherein:
   r is independently an integer selected from 1 to 16; and
   Z is an oligonucleoside moiety.
46. A compound of Formula (Xa*): 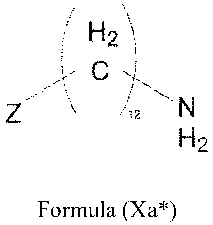
47. A compound of Formula (Xb*): 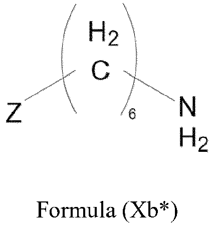
48. A compound of Formula (XI*): 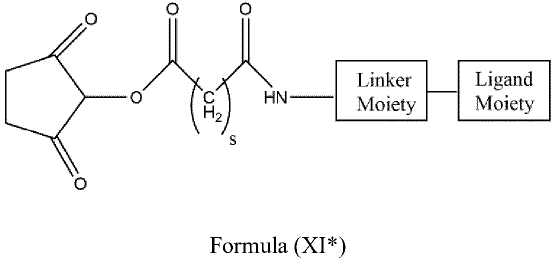 wherein:
   s is independently an integer selected from 1 to 16; and
   Z is an oligonucleoside moiety.
49. A compound of Formula (XIa*): 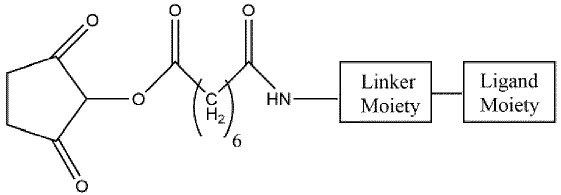 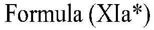
50. A compound of Formula (XIb*): 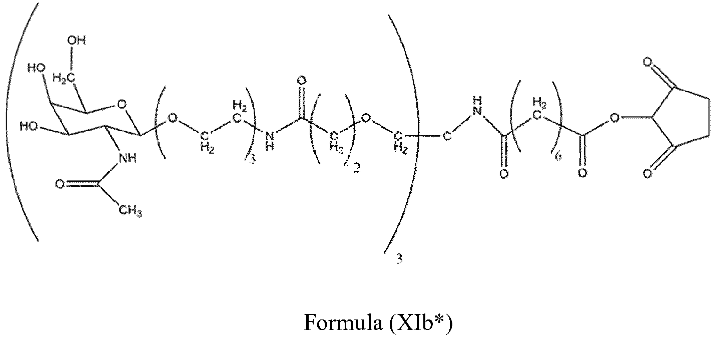
51. Use of a compound according to any of Clauses 45 and 48 to 50, for the preparation of a compound according to any of Clauses 1 to 40.
52. Use of a compound according to Clause 46, for the preparation of a compound according to any of Clauses 6, 8 to 14, 16 to 33, and 35 to 40.
53. Use of a compound according to Clause 47, for the preparation of a compound according to any of Clauses 5, 7, 9 to 13, 15 to 32, and 34 to 40.
54. A compound or composition obtained, or obtainable by a process according to any of Clauses 41 to 44.
55. A pharmaceutical composition comprising of a compound according to any of Clauses 1 to 40, together with a pharmaceutically acceptable carrier, diluent or excipient.
A compound according to any of Clauses 1 to 40, for use in therapy.

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

### Example 1 - Target identification

### Background

All biological functions arise from the co-ordinated interaction of hundreds of interacting molecules - primarily proteins - and can be thought of as the emergent functional consequence of protein-protein interaction networks where each node in the network is a protein and each edge linking the proteins can represent a range of possible interaction types from complex formation to catalytic activation etc.

Historically such functions have been represented as simplistic linear pathways. As knowledge has grown however, it has become clear that pathways are more complex and that the minimum level of complexity that adequately represents the functional properties of a biological process, and can capture the characteristics of resilience to perturbation and robustness to random damage of individual components, is a network.

Networks subserving biological function may consist of several interacting canonical pathways as well as additional proteins that are primarily engaged when the canonical function is perturbed. It is therefore critical to develop approaches that can model this complexity in a meaningful and tractable way; and generate target hypotheses that take account of the inherent resistance to change that is a consequence of network robustness.

Oversimplification of biology and the failure to make rational drug target choices based on realistic models of biological processes has contributed to the poor success rate of drug discovery. Furthermore, there has been a lack of rigorous objective methods both for making network models of processes and for prioritising protein targets within these models. As a consequence target selection decisions are frequently made on an ad hoc basis based on preference or evidence unrelated to the functional model.

### Process for identifying processes and targets

The starting point for the identification of the target SLC25A5/ANT2 was HepNet, a proprietary computational platform for the identification and validation of drug targets in hepatocytes. Two independent approaches were used for the identification of SLC25A5/ANT2, the first utilised omics data in the form of Genome Wide Association Studies (GWAS) metanalyses to identify targets for Non-Alcoholic Fatty Liver Disease (NAFLD) from experimental data. The second approach utilised the HepNet knowledge graph, a semantic network describing interactions between entities such as genes, drugs, diseases and/or biological processes in the context of hepatocytes.

For the GWAS-based approach metanalyses were QC'ed for sample size, target population, and quality of statistical analysis. More specifically, a sample size of more than 1,000 was used as a cut-off,
SNPs were taken forward if the p-value was reflecting multiple testing corrections. Finally, sex-combined and all-ancestries SNPs were selected (continental European, UK, other); SNPs with gender-specific significance were excluded.

The resulting selected GWAS were used to extract the SNPs with strong association with the trait of interest, in this case NAFLD pathophysiology as well as associated metabolic dysfunction (for example, NAFLD and cardiometabolic diseases or steatosis and risk of NAFLD).

As a subsequent step, the SNPs were mapped to genes using the Variant-to-Gene (V2G) pipeline data from Open Targets Genetics (https://genetics.opentargets.org/). More specifically, for each SNP of interest the gene with the highest score (or genes, in case of a top-score tie) based on the V2G pipeline were selected, along with those genes with a score above 0.2.

This generated a set of genes that could be used as an input for building protein-protein interaction networks, using a proprietary algorithm (algorithm A). Impact analysis (algorithm B) was then performed on the resulting networks to identify the biological processes enriched in the networks. These processes were then manually assessed by an internal team to identify the most relevant biological processes for NAFLD target identification.

Protein-protein interaction networks were then built around the selected processes and the nodes scored for their importance to the network using a proprietary algorithm (algorithm C). The highest scoring nodes from algorithm C were manually assessed by an internal team to identify targets of interest for the treatment of NAFLD metabolic diseases.

For the knowledge graph-based approach machine learning algorithms were used to predict interactions between genes and disease based upon the structure of the knowledge graph, a task commonly referred to as 'link prediction'. This generated a set of genes that could be used for network-based target identification using proprietary algorithms A-C as described above. The networks and other outputs from these algorithms were different from the ones generated by the GWAS-based analysis and were assessed by a different internal team.

Both analyses independently identified ANT2/SLC25A5 as being an important node in a biological process involved in "Adipogenesis/Non-alcoholic fatty liver disease" (termed Supercluster 574 in algorithm B).

The inventors have thus analysed these network models using proprietary analytical methods. These methods use directional information to capture key 'target' properties such as whether a protein is an integrator of information, a key conduit of information to other parts of the network, an influencer of key proteins and the extent to which an influencer is influenced or influences other proteins (based on absolute and relative number and direction of inputs and outputs). The directional information also enables hierarchical relationships between proteins to be imputed. Proteins higher in the hierarchy and with certain properties may be preferred over others with otherwise similar properties. The relative specificity and magnitude of each property relative to the others made it possible for the inventors to score and rank proteins in terms of their target suitability.

The ability to characterise the properties of these targets in terms of network relationships enables judgements to be made on the selectivity and magnitude of effect in the chosen context and hence the suitability of each for a given indication.

Proprietary analytical techniques were then applied to the network models to identify pharmacologically viable targets from within the networks whose knockdown will have a significant influence on the network and by extension on the biological function being modelled. The algorithms make extensive use of directional information and hierarchical relationships to identify targets with a range of specific properties that will make them good siRNA targets. Targets were then further filtered by protein class and hepatocyte specific properties according to the therapeutic requirements.

The above workflow leveraging proprietary data resources and network node metrics has identified the provided target for the provided uses.

The outcome of the network approach is shown in Table 6. ANT2 was surprisingly identified as a drug target for NAFLD among various other targets that have been previously associated with metabolic disorders, such NAFLD (APOA5, HMDH, APOC3, NR1H3, MTP, PCSK9, SOAT1 and ABCA1).

**Table 6**

| **Protein name** | **Accession number** | **Prediction score for NAFLD** | **Hepatocellular expression rank** |
|---|---|---|---|
| APOA5 | Q6Q788 | 93.6 | 0.46 |
| HMDH | P04035 | 91.3 | 0.54 |
| APOC3 | P02656 | 91.2 | 0.95 |
| NR1H3 | Q13133 | 91.1 | 0.22 |
| MTP | P55157 | 88.1 | 0.96 |
| **ANT2** | **P05141** | **80** | **0.98** |
| PCSK9 | Q8NBP7 | 79.3 | 0.21 |
| SOAT1 | P35610 | 77.7 | 0.27 |
| ABCA1 | O095477 | 77.0 | 0.46 |

### Example 2: Synthesis of tether 1

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Sfär Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 x 100 mm) at 60 °C. The solvent system consisted of solvent A with H₂O containing 0.1% formic acid and solvent B with acetonitrile (ACN) containing 0.1% formic acid. A gradient from 5-100% of B over 15 min with a flow rate of 0.4 mL/min was employed.

Detector and conditions: Corona ultra-charged aerosol detection (from esa). Nebulizer Temp.: 25 °C. N₂ pressure: 35.1 psi. Filter: Corona.

₁H and ₁₃C NMR spectra were recorded at room temperature on a Varian spectrometer at 500 MHz (₁H NMR) and 125 MHz (₁₃C NMR). Chemical shifts are given in ppm referenced to the solvent residual peak (CDCl₃ - ₁H NMR: δ at 7.26 ppm and ₁₃C NMR δ at 77.2 ppm; DMSO-d6 - ₁H NMR: δ at 2.50 ppm and ₁₃C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc Tether1:

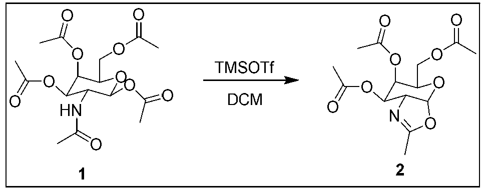

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO₃ (100 mL) and water (100 mL). The organic layer was separated, dried over Na₂SO₄ and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

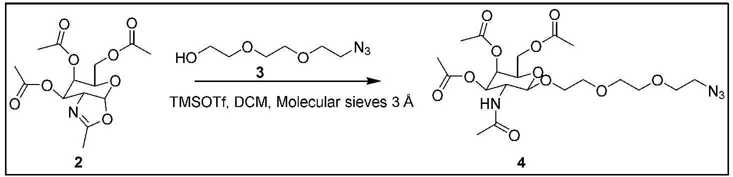

Preparation of compound 4: Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na₂SO₄ and the solvent was removed under reduced pressure. The crude material was purified by flash chromatography (gradient elution: 0-3% MeOH in DCM in 10 CV) to afford the title product as light yellow oil (3.10 g, 88%, rf = 0.25 (2% MeOH in DCM)). MS: calculated for C₂₀H₃₂N₄O₁₁, 504.21. Found 505.4. 1H NMR (500 MHz, CDCl3) δ 6.21-6.14 (m, 1H), 5.30 (dd, J = 3.4, 1.1 Hz, 1H), 5.04 (dd, J = 11.2, 3.4 Hz,1H), 4.76 (d, J = 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, J = 4.2 Hz, 6H). 13C NMR (125 MHz, CDCl3) δ 170.6 (C), 170.5 (C), 170.4 (C), 170.3 (C), 102.1 (CH), 71.6 (CH), 70.8 (CH), 70.6 (CH), 70.5 (CH), 70.3 (CH2), 69.7 (CH2), 68.5 (CH2), 66.6 (CH2), 61.5 (CH2), 23.1 (CH3), 20.7 (3xCH3).

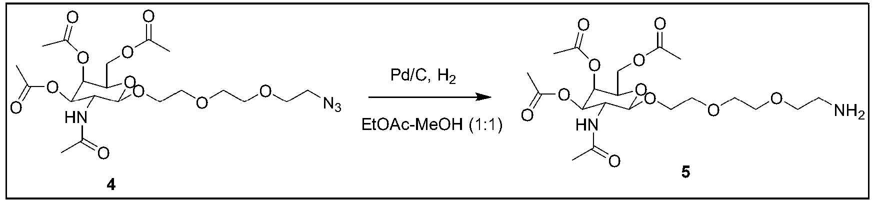

Preparation of compound 5: Compound 4 (1.00 g, 1.98 mmol, 1.0 eq.) was dissolved in a mixture of ethyl acetate (EtOAc) and MeOH (30 mL 1:1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

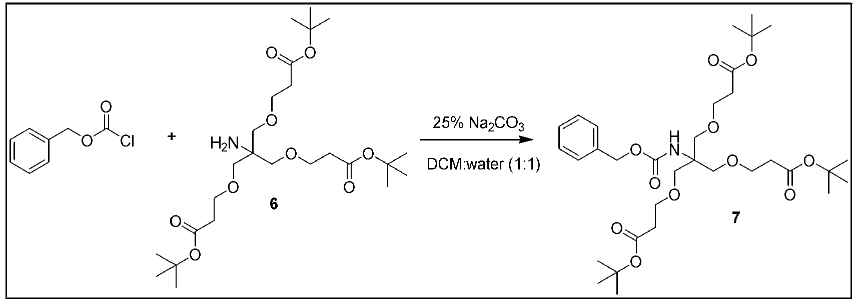

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na2SO4. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. 1H NMR (500 MHz, DMSO-d6) δ 7.38-7.26 (m, 5H), 4.97 (s, 2H), 3.54 (t, 6H), 3.50 (s, 6H), 2.38 (t, 6H), 1.39 (s, 27H). 13C NMR (125 MHz, DMSO-d6) δ 170.3 (3xC), 154.5 (C), 137.1 (C), 128.2 (2xCH), 127.7 (CH), 127.6 (2xCH), 79.7 (3xC), 68.4 (3xCH2), 66.8 (3xCH2), 64.9 (C), 58.7 (CH2), 35.8 (3xCH2), 27.7 (9xCH3).

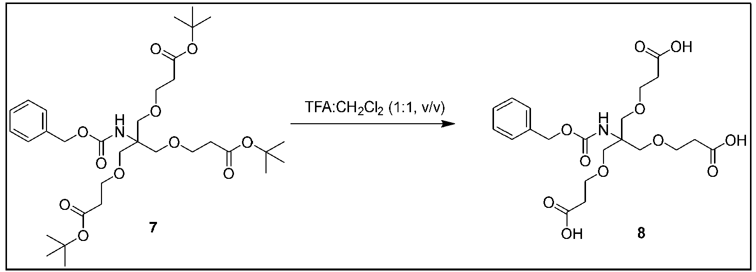

Preparation of compound 8: Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

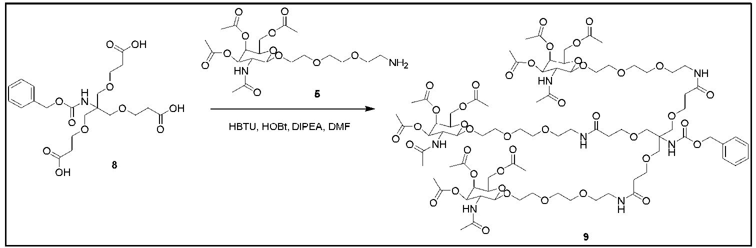

Preparation of compound 9: CbzNH-tris-COOH 8 (0.72 g, 1.49 mmol, 1.0 eq.) and GalNAc-PEG3-NH₂ 5 (3.56 g, 7.44 mmol, 5.0 eq.) were dissolved in N,N-dimethylformamide (DMF) (25 mL). Then N,N,N',N'-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and N,N-diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO₃ (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C_{S1}H₁₂₅N₇O₄₁, 1852.9. Found 1854.7. 1H NMR (500 MHz, DMSO-d6) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). 13C NMR (125 MHz, DMSO-d6) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH2), 69.0 (CH2), 68.2 (CH2), 67.2 (CH2), 66.7 (CH2), 61.4 (CH2), 22.6 (CH2), 22.4 (3xCH3), 20.7 (9xCH3).

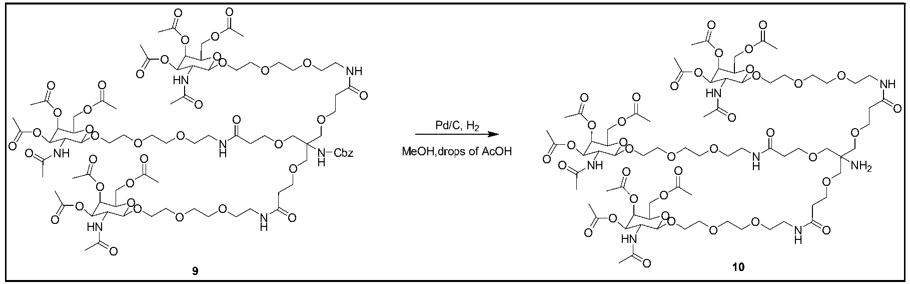

Preparation of compound 10: Triantennary GalNAc compound 9 (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

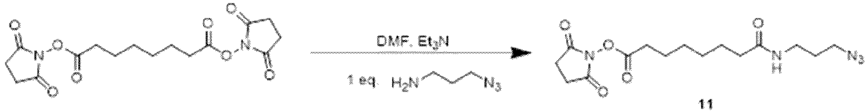

Preparation of compound 11: Commercially available suberic acid bis(N-hydroxysuccinimide ester) (3.67 g, 9.9 mmol, 1.0 eq.) was dissolved in DMF (5 mL) and triethylamine (1.2 mL) was added. To this solution was added dropwise a solution of 3-azido-1-propylamine (1.0 g, 9.9 mmol, 1.0 eq.) in DMF (5 mL). The reaction was stirred at room temperature for 3 h. The reaction mixture was diluted with EtOAc (100 mL) and washed with water (50 mL). The organic layer was separated, dried over Na₂SO₄ and the solvent was removed under reduced pressure. The crude material was purified by flash chromatography (gradient elution: 0-5% MeOH in DCM in 16 CV). The product was obtained as white solid (1.54 g, 43%, rf = 0.71 (5% MeOH in DCM)). MS: calculated for C₁₅H₂₃N₅O₅, 353.4. Found 354.3.

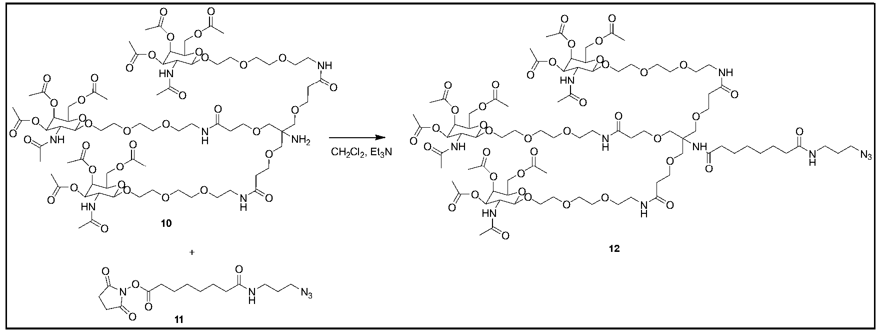

Preparation of TriGalNAc (12): Triantennary GalNAc compound 10 (0.35 g, 0.24 mmol, 1.0 eq.) and compound 11 (0.11 g, 0.31 mmol, 1.5 eq.) were dissolved in DCM (5 mL) under argon and triethylamine (0.1 mL, 0.61 mmol, 3.0 eq.) was added. The reaction was stirred at room temperature overnight. The solvent was removed under reduced pressure, the residue was dissolved in EtOAc (100 mL) and washed with water (100 mL). The organic layer was separated and dried over Na₂SO₄. The solvent was evaporated and the resulting crude material was purified by flash chromatography (elution gradient: 0-10% MeOH in DCM in 20 CV) to afford the title compound as white fluffy solid (0.27 g, 67%, rf = 0.5 (10% MeOH in DCM)). MS: calculated for C₈₄H₁₃₇N₁₁O₄₁, 1957.1. Found 1959.6.

### Conjugation of Tether 1 to a siRNA strand: Monofluoro cyclooctyne (MFCO) conjugation at 5'- or 3'-end

### 5'-end MFCO conjugation

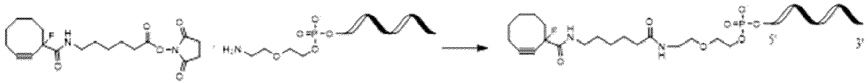

### 3'-end MFCO conjugation

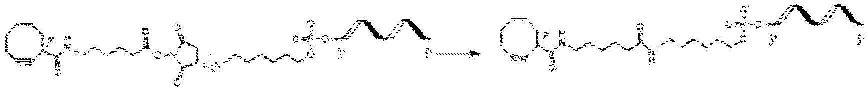

General conditions for MFCO conjugation: Amine-modified single strand was dissolved at 700 OD/mL in 50 mM carbonate/bicarbonate buffer pH 9.6/dimethyl sulfoxide (DMSO) 4:6 (v/v) and to this solution was added one molar equivalent of a 35 mM solution of MFCO-C6-NHS ester (Berry&Associates, Cat. # LK 4300) in DMF. The reaction was carried out at room temperature and after 1 h another molar equivalent of the MFCO solution was added. The reaction was allowed to proceed for an additional hour and was monitored by LC/MS. At least two molar equivalent excess of the MFCO NHS ester reagent relative to the amino modified oligonucleotide were needed to achieve quantitative consumption of the starting material. The reaction mixture was diluted 15-fold with water, filtered through a 1.2 µm filter from Sartorius and then purified by reserve phase (RP HPLC) on an Äkta Pure instrument (GE Healthcare).

Purification was performed using a XBridge C18 Prep 19 x 50 mm column from Waters. Buffer A was 100 mM TEAAc pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full length conjugated oligonucleotide were pooled, precipitated in the freezer with 3 M NaOAc, pH 5.2 and 85% ethanol and the collected pellet was dissolved in water. Samples were desalted by size exclusion chromatography and concentrated using a speed-vac concentrator to yield the conjugated oligonucleotide in an isolated yield of 40-80%.

### 5'-GalNAc-T1 conjugates

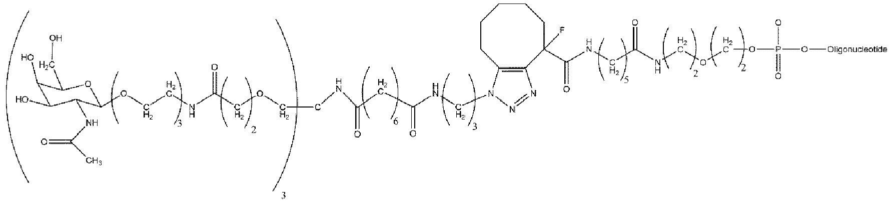

### 3'-GalNAc-T1 conjugates

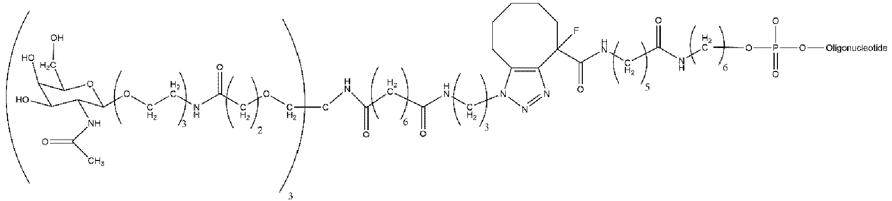

General procedure for TriGalNAc conjugation: MFCO-modified single strand was dissolved at 2000 OD/mL in water and to this solution was added one equivalent solution of compound 12 (10 mM) in DMF. The reaction was carried out at room temperature and after 3 h 0.7 molar equivalent of the compound 12 solution was added. The reaction was allowed to proceed overnight and completion was monitored by LCMS. The conjugate was diluted 15-fold in water, filtered through a 1.2 µm filter from Sartorius and then purified by RP HPLC on an Äkta Pure instrument (GE Healthcare).

RP HPLC purification was performed using a XBridge C18 Prep 19 x 50 mm column from Waters. Buffer A was 100 mM triethylammonium acetate pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleotide were pooled, precipitated in the freezer with 3 M NaOAc, pH 5.2 and 85% ethanol and the collected pellet was dissolved in water to give an oligonucleotide solution of about 1000 OD/mL. The O-acetates were removed by adding 20% aqueous ammonia. Quantitative removal of these protecting groups was verified by LC-MS.

The conjugates were desalted by size exclusion chromatography using Sephadex G25 Fine resin (GE Healthcare) on an Äkta Pure (GE Healthcare) instrument to yield the conjugated oligonucleotides in an isolated yield of 50-70%.

The following schemes further set out the routes of synthesis:

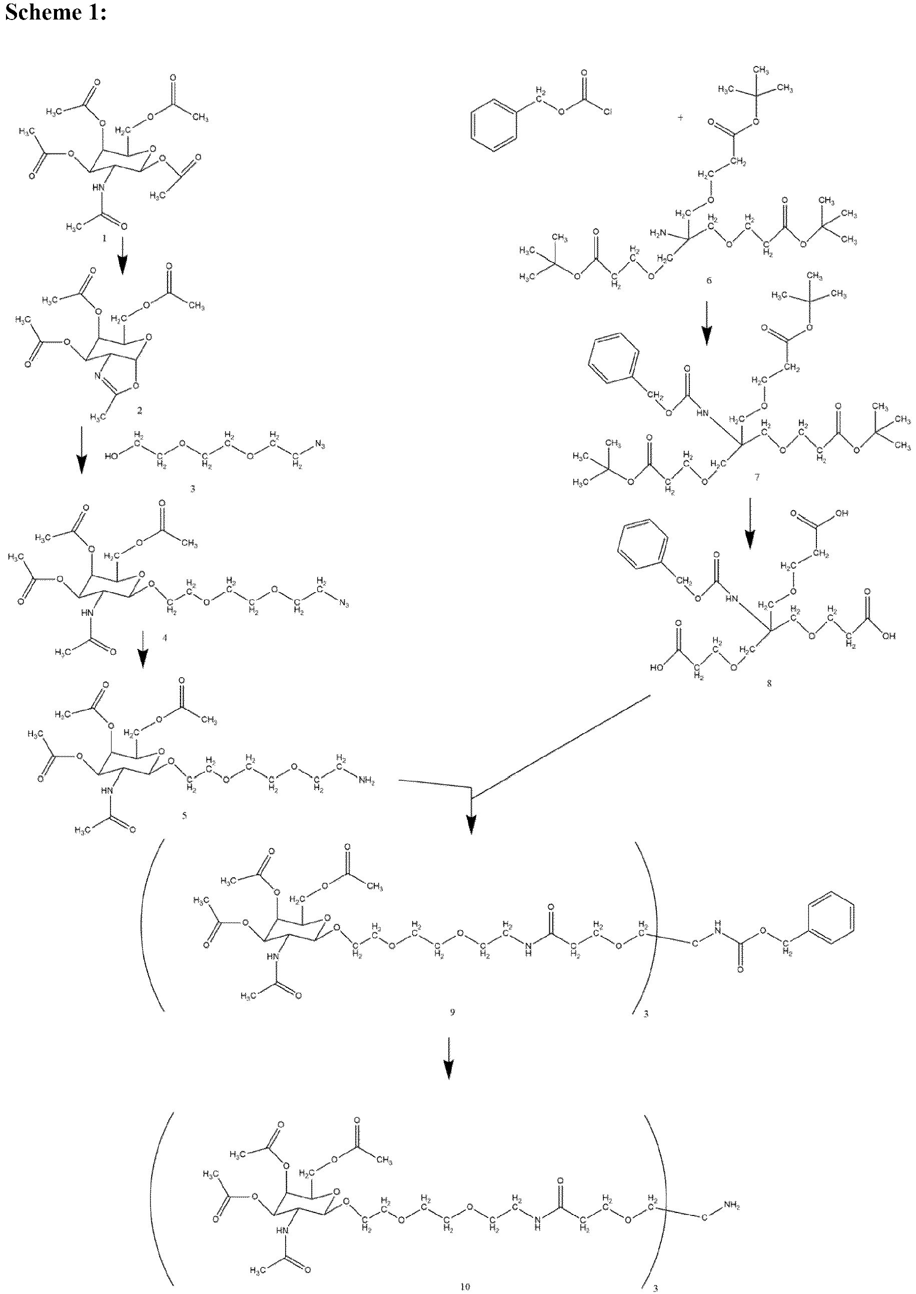

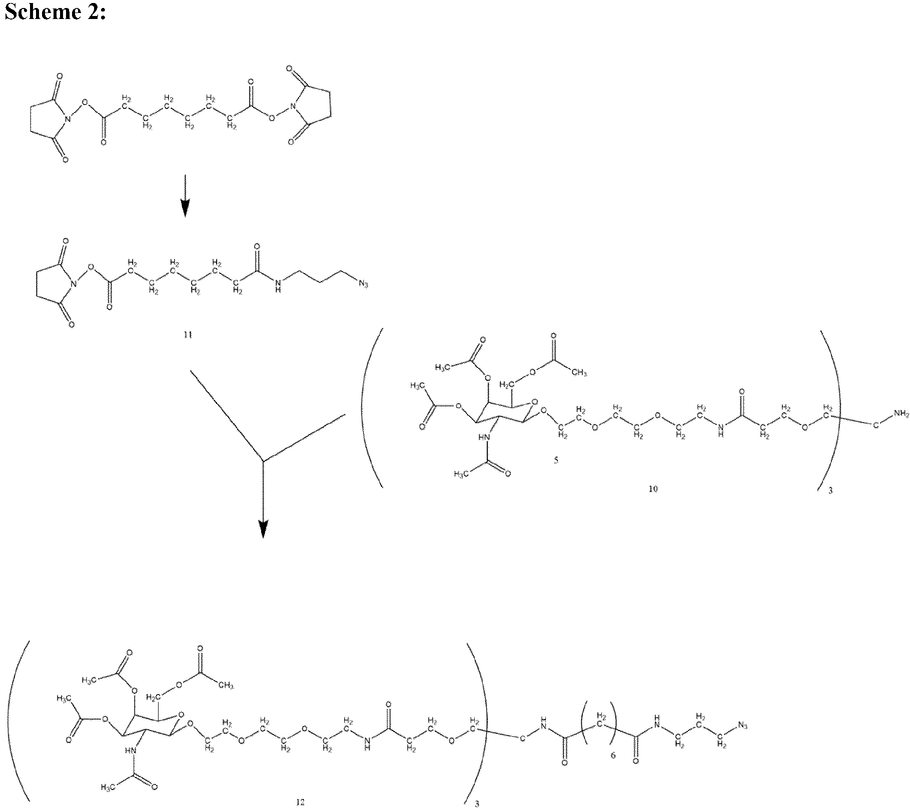

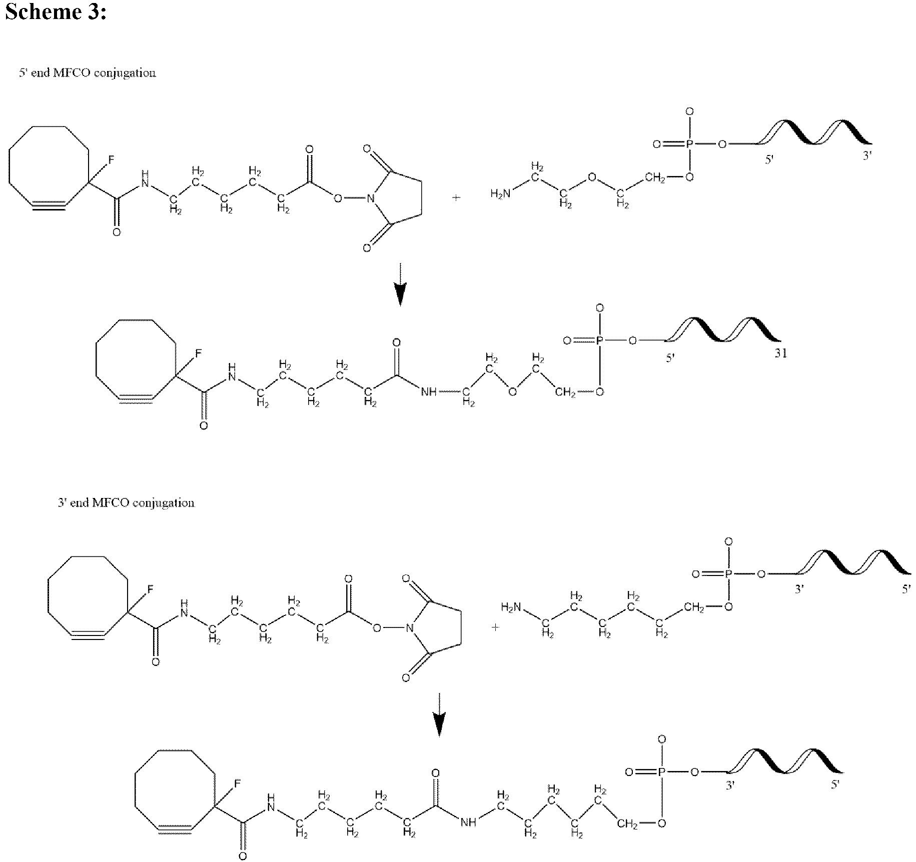

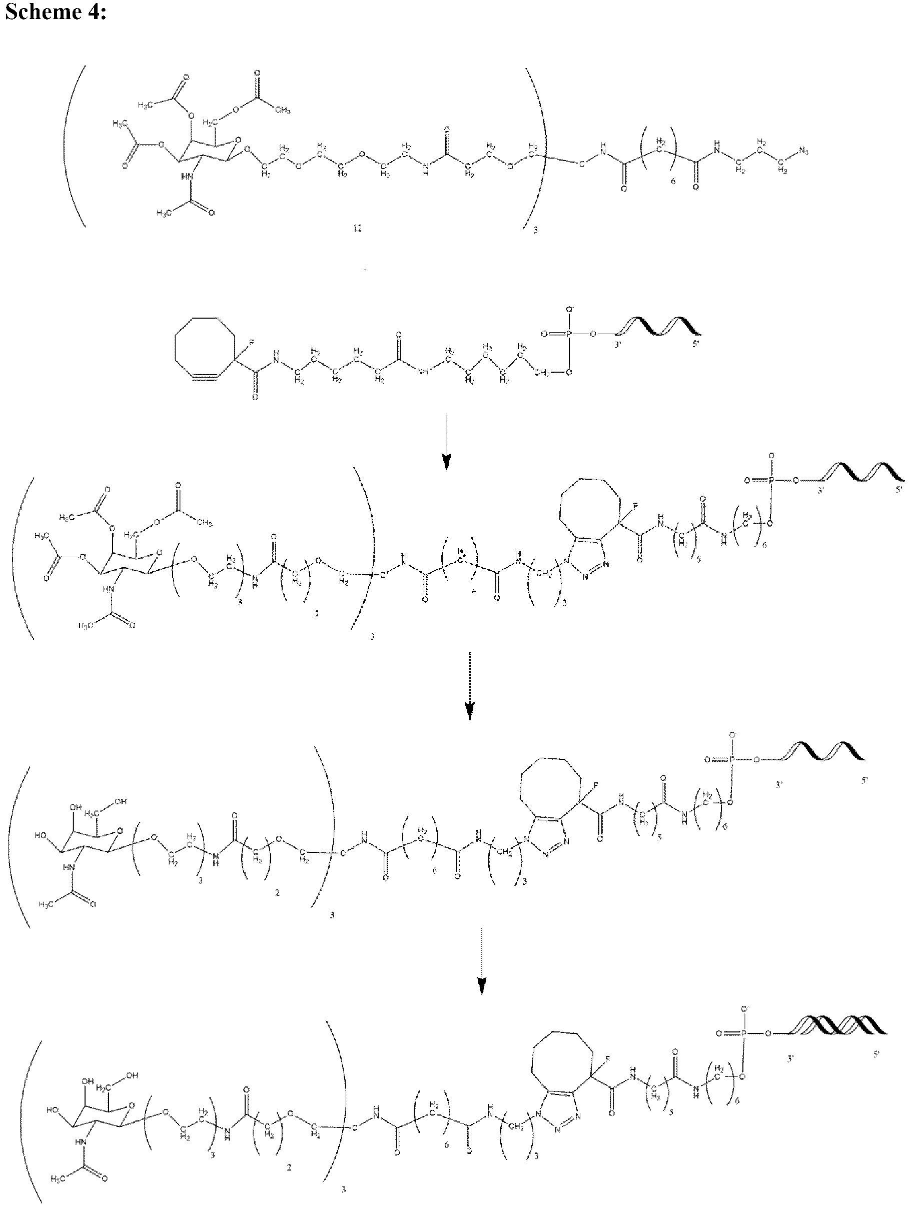

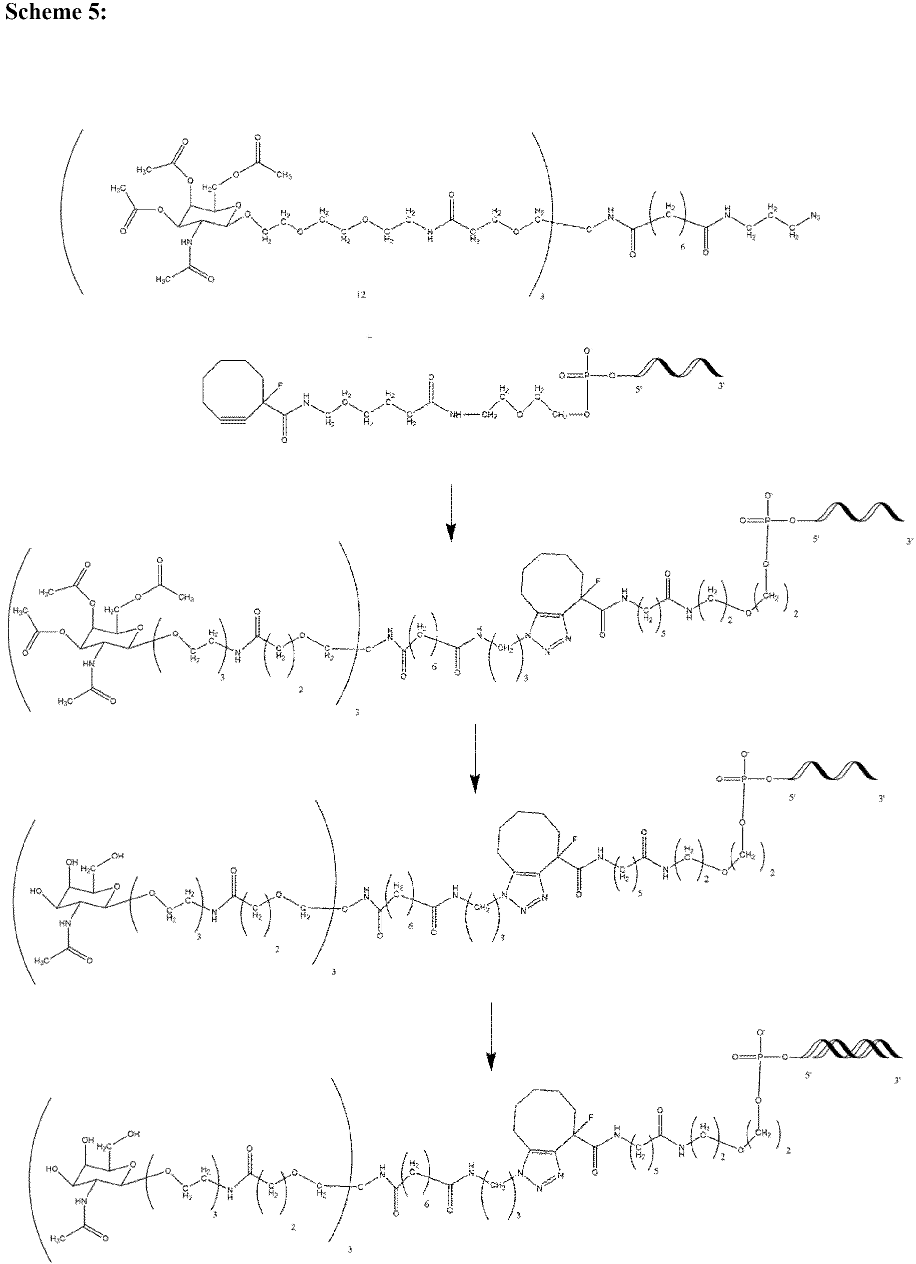

### Example 3: Duplex Annealing

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### Example 4: Synthesis of tether 2

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Sfär Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 x 100 mm) at 60 °C. The solvent system consisted of solvent A with H2O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25 °C. N₂ pressure: 35.1 psi. Filter: Corona.

₁H and ₁₃C NMR spectra were recorded at room temperature on a Varian spectrometer at 500 MHz (₁H NMR) and 125 MHz (₁₃C NMR). Chemical shifts are given in ppm referenced to the solvent residual peak (CDCl₃ - ₁H NMR: δ at 7.26 ppm and ₁₃C NMR δ at 77.2 ppm; DMSO-d6 - ₁H NMR: δ at 2.50 ppm and ₁₃C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc Tether2:

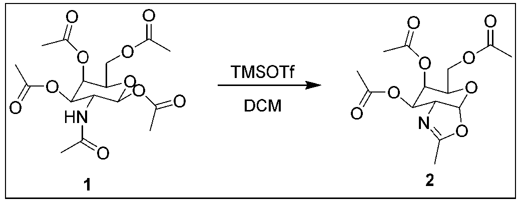

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO₃ (100 mL) and water (100 mL). The organic layer was separated, dried over Na₂SO₄, and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

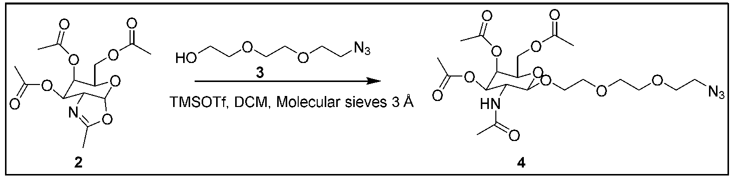

Preparation of compound 4: Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na2SO4 and the solvent was removed under reduced pressure. The crude material was purified by flash chromatography (gradient elution: 0-3% MeOH in DCM in 10 CV) to afford the title product as light-yellow oil (3.10 g, 88%, rf = 0.25 (2% MeOH in DCM)). MS: calculated for C₂₀H₃₂N₄O₁₁, 504.21. Found 505.4. 1H NMR (500 MHz, CDCl3) δ 6.21-6.14 (m, 1H), 5.30 (dd, J = 3.4, 1.1 Hz, 1H), 5.04 (dd, J = 11.2, 3.4 Hz,1H), 4.76 (d, J = 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, J = 4.2 Hz, 6H). 13C NMR (125 MHz, CDCl3) δ 170.6 (C), 170.5 (C), 170.4 (C), 170.3 (C), 102.1 (CH), 71.6 (CH), 70.8 (CH), 70.6 (CH), 70.5 (CH), 70.3 (CH2), 69.7 (CH2), 68.5 (CH2), 66.6 (CH2), 61.5 (CH2), 23.1 (CH3), 20.7 (3xCH3).

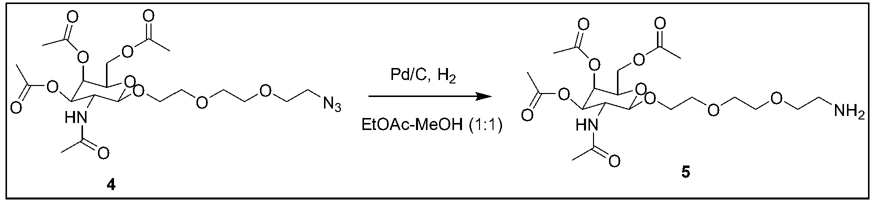

Preparation of compound 5: Compound 4 (1.00 g, 1.98 mmol, 1.0 eq.) was dissolved in a mixture of ethyl acetate (EtOAc) and MeOH (30 mL 1:1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

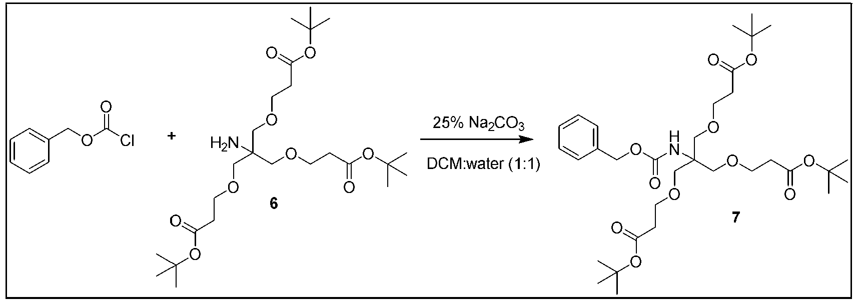

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na₂SO₄. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. 1H NMR (500 MHz, DMSO-d6) δ 7.38-7.26 (m, 5H), 4.97 (s, 2H), 3.54 (t, 6H), 3.50 (s, 6H), 2.38 (t, 6H), 1.39 (s, 27H). 13C NMR (125 MHz, DMSO-d6) δ 170.3 (3xC), 154.5 (C), 137.1 (C), 128.2 (2xCH), 127.7 (CH), 127.6 (2xCH), 79.7 (3xC), 68.4 (3xCH2), 66.8 (3xCH2), 64.9 (C), 58.7 (CH2), 35.8 (3xCH2), 27.7 (9xCH3).

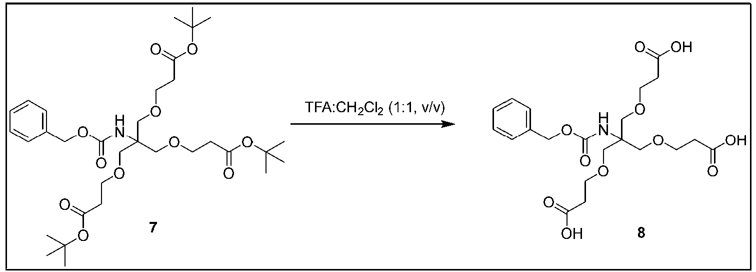

Preparation of compound 8: Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

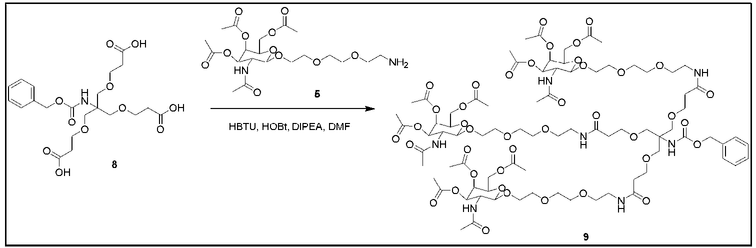

Preparation of compound 9: CbzNH-tris-COOH 8 (0.72 g, 1.49 mmol, 1.0 eq.) and GalNAc-PEG3-NH2 5 (3.56 g, 7.44 mmol, 5.0 eq.) were dissolved in N,N-dimethylformamide (DMF) (25 mL). Then N,N,N',N'-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and N,N-diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO3 (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. 1H NMR (500 MHz, DMSO-d6) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). 13C NMR (125 MHz, DMSO-d6) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH2), 69.0 (CH2), 68.2 (CH2), 67.2 (CH2), 66.7 (CH2), 61.4 (CH2), 22.6 (CH2), 22.4 (3xCH3), 20.7 (9xCH3).

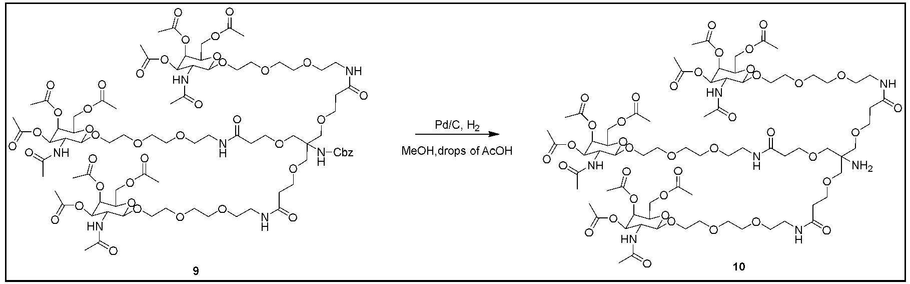

Preparation of compound 10: Triantennary GalNAc compound 9 (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated, and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

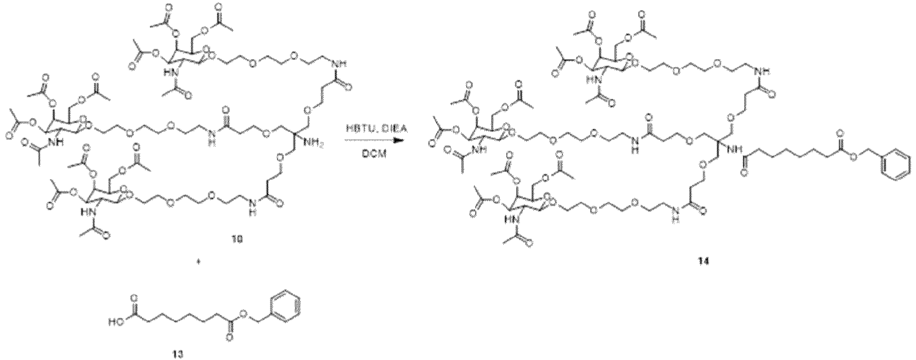

Preparation of compound 14: Triantennary GalNAc compound 10 (0.45 g, 0.26 mmol, 1.0 eq.), HBTU (0.19 g, 0.53 mmol, 2.0 eq.) and DIPEA (0.23 mL, 1.3 mmol, 5.0 eq.) were dissolved in DCM (10 mL) under argon. To this mixture, it was added dropwise a solution of compound 13 (0.14 g, 0.53 mmol, 2.0 eq.) in DCM (5 mL). The reaction was stirred at room temperature overnight. The solvent was removed, and the residue was dissolved in EtOAc (50 mL), washed with water (50 mL) and dried over Na₂SO₄. The solvent was evaporated, and the crude material was purified by flash chromatography (gradient elution: 0-5% MeOH in DCM in 20 CV). The product was obtained as white fluffy solid (0.25 g, 48%, rf = 0.4 (10% MeOH in DCM)). MS: calculated for C₈₈H₁₃₇N₇O₄₂, 1965.1. Found 1965.6.

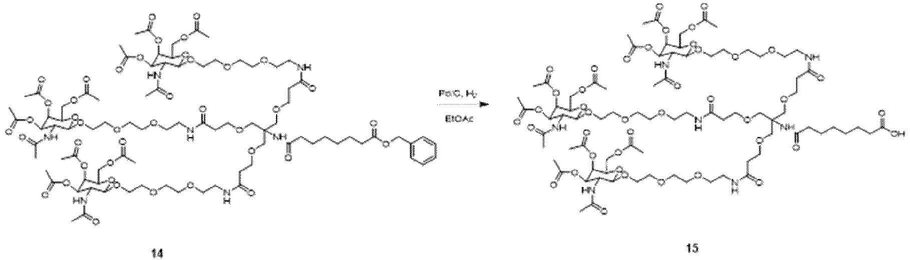

Preparation of TriGalNAc (15): Triantennary GalNAc compound 14 (0.31 g, 0.15 mmol, 1.0 eq.) was dissolved in EtOAc (15 mL) and Pd/C (40 mg) was added. The reaction mixture was degassed by using vacuum/argon cycles (3x) and hydrogenated under balloon pressure overnight. The completion of the reaction was monitored by mass spectrometry and the resulting mixture was filtered through a thin pad of celite. The solvent was removed under reduced pressure and the resulting residue was dried under high vacuum overnight. The residue was used for conjugations to oligonucleosides without further purification (0.28 g, quantitative yield). MS: calculated for C₈₁H₁₃₁N₇O₄₂, 1874.9. Found 1875.3.

Conjugation of Tether 2 to a siRNA strand: TriGalNAc tether 2 (GalNAc-T2) conjugation at 5'-end or 3'-end

### 5'-GalNAc-T2 conjugates

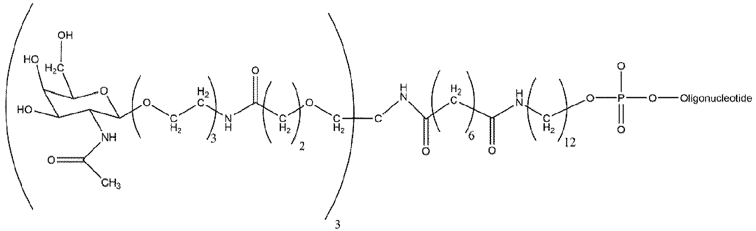

### 3'-GalNAc-T2 conjugates

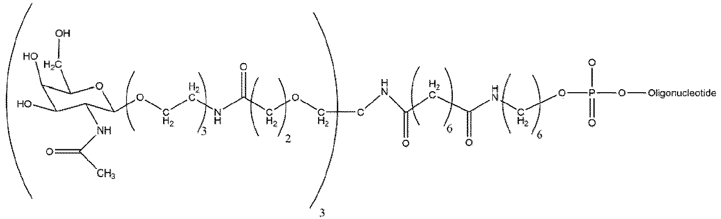

Preparation of TriGalNAc tether 2 NHS ester: To a solution of carboxylic acid tether 2 (compound 15, 227 mg, 121 µmol) in DMF (2.1 mL), N-hydroxysuccinimide (NHS) (15.3 mg, 133 µmol) and N,N'-diisopropylcarbodiimide (DIC) (19.7 µL, 127 µmol) were added. The solution was stirred at room temperature for 18 h and used without purification for the subsequent conjugation reactions.

General procedure for triGalNAc tether 2 conjugation: Amine-modified single strand was dissolved at 700 OD/mL in 50 mM carbonate/bicarbonate buffer pH 9.6/DMSO 4:6 (v/v) and to this solution was added one molar equivalent of Tether 2 NHS ester (57 mM) solution in DMF. The reaction was carried out at room temperature and after 1 h another molar equivalent of the NHS ester solution was added. The reaction was allowed to proceed for one more hour and reaction progress was monitored by LCMS. At least two molar equivalent excess of the NHS ester reagent relative to the amino modified oligonucleoside were needed to achieve quantitative consumption of the starting material. The reaction mixture was diluted 15-fold with water, filtered once through 1.2 µm filter from Sartorius and then purified by reserve phase (RP HPLC) on an Äkta Pure (GE Healthcare) instrument.

The purification was performed using a XBridge C18 Prep 19 x 50 mm column from Waters. Buffer A was 100 mM TEAA pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleosides were pooled together, precipitated in the freezer with 3 M NaOAc, pH 5.2 and 85% ethanol and then dissolved at 1000 OD/mL in water. The O-acetates were removed with 20% ammonium hydroxide in water until completion (monitored by LC-MS).

The conjugates were desalted by size exclusion chromatography using Sephadex G25 Fine resin (GE Healthcare) on an Äkta Pure (GE Healthcare) instrument to yield the conjugated oligonucleotides in an isolated yield of 60-80%.

The conjugates were characterized by HPLC-MS analysis with a 2.1 x 50 mm XBridge C18 column (Waters) on a Dionex Ultimate 3000 (Thermo Fisher Scientific) HPLC system equipped with a Compact ESI-Qq-TOF mass spectrometer (Bruker Daltonics). Buffer A was 16.3 mM triethylamine, 100 mM HFIP in 1% MeOH in H2O and buffer B contained 95% MeOH in buffer A. A flow rate of 250 µL/min and a temperature of 60°C were employed. UV traces at 260 and 280 nm were recorded. A gradient of 1-100% B within 31 min was employed.

The following schemes further set out the routes of synthesis:

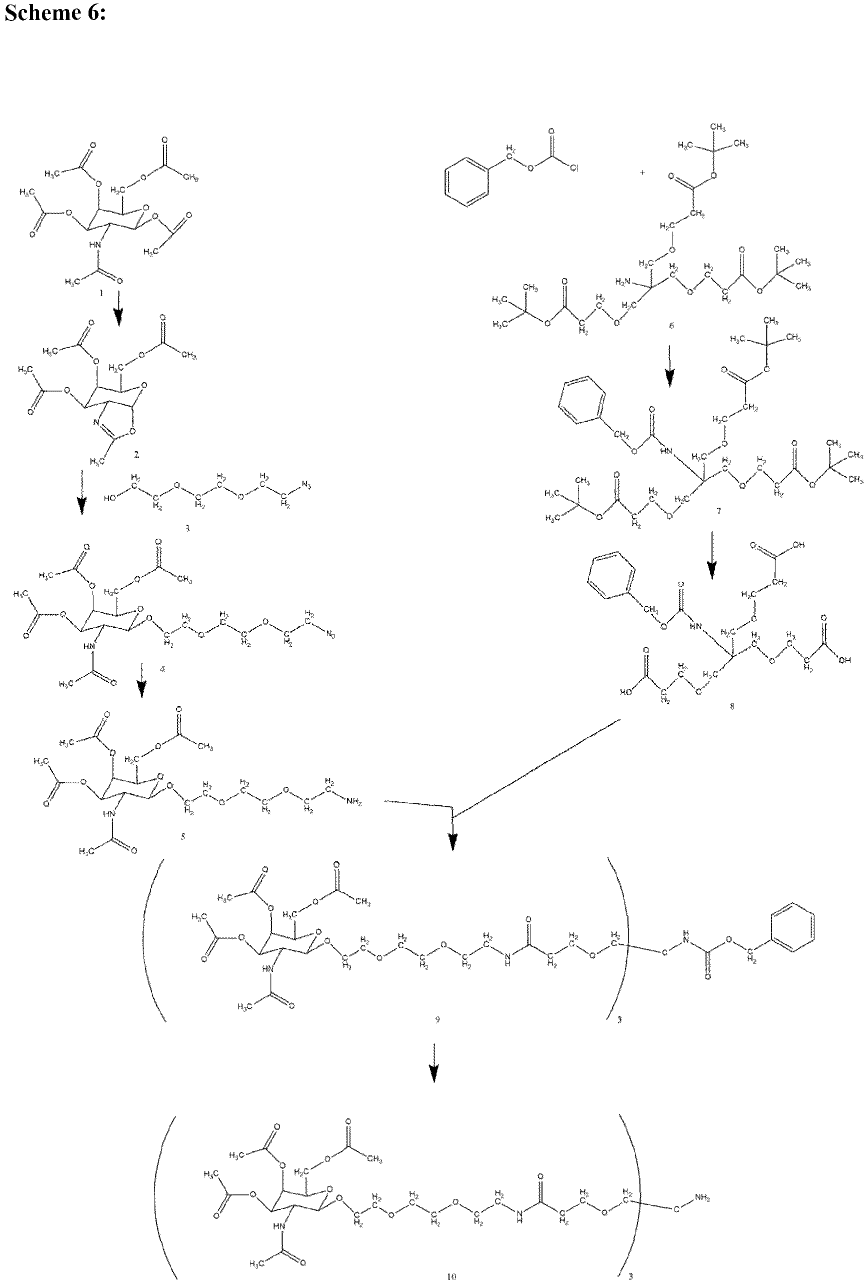

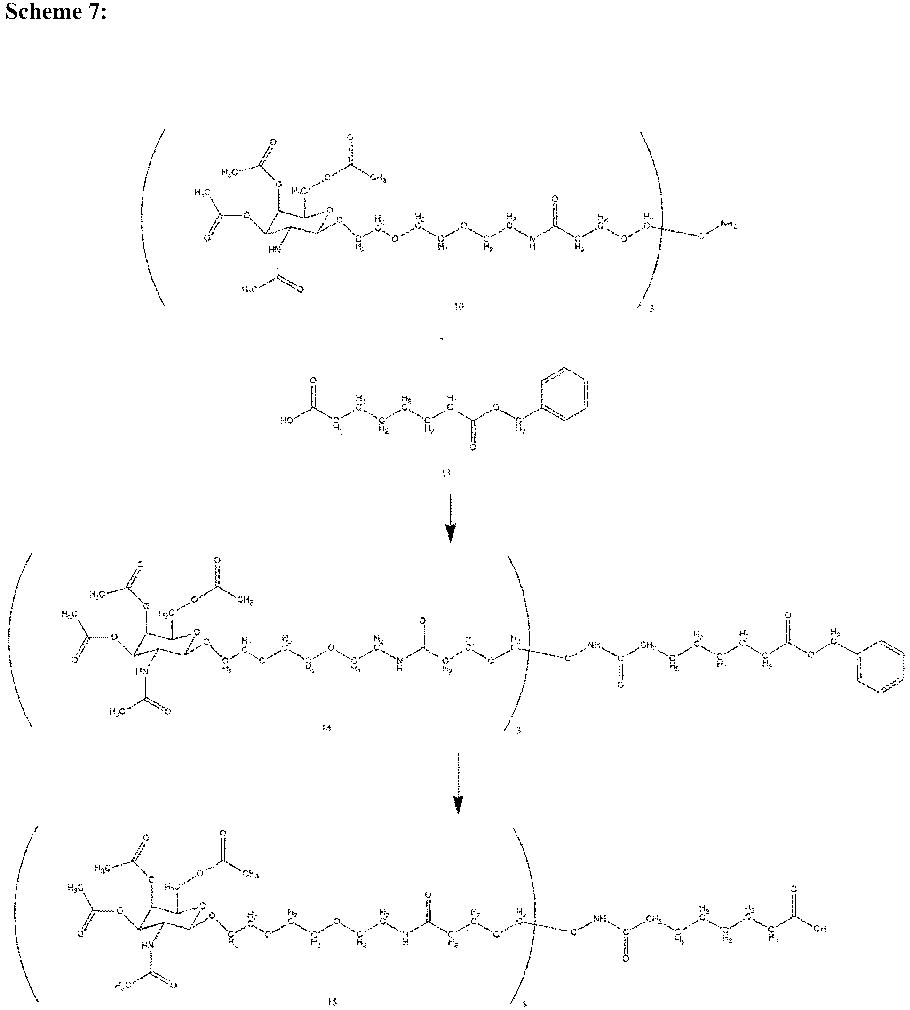

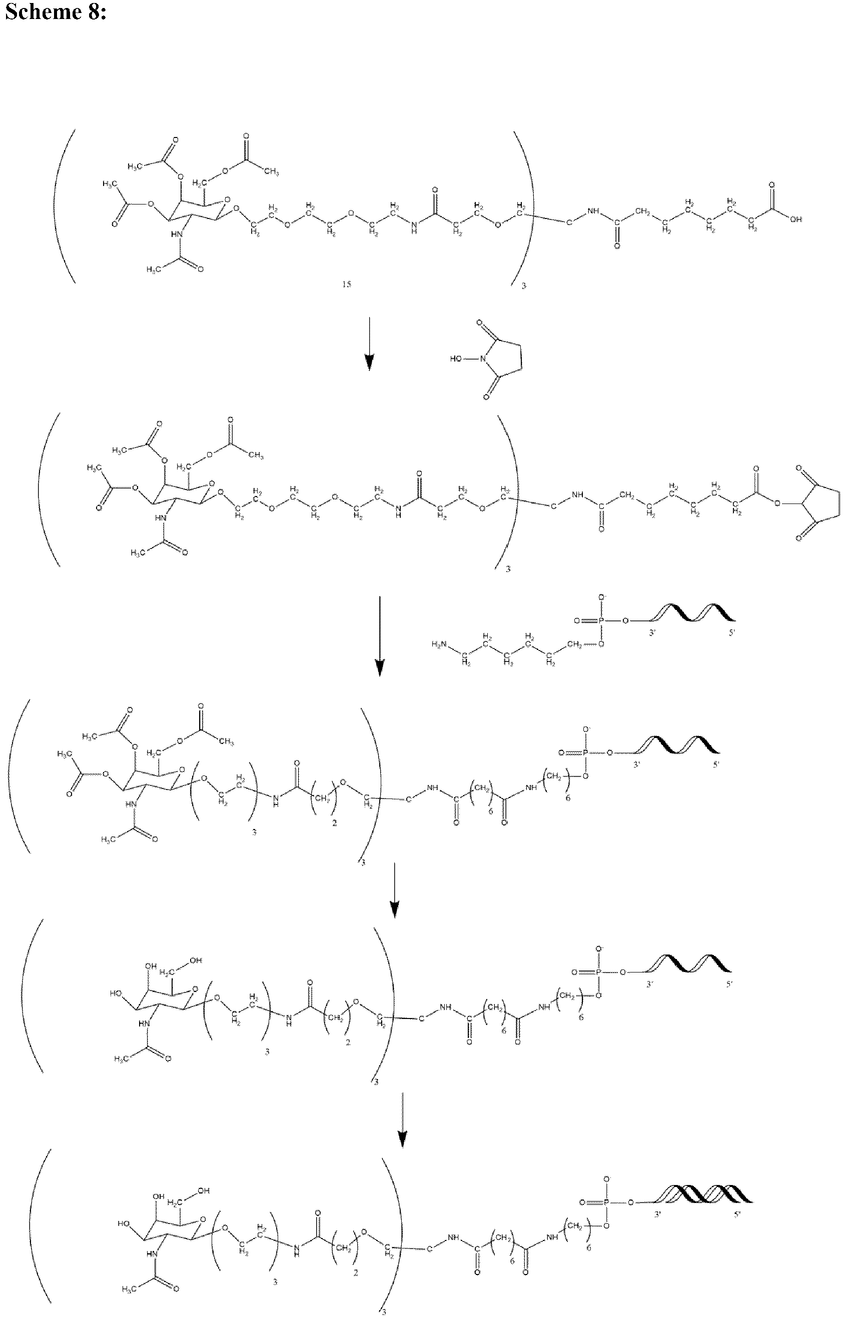

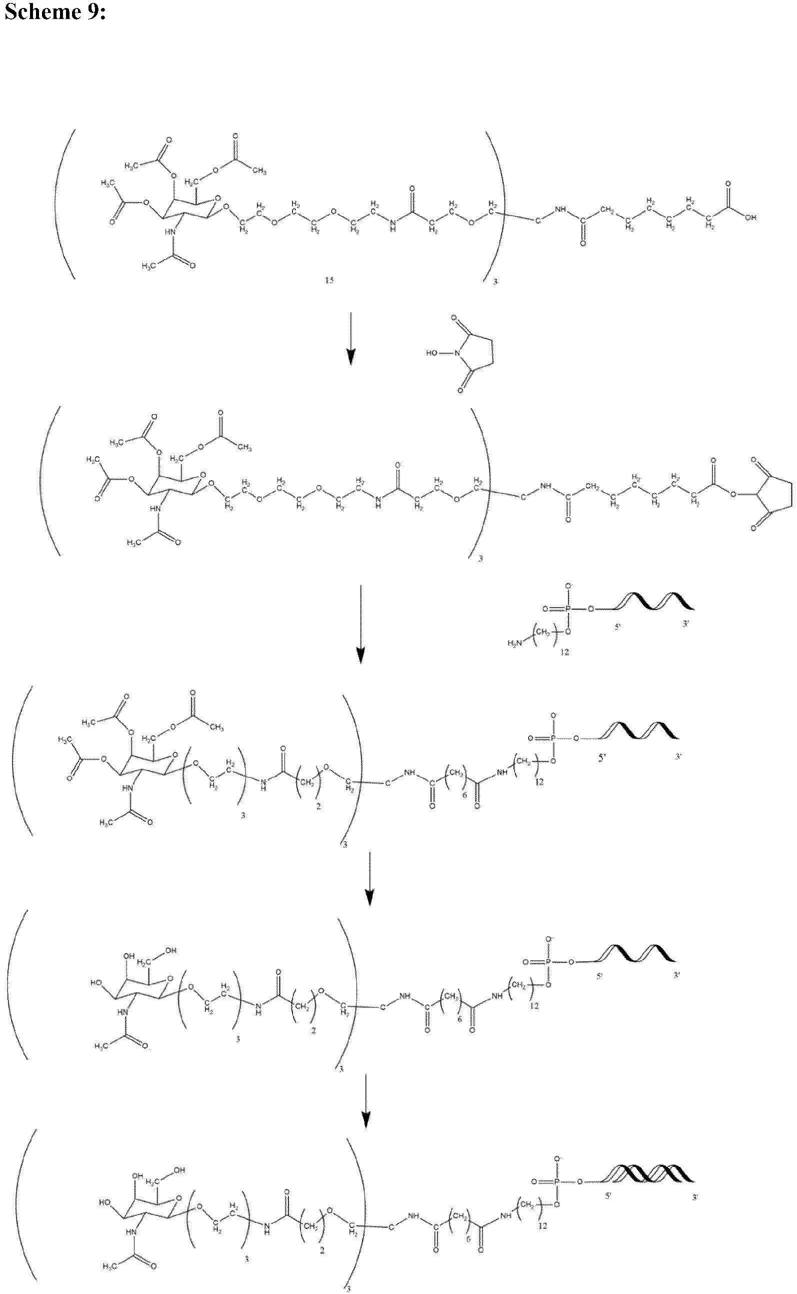

### Example 5: Duplex Annealing

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### Example 6: Alternative synthesis route for the conjugate building block TriGalNAc _Tether2

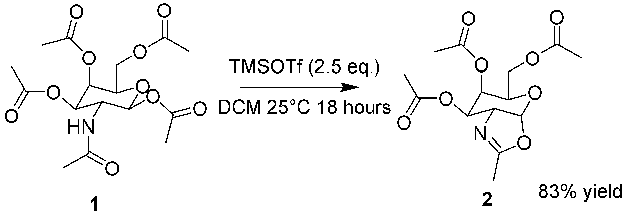

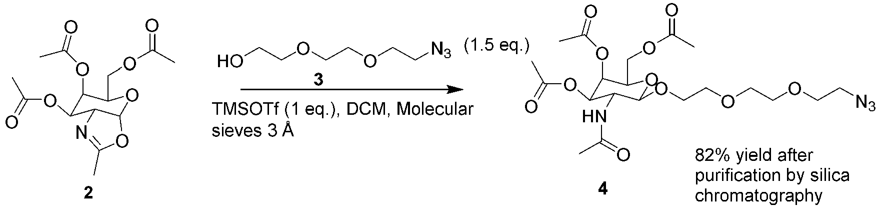

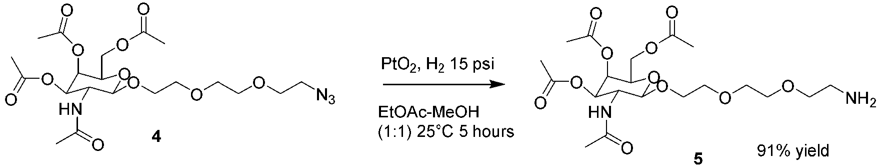

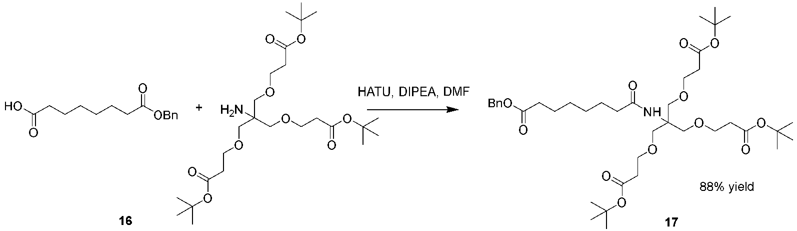

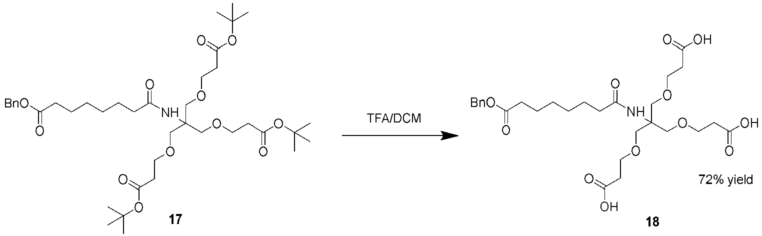

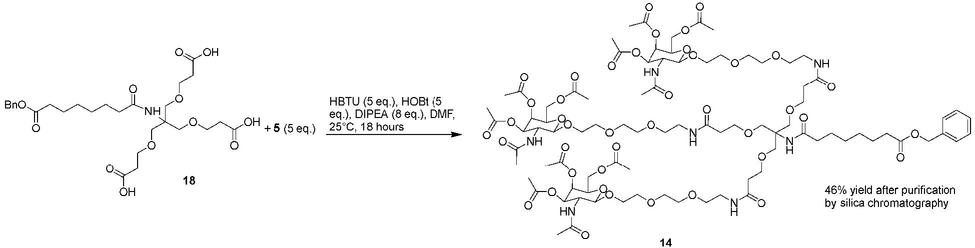

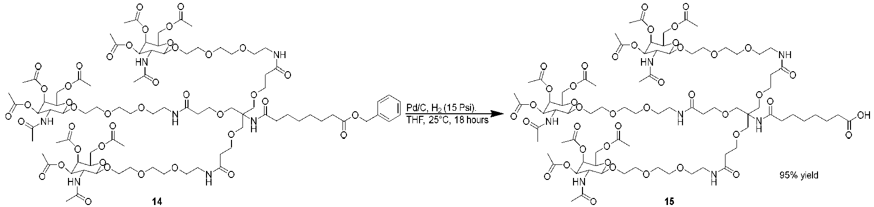

### Conjugation of Tether 2 to a siRNA strand: TriGalNAc tether 2 (GalNAc-T2) conjugation at 5'-end or 3'-end

### Conjugation conditions

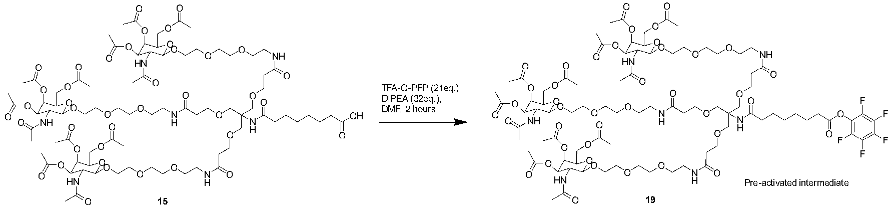

Pre-activation: To a solution of compound 15 (16 umol, 4 eq.) in DMF (160 µL) was added TFA-O-PFP (15 µl, 21 eq.) followed by DIPEA (23 µl, 32 eq.) at 25°C. The tube was shaken for 2 h at 25°C. The reaction was quenched with H2O (10 µL).

Coupling: The resulting mixture was diluted with DMF (400 µl), followed by addition of oligo-amine solution (4.0 µmol in 10 x PBS, pH 7.4, 500 µL; final oligo concentration in organic and aqueous solution: 4 µmol/ml = 4 mM). The tube was shaken at 25°C for 16 h and the reaction was analysed by LCMS. The resulting mixture was treated with 28% NH4OH (4.5 ml) and shaken for 2 h at 25°C. The mixture was analysed by LCMS, concentrated, and purified by IP-RP HPLC to produce the oligonucleotides conjugated to tether 2 GalNAc.

### 5'-GalNAc-T2 conjugates

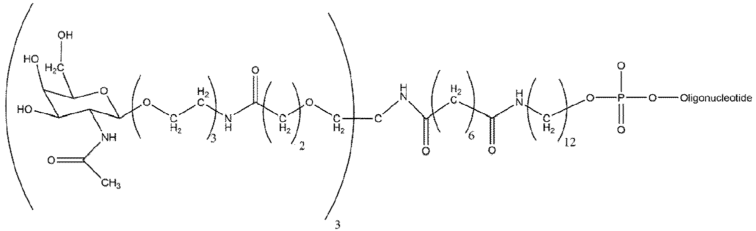

### 3'-GalNAc-T2 conjugates

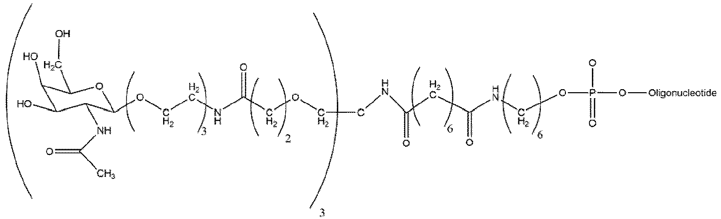

### Example 7: Solid phase synthesis method: scale ≤1µmol

Syntheses of siRNA sense and antisense strands were performed on a MerMade192X synthesiser with commercially available solid supports made of controlled pore glass with universal linker (Universal CPG, with a loading of 40 µmol/g; LGC Biosearch or Glen Research).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H₂O (DNAchem) was used as oxidizing reagent. Thiolation for phosphorothioate linkages was performed with 0.2 M PADS (TCI) in acetonitrile/pyridine 1:1 v/v. 5-Ethyl thiotetrazole (ETT), 0.25M mM in acetonitrile was used as activator solution.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

At each cycle, the DMT was removed by deblock solution, 3% TCA in DCM (DNAchem).

The coupling time was 180 seconds. The oxidizer contact time was set to 80 seconds and thiolation time was 2*100 seconds.

At the end of the synthesis, the oligonucleotides were cleaved from the solid support using a NH₄OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by ultracentrifugation using Amicon Ultra-2 Centrifugal Filter Unit; PBS buffer (10x, Teknova, pH 7.4, Sterile) or by EtOH precipitation from 1M sodium acetate.

The single strands identity were assessed by MS ESI- and then, were annealed in water to form the final duplex siRNA and duplex purity were assessed by size exclusion chromatography.

### Example 8: Solid phase synthesis method: scale ≥5 µmol

Syntheses of siRNA sense and antisense strands were performed on a MerMade12 synthesiser with commercially available solid supports made of controlled pore glass with universal linker (Universal CPG, with a loading of 40 µmol/g; LGC Biosearch or Glen Research) at 5 µmol scale. Sense strand destined to 3' conjugation were sytnthesised at 12 µmol on 3'-PT-Amino-Modifier C6 CPG 500 Å solid support with a loading of 86 µmol/g (LGC).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H₂O (DNAchem) was used as oxidizing reagent. Thiolation for phosphorothioate linkages was performed with 0.2 M PADS (TCI) in acetonitrile/pyridine 1:1 v/v. 5-Ethyl thiotetrazole (ETT), 0.25M mM in acetonitrile was used as activator solution.

At each cycle, the DMT was removed by deblock solution, 3% TCA in DCM (DNAchem).

For strands synthesised on universal CPG the coupling was performed with 8 eq. of amidite for 130 seconds. The oxidation time was 47 seconds, the thiolation time was 210 seconds.

For strands synthesised on 3'-PT-Amino-Modifier C6 CPG the coupling was performed with 8 eq. of amidite for 2*150 seconds. The oxidation time was 47 seconds, the thiolation time was 250 seconds

At the end of the synthesis, the oligonucleotides were cleaved from the solid support using a NH4OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by EtOH precipitation from 1M sodium acetate.

The single strand oligonucleotides were purified by IP-RP HPLC on Xbridge BEH C18 5 µm, 130 Å, 19x150 mm (Waters) column with an increasing gradient of B in A. Mobile phase A: 240 mM HFIP, 7 mM TEA and 5% methanol in water; mobile phase B: 240 mM HFIP, 7 mM TEA in methanol.

The single strands purity and identity were assessed by UPLC/MS ESI- on Xbridge BEH C18 2.5 µm, 3x50 mm (Waters) column with an increasing gradient of B in A. Mobile phase A: 100 mM HFIP, 5 mM TEA in water; mobile phase B: 20% mobile phase A: 80% Acetonitrile (v/v).

Sense strands were conjugated as per protocol provided in any of Examples 2, 4, 6.

Sense and Antisense strands were then annealed in water to form the final duplex siRNA and duplex purity were assessed by size exclusion chromatography.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### Example 9: Dose-response for Inhibition of SLC25A5 Expression in Human Huh7 Cells

Huh7 cells (human hepatocyte-derived cell line, obtained from JCRB Cell Bank) were maintained in Dulbecco's Modified Eagle Medium (DMEM) supplemented with 10% FBS and 1% non-essential amino acids at 37°C, 5% CO₂, 95% humidity. Cells were transfected with siRNA duplexes targeting either SLC25A5 mRNA or a negative control siRNA (siRNA-control; sense strand 5'- GCCTGTACCAAGGCTTTAA-3' (SEQ ID NO:1383), antisense strand 5'-TTAAAGCCTTGGTACAGGC-3' (SEQ ID NO:1382)) in a 6-point, log dose response curve to give final in assay concentrations of 3nM to 0.03pM. Transfection was carried out by diluting Lipofectamine RNAiMAX (ThermoFisher) in Opti-MEM (ThermoFisher) medium at a ratio of 48.5:1.5. This solution was added to an equal volume of siRNA, diluted to the required concentration in phosphate buffered saline. The lipofectamine RNAiMAX and siRNA mixture was incubated at room temperature for 15 minutes before 20 µL was added to wells of a 96 well plate. Huh7 cells were dissociated from flasks using trypsin and resuspended at a density of 200,000 cells/mL. 100 µL of Huh7 cell suspension was added to each well of the siRNA-containing 96-well plates. Cells were incubated for 24 hours at 37°C, 5% CO₂,95% humidity. Each siRNA was tested in triplicate wells and on two separate days for a total of six replicates.

Intracellular RNA was isolated using an RNeasy kit (Qiagen) according to the manufacturer's instructions. cDNA synthesis was performed using a FastKing RT kit, with gDNase (Tiangen). Target cDNA was the quantified by qPCR on an ABI Prism 7900HT or ABI QuantStudio 7 with primers specific for human SLC25A5 (forward: ACTGACATCATGTACACAGGCAC (SEQ ID NO:1384), reverse: ACCCATGCCTCTGAGAACATT (SEQ ID NO:1385)) and human GAPDH (forward: GAAGGTGAAGGTCGGAGTC (SEQ ID NO:1386), reverse: GAAGATGGTGATGGGATTTC (SEQ ID NO:1387)) using a SensiFAST SYBR Hi-ROX kit (Meridian).

qPCR was performed in duplicate on cDNA derived from each well and the mean Ct calculated. Relative SLC25A5 expression was calculated from mean Ct values using the comparative Ct (ΔΔCt) method, 229ormalized to GAPDH and relative to untreated cells. Maximum percent inhibition of SLC25A5 expression and pEC50 values (-log₁₀ of the EC50) were calculated using a four parameter (variable slope) model using NumPy (Python). Results are shown in Table 6. Sequences of RNAi molecules are depicted in the relevant Tables herein.

**Table 6 - Results of dose-response experiments for inhibition of SLC25A5 mRNA expression in human Huh7 cells. Observed and fitted pEC50s are reported with observed minimum and maximum relative expression values (relative to medium control) for two experimental replicates (repeated on separate days with three repeats per day).**

| ETXM ID | Minimum r1 | Maximum r1 | pEC50 r1 | Minimum r2 | Maximum r2 | pEC50 r2 |
|---|---|---|---|---|---|---|
| ETX-M00001351 | 0.095738517 | 1.012076322 | 9.74 | 0.16593971 | 1.135465148 | 9.46 |
| ETX-M00001352 | 0.068977171 | 0.832613609 | 10.36 | 0.107726125 | 1.031889622 | 10.19 |
| ETX-M00001353 | 0.076601856 | 0.942804039 | 10.09 | 0.105081317 | 0.866486243 | 9.79 |
| ETX-M00001354 | 0.04160111 | 0.352837401 | 12.42 | 0.054758242 | 0.528360743 | 10.99 |
| ETX-M00001355 | 0.286834824 | 1.105769257 | 9.36 | 0.422953861 | 1.267175414 | 9.08 |
| ETX-M00001356 | 0.062109246 | 0.683699132 | 10.89 | 0.082971501 | 0.990778092 | 10.6 |
| ETX-M00001357 | 0.260371194 | 1.047615584 | 9.05 | 0.330270419 | 1.227520719 | 9.11 |
| ETX-M00001358 | 0.126012018 | 1.005286574 | 10.49 | 0.097256411 | 0.933587755 | 10.17 |
| ETX-M00001359 | 0.086718222 | 0.886522538 | 10.16 | 0.143556553 | 1.077345717 | 9.88 |
| ETX-M00001360 | 0.197501054 | 1.021858054 | 9.44 | 0.444312718 | 1.286204781 | 9.33 |
| ETX-M00001361 | 0.155005218 | 1.010333551 | 9.41 | 0.337064444 | 1.310022413 | 8.88 |
| ETX-M00001362 | 0.602601339 | 1.065764399 | 9.79 | 0.782613101 | 1.537826561 | 9.69 |
| ETX-M00001363 | 0.267572294 | 1.072128134 | 9.35 | 0.294825935 | 0.993647402 | 9.12 |
| ETX-M00001364 | 0.06269171 | 0.884419453 | 10.17 | 0.099587992 | 0.789694685 | 10.08 |
| ETX-M00001365 | 0.162554032 | 1.032331404 | 9.49 | 0.230453664 | 0.874107567 | 9.12 |
| ETX-M00001366 | 0.6460043 | 1.101955001 | 8.88 | 0.560386396 | 0.86269628 | 6.93 |
| ETX-M00001367 | 0.071999043 | 0.945858821 | 9.64 | 0.153954817 | 1.043108396 | 9.61 |
| ETX-M00001368 | 0.308068257 | 0.9872225 | 9.47 | 0.405284264 | 1.139893375 | 9.4 |
| ETX-M00001369 | 0.156575267 | 0.90043545 | 9.83 | 0.225683816 | 1.020341168 | 9.72 |
| ETX-M00001370 | 0.470403615 | 1.014232608 | 9.5 | 0.563112577 | 1.122335299 | 9.31 |
| ETX-M00001371 | 0.098907568 | 0.787402535 | 10.35 | 0.153074794 | 0.798991053 | 10.32 |
| ETX-M00001372 | 0.232940452 | 20.51401011 | 9.15 | 0.29985096 | 0.937317894 | 9.23 |
| ETX-M00001373 | 0.672653114 | 1.084781814 | 8.98 | 0.709952949 | 1.044549135 | 6.47 |
| ETX-M00001374 | 0.129831319 | 0.858799681 | 10.42 | 0.184951482 | 0.991071046 | 10.47 |
| ETX-M00001375 | 0.638666505 | 1.178135966 | 9.09 | 0.833987501 | 1.394790706 | 8.96 |
| ETX-M00001376 | 0.173010986 | 1.014074132 | 9.55 | 0.271870984 | 1.159800931 | 9.44 |
| ETX-M00001377 | 0.029866025 | 0.461845856 | 11.42 | 0.082112622 | 0.405412255 | 11.37 |
| ETX-M00001378 | 0.033539757 | 0.391435718 | 11.38 | 0.074342373 | 0.394622732 | 11.41 |
| ETX-M00001379 | 0.163436084 | 0.971055681 | 9.56 | 0.205951766 | 0.969382997 | 9.48 |
| ETX-M00001380 | 0.670568552 | 1.034799511 | 9.0 | 0.703395629 | 1.344662124 | 8.94 |
| ETX-M00001381 | 0.110474151 | 0.94017161 | 9.78 | 0.166686735 | 0.848694146 | 9.75 |
| ETX-M00001382 | 0.122417733 | 1.010395959 | 9.73 | 0.270001915 | 1.09501631 | 9.56 |
| ETX-M00001383 | 0.221764768 | 1.090018663 | 9.27 | 0.327849656 | 1.16764907 | 9.0 |
| ETX-M00001384 | 0.287275636 | 1.053461449 | 9.3 | 0.407959766 | 1.098005644 | 9.4 |
| ETX-M00001385 | 0.216868684 | 1.02420622 | 9.14 | 0.279940004 | 1.109063691 | 9.31 |
| ETX-M00001386 | 0.148103725 | 1.112208683 | 10.22 | 0.201395101 | 1.026732832 | 9.9 |
| ETX-M00001387 | 0.452045611 | 1.214447835 | 9.11 | 0.440417532 | 1.065952197 | 8.99 |
| ETX-M00001388 | 0.800140403 | 1.158463785 | 9.01 | 0.76715024 | 0.967428082 | 8.9 |
| ETX-M00001389 | 0.800162618 | 1.419723412 | 0.0 | 0.773333933 | 1.392230032 | 9.5 |
| ETX-M00001390 | 0.78279321 | 1.267998923 | 9.0 | 0.678338137 | 1.053988924 | 9.01 |
| ETX-M00001391 | 0.205170765 | 1.226167435 | 9.59 | 0.333949624 | 1.105578037 | 9.72 |
| ETX-M00001392 | 0.158295637 | 1.087596385 | 9.47 | 0.257646919 | 0.954021904 | 9.08 |
| ETX-M00001393 | 0.194066175 | 3.342535483 | 9.04 | 0.313998274 | 0.971069253 | 9.22 |
| ETX-M00001394 | 0.180283743 | 1.118030944 | 9.28 | 0.260329094 | 1.082753479 | 9.18 |
| ETX-M00001395 | 0.309352861 | 1.137365201 | 9.28 | 0.52067815 | 1.193534411 | 9.11 |
| ETX-M00001396 | 0.40019968 | 1.13373136 | 9.49 | 0.563866817 | 1.334643135 | 8.99 |
| ETX-M00001397 | 0.073086557 | 0.940567178 | 10.12 | 0.146815712 | 1.080179879 | 9.62 |
| ETX-M00001398 | 0.496678766 | 1.275410449 | 9.11 | 0.569827975 | 1.224383752 | 9.0 |
| ETX-M00001399 | 0.646326737 | 1.261626778 | 8.25 | 0.783692857 | 1.127135396 | 9.37 |
| ETX-M00001400 | 0.28591739 | 1.049050528 | 10.22 | 0.455156391 | 1.119328067 | 9.26 |
| ETX-M00001401 | 0.335759007 | 1.305561354 | 9.14 | 0.548618029 | 1.073617382 | 8.98 |
| ETX-M00001402 | 0.660927117 | 1.250923252 | -0.32 | 0.811938783 | 1.135108226 | ? |
| ETX-M00001403 | 0.272226606 | 1.247723091 | 9.26 | 0.332815624 | 1.063614207 | 9.1 |
| ETX-M00001404 | 0.94795512 | 1.325845624 | 8.4 | 0.882313118 | 1.159683253 | ? |
| ETX-M00001405 | 0.216376035 | 1.081756406 | 9.38 | 0.353142531 | 1.074248491 | 9.08 |
| ETX-M00001406 | 0.425361623 | 1.388509882 | 9.03 | 0.520891691 | 1.290040521 | 9.0 |
| ETX-M00001407 | 0.360201247 | 1.028556712 | 9.36 | 0.530895788 | 1.15606022 | 8.54 |
| ETX-M00001408 | 0.790249405 | 1.164609515 | 9.04 | 0.822216806 | 1.000182763 | 8.25 |
| ETX-M00001409 | 0.384042177 | 1.109734635 | 9.07 | 0.588229001 | 0.984025099 | 8.11 |
| ETX-M00001410 | 0.458907902 | 1.110918523 | 8.99 | 0.661359549 | 1.203285258 | 8.99 |
| ETX-M00001411 | 0.75868181 | 1.269040526 | 8.79 | 0.810606369 | 1.180662648 | 9.95 |
| ETX-M00001412 | 0.15105394 | 1.235349129 | 9.46 | 0.260396517 | 0.975273705 | 8.96 |
| ETX-M00001413 | 0.160985877 | 1.678177568 | 9.61 | 0.26980012 | 0.961196934 | 8.94 |
| ETX-M00001414 | 0.768241259 | 1.170215924 | ? | 0.715623854 | 1.158138413 | 7.59 |
| ETX-M00001415 | 0.158415985 | 0.863652199 | 9.01 | 0.327047507 | 1.082230484 | 9.23 |
| ETX-M00001416 | 0.501585231 | 0.925841695 | 8.16 | 0.696435893 | 1.067735112 | 8.99 |
| ETX-M00001417 | 0.079029021 | 0.804039145 | 10.19 | 0.129634235 | 0.972886998 | 9.97 |
| ETX-M00001418 | 0.215872805 | 0.953565365 | 9.4 | 0.297826739 | 0.997706738 | 9.21 |
| ETX-M00001419 | 0.934639334 | 1.346565751 | 0.25 | 0.93139409 | 1.274261754 | 0.0 |
| ETX-M00001420 | 0.612369112 | 1.827760146 | 9.14 | 0.657453694 | 1.36190322 | 8.99 |
| ETX-M00001421 | 0.091032944 | 0.929188108 | 9.92 | 0.13364019 | 1.220179968 | 9.78 |
| ETX-M00001422 | 0.101499366 | 1.062852058 | 9.77 | 0.139991669 | 1.09309642 | 9.63 |
| ETX-M00001423 | 0.030768893 | 0.602673809 | 10.94 | 0.114329435 | 0.807419623 | 10.28 |
| ETX-M00001424 | 0.041897446 | 0.706970326 | 10.98 | 0.119111702 | 0.787803206 | 10.56 |
| ETX-M00001425 | 0.053728018 | 0.473666552 | 11.18 | 0.130755094 | 0.684291085 | 10.44 |
| ETX-M00001426 | 0.027792257 | 0.69772576 | 10.99 | 0.1330014 | 0.805546258 | 10.34 |
| ETX-M00001427 | 0.042372721 | 0.768245564 | 10.56 | 0.15004026 | 0.98645508 | 9.71 |
| ETX-M00001428 | 0.038274421 | 0.540155754 | 11.12 | 0.131942785 | 0.810898502 | 10.3 |
| ETX-M00001429 | 0.071583565 | 0.925950293 | 9.9 | 0.291507299 | 1.13672904 | 9.45 |
| ETX-M00001430 | 0.023816791 | 0.215234943 | -78.66 | 0.135367433 | 0.607962593 | 10.78 |
| ETX-M00001431 | 0.435973061 | 1.411804601 | 8.46 | 0.694683789 | 1.248311443 | -0.01 |
| ETX-M00001432 | 0.177808018 | 1.234507806 | 9.88 | 0.338993675 | 1.056091296 | 9.3 |
| ETX-M00001433 | 0.344988132 | 1.299711934 | 9.0 | 0.627298879 | 1.201312427 | 9.02 |
| ETX-M00001434 | 0.028180714 | 0.40484356 | 11.55 | 0.103829996 | 0.703970412 | 10.3 |
| ETX-M00001435 | 0.216966986 | 1.11371214 | 9.77 | 0.330384089 | 1.112557791 | 9.8 |
| ETX-M00001436 | 0.115218504 | 0.707893402 | 10.87 | 0.142475213 | 0.768763289 | 10.65 |
| ETX-M00001437 | 0.103217781 | 0.70456761 | 10.07 | 0.143716816 | 1.035354144 | 10.14 |
| ETX-M00001438 | 0.132112484 | 1.005363409 | 9.72 | 0.218409608 | 1.10831212 | 9.78 |
| ETX-M00001439 | 0.221764768 | 1.090018663 | 9.27 | 0.123223569 | 0.957429878 | 9.87 |
| ETX-M00001440 | 0.287275636 | 1.053461449 | 9.3 | 0.163879147 | 0.817019676 | 10.42 |
| ETX-M00001441 | 0.216868684 | 1.02420622 | 9.14 | 0.306020744 | 1.065748137 | 9.97 |
| ETX-M00001442 | 0.148103725 | 1.112208683 | 10.22 | 0.576438032 | 1.124823589 | 8.99 |
| ETX-M00001443 | 0.452045611 | 1.214447835 | 9.11 | 0.2936311 | 1.049463941 | 9.54 |
| ETX-M00001444 | 0.800140403 | 1.158463785 | 9.01 | 0.365630694 | 1.172872599 | 9.29 |
| ETX-M00001445 | 0.800162618 | 1.419723412 | 0.0 | 0.197824563 | 0.916524711 | 9.95 |
| ETX-M00001446 | 0.78279321 | 1.267998923 | 9.0 | 0.700984039 | 1.099422182 | 8.58 |
| ETX-M00001447 | 0.100102106 | 0.854701522 | 10.39 | 0.168763678 | 0.751253787 | 10.26 |
| ETX-M00001448 | 0.15894435 | 1.043784315 | 9.89 | 0.223273212 | 0.883386035 | 10.42 |
| ETX-M00001449 | 0.249364194 | 1.077980073 | 9.52 | 0.351716637 | 0.93335442 | 9.79 |
| ETX-M00001450 | 0.368410731 | 1.275252679 | 9.17 | 0.55019584 | 1.169170461 | 8.93 |
| ETX-M00001451 | 0.308919609 | 1.192696082 | 8.97 | 0.490813235 | 1.211992211 | 9.01 |
| ETX-M00001452 | 0.238825279 | 1.127361753 | 9.5 | 0.267021778 | 1.07609102 | 9.47 |
| ETX-M00001453 | 0.217260602 | 1.051652684 | 9.46 | 0.230084869 | 1.042460178 | 9.37 |
| ETX-M00001454 | 0.234446288 | 1.169298234 | 9.41 | 0.271443049 | 1.179742065 | 9.19 |
| ETX-M00001455 | 0.746843593 | 1.367585649 | 8.29 | 0.968150833 | 1.336735444 | -0.17 |
| ETX-M00001456 | 0.183031984 | 1.064959801 | 9.29 | 0.289254915 | 1.293047811 | 8.99 |
| ETX-M00001457 | 0.238616955 | 1.114849179 | 9.25 | 0.397275929 | 1.264765125 | 9.16 |
| ETX-M00001458 | 0.210942479 | 1.125762774 | 9.28 | 0.303795657 | 1.299295709 | 9.11 |
| ETX-M00001459 | 0.489740872 | 1.694856765 | 8.55 | 0.584916867 | 1.104498189 | 8.98 |
| ETX-M00001460 | 0.285056768 | 1.294302518 | 9.14 | 0.441451608 | 1.188362675 | 9.08 |
| ETX-M00001461 | 0.110882254 | 0.727086504 | 10.3 | 0.17207752 | 0.838528119 | 10.5 |
| ETX-M00001462 | 0.200805398 | 1.214763555 | 9.35 | 0.280317345 | 1.012046941 | 9.24 |
| ETX-M00001463 | 0.320540907 | 1.810561879 | 8.88 | 0.402634571 | 1.290068698 | 9.2 |
| ETX-M00001464 | 0.159637206 | 1.271535485 | 9.55 | 0.265979152 | 1.23028713 | 9.39 |
| ETX-M00001465 | 0.454641221 | 1.443074714 | 9.0 | 0.56238986 | 1.35228576 | 9.0 |
| ETX-M00001466 | 0.171636779 | 1.335353607 | 9.74 | 0.196300204 | 1.171968934 | 9.57 |
| ETX-M00001467 | 0.778757355 | 1.553862978 | 8.43 | 0.851261054 | 1.271042398 | 8.29 |
| ETX-M00001468 | 0.401989061 | 1.057827275 | 9.46 | 0.577060998 | 1.186666801 | 9.73 |
| ETX-M00001469 | 0.396568651 | 1.171359199 | 9.16 | 0.523167163 | 1.175507738 | 9.18 |
| ETX-M00001470 | 0.113717947 | 0.907199722 | 9.62 | 0.218182315 | 1.063325306 | 9.57 |
| ETX-M00001471 | 0.120533094 | 0.828923044 | 10.57 | 0.244872851 | 1.072996958 | 9.86 |
| ETX-M00001472 | 0.113811368 | 0.815070157 | 9.95 | 0.240436689 | 1.012257113 | 10.24 |
| ETX-M00001473 | 0.283579604 | 0.963540287 | 9.45 | 0.426049534 | 1.120006437 | 9.28 |
| ETX-M00001474 | 0.248817244 | 1.007768806 | 9.29 | 0.364067497 | 1.049510075 | 9.27 |
| ETX-M00001475 | 0.121434646 | 1.137384843 | 10.19 | 0.220525582 | 1.722171527 | 9.66 |
| ETX-M00001476 | 0.174490948 | 0.988894599 | 9.4 | 0.295644685 | 1.17090225 | 9.28 |
| ETX-M00001477 | 0.171943987 | 1.188054484 | 9.66 | 0.274977759 | 1.18087502 | 9.28 |
| ETX-M00001478 | 0.177969128 | 1.021092284 | 9.67 | 0.331721708 | 1.093505269 | 9.62 |
| ETX-M00001479 | 0.092930861 | 0.824855043 | 10.54 | 0.204343598 | 0.969385677 | 9.84 |
| ETX-M00001480 | 0.141566218 | 0.745036939 | 10.32 | 0.233151152 | 0.895095386 | 9.84 |
| ETX-M00001481 | 0.138921202 | 0.736618352 | 10.51 | 0.226481701 | 0.841155311 | 10.13 |
| ETX-M00001482 | 0.388745982 | 1.162255498 | 9.06 | 0.568413041 | 1.123140442 | 9.12 |
| ETX-M00001483 | 0.072424585 | 0.938831484 | 10.58 | 0.109836142 | 0.783990627 | 10.89 |
| ETX-M00001484 | 0.385456815 | 1.337734578 | 8.99 | 0.407593444 | 1.115493506 | 8.59 |
| ETX-M00001485 | 0.644075114 | 1.33239794 | 9.0 | 0.696154137 | 1.207585965 | 9.09 |
| ETX-M00001486 | 0.209202376 | 1.331221759 | 9.02 | 0.294101713 | 1.014341711 | 9.77 |
| ETX-M00001487 | 0.108356429 | 0.947812285 | 10.31 | 0.166435673 | 1.481408137 | 9.55 |
| ETX-M00001488 | 0.47465733 | 1.14219708 | 8.96 | 0.628623935 | 1.900515639 | 9.0 |
| ETX-M00001489 | 0.434499739 | 1.167375381 | 9.13 | 0.440779731 | 1.591624543 | 8.71 |
| ETX-M00001490 | 0.19610501 | 1.205713394 | 9.56 | 0.274297457 | 1.103030792 | 9.3 |
| ETX-M00001491 | 0.426861836 | 1.463737558 | 8.63 | 0.643380462 | 2.286311572 | 8.46 |
| ETX-M00001492 | 0.224798533 | 1.081459718 | 9.49 | 0.233644026 | 1.400814023 | 9.2 |
| ETX-M00001493 | 0.906094114 | 1.191672581 | 9.5 | 0.976893745 | 1.264085464 | -1.4 |
| ETX-M00001494 | 0.08192495 | 0.94445998 | 10.19 | 0.17525009 | 0.993388621 | 10.2 |
| ETX-M00001495 | 0.080857184 | 0.597337943 | 10.8 | 0.049498544 | 0.491324095 | 11.23 |
| ETX-M00001496 | 0.094063464 | 0.764050166 | 10.18 | 0.071457482 | 0.720299294 | 10.4 |
| ETX-M00001497 | 0.075678813 | 0.50449811 | 11.45 | 0.063012829 | 0.455447572 | 11.57 |
| ETX-M00001498 | 0.510534822 | 1.259204211 | 8.99 | 0.463093976 | 1.048341966 | 9.01 |
| ETX-M00001499 | 0.418784302 | 1.1920872 | 9.0 | 0.374906799 | 1.073669819 | 9.03 |
| ETX-M00001500 | 0.200723546 | 1.060111323 | 9.52 | 0.175118984 | 0.899436225 | 9.63 |
| ETX-M00001501 | 0.882334298 | 1.226476653 | 0.16 | 0.848469024 | 1.095005712 | -5.02 |
| ETX-M00001502 | 0.126837941 | 0.919482021 | 9.95 | 0.115173834 | 0.973821256 | 10.23 |
| ETX-M00001503 | 0.231044552 | 1.082675933 | 9.24 | 0.176195581 | 1.075046561 | 9.61 |
| ETX-M00001504 | 0.114326676 | 0.733485819 | 10.86 | 0.083699606 | 0.693000187 | 10.9 |
| ETX-M00001505 | 0.197915702 | 1.103229148 | 9.48 | 0.13713965 | 0.998946726 | 9.9 |
| ETX-M00001506 | 0.176746491 | 0.987150514 | 9.97 | 0.135915382 | 0.873482162 | 10.34 |
| ETX-M00001507 | 0.08160249 | 0.71798817 | 10.37 | 0.087207447 | 0.670526217 | 10.66 |
| ETX-M00001508 | 0.364040402 | 1.280998227 | 9.3 | 0.348836799 | 1.050224419 | 9.3 |
| ETX-M00001509 | 0.31606101 | 0.979201368 | 9.69 | 0.313530488 | 0.975783603 | 9.52 |
| ETX-M00001510 | 0.071968429 | 0.581647292 | 10.78 | 0.060296712 | 0.602027438 | 10.82 |
| ETX-M00001511 | 0.144734418 | 1.030898602 | 9.95 | 0.10386541 | 0.925246827 | 10.2 |
| ETX-M00001512 | 0.104870364 | 0.75616524 | 10.37 | 0.059908818 | 0.516885518 | 11.64 |
| ETX-M00001513 | 0.179163038 | 1.072057612 | 9.61 | 0.105146715 | 0.742414703 | 9.93 |
| ETX-M00001514 | 0.112349798 | 0.769550516 | 10.29 | 0.071720467 | 0.693729042 | 10.6 |
| ETX-M00001515 | 0.067451248 | 0.568081347 | 10.82 | 0.114948551 | 0.602883724 | 11.12 |
| ETX-M00001516 | 0.226102671 | 1.151276558 | 9.24 | 0.272063757 | 1.323051929 | 9.3 |
| ETX-M00001517 | 0.130588516 | 0.890962698 | 9.83 | 0.138718171 | 1.147955078 | 9.68 |
| ETX-M00001518 | 0.168426311 | 1.001374024 | 9.58 | 0.248305081 | 1.46112812 | 9.24 |
| ETX-M00001519 | 0.809829349 | 1.456027731 | 0.0 | 0.749409682 | 1.281428091 | 8.05 |
| ETX-M00001520 | 0.179840061 | 1.141350447 | 9.62 | 0.201821884 | 1.052988019 | 10.21 |
| ETX-M00001521 | 0.203198525 | 1.289622065 | 9.07 | 0.180323649 | 1.117605884 | 9.29 |
| ETX-M00001522 | 0.077505813 | 0.685343817 | 10.34 | 0.130822448 | 0.877167048 | 10.83 |
| ETX-M00001523 | 0.117059655 | 0.981224957 | 9.91 | 0.105714855 | 0.803061856 | 10.02 |
| ETX-M00001524 | 0.103347245 | 0.893605415 | 10.29 | 0.117905549 | 0.809822999 | 10.02 |
| ETX-M00001525 | 0.13029678 | 0.941109066 | 9.54 | 0.160804315 | 0.832443362 | 9.61 |
| ETX-M00001526 | 0.062744305 | 0.520379788 | 11.18 | 0.126635651 | 0.597941198 | 10.88 |
| ETX-M00001527 | 0.099571218 | 0.465361604 | 11.17 | 0.072424535 | 0.507056471 | 11.76 |
| ETX-M00001528 | 0.149902577 | 0.935776294 | 9.56 | 0.14818156 | 0.973651465 | 9.73 |
| ETX-M00001529 | 0.732102262 | 1.130692465 | 9.0 | 0.634056731 | 0.979029905 | 9.39 |
| ETX-M00001530 | 0.347540036 | 0.993951999 | 9.44 | 0.309187379 | 0.881776133 | 9.66 |
| ETX-M00001531 | 0.493193123 | 1.35093671 | 9.2 | 0.373880033 | 1.205940588 | 9.4 |
| ETX-M00001532 | 0.17481454 | 0.873636227 | 9.97 | 0.173560413 | 1.029329111 | 9.82 |
| ETX-M00001533 | 0.10594013 | 0.862763224 | 10.61 | 0.124919693 | 0.800477542 | 9.94 |
| ETX-M00001534 | 0.456821858 | 1.377927122 | 9.43 | 0.458928031 | 1.28999398 | 9.32 |
| ETX-M00001535 | 0.157627223 | 0.765275769 | 10.57 | 0.127689025 | 0.667310419 | 10.54 |
| ETX-M00001536 | 0.685316166 | 1.082851287 | 9.04 | 0.597891015 | 1.019780343 | 9.06 |
| ETX-M00001537 | 0.381668923 | 1.062754689 | 9.09 | 0.396259914 | 1.000956262 | 8.73 |
| ETX-M00001538 | 0.180573755 | 1.055378958 | 9.51 | 0.201395581 | 0.991416335 | 9.48 |
| ETX-M00001539 | 0.197517396 | 0.744389507 | 10.38 | 0.16862501 | 0.775077746 | 10.54 |
| ETX-M00001540 | 0.563176143 | 1.011627826 | 9.45 | 0.64089012 | 1.314587739 | 9.51 |
| ETX-M00001541 | 0.197125268 | 0.945899049 | 9.98 | 0.206910811 | 1.109952497 | 9.59 |
| ETX-M00001542 | 0.542663818 | 1.05735558 | 8.41 | 0.719819173 | 1.258341437 | 8.9 |
| ETX-M00001543 | 0.777761827 | 1.26233632 | 9.0 | 0.554219931 | 1.108749535 | 8.72 |
| ETX-M00001544 | 0.89375873 | 1.302943327 | 8.41 | 0.788780887 | 1.163578464 | 8.43 |
| ETX-M00001545 | 0.576131016 | 1.119549124 | 9.08 | 0.503221337 | 1.264839213 | 8.97 |
| ETX-M00001546 | 0.483426456 | 0.883052326 | 8.78 | 0.308175911 | 1.054578142 | 9.37 |
| ETX-M00001547 | 0.480114307 | 1.051566679 | 8.54 | 0.359316228 | 1.094484103 | 8.78 |
| ETX-M00001548 | 0.70132732 | 1.065456235 | 9.02 | 0.602001297 | 1.04393653 | 8.85 |
| ETX-M00001549 | 0.386814514 | 0.993180839 | 9.33 | 0.288733418 | 0.998632663 | 9.19 |
| ETX-M00001550 | 0.184467342 | 0.910668466 | 9.72 | 0.145861597 | 0.919265817 | 9.78 |
| ETX-M00001551 | 0.386743581 | 0.991704529 | 9.25 | 0.367859285 | 0.974739364 | 9.29 |
| ETX-M00001552 | 0.445317048 | 1.090403237 | 9.23 | 0.360788664 | 1.05755516 | 8.99 |
| ETX-M00001553 | 0.741525263 | 1.205512199 | 9.02 | 0.486200881 | 0.930696646 | 8.29 |
| ETX-M00001554 | 0.494077496 | 1.150159346 | 9.29 | 0.349603107 | 0.900009674 | 8.8 |
| ETX-M00001555 | 0.178659403 | 0.848413657 | 9.97 | 0.170464758 | 0.939955207 | 9.57 |
| ETX-M00001556 | 0.828543443 | 1.114700202 | 10.99 | 0.856549135 | 1.096966838 | 9.47 |
| ETX-M00001557 | 0.470368629 | 1.276291834 | 9.27 | 0.338719844 | 1.019021445 | 9.0 |
| ETX-M00001558 | 0.171496525 | 0.771925354 | 10.13 | 0.078951578 | 0.753412781 | 10.25 |
| ETX-M00001559 | 0.181284051 | 0.908321484 | 9.55 | 0.168754413 | 1.14125101 | 9.37 |
| ETX-M00001560 | 0.108847895 | 0.563787309 | 11.12 | 0.094853422 | 0.844001013 | 10.32 |
| ETX-M00001561 | 0.148083049 | 0.650576181 | 10.49 | 0.119327018 | 0.933218386 | 10.08 |
| ETX-M00001562 | 0.579285681 | 0.989126818 | 9.01 | 0.463948037 | 1.139016097 | 8.89 |
| ETX-M00001563 | 0.098812665 | 0.941552386 | 10.34 | 0.214112909 | 1.162380727 | 9.47 |
| ETX-M00001564 | 0.052725485 | 0.833096066 | 10.67 | 0.107121702 | 0.914576774 | 9.74 |
| ETX-M00001565 | 0.047907714 | 0.581064923 | 10.91 | 0.067270985 | 0.552200534 | 10.47 |
| ETX-M00001566 | 0.214032943 | 0.818838176 | 9.89 | 0.311818988 | 0.981055179 | 9.2 |
| ETX-M00001567 | 0.199889178 | 0.968529415 | 10.04 | 0.319098269 | 1.409166113 | 9.13 |
| ETX-M00001568 | 0.176969332 | 1.06278721 | 9.73 | 0.29353383 | 1.103629318 | 9.04 |
| ETX-M00001569 | 0.407251176 | 1.186700827 | 8.54 | 0.672469941 | 1.17041867 | 8.97 |
| ETX-M00001570 | 0.115382895 | 0.860648931 | 10.34 | 0.164460348 | 0.92337603 | 9.32 |
| ETX-M00001571 | 0.05473803 | 0.438738377 | 11.93 | 0.084787702 | 0.633491573 | 10.88 |
| ETX-M00001572 | 0.067802596 | 0.617781602 | 10.57 | 0.18910512 | 0.906705118 | 9.59 |
| ETX-M00001573 | 0.312545863 | 1.17445612 | 9.2 | 0.708548657 | 1.189540452 | 9.29 |
| ETX-M00001574 | 0.057184217 | 0.552280966 | 10.67 | 0.115227409 | 0.824190268 | 9.92 |
| ETX-M00001575 | 0.04317709 | 0.27527029 | 11.85 | 0.09050152 | 0.772109748 | 10.47 |
| ETX-M00001576 | 0.152649557 | 0.841047573 | 9.93 | 0.400363493 | 1.223773728 | 9.27 |
| ETX-M00001577 | 0.287805848 | 0.877207354 | 9.48 | 0.512663586 | 1.095392064 | 8.83 |
| ETX-M00001578 | 0.099624046 | 0.526723164 | 10.86 | 0.197297808 | 0.983353018 | 10.02 |
| ETX-M00001579 | 0.863418654 | 1.46395648 | 10.84 | 0.84901866 | 1.227113901 | -6.58 |
| ETX-M00001580 | 0.403486041 | 1.096554233 | 9.66 | 0.520411651 | 1.02518741 | 9.03 |
| ETX-M00001581 | 0.320598408 | 0.811449753 | 9.81 | 0.425870046 | 0.928399188 | 9.2 |
| ETX-M00001582 | 0.215381862 | 0.820981528 | 9.58 | 0.256176522 | 0.914370438 | 9.42 |
| ETX-M00001583 | 0.392932707 | 1.360432302 | 9.32 | 0.412439429 | 1.216383934 | 8.99 |
| ETX-M00001584 | 0.402751165 | 1.132555042 | 9.3 | 0.408138628 | 1.048518391 | 9.07 |
| ETX-M00001585 | 0.08435408 | 0.383878766 | 11.68 | 0.086676618 | 0.357384673 | 11.58 |
| ETX-M00001586 | 0.269361892 | 0.948358322 | 9.22 | 0.338865735 | 0.990278462 | 9.47 |
| ETX-M00001587 | 0.277665435 | 1.143162503 | 9.2 | 0.378850337 | 1.078608968 | 8.59 |
| ETX-M00001588 | 0.841480649 | 1.220938473 | 9.26 | 0.764889069 | 1.058900061 | 9.0 |
| ETX-M00001589 | 0.208434212 | 0.892736613 | 9.4 | 0.298104578 | 0.797916085 | 9.03 |
| ETX-M00001590 | 0.716619149 | 1.25745607 | -4.12 | 0.784044223 | 0.987616219 | 26.18 |
| ETX-M00001591 | 0.288322534 | 1.08649535 | 9.43 | 0.307111179 | 0.965734776 | 9.11 |
| ETX-M00001592 | 0.201705314 | 1.083891991 | 9.42 | 0.374456992 | 0.890546244 | 9.08 |
| ETX-M00001593 | 0.18228958 | 1.111277792 | 9.33 | 0.256884978 | 0.862583147 | 8.99 |
| ETX-M00001594 | 0.205552739 | 1.155201884 | 9.14 | 0.361292556 | 0.90636084 | 8.58 |
| ETX-M00001595 | 0.206440906 | 0.991323799 | 9.56 | 0.163368706 | 0.955356479 | 9.57 |
| ETX-M00001596 | 0.589633448 | 2.132271683 | 8.97 | 0.639093563 | 1.181792348 | 8.95 |
| ETX-M00001597 | 0.367440045 | 1.094506689 | 9.35 | 0.407310825 | 1.059163317 | 8.71 |
| ETX-M00001598 | 0.655942506 | 1.235746886 | 9.53 | 0.831850723 | 1.240753963 | 8.92 |
| ETX-M00001599 | 0.352272258 | 1.111112788 | 8.58 | 0.362658499 | 1.037247416 | 8.79 |
| ETX-M00001600 | 0.248248305 | 1.097005509 | 9.12 | 0.223276615 | 0.888735217 | 9.17 |
| ETX-M00001601 | 0.475783706 | 1.098854043 | 8.51 | 0.465953232 | 1.082429874 | 9.0 |
| ETX-M00001602 | 0.509886049 | 1.118393415 | 8.55 | 0.50385973 | 1.026034017 | 8.8 |
| ETX-M00001603 | 0.643839605 | 1.137055937 | 9.01 | 0.529177461 | 1.064149629 | 9.13 |
| ETX-M00001604 | 0.504469772 | 1.118067066 | 9.02 | 0.464379123 | 1.059705967 | 9.18 |
| ETX-M00001605 | 0.567162373 | 1.14875507 | 9.01 | 0.551503146 | 1.16342646 | 8.98 |
| ETX-M00001606 | 0.208344328 | 0.89822559 | 9.3 | 0.226732801 | 0.919876777 | 9.14 |
| ETX-M00001607 | 0.415849594 | 1.050395564 | 9.24 | 0.327137947 | 1.113661679 | 9.06 |
| ETX-M00001608 | 0.808421575 | 1.046724469 | 9.02 | 0.772163102 | 1.236522954 | 9.11 |
| ETX-M00001609 | 0.554494567 | 1.02478462 | 9.0 | 0.577937353 | 1.129353801 | 9.03 |
| ETX-M00001610 | 0.39828919 | 1.021515595 | 9.14 | 0.36913694 | 1.096504339 | 9.28 |
| ETX-M00001611 | 0.848566644 | 1.161099312 | 0.0 | 0.940978555 | 1.380812328 | 0.0 |
| ETX-M00001612 | 0.892481009 | 1.244147363 | 9.08 | 0.88917405 | 1.240736924 | 0.0 |
| ETX-M00001613 | 0.656003481 | 1.194774105 | 9.05 | 0.829473474 | 1.273747001 | 9.0 |
| ETX-M00001614 | 0.742166545 | 1.243436536 | 9.0 | 0.812683918 | 1.209821119 | 9.01 |
| ETX-M00001615 | 0.595695462 | 1.14232952 | 9.51 | 0.70827781 | 1.158039329 | 9.02 |
| ETX-M00001616 | 0.904920762 | 1.476650471 | 8.99 | 0.774445301 | 1.154820201 | 9.01 |
| ETX-M00001617 | 0.861617152 | 1.244273276 | -0.01 | 0.856886335 | 1.063775894 | ? |
| ETX-M00001618 | 0.593920146 | 1.119007631 | 8.98 | 0.557719551 | 1.091604502 | 8.87 |
| ETX-M00001619 | 0.517851725 | 1.515200255 | 8.96 | 0.373553248 | 1.145052949 | 8.99 |
| ETX-M00001620 | 0.85505106 | 1.147959289 | 8.99 | 0.733229539 | 1.166700543 | 8.97 |
| ETX-M00001621 | 0.589689324 | 1.183170083 | 8.73 | 0.613296368 | 1.044645764 | 9.01 |
| ETX-M00001622 | 0.892328287 | 1.071820251 | 9.71 | 0.826525258 | 1.080202515 | 9.1 |
| ETX-M00001623 | 0.36492363 | 1.116179441 | 8.78 | 0.376059882 | 1.087345416 | 8.96 |
| ETX-M00001624 | 0.422346201 | 0.94353241 | 8.85 | 0.405757963 | 1.066069735 | 8.82 |
| ETX-M00001625 | 0.257167276 | 1.085491538 | 9.11 | 0.321340742 | 1.080066253 | 8.93 |
| ETX-M00001626 | 0.32109742 | 0.969305592 | 9.13 | 0.804305401 | 1.144015273 | 8.78 |

### Example 10: Pharmacological studies in normal mice

### In vivo pharmacology study with five selected GalNAc-siRNAs

The pharmacodynamic activity of five selected ANT2 GalNAc-siRNAs was measured in vivo. Thirty-two C57BL/6 male mice were allocated for each of the five GalNAc-siRNAs, ETX-M00001397, ETX-M00001570, ETX-M00001378, ETX-M00001513, and ETX-M00001527. 20 mice were allocated as a control group (saline) and 16 mice were allocated for a negative control siRNA group (ETXM1198). Mice were subcutaneously dosed with ETXMs (1 mg/kg or 3 mg/kg) on day 0, defined as the day mice were first dosed. Four mice from saline control group were sacrificed on day 0 prior to dosing and subsequently, four mice in each treatment group at each dose level were sacrificed on day 7, 14, 21, and 28. Upon termination, liver tissues and plasma samples were harvested for further analysis.

### SLC25A5 mRNA knockdown in mouse liver

Harvested liver samples were used to measure the SLC25A5 mRNA knockdown level by RT-qPCR. Upon collection, each tissue was treated with RNAlater and stored at 4 °C overnight then at -80 °C until the further analysis. Liver tissues were homogenized with TRIZOL for RNA extraction. RNA samples, adjusted to 400 ng/µL, were reverse transcribed to cDNA using FastKing RT Kit, manufactured by TIANGEN. After reverse transcription, real-time quantitative PCR may be performed using an ABI Prism 7900HT to detect the relative abundance of SLC25A5 mRNA normalized to the housekeeping gene GAPDH. The expression of the target gene in each test sample may be determined by relative quantitation using the comparative Ct (ΔΔCt) method. This method measures the Ct differences (ΔCt) between target gene and housekeeping gene. The formula is as follows: ΔCt = average Ct of SLC25A5-average Ct of GAPDH, ΔΔCt=ΔCt (sample) - average ΔCt (untreated control), relative expression of target gene mRNA = 2-ΔΔCt.

### ANT2 protein knockdown in mouse liver

A second sample of liver tissue was used to measure ANT2 protein knockdown by LC-MS. Forty milligrams of mouse liver with 1.5 mL of RIPA buffer were used for protein extraction. Additionally, 1X of protease inhibitor, 1X of phosphatase inhibitor and 1X of PMSF were included in the RIPA buffer. QIAGEN Tissue Lyser was used to homogenize the liver until lysate becomes transparent. After high-speed centrifugation at 4 °C, the protein concentration in liver lysate was measured by BCA quantification kit. Three hundred microgram protein was taken from each liver lysate sample for digestion using the S-TrapTM 96-well MS sample prep kit according to the manufacturer's instruction with one exception: DTT and IAA were used as reductant and alkylation reagents instead of TCEP and MMTS.

For LC-MS/MS analysis, five hundred nanogram peptide was run on a 60 minute LC method in Easy Nano 1200 (mobile phase A: 0.1% formic acid in H2O, mobile phase B: 0.1% formic acid in 80% acetonitrile). The separation gradient is: 3-28%B from 0 to 40 min, 28-46%B from 40 to 45 min, 46 -100%B from 45-50 min and maintain at 100 %B to the end. A 25 cm of C18 column was used for peptide separation. The eluted peptide was analyzed in Orbitrap Eclipse at scan range of 350-1500 m/z with PRM-MS/MS method.

The raw data was processed in Skyline and the peptide quality was manually checked. The SLC25a5 protein database from UniProt was uploaded as library search. The final selection of peptides should have the following characters: 1. Elution peak without tailing; 2. Well-matched product ions; 3. Quantified peak area greater than 1e6. Consequently, the following peptides, QIFLGGVDK and EQGVLSFWR, were chosen for SLC25a5 protein quantitation. Relative protein expression is calculated via time-matched normalisation of the average target peptide counts to the Vehicle Control group. Peptide counts corresponding to the reference protein (GAPDH) are not considered.

The results of mRNA and protein knockdown are shown in Figure 10.

### Example 11: Pharmacological studies in Gubra-Amylin NASH (GAN) diet-induced obese (DIO) mice

In this Example, the activity of ETX-312 (ETX-M00001378), an inhibitor of SLC25A5 expression, on metabolic parameters, hepatic pathology, and NAFLD Activity Score (NAS) including fibrosis stage in male biopsy-confirmed GAN DIO-NASH mice was tested. A total of 9 DIO-NASH groups (n=16) and 1 LEAN-CHOW group (n=10) of male C57BL/6JRj mice were fed 40% HFD, 40% carbohydrate (22% fructose), 2% Cholesterol (GAN) diet or normal chow (Altromin 1324) for 34 weeks prior to study start.

All mice were pre-biopsied at week -4 and stratified based on liver biopsy (only animals with fibrosis stage =F2-F3, steatosis score =3 and inflammation score ≥2 were included). Mice were anesthetized by inhalation anesthesia using isoflurane (2-3%). A small abdominal incision was made in the midline and the left lateral lobe of the liver exposed. A cone shaped wedge of liver tissue (approximately 50 mg) was excised from the distal portion of the lobe and fixated in 10% neutral buffered formalin (10% NBF) for histology. The cut surface of the liver was instantly electrocoagulated using bipolar coagulation (ERBE VIO 100 electrosurgical unit). The liver was returned to the abdominal cavity, the abdominal wall sutured and the skin closed with staplers. For post-operative recovery, mice received carprofen (5mg/kg) administered subcutaneously on the day of the procedure and on post-operation days 1 and 2.

Animals were randomized into groups based on fibrosis stage and picrosirius red (PSR) staining (% fractional area). Mice received a total of 12 weeks of dosing and were divided into the following groups: 1) LEAN-CHOW control (saline) (subcutanoues (SC), once weekly (QW)), 2) DIO-NASH control (saline) (SC, QW), 3) SiCtrl, 5 mg/kg (SC, QW), 4) ETX-312, 5 mg/kg (SC, QW), 5) Semaglutide, 30 nmol/kg (SC, once a day (QD)), 6) Semaglutide, 30 nmol/kg (SC, QD) + ETX-312, 5 mg/kg (SC, QW), 7) Vehicle (per os (PO), QD), 8) HU6, 5 mg/kg (PO, QD), 9) Resmetirom, 3 mg/kg (PO, QD), 10) Resmetirom, 3 mg/kg (PO, QD) + ETX-312, 5 mg/kg (SC, QW).

Body weight was recorded daily and food intake recorded once weekly for 24 hours during the study period. The body composition of mice was be assessed by an EchoMRI 3-1 Body composition analyzer (EchoMRI, US) at week 12. Non-anaesthetised animals were placed in a plastic tube inside the MRI scanner for approximately 80 seconds. The body composition was expressed as fat mass, fat free mass (lean mass) and water. Water is normally excluded.

At the end of the study period, preterminal tail vein blood collection was conducted for non-fasting plasma Alanine transaminase (ALT), Aspartate transaminase (AST), Triglycerides (TG) and Total Cholesterol (TC) analysis. Tail-vein, tongue or cheek blood was collected in heparinized tubes and mixed by inversion 5 times. Blood was placed at 4°C until it was centrifuged at 3000 g for 10 minutes. The plasma supernatants were transferred to new tubes and immediately frozen on dry ice. The samples were stored at -70°. Terminal plasma samples were prepared during anesthesia with isoflurane with the abdominal cavity opened and cardiac blood drawn with a syringe into a Microvette/Vacuette of appropriate dimensions with anticoagulant and mixed by inversion 5 times. Blood was placed at 4°C until it was centrifuged at 3000 g for 10 minutes. The plasma supernatants were transferred to new tubes and immediately frozen on dry ice. The samples were stored at -70°C.

ALT, AST, TG, and TC assays were measured using commercial kits (Roche Diagnostics), on the Cobas c 501 autoanalyzer. Insulin was measured in plasma collected in heparinized tubes using the commercial MSD platform (Meso Scale Diagnostics). TIMP -1 was measured in plasma collected in EDTA tubes using a commercial ELISA kit (R&D Systems). PIIINP was measured in plasma collected in EDTA tubes using a commercial ELISA kit (Cusabio).

After study termination, the liver was collected and weighed. Terminal adipose tissue weight was also measured. Specific liver samples and biopsies were dissected and prepared as follows. The liver was divided into a left lateral lobe, medial lobe, right lateral lobe, and caudate lobe. The Post-biopsy piece (~200 mg, less than 0.7 x 0.5 cm) was cut from the left lateral lobe, 4 mm from the prebiopsy site with an edge. The tissue was collected in 10% neutral buffered formalin. A liver sample collected for future analysis of mRNA expression (~150 mg) was dissected from the left medial lobe, collected in tubes and frozen in liquid nitrogen. The samples was stored at - 70°C. A second piece of liver was dissected from the center of the left lateral lobe, put in a tube and frozen in liquid nitrogen for future analysis. The samples were stored at -70°C.

Data were analysed via fitting of a simple linear model with fitted coefficients for the mean effect of the treatment on the biomarker, compared to a baseline comparator. p-values correspond to one-tailed t-tests on the regression coefficients, testing the alternate hypothesis that the coefficient in question is less than 01 (one-tailed) for non-control groups (equivalent to testing there being a reductive effect versus the comparator). Where significant heteroskedasticity was observed between treatment groups, p-values were calculated using robust estimation to the variance-covariance matrix. p-values were subsequently corrected for multiple comparisons via the FDR method.

FFPE biopsies were placed in 10% neutral buffered formalin (10% NBF) for approximately 24 hours and then transferred to 70% ethanol and stored at 4°C. The FFPE biopsies were then placed in the Histokinette to infiltrate prior to embedding in blocks. Biopsy tissues were cut at 3µm on a microtome and the sections were mounted on Superfrost Plus slides and stored at 4 ⁰C.

Glass slides with paraffin embedded sections were deparaffinated in xylene and rehydrated in series of graded ethanol. For Hematoxylin & Eosin (H&E) staining, slides are incubated in Mayer's Hematoxylin (Dako), washed in tap water, stained in Eosin Y solution (Sigma-Aldrich), dehydrated in graded ethanol and cover slipped. For Picrosirius red (PSR) staining, slides were incubated in Weigert's iron hematoxylin (Sigma-Aldrich), washed in tap water, stained in Picrosirius red (Sigma-Aldrich) and washed twice in acidified water. Excess water was removed by shaking the slides and the slides were then dehydrated in three changes of 100% ethanol, cleared in xylene and cover slipped. Immunohistochemistry (IHC) was performed using standard procedures. Briefly, after antigen retrieval and blocking of endogenous peroxidase activity, slides were incubated with primary antibody. The primary antibody was detected using a polymeric HRP-linker antibody conjugate. Next, the primary antibody was visualized with DAB as chromogen. Finally, sections were counterstained in hematoxylin and cover slipped. Slides were scanned under a 20x objective in a ScanScope AT slide scanner (Aperio).

### Antibodies Used:

| **Antigen** | **Primary Antibody** | **Vendor** |
|---|---|---|
| Col1a1 | Goat anti-type I Collagen | Southern Biotech, Cat. 1310-01 |
| Galectin-3 (mouse) | Rat anti-Galectin 3 | Biolegend, Cat. 125402 |
| α-SMA | Rabbit anti-alpha smooth muscle actin [EPR5368] | Abcam, Cat. ab124964 |

Liver samples stained with H&E or PSR were given a score for NAFLD Activity Score (NAS) and fibrosis stage, respectively, using the clinical criteria outlined by Kleiner et al. (Design and validation of a histological scoring system for nonalcoholic fatty liver disease, Hepatology, 2005) shown in the table below. Total NAS represents the sum of scores for steatosis, inflammation, and ballooning, and ranges from 0-8. NAS and fibrosis stage was determined by Gubra Histopathological Objective Scoring Technology (GHOST) deep learning app developed by Gubra using the VIS software (Visiopharm, Denmark) for a more accurate and objective method for staging disease in DIO-NASH mouse models. The app scores were reviewed by a histopathologist blinded to the results. Terminal liver biopsy histomorphometric analysis was also conducted by immunohistochemistry (IHC) for inflammation (Gal-3), Collagen (Col1a1), and activated stellate cells (a-SMA).

### NAS Scoring:

| **Feature** | **Degree** | **Score** |
|---|---|---|
| Steatosis (percentage of hepatocytes with lipid droplets) | <5% | 0 |
| | 5-33% | 1 |
| | >33-66% | 2 |
| | >66% | 3 |
| Lobular inflammation | No foci | 0 |
| | <2 foci/200x | 1 |
| | 2-4 foci/200x | 2 |
| | >4 foci/200x | 3 |
| Ballooning degeneration | None | 0 |
| | Few | 1 |
| | Many cells/prominent | 2 |
| | ballooning | |
| Fibrosis stage | None | 0 |
| | Perisinusoidal or periportal | 1 |
| | Perisinusoidal and | 2 |
| | portal/periportal | 3 |
| | Bridging fibrosis | 4 |
| | Cirrhosis | |

As shown in Figure 11, the inhibitor ETX-312 resulted in a significant improvement of the NAFLD activity score (NAS). Of note, the effect obtained with ETX-312 was greater than the effects obtained with both positive controls, i.e., the GLP-1 agonist semaglutide and the THR-beta agonist resmetirom. Surprisingly, the most significant improvement was obtained when ETX-312 was combined with semaglutide or resmetirom.

The impact of the inhibitor ETX-312 on the blood markers ALT, AST, TIMP-1 and PIIINP is shown in Figures 12 and 13. The terminal liver weight to body weight ratio in DIO-NASH mice that received different treatments is shown in Figure 14.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.
**Aspects of the invention are disclosed in the following numbered clauses:**
1. An inhibitor of expression and / or function of SLC25A5/ANT2, wherein said inhibitor is conjugated to one or more ligand moieties, preferably wherein the ligand moiety allows targeting of hepatocytes.
2. An inhibitor according to clause 1, wherein said inhibitor is an siRNA oligomer.
3. An inhibitor of expression and / or function of SLC25A5/ANT2, wherein said inhibitor is an siRNA oligomer.
4. An inhibitor according to clause 3, wherein said inhibitor comprises an siRNA oligomer conjugated to one or more ligand moieties, preferably wherein the ligand moiety allows targeting of hepatocytes.
5. An inhibitor according to clause 1, 2 or 4, wherein said one or more ligand moieties comprise one or more GalNAc ligands or comprise one more GalNAc ligand derivatives.
6. An inhibitor according to clause 1, 2 or 4 wherein said one or more ligand moieties comprise one or more GalNAc ligand derivatives.
7. An inhibitor according to one or more preceding clauses, wherein the target of the inhibitor is SLC25A5/ANT2.
8. An inhibitor according to one or more preceding clauses, wherein the inhibitor is a nucleic acid for inhibiting expression of SLC25A5 comprising a duplex region that comprises a first strand and a second strand that is at least partially complementary to the first strand, wherein
(i) at least partially complementary to a portion of RNA transcribed from the SLC25A5 gene, and
(ii) comprises at least 17 contiguous nucleosides differing by 0 or 1 nucleosides from any one of the first strand sequences as listed in Table 2.
9. An inhibitor according to one or more preceding clauses, wherein the inhibitor is a nucleic acid for inhibiting expression of SLC25A5 comprising a duplex region that comprises a first strand and a second strand that is at least partially complementary to the first strand, wherein said first strand is:
(i) at least partially complementary to a portion of RNA transcribed from the SLC25A5 gene, and
(ii) comprises at least 17 contiguous nucleosides differing by 0 or 1 nucleosides from any one of the first strand modified sequences as listed in Table 3.
10. An inhibitor according clause 8 or 9, wherein the first strand comprises nucleosides 2-18 of any one of the sequences defined in clause 8 or 9, in particular wherein the first strand comprises nucleosides 2-18 of any one of the sequences defined in Tables 2 or 3.
11. An inhibitor according to clause 8, wherein the second strand comprises a nucleoside sequence of at least 17 contiguous nucleosides differing by 0 or 1 nucleosides from any one of the second strand sequences as listed in Table 2, and wherein the second strand has a region of at least 85% complementarity over the 17 contiguous nucleosides to the first strand.
12. An inhibitor according to clause 9, wherein the second strand comprises a nucleoside sequence of at least 17 contiguous nucleosides differing by 0 or 1 nucleosides from any one of the second strand modified sequences as listed in Table 4, and wherein the second strand has a region of at least 85% complementarity over the 17 contiguous nucleosides to the first strand.
13. An inhibitor according to clause 8, wherein the first strand comprises any one of the first strand sequences as listed in Table 2.
14. An inhibitor according to clause 9, wherein the first strand comprises any one of the first strand modified sequences as listed in Table 3.
15. An inhibitor according to clause 11, wherein the second strand comprises any one of the second strand sequences as listed in Table 2.
16. An inhibitor according to clause 12, wherein the second strand comprises any one of the first strand modified sequences as listed in Table 4.
17. An inhibitor according to clause 13, wherein the first strand comprises any one of the following sequences: SEQ ID NO: 304, SEQ ID NO: 323, SEQ ID NO: 439, SEQ ID NO: 453 and SEQ ID NO: 496.
18. An inhibitor according to clause 14, wherein the first strand comprises any one of the following sequences: SEQ ID NO: 856, SEQ ID NO: 875, SEQ ID NO: 991, SEQ ID NO: 1005 and SEQ ID NO: 1048.
19. An inhibitor according to clause 15, wherein the second strand comprises any one of the following sequences: SEQ ID NO:580, SEQ ID NO:599, SEQ ID NO:715, SEQ ID NO:729 and SEQ ID NO:772.
20. An inhibitor according to clause 16, wherein the second strand comprises any one of the following sequences: SEQ ID NO:1132, SEQ ID NO:1151, SEQ ID NO:1267, SEQ ID NO:1281 and SEQ ID NO:1324.
21. An inhibitor according any one of clauses 8 and 11, comprising first and second strands that comprise, consist of, or consist essentially of a nucleoside sequence differing by 0 or 1 nucleosides from any one of the following combinations of first and second sequences:

| Unmodified first strand | Unmodified second strand |
|---|---|
| SEQ ID NO: 304 | SEQ ID NO: 580 |
| SEQ ID NO: 323 | SEQ ID NO: 599 |
| SEQ ID NO: 439 | SEQ ID NO: 715 |
| SEQ ID NO: 453 | SEQ ID NO: 729 |
| SEQ ID NO: 496 | SEQ ID NO: 772 |

22. An inhibitor according any one of clauses 9 and 12, comprising first and second strands that comprise, consist of, or consist essentially of a nucleoside sequence differing by 0 or 1 nucleosides from any one of the following combinations of first and second sequences:

| Unmodified first strand | Unmodified second strand |
|---|---|
| SEQ ID NO: 856 | SEQ ID NO: 1132 |
| SEQ ID NO: 875 | SEQ ID NO: 1151 |
| SEQ ID NO: 991 | SEQ ID NO: 1267 |
| SEQ ID NO: 1005 | SEQ ID NO: 1281 |
| SEQ ID NO: 1048 | SEQ ID NO: 1324 |

23. An inhibitor according any one of clauses 8 to 22, wherein the first strand has a length in the range of 17 to 30 nucleosides, preferably 19 to 25 nucleosides, more preferably 19 or 23 nucleosides.
24. An inhibitor according any one of clauses 8 to 23, wherein the second strand has a length in the range of 17 to 30 nucleosides, preferably 19 to 25 nucleosides, more preferably 19 or 21 or 23 nucleosides.
25. An inhibitor according any one of clauses 8 to 24, wherein the duplex region of the nucleic acid is between 17 and 30 nucleosides in length, more preferably is 19 or 21 or 23 nucleosides in length.
26. An inhibitor according any one of clauses 8 to 25, wherein the region of complementarity between the first strand and the portion of RNA transcribed from the SLC25A5 gene is between 17 and 30 nucleosides in length.
27. An inhibitor according any one of clauses 8 to 26, wherein the nucleic acid further comprises one or more single-stranded nucleoside overhangs, optionally wherein the overhang is present on the first or second strand, preferably at the 3' terminus of the first or second strand, and/or wherein the overhang comprises 1 to 4 nucleosides, more preferably 2 nucleosides.
28. An inhibitor according any one of clauses 8 to 27, wherein the nucleic acid is an siRNA oligonucleoside.
29. An inhibitor according to any one of clauses 8 to 28, wherein the second sense strand further comprises one or more abasic nucleosides in a terminal region of the second strand, and wherein said abasic nucleoside(s) is / are connected to an adjacent nucleoside through a reversed internucleoside linkage.
30. An inhibitor according to any one of clauses 8 to 29, wherein the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein:
(a) said penultimate abasic nucleoside is connected to an adjacent first basic nucleoside in an adjacent 5' near terminal region through a reversed internucleoside linkage; and
(b) the reversed linkage is a 5-5' reversed linkage; and
(c) the linkage between the terminal and penultimate abasic nucleosides is 3'5' when reading towards the terminus comprising the terminal and penultimate abasic nucleosides.12. An inhibitor, or inhibitor for use, according clause 10 or 11, wherein the reversed internucleoside linkage is at a terminal region which is distal to the 5' terminal region of the second strand, or at a terminal region which is distal to the 3' terminal region of the second strand.
31. An inhibitor according to clause 30, wherein
(i) the first strand and the second strand each has a length of 23 nucleosides;
(ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand;
(iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and
(iv) the second strand of the nucleic acid is conjugated directly or indirectly to one or more ligand moieties at the 3' terminal region of the second strand.
32. An inhibitor according to clause 30 or 31, wherein, wherein the 2 consecutive inverted abasic nucleosides in the 5' terminal region of the second strand present as the following 5' terminal motif 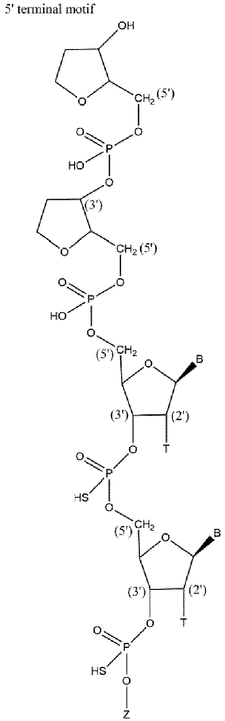 wherein:
T represents a 2'Me ribose modification,
B represents the nucleoside bases of the first two basic nucleosides in the 5' terminal region of the second strand, and
Z represents the remaining 19 contiguous basic nucleosides of said second strand.
33. An inhibitor according to any one of clauses 8 to 32, wherein the nucleic acid is conjugated directly or indirectly to one or more ligand moieties, optionally wherein said ligand moiety is present at a terminal region of the second strand, preferably at the 3' terminal region thereof.
34. An inhibitor according to clause 33, wherein the ligand moiety comprises:
(i) one or more N-acetyl galactosamine (GalNAc) ligands, and / or
(ii) one or more N-acetyl galactosamine (GalNAc) ligand derivatives.
35. An inhibitor according to clause 34, wherein said one or more GalNAc ligands and / or GalNAc ligand derivatives are conjugated directly or indirectly to the 5' or 3' terminal region of the second strand of the nucleic acid, preferably at the 3' terminal region thereof.
36. An inhibitor according to any one of clauses 33 to 35, comprising the structure: 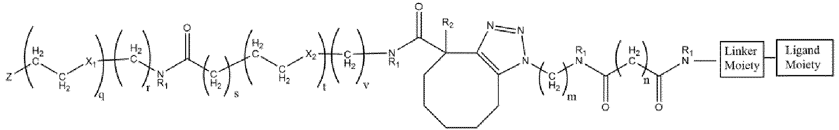 wherein:
R₁ at each occurrence is independently selected from the group consisting of hydrogen, methyl and ethyl;
R₂ is selected from the group consisting of hydrogen, hydroxy, -OC₁₋₃alkyl, -C(=O)OC₁₋₃alkyl, halo and nitro;
X₁ and X₂ at each occurrence are independently selected from the group consisting of methylene, oxygen and sulfur;
m is an integer of from 1 to 6;
n is an integer of from 1 to 10;
q, r, s, t, v are independently integers from 0 to 4, with the proviso that:
   (i) q and r cannot both be 0 at the same time; and
   (ii) s, t and v cannot all be 0 at the same time;
Z is an oligonucleoside moiety.
37. An inhibitor according to clause 36, comprising the structure 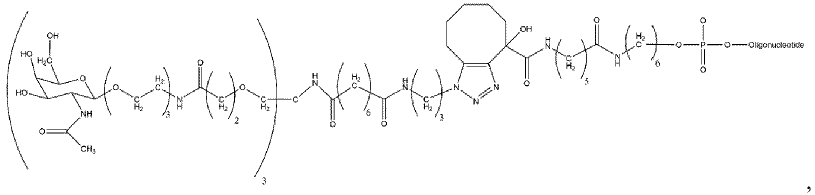 wherein oligonucleotide represents the contiguous nucleosides of the second strand.
38. An inhibitor according to any one of clauses 33 to 35, comprising the structure: 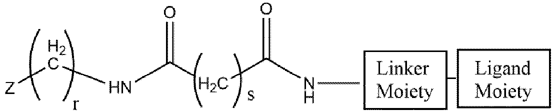 wherein:
r and s are independently an integer selected from 1 to 16; and
Z is an oligonucleoside moiety.
39. An inhibitor according to clause 38, comprising the structure 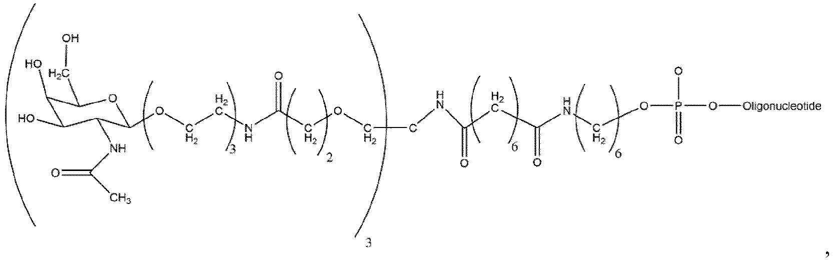 wherein oligonucleotide represents the contiguous nucleosides of the second strand.
40. An inhibitor according to any one of clauses 37 or 39, wherein the structure is conjugated to the 3' terminal region of the second strand.
41. An inhibitor according to any one of clauses 22, 32, 37 and 40.
42. An inhibitor according to any one of clauses 22, 32, 39 and 40.
43. An inhibitor according to one or more preceding clauses, formulated as a pharmaceutical composition with an excipient and / or carrier.
44. A pharmaceutical composition comprising an inhibitor according to one or more preceding clauses, in combination with a pharmaceutically acceptable excipient or carrier.
45. An inhibitor according to any one of clauses 1 to 43 or a pharmaceutical composition according to clause 44, for use in therapy.
46. An inhibitor according to any one of clauses 1 to 43 or a pharmaceutical composition according to clause 44, for use in prevention and/or treatment of a metabolic disease or disorder, such as a metabolic disease or disorder associated with non-alcoholic fatty liver disease (NAFLD), and/or obesity and/or a disease or disorder associated with adipogenesis and/or for use in reducing adipogenesis.
47. Use of SLC25A5/ANT2 as a target for identifying one or more therapeutic agents for the prevention and/or treatment of a metabolic disease or disorder, such as a metabolic disease or disorder associated with non-alcoholic fatty liver disease (NAFLD), and/or obesity and/or a disease or disorder associated with adipogenesis and/or for reducing adipogenesis.
48. A method of treating or preventing a metabolic disease or disorder, such as a metabolic disease or disorder associated with non-alcoholic fatty liver disease (NAFLD), and/or obesity and/or a disease or disorder associated with adipogenesis and/or a method of reducing adipogenesis, which comprises administering to a patient an inhibitor of SLC25A5/ANT2, such as an inhibitor as defined according to any one of clauses 1 to 43, or a pharmaceutical composition comprising an inhibitor of SLC25A5/ANT2, such as a composition as defined according to clause 44.
49. Use of an inhibitor according to any one of clauses 1 to 43 or a pharmaceutical composition according to clause 44, in the preparation of a medicament for the treatment of a metabolic disease or disorder, such as a metabolic disease or disorder associated with non-alcoholic fatty liver disease (NAFLD) and/or obesity and/or a disease or disorder associated with adipogenesis and/or for reducing adipogenesis.
50. SLC25A5/ANT2 for use as a biomarker of a metabolic disease or disorder, such as a metabolic disease or disorder associated with non-alcoholic fatty liver disease (NAFLD) and/or obesity and/or a disease or disorder associated with adipogenesis.
51. SLC25A5/ANT2 for use in an *in vivo* method of predicting susceptibility to a metabolic disease or disorder, such as a metabolic disease or disorder associated with non-alcoholic fatty liver disease (NAFLD) and/or obesity and/or a disease or disorder associated with adipogenesis, typically by monitoring the sequence and/ or level of expression and / or function of SLC25A5/ANT2 in a sample obtained from a patient.
53. A method of predicting susceptibility to a metabolic disease or disorder, such as a metabolic disease or disorder associated with non-alcoholic fatty liver disease (NAFLD) and/or obesity and/or a disease or disorder associated with adipogenesis, and optionally treating said metabolic disease or disorder, in a patient, said method comprising:
(a) obtaining a sample from the patient,
(b) detecting the sequence and / or expression and / or function of SLC25A5/ANT2 in said sample obtained from the patient,
(c) predicting susceptibility to a metabolic disease or disorder, such as a metabolic disease or disorder associated with non-alcoholic fatty liver disease (NAFLD) and/or obesity and/or a disease or disorder associated with adipogenesis, based on the sequence and / or expression and / or function of SLC25A5/ANT2 in said sample obtained from the patient,
(d) preferably administering to the diagnosed patient an effective amount of an inhibitor of SLC25A5/ANT2, preferably an inhibitor of SLC25A5/ANT2 according to any one of clauses 1 to 43, or of a pharmaceutical composition comprising an inhibitor of SLC25A5/ANT2, such as a composition as defined according to clause 44.

## Claims

1. An inhibitor of expression and / or function of SLC25A5/ANT2, wherein said inhibitor is a nucleic acid, for use in prevention and/or treatment of fatty liver disease.

2. An inhibitor for use according to claim 1, wherein the nucleic acid is conjugated to one or more ligand moieties, preferably wherein the ligand moiety allows targeting of hepatocytes.

3. An inhibitor for use according to claim 2, wherein said one or more ligand moieties comprise one or more GalNAc ligands or one or more GalNAc ligand derivatives.

4. An inhibitor for use according to claim 2, wherein said one or more ligand moieties comprise one or more GalNAc ligand derivatives.

5. An inhibitor for use according to any one of the preceding claims, wherein the nucleic acid is an siRNA oligomer.

6. An inhibitor for use according to any one of the preceding claims, wherein the inhibitor is formulated as a pharmaceutical composition with an excipient and / or carrier.

7. A pharmaceutical composition for use in prevention and/or treatment of fatty liver disease, wherein the pharmaceutical composition comprises the inhibitor of any one of the preceding claims, wherein the inhibitor is in combination with a pharmaceutically acceptable excipient or carrier.

8. An inhibitor for use or a pharmaceutical composition for use according to any one of the preceding claims, wherein the fatty liver disease is Non-Alcoholic SteatoHepatitis (NASH) or non-alcoholic fatty liver disease (NAFLD).

9. An inhibitor for use or a pharmaceutical composition for use according to claim 8, wherein the fatty liver disease is Non-Alcoholic SteatoHepatitis (NASH).

10. An inhibitor for use or a pharmaceutical composition for use according to claim 8, wherein the fatty liver disease is non-alcoholic fatty liver disease (NAFLD).
